# DEMANDE DE BREVET EUROPEEN

(11) **EP 1 997 895 A1**
(43) Date de publication de la demande: **03.12.2008**
(21) Numéro de dépôt: 08010138.9
(22) Date de dépôt: 23.06.1999
(51) Int. Cl.: C12N 15/48, C12Q 1/70, C07K 14/15, A61K 31/70, C07K 14/16

(54) **Famille de séquences nucléiques et de séquences protéiques déduites presentant des motifs rétroviraux endogènes humains, et leurs applications**

(30) Priorité: 23.06.1998 FR 9807920
(62) Demande divisionnaire de: 99926538.2
(71) Demandeur: Institut National de la Santé et de la Recherche Médicale (INSERM), 75013 Paris (FR)
(72) Inventeur: Alliel, Patrick M., 92140 Clamart (FR); Perin, Jean-Pierre, 92350 Le Plessis-Robinson (FR); Rieger, François, 92100 Boulogne (FR)
(74) Mandataire: Leblois-Préhaud, Hélène Marthe Georgette

(57) **Abrégé**

Nouvelle famille de séquences nucléiques et de séquences protéiques déduites, qui présentent des motifs rétroviraux endogènes humains complets ou partiels.

Détection et/ou utilisation desdites séquences nucléiques et desdites séquences protéiques correspondantes ou de fragments de ces séquences, dans le cadre d'applications diagnostiques, prophylactiques et thérapeutiques, en particulier pour des neuropathologies à composante autoimmune comme la sclérose en plaques.

Utilisation des séquences flanquantes ou adjacentes desdites séquences et contrôlées par ces dernières, comme réactifs de diagnostic.

LISTE DE SEQUENCES

<110> INSERM
      ALLIEL, Patrick
      PERIN, Jean-Pierre
      RIEGER, Francois

<120> FAMILLE DE SEQUENCES NUCLEIQUES ET DE SEQUENCES
      PROTEIQUES DEDUITES PRESENTANT DES MOTIFS RETROVIRAUX
      ENDOGENES HUMAINS, LEURS SEQUENCES FLANQUANTES ET LEURS
      APPLICATIONS.

<130> 598EXT21

<140>
<141>

<150> 9807920
<151> 1998-06-23

<160> 122

<170> PatentIn Ver. 2.1

<210> 1
<211> 2599
<212> ADN
<213> Homo sapiens

<400> 1
atcccctgcc ttaatcgcca agctccttca ggagaacaaa gaacaggcca ttaccctgga 60
gaagactggc aactgatttt acccacaagc ccaaacctca gggatttcag tatctactag 120
tctgggtaga tactttcacg ggttgggcag aggccttccc ctgtaggaca gaaaaggccc 180
aagaggtaat aaaggcacta gttcatgaaa taattcccag attcggactt ccccgaggct 240
tacagagtga caatagccct gctttccagg ccacagtaac ccagggagta tcccaggcgt 300
taggtatacg atatcactta cactgcgcct gaaggccaca gtcctcaggg aaggtcgaga 360
aaatgaatga aacactcaaa ggacatctaa aaaagcaaac ccaggaaacc cacctcacat 420
ggcctgctct gttgcctata gccttaaaaa gaatctgcaa ctttccccaa aaagcaggac 480
ttagcccata cgaaatgctg tatggaaggc ccttcataac caatgacctt gtgcttgacc 540
caagacagcc aacttagttg cagacatcac ctccttagcc aaatatcaac aagttcttaa 600
aacattacaa ggaacctatc cctgagaaga gggaaaagaa ctattccacc cttgtgacat 660
ggtattagtc aagtcccttc cctctaattc cccatcccta gatacatcct gggaaggacc 720
ctacccagtc attttatcta ccccaactgc ggttaaagtg gctggagtgg agtcttggat 780
acatcacact tgagtcaaat cctggatact gccaaaggaa cctgaaaatc caggagacaa 840
cgctagctat tcctgtgaac ctctagagga tttgcgcctg ctcttcaaac aacaaccagg 900
aggaaagtaa ctaaaatcat aaatccccat ggccctccct tatcatattt ttctctttac 960
tgttctttta ccctctttca ctctcactgc accccctcca tgccgctgta tgaccagtag 1020
ctccccttac caagagtttc tatggagaat gcagcgtccc ggaaatattg atgccccatc 1080
gtataggagt ctttctaagg gaacccccac cttcactgcc cacacccata tgccccgcaa 1140
ctgctatcac tctgccactc tttgcatgca tgcaaatact cattattgga caggaaaaat 1200
gattaatcct agttgtcctg gaggacttgg agtcactgtc tgttggactt acttcaccca 1260
aactggtatg tctgatgggg gtggagttca agatcaggca agagaaaaac atgtaaaaga 1320
agtaatctcc caactcaccc gggtacatgg cacctctagc ccctacaaag gactagatct 1380
ctcaaaacta catgaaaccc tccgtaccca tactcgcctg gtaagcctat ttaataccac 1440
cctcactggg ctccatgagg tctcggccca aaaccctact aactgttgga tatgcctccc 1500
cctgaacttc aggccatatg tttcaatccc tgtacctgaa caatggaaca acttcagcac 1560
agaaataaac accacttccg ttttagtagg acctcttgtt tccaatctgg aaataaccca 1620
tacctcaaac ctcacctgtg taaaatttag caatactaca tacacaacca actcccaatg 1680
catcaggtgg gtaactcctc ccacacaaat agtctgccta ccctcaggaa tattttttgt 1740
ctgtggtacc tcagcctatc gttgtttgaa tggctcttca gaatctatgt gcttcctctc 1800
attcttagtg ccccctatga ccatctacac tgaacaagat ttatacagtt atgtcatatc 1860
taagccccgc aacaaaagag tacccattct tccttttgtt ataggagcag gagtgctagg 1920
tgcactaggt actggcattg gcggtatcac aacctctact cagttctact acaaactatc 1980
tcaagaacta aatggggaca tggaacgggt cgccgactcc ctggtcacct tgcaagatca 2040
acttaactcc ctagcagcag tagtccttca aaatcgaaga gctttagact tgctaaccgc 2100
tgaaagaggg ggaacctgtt tatttttagg ggaagaatgc tgttattatg ttaatcaatc 2160
cggaatcgtc actgagaaag ttaaagaaat tcgagatcga atacaacgta gagcagagga 2220
gcttcgaaac actggaccct ggggcctcct cagccaatgg atgccctgga ttctcccctt 2280

cttaggacct ctagcagcta taatattgct actcctcttt ggaccctgta tctttaacct 2340
ccttgttaac tttgtctctt ccagaatcga agctgtaaaa ctacaaatgg agcccaagat 2400
gcagtccaag actaagatct accgcagacc cctggaccgg cctgctagcc cacgatctga 2460
tgttaatgac atcaaaggca cccctcctga ggaaatctca gctgcacaac ctctactacg 2520
ccccaattca gcaggaagca gttagagcgg tctcggccaa cctccccaac agcacttagg 2580
ttttcctgtt gagatgggg 2599

<210> 2
<211> 1326
<212> ADN
<213> Homo sapiens

<400> 2
gccgcctggc actcctgagg gaagtataaa ttataacacc atcttacagc tagacctctt 60
ttgtagaaaa ggcaaatgga gtgaagtgcc ataagtacaa actttctttt cattaagaga 120
caactcacaa ttatgtaaaa agtgtgattt atgccctaca ggaagccttc agagtctacc 180
tccctatccc agcatccccg actccttccc caactaataa ggacccccct tcaacccaaa 240
tggtccaaaa ggagatagac aaaagggtaa acagtgaacc aaagagtgcc aatattcccc 300
aattatgacc cctccaagca gtgggaggaa gagaattcgg cccagccaga gtgcatgtgc 360
ctttttctct cccagactta aagcaaataa aaacagactt aggtaaattc tcagataacc 420
ctgatggcta tattgatgtt ttacaagggt taggacaatt ctttgatctg acatggagag 480
atataatgtc actgctaaat cagacactaa ccccaaatga gagaagtgcc accataactg 540
cagcctgaga gtttggcgat ctctggtatc tcagtcaggt caatgatagg atgacaacag 600
aggaaagaga atgattcccc acaggccagc aggcagttcc cagtctagac cctcattggg 660
acacagaatc agaacatgga gattggtgct gcagacattt gctaacttgt gtgctagaag 720
gactaaggaa aactaggaag aagtctatga attactcaat gatgtccacc ataacacagg 780
gaagggaaga aaatcctact gcctttctgg agagactaag ggaggcattg aggaagcgtg 840
cctctctgtc acctgactct tctgaaggcc aactaatctt aaagcgtaag tttatcactc 900
agtcagctgc agacattaga aaaaaacttc aaaagtctgc cgtaggcccg gagcaaaact 960
tagaaaccct attgaacttg gcaacctcgg ttttttataa tagagatcag gaggagcagg 1020
cggaacagga caaaacggat taaaaaaaag gccaccgctt tagtcatgac cctcaggcaa 1080
gtggactttg gaggctctgg aaaagggaaa agctgggcaa attgaatgcc taatagggct 1140
tgcttccagt gcggtctaca aggacacttt aaaaaagatt gtccaagtag aagtaagccg 1200
ccccctcgtc catgcccctt atttcaaggg aatcactgga aggcccactg ccccagggga 1260
caaaggtcct ctgagtcaga agccactaac cagatgatcc agcagcagga ctgagggtgc 1320
ctgggg 1326

<210> 3
<211> 10499
<212> ADN
<213> Homo sapiens

<400> 3
ccctggggcg ggcttccttt ctgggatgag ggcaaaacgc ctggagatac agcaattatc 60
ttgcaactga gagacaggac tagctggatt tcctaggccg actaagaatc cctaagccta 120
gctgggaagg tgaccacgtc cacctttaaa cacggggctt gcaacttagc tcacacctga 180
ccaatcagag agctcactaa aatgctaatt aggcaaagac aggaggtaaa gaaatagcca 240
atcatctatt gcctgagagc acagcaggag ggacaacaat cgggatataa acccaggcat 300
tcgagctggc aacagcagcc cccctttggg tcccttccct ttgtatggga gctgttttca 360
tgctatttca ctctattaaa tcttgcaact gcactcttct ggtccatgtt tcttacggct 420
cgagctgagc ttttgctcac cgtccaccac tgctgtttgc caccaccgca gacctgccgc 480
tgactcccat ccctctggat cctgcagggt gtccgctgtg ctcctgatcc agcgaggcgc 540
ccattgccgc tcccaattgg gctaaaggct tgccattgtt cctgcacggc taagtgcctg 600
ggtttgttct aattgagctg aacactagtc actgggttcc atggttctct tctgtgaccc 660
acggcttcta atagaactat aacacttacc acatggccca agattccatt ccttggaatc 720
cgtgaggcca agaactccag gtcagagaat acgaggcttg ccaccatctt ggaagcggcc 780
tgctaccatc ttggaagtgg ttcaccacca tcttgggagc tctgtgagca aggacccccc 840
ggtaacattt tggcaaccac gaacggacat ccaaagtggt gagtaatatt ggaccacttt 900
cacttgctat tctgtcctat ccttccttag aattggagga aaataccggg cacttgtcgg 960
ccagttaaaa acgattagtg tggccaccgg acttaagact caggtgtgag gctatctggg 1020
gaagggcttt ctaacaaccc ccaacccttc tgggttgggg acttggtttg cctcaagcca 1080
gcttccactt tcagttttct tggggaagcc gagggccgac tagaggcaga aagctgtcgt 1140
cctgaactcc cggcagtagc cggttgagat catggtgtag ccagaagtct caacagtcgc 1200
ccatgcatgc acccctatct ttccttctga cccatacctc ctgggtccca accacaactt 1260
tcttcaaagt gtagccccaa aattctcctt acctctgaat atacttcctc tgatccctgc 1320
ctcctaggta ctattggttc agacttccat ttcctctagc aagttgtatc tccaaaggga 1380

tctaaggaag ctctgcgctg cgtccttagg cacctaggct ataacccagg gagtcttatc 1440
cctggtgtcc ctcccaattt aggcatacag ctcttgacat gggcagttat gtaggaccca 1500
ctccccacca cccttgccag ggccccaagt ttgtaaatgg ctgagggaaa agagagacag 1560
aggagagaga gagaaatgga ggagaaagag agagagacag agaggagaga gagacagtga 1620
gagagacaga agagagagag agacaaagag gagagagaga gagtcaaaga gagaaagaaa 1680
gagaaagaaa tagtaaaaaa cagtgtgccc tattccttta aaagccaggg taaatttaaa 1740
acctgtactt gataattgaa ggtcttctct gtgaccctat agcactccaa tccactttgt 1800
ggtcagtgta aataagagca taggccgaaa gcactgaggc cattgacaac ccgtagcttc 1860
cctatcaaaa atccttaacc cagtaacccg cagatggacc aaatgcattc agtcggtagc 1920
gcaactgctt tgctaaaagt agaaaagtaa cttttagagg aaacctcatt gtgagcacac 1980
ctcacctgtt cagaattatt ctaataaaaa aagcaaaaag gtagcttact aactcaaaaa 2040
tcttaaagta tggggctatt ctgttagaaa aaggtaatgt aactccaacc actgataatt 2100
cccttaaccc agcagatttc ctaacgggat ttaaatctta attaccatac aaaggtccga 2160
ccagacctag gcggaactcc cttcaggaca ggacgataga tggttcctcc caggtgattg 2220
aggaaaaaaa ccacaatggg tattcagtaa ttgatacggg gactcttgtg gaagcagagt 2280
tagaaaaatt gcctaataac tggtctcctc aaacgtgtga gctgtttgca ctcagccaag 2340
ccttaaagta cttacagaat caaaagacta tctcaatcct gattcaaaag gttagctaca 2400
ccctctctgt aatgcatttg cataagaact tgtttatggg aatgcatctt gatggggcag 2460
ctgggttgtt ataaaatagg aacccagccc agctctagga ctcacccctg agcgcaaagg 2520
caatgttggg catgctggta aaggaccact agaatccagc agcccagacc cctttctttg 2580
tggtcaagaa aggcgggaaa aggggtgcag gactgctaca tcggtaagca taactaatcc 2640
gataaacaga ggtccatggg tggttacgca ccctggaaag gaactcaccc ctgagcacaa 2700
aggcaatgtt gggcacgctg gtaaaggacc actagaatcc agcagcctgg acccctttct 2760
ttgtggtcaa gagaggcagg aaaacaggtg caggactgca acatcagtga gcataactaa 2820
ttcgataagc agaggtccat gggtggtgat gcaccctgga aagaataagc attaggacca 2880
tagaggacac tccaggacta aagctcatcg gaaaatgact agggttgctg gcatccctat 2940
gttctttttt cagatgggaa acgttccccg caagacaaaa acgcccctaa gacgtattct 3000
ggagaattgg gaccaatttg accctcagac actaagaaag aaacgactta tattcttctg 3060
cagtgccgcc tggcactcct gagggaagta taaattataa caccatctta cagctagacc 3120
tcttttgtag aaaaggcaaa tggagtgaag tgccataagt acaaactttc ttttcattaa 3180
gagacaactc acaattatgt aaaaagtgtg atttatgccc tacaggaagc cttcagagtc 3240
tacctcccta tcccagcatc cccgactcct tccccaacta ataaggaccc cccttcaacc 3300
caaatggtcc aaaaggagat agacaaaagg gtaaacagtg aaccaaagag tgccaatatt 3360
ccccaattat gacccctcca agcagtggga ggaagagaat tcggcccagc cagagtgcat 3420
gtgccttttt ctctcccaga cttaaagcaa ataaaaacag acttaggtaa attctcagat 3480
aaccctgatg gctatattga tgttttacaa gggttaggac aattctttga tctgacatgg 3540
agagatataa tgtcactgct aaatcagaca ctaaccccaa atgagagaag tgccaccata 3600
actgcagcct gagagtttgg cgatctctgg tatctcagtc aggtcaatga taggatgaca 3660
acagaggaaa gagaatgatt ccccacaggc cagcaggcag ttcccagtct agaccctcat 3720
tgggacacag aatcagaaca tggagattgg tgctgcagac atttgctaac ttgtgtgcta 3780
gaaggactaa ggaaaactag gaagaagtct atgaattact caatgatgtc caccataaca 3840
cagggaaggg aagaaaatcc tactgccttt ctggagagac taagggaggc attgaggaag 3900
cgtgcctctc tgtcacctga ctcttctgaa ggccaactaa tcttaaagcg taagtttatc 3960
actcagtcag ctgcagacat tagaaaaaaa cttcaaaagt ctgccgtagg cccggagcaa 4020
aacttagaaa ccctattgaa cttggcaacc tcggtttttt ataatagaga tcaggaggag 4080
caggcggaac aggacaaacg ggattaaaaa aaaggccacc gctttagtca tgaccctcag 4140
gcaagtggac tttggaggct ctggaaaagg gaaaagctgg gcaaattgaa tgcctaatag 4200
ggcttgcttc cagtgcggtc tacaaggaca ctttaaaaaa gattgtccaa gtagaagtaa 4260
gccgccccct cgtccatgcc ccttatttca agggaatcac tggaaggccc actgccccag 4320
gggacaaagg tcctctgagt cagaagccac taaccagatg atccagcagc aggactgagg 4380
gtgcctgggg caagcgccat cccatgccat caccctcaca gagccctggg tatgcttgac 4440
cattgagggc caggaggttg tctcctggac actggtgcgg tcttcttagt cttactcttc 4500
tgtcccggac aactgtcctc cagatctgtc actatctgag gggtcctaa gacgggcagt 4560
cactagatac ttctcccagc cactaagtta tgactgggga gctttattct tttcacatgc 4620
ttttctaatt atgcttgaaa gccccactac cttgttaggg agagacattc tagcaaaagc 4680
aggggccatt atacacctga acataggaga aggaacaccc gtttgttgtc ccctgcttga 4740
ggaaggaatt aatcctgaag tctgggcaac agaaggacaa tatggacgag caaagaatgc 4800
ccgtcctgtt caagttaaac taaaggattc cacctccttt ccctaccaaa ggcagtaccc 4860
cctcagaccc aaggcccaac aaggactcca aaagattgtt aaggacctaa aagcccaagg 4920
cctagtaaaa ccatgcagta accccctgcag tactccaatt ttaggagtac agaaacccaa 4980
cagacagtgg aggttagtgc aagatctcag gattatcaat gaggctgttg ttcctctata 5040
gccagctgta cctagccctt atactctgct ttcccaaata ccagaggaag cagagtggtt 5100
tacagtcctg gaccttcagg atgccttctt ctgcatccct gtacatcctg actctcaatt 5160
cttgtttgcc tttgaagata cttcaaaccc aacatctcaa ctcacctgga ctattttacc 5220
ccaagggttc agggatagtc cccatctatt tggccaggca ttagcccaag acttgagcca 5280
atcctcatac ctggacactt gtccttcggt aggtggatga tttacttttg gccgcccatt 5340
cagaaacctt gtgccatcaa gccacccaag cgctcttcaa tttcctcgct acctgtggct 5400
acatggtttc caaaccaaag gctcaactct gctcacagca ggttacttag ggctaaaatt 5460

atccaaaggc accagggccc tcagtgagga acacatccag cctatactgg cttatcctca 5520
tcccaaaacc ctaaagcaac taaggggatt ccttggcgta ataggtttct gccgaaaatg 5580
gattcccagg tatggcgaaa tagccaggtc attaaataca ctaattaagg aaactcagaa 5640
agccaatacc catttagtaa gatggacaac tgaagtagaa gtggctttcc aggccctaac 5700
ccaagcccca gtgttaagtt tgccaacagg gcaagacttt tcttcatatg tcacagaaaa 5760
aacaggaata gctctaggag tccttacaca gatccgaggg atgagcttgc aacctgtggc 5820
atacctgact aaggaaattg atgtagtggc aaagggttga cctcattgtt tacgggtagt 5880
ggtggcagta gcagtcttag tatctgaagc agttaaaata atacagggaa gagatcttac 5940
tgtgtggaca tctcatgatg tgaatggcat actcactgct aaaggagact tgtggctgtc 6000
agacaactgt ttacttaaat gtcaggctct attacttgaa gggccagtgc tgcgactgtg 6060
cacttgtgca actcttaacc cagccacatt tcttccagac aatgaagaaa agataaaaca 6120
taactgtcaa caagtaattt ctcaaaccta tgccactcga ggggaccttt tagaggttcc 6180
tttgactgat cccgacctca acttgtatac tgatggaagt tcctttgtag aaaaaggact 6240
tcgaaaagtg gggtatgcag tggtcagtga taatggaata cttgaaagta atcccctcac 6300
tccaggaact agtgctcagc tagcagaact aatagccctc acttgggcac tagaattagg 6360
agaagaaaaa agggcaaata tatatacaga ctctaaatat gcttacctag tcctccatgc 6420
ccatgcagca atatggaaag aaagggaatt cctaacttct gagagaacac ctatcaaaca 6480
tcaggaagcc attaggaaat tattattggc tgtacagaaa cctaaagagg tggcagtctt 6540
acactgccgg ggtcatcaga aaggaaagga aagggaaata gaagagaact gccaagcaga 6600
tattgaagcc aaaagagctg caaggcagga ccctccatta gaaatgctta taaaacaacc 6660
cctagtatag ggtaatcccc tccgggaaac caagccccag tactcagcag gagaaacaga 6720
atggggaacc tcacgaggac agttttctcc cctcgggacg gctagccact gaagaaggga 6780
aaatactttt gcctgcaact atccaatgga aattacttaa aacccttcat caaacctttc 6840
acttaggcat cgatagcacc catcagatgg ccaaatcatt atttactgga ccaggccttt 6900
tcaaaactat caagcagata gtcagggcct gtgaagtgtg ccagagaaat aatcccctgc 6960
cttatcgcca agctccttca ggagaacaaa gaacaggcca ttaccctgga gaagactggc 7020
aactgatttt acccacaagc ccaaacctca gggatttcag tatctactag tctgggtaga 7080
tactttcacg ggttgggcag aggccttccc ctgtaggaca gaaaaggccc aagaggtaat 7140
aaaggcacta gttcatgaaa taattcccag attcggactt ccccgaggct tacagagtga 7200
caatagccct gctttccagg ccacagtaac ccagggagta tcccaggcgt taggtatacg 7260
atatcactta cactgcgcct gaaggccaca gtcctcaggg aaggtcgaga aaatgaatga 7320
aacactcaaa ggacatctaa aaaagcaaac ccaggaaacc cacctcacat ggcctgctct 7380
gttgcctata gccttaaaaa gaatctgcaa ctttccccaa aaagcaggac ttagcccata 7440
cgaaatgctg tatggaaggc ccttcataac caatgacctt gtgcttgacc caagacagcc 7500
aacttagttg cagacatcac ctccttagcc aaatatcaac aagttcttaa aacattacaa 7560
ggaacctatc cctgagaaga gggaaaagaa ctattccacc cttgtgacat ggtattagtc 7620
aagtcccttc cctctaattc cccatcccta gatacatcct gggaaggacc ctacccagtc 7680
attttatcta ccccaactgc ggttaaagtg gctggagtgg agtcttggat acatcacact 7740
tgagtcaaat cctggatact gccaaaggaa cctgaaaatc caggagacaa cgctagctat 7800
tcctgtgaac ctctagagga tttgcgcctg ctcttcaaac aacaaccagg aggaaagtaa 7860
ctaaaatcat aaatccccat ggccctccct tatcatattt ttctctttac tgttctttta 7920
ccctctttca ctctcactgc accccctcca tgccgctgta tgaccagtag ctccccttac 7980
caagagtttc tatggagaat gcagcgtccc ggaaatattg atgccccatc gtataggagt 8040
ctttctaagg gaacccccac cttcactgcc cacacccata tgccccgcaa ctgctatcac 8100
tctgccactc tttgcatgca tgcaaatact cattattgga caggaaaaat gattaatcct 8160
agttgtcctg gaggacttgg agtcactgtc tgttggactt acttcaccca aactggtatg 8220
tctgatgggg gtggagttca agatcaggca agagaaaaac atgtaaaaga agtaatctcc 8280
caactcaccc gggtacatgg cacctctagc ccctacaaag gactagatct ctcaaaacta 8340
catgaaaccc tccgtaccca tactcgcctg gtaagcctat ttaataccac cctcactggg 8400
ctccatgagg tctcggccca aaaccctact aactgttgga tatgcctccc cctgaacttc 8460
aggccatatg tttcaatccc tgtacctgaa caatggaaca acttcagcac agaaataaac 8520
accacttccg ttttagtagg acctcttgtt tccaatctgg aaataaccca tacctcaaac 8580
ctcacctgtg taaaatttag caatactaca tacacaacca actcccaatg catcaggtgg 8640
gtaactcctc ccacacaaat agtctgccta ccctcaggaa tattttttgt ctgtggtacc 8700
tcagcctatc gttgtttgaa tggctcttca gaatctatgt gcttcctctc attcttagtg 8760
ccccctatga ccatctacac tgaacaagat ttatacagtt atgtcatatc taagccccgc 8820
aacaaaagag tacccattct tccttttgtt ataggagcag gagtgctagg tgcactaggt 8880
actggcattg gcggtatcac aacctctact cagttctact acaaactatc tcaagaacta 8940
aatggggaca tggaacgggt cgccgactcc ctggtcacct tgcaagatca acttaactcc 9000
ctagcagcag tagtccttca aaatcgaaga gctttagact tgctaaccgc tgaaagaggg 9060
ggaacctgtt tatttttagg ggaagaatgc tgttattatg ttaatcaatc cggaatcgtc 9120
actgagaaag ttaaagaaat tcgagatcga atacaacgta gagcagagga gcttcgaaac 9180
actggaccct ggggcctcct cagccaatgg atgccctgga ttctcccctt cttaggacct 9240
ctagcagcta taatattgct actcctcttt ggaccctgta tctttaacct ccttgttaac 9300
tttgtctctt ccagaatcga agctgtaaaa ctacaaatgg agcccaagat gcagtccaag 9360
actaagatct accgcagacc cctggaccgg cctgctagcc cacgatctga tgttaatgac 9420
atcaaaggca cccctcctga ggaaatctca gctgcacaac ctctactacg ccccaattca 9480
gcaggaagca gttagagcgg tctcggccaa cctccccaac agcacttagg ttttcctgtt 9540

gagatggggg actgagagac aggactagct ggatttccta ggctgactaa gaatccctaa 9600
gcctagctgg gaaggtgacc acatccacct ttaaacacgg ggcttgcaac ttagctcaca 9660
cctgaccaat cagagagctc actaaaatgc taattaggca aagacaggag gtaaagaaat 9720
agccaatcat ctattgcctg agagcacagc aggagggaca atgatcggga tataaaccca 9780
agtcttcgag ccggcaacgg caaccccctt tgggtcccct ccctttgtat gggagctctg 9840
ttttcatgct atttcactct attaaatctt gcaactgcac tcttctggtc catgtttctt 9900
acggcttgag ctgagctttc gctcgccatc caccactgct gtttgccgcc accgcagacc 9960
cgccgctgac tcccatccct ctggatcatg cagggtgtcc gctgtgctcc tgatccagcg 10020
aggcacccat tgccgctccc aatcgggcta aaggcttgcc attgttcctg catggctaag 10080
tgcctgggtt catcctaatt gagctgaaca ctagtcactg ggttccatgg ttctcttctg 10140
tgacccacag cttctaatag agctataaca ctcaccgcat ggcccaaggt tccattcctt 10200
gaatccataa ggccaagaac cccaggtcag agaacacgag gcttgccacc atcttgggag 10260
ctctgtgagc aaggaccccc aagtaacaca accatgaggg tgcaaatgca tgggccacta 10320
atggtagagc aagaaaacag aagggccctg gttcctcgaa ggcatcagtg agctgaaatg 10380
cctgccctgg atgtcctatt cctaggtgtt tttctgcctg aagcagatta aaccctttgt 10440
tcacttctcc aagtagggct tctattacag cccaaatcaa tccccacccc agatgacat 10499

<210> 4
<211> 2784
<212> ADN
<213> Homo sapiens

<400> 4
ctccttcagg agaacaaaga acaggccact acccaagaga agactggcaa ctagatttta 60
cccatatgcc caaatctcag ggatttcagt atctactagt ttgggtagat actttcactg 120
gttgggcaga ggccttcccc tgtaggacag aaaaggccca agaggtaata aacgttcatg 180
aaataattcc cagattcgga cttccccaag gcttacagag tgacaatggc cctgctttca 240
aggctacagt aacccaagga gtatcccagg tgttaggtat acaatatcac tcacactgcg 300
cctggaggcc acagtcctca ggaaaggtgg agaaaatgaa caaaacactc aaatgacatc 360
taaaaaagct aatccaggaa acccacctcg catggcctgc tctgttgcct atagccttac 420
taagaatccg aaactctccc caaaaagcag gacttagtcc atacaaaatg ctgtatggac 480
ggcccttcct aaccaatgaa cttgggcttg accgagagac agccaactta gttgcagaca 540
tcatctcctt agccaaatat caacaggttc ttaaaacatt acagggagcc tgtccccaag 600
aagagggaaa ggaactattc caccctggtg acatggtatt agtcaagtcc cttccctcta 660
attccccatc cctagataca tcctgggaag gaaactaccc agccatttta tctaccctaa 720
cggcagttaa agtggctgga gcggagtctt ggatacatca cactcaagtc aaaccctgga 780
tactgccaaa ggaactcaaa aatccatgag acaatgctag ctattcctgt gaacctctag 840
aggatctgcg cctgctcttc aaatgacaac cagggggaaa gtaactaaaa tcgtaaatcc 900
cctggccctc ccttatcata tttttctctt tactgttctc ttaccccctt tcactctcac 960
tgcaccccgt ccatgccact gcaccccgtc catgccccgt ccatgccagt agctcccctt 1020
agcaagagtt tctatggaga atgcagcgtc ccggaaatat tgatgcccca ttgtatagga 1080
gtttatctaa gggaaccccc accttcactg cccacaccca tatgccccac aactgctata 1140
actctgccac tctttgcatg catgcaaata ctcattattg gacaggaaaa acgattaatc 1200
ccagttgtcc tggaggactt ggaggactca cttcactcat accagtatgt ctgatggggg 1260
tggagttcaa gatcaggcaa cagaaaaaca cataaaggaa gtaatctccc aactgacctg 1320
ggtacatagc acccctggcc cctacaaagg actagatctc tcaaaactac atgaaaccct 1380
ccatacccat actggcctgg taagcctatt taataccacc ctgactgggc tccatgaggt 1440
ctcggcccaa aaccctacta actgttggat gtgcctcccc ctgcacttta ggccatacat 1500
ttcaatccct atacctgaac aatggaacaa cttcagcaca gaaataaaca ccacttctgt 1560
tttagtaggt cctctttcca atctggaaat aacccatacc tcaaacctca cctgtgtaaa 1620
atttagcaat actatagaca cagccaactc ccaatgcatc aggtgggtaa ctcctcccac 1680
acgaatagtc tgcctaccct caggaatatt ttttgtctgt ggtacctcag cctatcattg 1740
tttgaatggc tcttcagaat ctgtgtgctt cctctcattc ttagtggccc ctatgcccat 1800
ctacactgaa caagatttat acaatcatgt catacctaag ccccgcaaca aaagagtacc 1860
cattcttcct tttgttattg gagcaggagt gctaggcgga gtagctactg gcattggcgg 1920
tatcacaacc tctactcagt tctactacaa actgtctcaa gaactaaatg gtgacatgga 1980
atgggtcgct gataccctgg tcaccttgca agatcaactt aactccctag cagcagtagt 2040
ccttcaaaat cgaagagctt tagacttgct aaccgcggaa agcgggggaa cctttttatt 2100
tttagaggaa aaatgctgtt gttatgttaa tcaatccgga atcatcaccg agaaagttaa 2160
agaaattcaa ggtcgaatat aacgtagagc aaaggagctg caaaacactg gaccctgggg 2220
cctcctcagc caatggatgc cctggattct ccccttctta ggacctctag cagctataat 2280
attgttactc ctctttggac cctgtatctt taacctcctt gttaagtttg tcttttccag 2340
aatcgaagca gtaaaactac aaatcgttct tcaaatggag ccccagatgc agtccatgag 2400
taaaatctac cacggacccc tggaccggcc tgctagccca tgctctgatg ttaatgacat 2460
caaaggcacc cctcccgagg aaatctcaac tgcacaacct ctactacgcc ccaattcagc 2520
aggaagcagt tagagtggtt gttggccaac ctccccaaca gcagttgggt tttcctgttg 2580
agagggggga ctgagagaca ggaataacta gatttcctag accaactaag aatccctaag 2640

actagctggg aaggtgaccg cttccacctt taaacaccgg gcttgcaact tagctcacgc 2700
ccaaccaatc agatactaaa gagagctcac taaaatgcta attaggcaaa aacaggagat 2760
aaagaaatag ccaatcatct gttg 2784

<210> 5
<211> 1799
<212> ADN
<213> Homo sapiens

<400> 5
gggattctta gtcggcctag gaaatccagc taatcctgtc tctcagtccc cccactcaac 60
aggaaaaccc aagtgctgtt ggggaggttg gctgacgacc agtctaactg cttcctgcgg 120
aattggggca tagtaggggt tgtgcagttg agatttcctc gggaggggtg cgttcgatat 180
cattacaatt ggagcatggg ctagtaggcc ggtccagggg tccacggtag atcttagtca 240
tggacttcat ctggggttcc atttgaagaa cgatttgtag ctttacaact ttgattctgg 300
aagagacaaa cttaacaagg aggttaaaga tacagggtcc aaagaggagt atcaatatta 360
gagctgctag agatcctaag aaggggagaa tccagggcat ccattggctg aggaggcccc 420
agggtctggt gtttttgaag ctcctctgtt ctacgttgta ttcaatctcg aatttcttca 480
actttctctg tgacaattca ggattgatta acataataac aacattcttc cgctaaaata 540
acataataac aacattcttc ccctaaaaat aaacagcttc cccctctttc agaggttagc 600
aagtctaaag ctcttcaatt ttgaaggact actgatgcta ggaagttaag ttgatcttgc 660
aaggtgacca gggagtcggc aacccattcc atgtcaccat tgagttcttg agatagtttg 720
tagtagaact gagtagaggt tgtggtaccg ccaatgccag aacctagtcc acctagcact 780
cctgctccga taacaaaagg aagaatgagt actcttttgt tgtggggctt aggtacaaca 840
taattgtata aatcttgttc agtgtaaatg gtcatggggg cactaagaat gagaggaagc 900
acatagattc tgaagagcca ttcaaacaac gataggctaa ggtaccacag acaaaaaata 960
ttcctgaggg taggcagact attcgtgtgg gaggagttac ccacctgatg cattgggagt 1020
tggttgtgtc tacagtattg ctaaatttta cacaggtgag gtttgaggta tgggttattt 1080
ccagattgga aacaagaggt cctactaaaa cggaagtggt gtttatttct gtgctgtagt 1140
tgttccattg ttcaggtaca gggattgaaa tgcatggcct gaaatacagg gggaggcaca 1200
accaacagtt agtagggttt tggaccgaga cctcatggag cccagtgagg gtggtattaa 1260
ataggcttac caggcaagta tgggtatgga gggtttcatg tagttttaag agatctagtc 1320
ctttgtaggg gctaggggtg ctatgtaccc gggtcagttg ggaggttact tcctttacat 1380
gtttttctct tgcctgatct tgaactccac ccccctcaga cataccagta tgggtgaagt 1440
aagtccgaca gacagtggct ccaagtcttc caggacaact aggattaatc attttccctg 1500
tccaataatg agtatttgca tgcatgcaaa gagtggcaga gttatagcag ttgtggggca 1560
tatgggtgtg ggcagtgaag gtggagtttc ctttaggtaa actcctattt gatggggcat 1620
caatatttct gggaagccgc attcttcata gaaactcttg gtaaggggag ctgctggttg 1680
tacagcagca tggagggggt gcagtgagag tgaaaggggg taagagaaca gtaaagagaa 1740
aaatatgata agggagggcc atggggattt acgattttag ttactttcct cacggttgt 1799

<210> 6
<211> 1489
<212> ADN
<213> Homo sapiens

<400> 6
tggtgcttgc cccgggcact ctcagtcctg ctgctggatc atctggttag tggcttctga 60
ctcagaggac ctacgtcccc tggggcagtg ggccttacag tgattccctt gacacgaggt 120
gcatggacga gggggcggct tatttctatt tggacaatct tttttaaagt gtccttgtag 180
accgcactgg aagcaaaccc tattaggcat ttgatttgcc tagcttttcc cttttccagt 240
gcctccaaag tccgcttgcc tgagggccat gactaaagcg gtggcctttt ttttatccca 300
tttgtcccat tctgcctgct catcctgatc tctattataa aaaactgagg ttgccaagtt 360
caatagggtt tctaagtttt gttccgggcc taaggcagac ttttgaagtt ttttcctaat 420
gtctgtagct gactgagtga taaacttatc ctttaagatt agttggcctt cagtagagtc 480
agttgacaga gagaggtatg cttcctcaat gcctccgtta gtcactccag aaaggcggta 540
ggatttcttt cctttccctg tgttatagtg gacatcattg aataactcac aggcttcttt 600
ctagttttcc ttagtccttc tagcacgcaa gttagcaaat gtctgcggca ccaatctcca 660
tgttctgatt ctgtgtccca gtgagggtct acactgggaa ctgcctgctg gcctgtgggg 720
aatcgttctc tttcctctgt tgtcgaccta tcattgacct gactgagata ccagagatcg 780
ccaaactctc aggctgcagt tacggcgaca cttctgtcat ttggggttag tgtctgattt 840
agcagtaaca ttatatctct ccatatcaga tcaaaggatt gtcctaaacc ttgtaaaaca 900
tcaatatagc cattagggtt atctgagaat ttacctaggt ctattttaat ttaaagtctg 960
ggagagaaaa aggcacatgc actctggctg ggccgaattc tcttcctccc actgcgtctg 1020
agagagaaaa aggtacgtgc actctggctg ggccgaattc tcctcccacc gcttggaggg 1080
ggcataatcg gggaatattg gcattctttg gttagttgtt taccccttg tctatctcct 1140

tttggaccgt ttgggttgaa gggggtcct tattatttgg ggaaggagtc tgggggatgc 1200
tggggtaggg aggtagactc tgagggcttc ctgtagggca taaatcacac tttttacata 1260
attgcgagtt gtctcttaat gaaaagaaag tttgtacgta tgacacttca caccatttgc 1320
cttcttttct acaaaagagg tctagctgta agatggtgtt ataatttatg cttccctcag 1380
gatgccaggt ttctccccct taaagagtat atcgttgcca ggcggtactg cagaagaata 1440
tgtctttttt ttcttagcat ctgagagtca aattggtccc aattctcca 1489

<210> 7
<211> 1216
<212> ADN
<213> Homo sapiens

<400> 7
taaagataca gggattgaaa tgtatggcct gaagtgcagg gtcatatagg tgtgggtggt 60
gaaaatgggg tttcctttag aaaaactcct atacgatggg tcatcaatat ttccaggaag 120
ccgcattctc catagaagct cttggtaatg ggagctactg gtagtacagt ggcatggagg 180
gggtgcagtg agagtgaaag agggtaaaag aacagtaaag agaaaaatat gataagggag 240
gggttcagtg agagtgaaag ggggtaagag aacagtaaag aaaaaaatat gacaaggagg 300
gccatgagga tctacgattc tagttacttt cctcacggtt gtcgcttgaa gagcaggtgc 360
agatcctcta gaggttcaca ggaatagcta gcgttgtctc ctggattttc gggttccttt 420
ggcagtatac agagtttgac tcgagtgtga tgtattcaag actccactcc agccacttta 480
accgcagttg gggtagataa aatgactggg tagggtcctt cccaggatgt atctaaggat 540
ggggacttag aaggaaggga cttgactaat accatgtcac cagggtgcaa taattacttt 600
ccctcttctc gggaacaggt tccctgtaat gttttaagaa cttgttgata tttggccaag 660
gaggtgatgt ctgcaactaa gctggccatc tctcggtcaa gcacaaggtc cttggttagg 720
aagggccatc catacagcat tttgtatggg ctaagtcctg ctttttgggg agagttttgg 780
attcttagta aggctgtagg caacagagca ggccatgcaa ggtgggtttc ttgggttagc 840
ttttttaaat gtcgtttgag tgcttcattc attttcttga cttttcctga ggattgtggc 900
ctccacgcgc agtgtaagtg atattgtatg cctaatgcct gggatactcc ctgggttact 960
gtagccttga aaacggggcc attgtcactc tgtaagcctc ggggaagtcc gaatctggga 1020
attatttcat gaattagtgc ctttattaca tcttggtcct tttctgtcct acaaaggaag 1080
gcctctgccc aaccagtgaa aatatctacc cagactagta gatactgaaa tccctgagat 1140
ttgggcatgt gggtaaaatc tagttgccag tcttctcctg agtaatggcc tgttctttgt 1200
tctcctgaag gagctt 1216

<210> 8
<211> 976
<212> ADN
<213> Homo sapiens

<400> 8
   agtgataatg gaatacttga aagtaatccc ctcactccag gaactagtgc tgagctggcc 60
   aaactaatag ccctcactcg ggcactagaa ttaggagaag agaaaagggt aaatatatat 120
   acagactata agtatgctta cctagtcctt catgcccatg cagcaatatg gagagaaagg 180
   gaattcctaa cttccaaagg aacacctatc aaacatcagg aagccattag gatattatta 240
   ttggtggtac agaaacctaa agaggtggca gtcctacact gctggggtca tcagaaaaaa 300
   aaggaaaggg aaatagaagg gaactaccaa gcagatattg aagccaaaag agccgcaagg 360
   caggaccctc cattagaaat gcttatagaa ggacccctag tgtggggtaa ccccctccag 420
   gaaagcaatc cccagtactc agcaggagaa ataaaatgga gaacctcacg aggacatact 480
   ttcctcccct caggatggct agccaccaaa gaaggaaaaa tgcttttgcc tgcagctaac 540
   caatggaaat tacttaaaac ccttcaccaa acctttcact taggattgat agcacccatc 600
   agatggccaa attattattt actggatcag gccttttcaa aactatcaag caggtagtca 660
   gggcctgtaa agtgtgccaa agaaataatc tcctgcactg caagccatac atttcaatcc 720
   ctgtatcttt aacctccttg ttaagtttgt ctcttccaga atcaaagctg taaaactaca 780
   aatggttctt caaatggagt ctcagatgca gtccatgact aagatatacc gcagccccct 840
   ggagggggcc tgctagccca tgctccaatg ttaatgacat cgaaggcacc cctcccgggg 900
   aaatctcaac tgcacaaccc ctactatgtc ccaattcagc aggaagcagt taaagcggtc 960
   atcggccaac ctcccc 976

<210> 9
<211> 942
<212> ADN
<213> Homo sapiens

<400> 9

agaggagaac agcagcataa gcggctggca gaggtaggga aagaccagca agaagaaaag 60
agagaaagag aaagagaaag tcagagaaag agacagagag aggaagagac aaagagacag 120
aaagtcaaag aggtagtagt cagaaacaga gacaaaaaaa aggagtcaga aagagggaca 180
gacacagaaa gtcaaaaaaa aagttaagaa gaaaggaaaa gacaaagaag aagtcgaaga 240
ggagaaagag agagatagaa gtagtaaaga aaaaaacagc atatcccatt cctttaaagc 300
cagggtaaat ttctatctac ccagccaagg catattctac ttatgtggat cttcaaccca 360
tatctgcctc tcagacagtt tgcaagaaat aatgaaatct atccttactt tacaatccca 420
aatagactct ttggcagcag tgactctcca aaactgcaga ggcctagacc tcctcactgc 480
tgaaaaagga ggacactaca ccttcttagg ggaagaatgt tgtttttaca ctaaccagtc 540
ggggatagta tgagatgctg cccggagttt acaggaaaag gcttctgaaa tcagacaacg 600
cctttcaaat tcttatacca acttctggag ttaggcaaca tggcttctcc cctttctagg 660
tcctgtggca gccatcttgc tgttactcgc ctttgggccc tgtattttta accttcttgt 720
caaatttgtt tcctctagaa tcgaggccat caagctacag atggtcttac aaatggaacc 780
ccaaaagagt tcaactaaca acttctaccg aggacccctg gatcaaccca ctggcacttc 840
ccctggccta gagagttccc ctctgaagga caccgcaact gcagggccct tctttgcccc 900
atccagcagg agtagctaga gtggtcatcg gccaaattgc ca 942

<210> 10
<211> 1375
<212> ADN
<213> Homo sapiens

<400> 10
ccccaatatt ctctttctga tggggaaaaa tggccacctg agggaagcac aaattacaat 60
actatcctgc agcttgatct tttctgtaag agggaaggca aatggagtga aataccttat 120
gtccaagctt tcttttcatt gagggagaat acacaactat gcaaagcttg caatttacat 180
cccacaggag gacccctcag cttaccccca tatcctagcc tccctatagc ttcccttcct 240
attgatgata ctcctcctct aatctcccct gcccagaagg aaataagcaa agaaatctcc 300
aaaggtccac aaaaaccccc gggctatcgg ttatgtcccc ttcaagctgt agggggaggg 360
gaatttggcc caacccgggt gcatgtcccc ttctccctct ctgatttaaa gcagatcagg 420
cagacctggg gaagttttca gatgatcctg ataggtacat agatgtccta cagggtctag 480
ggcaaacctt tgacctcact tggagagacg tcatgctact gttagatcaa accctggcct 540
ttaatgaaaa gaatgcggct ttagctgcag cctgagagtt tggagatacc tggtatccta 600
gtcaagtaaa tgaaagaatg acagccgaag aaagggacaa cttccctact ggtcagcaag 660
ccatccccag tatggatccc cactgggact ttgactcaga tcatggggac tggagtcgta 720
aacatctgtt gatctgtgtt ctggaaggac taaggagaat tgggaaaaag cccatgaatt 780
attcaatgat atccaccata acccagggaa aggaagaaaa tccttctgcc ttcctcgagc 840
ggctacaaga ggccttaaga aaatatactc ccctgtcacc cgaatcactc gagggtcaat 900
tgattctaaa agataagttt attacccaat cagccacaga tatcaggaga aagctccaaa 960
agcaagccct gagccctgaa caaaatctag agacattatt aaacctggca accttggtgt 1020
tctataatag ggaccaagag gaacaggccc aaaaggaaaa gcgagatcag agaaaggccg 1080
cagccttagt catggccctc agacaaacaa accttggtgg ttcagagagg tcagaaaatg 1140
gagcaggcca atcacctggt acggcttgtt atcagtgcgg tttactagga cactttaaaa 1200
aagattgtcc aataagaaac aagctgcccc ctcatccgtg tccactatgc cgaggcaatc 1260
actggaaggt gcactgcccc agaggatgaa ggttccctgg gttagaagcc cccaaccaga 1320
tgatccaaca acaggactga gggtgcccgg ggcaagcacc agctcatgtc atcac 1375

<210> 11
<211> 944
<212> ADN
<213> Homo sapiens

<400> 11
acctaggagg aactgtcttc aggacaggac tatagatgct tcctcccagg cgattaaggg 60
aaaaagacac aatgggtatt cagtaagtga taaggaaact cttgtagaag cagagttagg 120
aaaattgcct aataattggt ctgctcaaat gtgcgagctg tttgcactca gccaaacctt 180
aaaagtatta cagaatcagg aagaagccat ctataccaat tctaagttaa tatggactga 240
acgagaactt attaatagca aagaataatt gaaatcccaa acttacaagg ttttcaacaa 300
aagcacagtt tgctaaaagt taactgtgta acatgtatta tcctactacc acaaactctc 360
aaatgatttc tcagacagtt tgcaagaaac aatgaaacct atccttactc tacaatccca 420
aatagactct ttggcagcag tgactctcca aaaccaccaa ggcctagacc tcctcactgc 480
tgagaaagga ggactctgca ccttcttagg ggaagattgt tgtttttaca ctaaccagtc 540
agggatagtg tgagatgcca cccagcgttt acaggaaaag gcttctgaaa tcagacacaa 600
tgcttttcaa accttatagc aacctctgga gttcggcgac tggcttttcc cctttctagg 660
tcctgtgaca gccatcttgc tattactcgc cttcgggccc tgtattttta acctcctcgt 720
caaatttgtt tcctctagga tcgaggccat caagctacag atggtcttac aaatggaacc 780

ccaaatgagc tcgactaaca acttctactg aggacccctg gaccgaccca ctggcccttt 840
aactggctta aagagtttcc ctctggagga cactacaact gcagggcccc ttctttgccc 900
catccacagg aagttagcta gagcagtcat cacccaattc ccaa 944

<210> 12
<211> 963
<212> ADN
<213> Homo sapiens

<400> 12
tacaggaacc ccataatacg tccttggcaa attctattca gctccaactg ctaggagtgg 60
cccatttgtc ctgaaccctc aaatcatggg aatgagaaat gaatttagac tgaccacagc 120
ccttatgagt tttcagctac aggggtgtat agaaccctga taaggagttt tctttgtgtg 180
tggaagatcc ttctatattt gcctccccac caactggaca ggaacttgta ctttagccta 240
catagtacct cctgtgactt atccttttca gaagaggcag tagctgtgcc cattcatgct 300
aagcttcagc cgagagcaat ctcactactt cctctattgg ctggtttagg atttactacc 360
acctaggaag tggactcaca gcctagatga aatctctctc caacttactc aaatccagga 420
ccaaatagac tcattagcag ctgtggttct ccgaaccagt gagcactaga tctccaatct 480
cctcactgcc gaaaggggag gaacatgcct ttttctgaac aaggaatgtt gtttttatgt 540
caataaatca ggcatagtga gagatggaat taaatgactt caggatagag ctagcagact 600
acatggtggg acaaccgaaa ctacctcagg gttctcacag cctgttctcc actggcttct 660
tccattttta ggtcccttcc ttatgattat tctaggagta acctttggcc catgtctttt 720
cagttccttc atcctttcgt ttcttcctga atagaatcaa tgaaactaga aatgttactg 780
cagatggaac ctcagatgac ttcaaccagc acctattatc aaggacccct aaaccagcct 840
gccggcccat acccggacgt tgacacccaa accacctctc acgaggaaac ctcagctaca 900
gaaccccttc tatgccccta ttcagcagga agcaattaga gtggtcatcc tcccacaccc 960
caa 963

<210> 13
<211> 1362
<212> ADN
<213> Homo sapiens

<400> 13
ccacaatatc ctcttccagg aggagaacga tggccacctg agggaagtat acactataat 60
accatcctgc aactagatct gttttgtaaa caagaaggca agtggattta ggtaccatat 120
gttcagacct ttttctcatt aagggatgat aacccacgat tgtgtaagac atgtaacctg 180
caccccacag ggagtcctca aattctaccc ccatacccag tcctccccac ggctcctcct 240
actaatgcca aaccctctct ggcttctaca gcccaaaagg gaacaaataa aagagccttc 300
agagagccaa gagaccccac tggcccctgg ctatgtcctc ttcaggctgt aggaggggaa 360
tttggcccaa cccgagtaca tgttcccttt tctctctctg atctaaagca aattaaggca 420
gacttggatg aaagttctca gatgacccca atagatacgt agatggcctg ctgggtctgg 480
gacaatcttt tgacctttcc tggagagaga tcatgttatt gcttgatcag acctaacctc 540
taatgagaag aatgctgctt taacaggagc ccgagagttt ggggatacct ggtacctcag 600
ttaagtaagt gatagaatga catcagaaga gagcagtttc ctactggcca gcaagcagtc 660
cccagtatgg atccccactg ggaccctgac tcggatcatg gggactggag tcacaaacat 720
ttactgacct gtatcctaga agggttaagg agaactagga aaaagcccat gaactattca 780
atgatgtcta ctataaccca agggaaggaa gaaaacccta ttgccttcct caaaaggctg 840
agggaggctt tgagaaaata tactcccctg tcaccagatt ccctcgaagg ccagttaatt 900
ttaaaggaca aatttattac tcagtcagct gcagacatta ggaaaaagct ccaaaagtta 960
gccttgggcc gagcaaaatt tggaggcatc attaaacctg gcaacctcag tgttctatca 1020
tagggaccaa gaggaacagg ccgaaaagga aaagcaggat aagagaaagg ctgcagattt 1080
agtcatgccc tcagacaaac cttggcggtt caaagaggag aaaaaatgga gcaggccaat 1140
cacccagcag ggcttattat cagtgcagtt tacaaggaca ctttaaacaa gattgtccaa 1200
agagaaataa gccgccctct cacccatgtc cactatgcca aggtgatcac tggaaggcac 1260
actgtcccag aggacaaagg ttctctgggc cagaagtccc caaccagatg atccagcaac 1320
aggatggagg gtgcccgggg caagcaccag ctcgtgttgt ca 1362

<210> 14
<211> 945
<212> ADN
<213> Homo sapiens

<400> 14
ttgcagatca atctcagact gctgtgctag caatgagtga ggcttcgtgg gcatgggacc 60

ctctgagcca ggcatgggat ataatgtcct tgtgtgccat ttgctaagac tgttggaata 120
gcacagtatt agggtgggag tggcccgatt ttccaggtgc tgtctgtcac cgcttccctt 180
ggctaggaaa gagaattccc tgaccccttg ttcttcccag gtaaggcagt gcctcaccct 240
gcttcagctc acactcaggt gactgcaccc actgtcctgc ccccactgtc ggacaagccc 300
cagtgagatg aacctggtac ctcagttgga aatgcagaaa tcacctgtct tctgcgtcac 360
tcacactggg agctgtagac tggagctgtt cctatttggc catcttggaa ccatctccca 420
aatagactct ttggcagcag tgactctcca aaaccaccaa ggcctagacc tcctcattgc 480
tgagaaagga ggactctgca ccttcttagg ggaggagtgt tgtttttata ctgaccagtc 540
agggatggta cgagatgcca cccgatgttt acaggaaaag gcttctgaaa tcacacaaca 600
cctttcaaac tcttatacca acctctggag ttgggcaaca tggcttctcc cctttctcgg 660
tcccattgca gccatcttgc tattactcgc cttcaggctg tgtattttta acctccttgt 720
caaatttgtt tcctctagaa ttgaggccgt caagctacag atggtcttac aaatgggacc 780
ccaaatgagc tcaactaaca acttctgcca aggacccctg gaccaacctg ctggcccttt 840
cactggcctt aagagttccc ctctggaggg cactacaact gcagggcccc ttctttgccc 900
ctatccagca ggaagtagct agagcagtca tcacccaatt cccaa 945

<210> 15
<211> 939
<212> ADN
<213> Homo sapiens

<400> 15
agagctacct tggcaagtac tctaggagta tgggaaaatg aaaacaacaa actcacacac 60
cattttaaca tacacaatca ggtctgccca cccagcaagg tatattcttt gtatgtggaa 120
catcgaccta tatctgcctc cccactaact agacagccac ctgaatctta gtctttctaa 180
gtcccaacag taacattgcc ccaggaaatc agaccatatc agtatccctc aaagctcaag 240
tctgtcagtg cagagccata caactaatac ccctacttat agggtaagga atggctactg 300
ctacaggaac cagaatagct agtttgttta cttcattatc ctactaccac acactctcaa 360
atgatttctc agacagtttg caagaaataa cgaaatctat ccttactcta caatcccaaa 420
tagactcctt ggcagcagtg accctccaaa acggctgagg cctagacctc ctcactgcca 480
agaaaggagg actctgcatt ttcttagggg aagagtgttt ttacactaac cagtcaggga 540
cagtatgaga tgccactcgg agtttacagg aaaaggcttc tgaagtcaga caatgccttt 600
caaactctat accaaactct ggagttgggc aacatggctt ctcccctttc taggtcccgt 660
gacagccatc ttgctattat ttgcctttga gccctgtatt tttaatctcc ttttcaaatt 720
tgtttcctct ggatcgaggc catcgagcta cagatggtct tcacaaatgg aaccccaaat 780
gagctcaact aacaacttct actgaggacc cctggactaa cctgctgacc ctttcactgg 840
cctgaagaat tcccctctgg aggacactac aactgcaggg ctccttcttt gcccctatcc 900
agcaggaagt agctagagct gtcattgcct aattcctaa 939

<210> 16
<211> 979
<212> ADN
<213> Homo sapiens

<400> 16
agtgataatg gaatacttga aagtaatccc ctcactcccc aggaactagt gctcagctgg 60
cagaactaat agccctcact cgggtactag aatcaggaga aggaaaaagg gtaaatatat 120
atacagactc taagtgtgct tacctagtcc tccatgccca tgcagcaata tggagagaaa 180
gggaattcct aacttccgag ggaacaccta tcaaacatca ggaagccatt aggaaattat 240
tattggctgt acagaaacct aaagaggtgg cagttttaca ctgccggggt catcagaaag 300
gaaaggaaag ggaaatacaa gggagccacc aagttgatat tgaagtcaaa agagccacaa 360
ggctggaccc tccattagaa atgcttatag gaggacccct agtatggggt aatcccctcc 420
gggaagccaa gccccagtac tcagcaggag aaatagaata gggaacttca tgaggacata 480
cttccctccc ctccagatgg ctagccacca ataaaggaaa aatacttttg cctgcagcta 540
accaatagaa attacttaaa acccttcatc aaaccttcca cttaggcatt gatagcaccc 600
atgagatggc caaattatta tttactggac caggcctttt caaaactatc aagcagatag 660
tcagggcctg taaagtctgc caaagaaata atcccctgca ctgcaggcca tacatttcaa 720
tccctgtatc tttaacctcc ttcttaaatt tgtctcttcc agaatcaaag ctgtaaaatt 780
acaaatagtt cttcaaatgg agccacagat gcagtccatg actaagatcc accacagacc 840
cctggaccag cctgctagcc catgctccaa tgttaatgac atcgaaggca ccccctcctg 900
aggaaatctc aactgcacaa cccctactac gccccaattc agcagaaagc agttagagtg 960
gtcatcagcc aacctcccc 979

<210> 17
<211> 1774

<212> ADN
<213> Homo sapiens

<400> 17
catgctggta aaggaccgct agaatccagc agccaggacc actttctttg tggtcaagaa 60
aggtgggaaa acaggtgcag gactgctaca ctggtaagca taactaatcc gataagcaga 120
ggtccatggg tggttacgca ccctggaaag gaataagcat taggactata gaggacactc 180
taggactaat gctcatcgga aaatgactag gggtactggc atccctatgt tcttttttca 240
gatgggaaat gttcccccca aggcagaaat gcccctaaga tgtattctgg agaaatggga 300
ccaatctgac catcagacac taagaaagaa atgacttata ttcttctgca gtaccacctg 360
gccacaatat cttcttcaag gggcagaaac ctggcctcct gagggaagta taaattataa 420
caccatctta cagctagacc tcttttgtag aaaagaaggc aaatggagtg aagtgccata 480
tgtacaaact ttcttttcat taagagataa ctcccaatta tgtaaaaagt gtgatttatg 540
ccctacagga agccctcaga gtctacctcc cgaccccagc aagaccccaa ctccttctcc 600
aactaataag gacccccctt caacccaaat ggtccaaaag gagatagaca aaggggtaaa 660
caatgaacca aagagtgcca atattacacg attatactcg ctccaagcag tgggaggaga 720
atttggccca gccagcgtgc atgtaccttt ttctctctca gatttaaagc aaattaaaat 780
agacctaggt aaattctcag ataaccctga tggctatatt gatgttttac aagggttagg 840
acaatccttt gatctgacat ggagagatat aatgttactg ctaaatcaga cactaacccc 900
aaatgaaaaa agtgctgcca taacagcagc ctgagagttt ggcgaactct ggtatctcag 960
tcaggtcaat gataggatga caacagatga aagagaatga ttccccacag gccagcaggc 1020
agttcccagt gtagaccctc attaggacac agaatcagaa cttggagatt ggtgccacag 1080
acatttgcta acttgcgtgc tagaaggact aaggaaaact aggaagaagc ccatgaatta 1140
ttcaatgatg tcccctataa cacagggaaa ggaagaaaat cctactgcct ttctggagag 1200
actaagggaa ggattgagga agcatacctc cctgtcacct gactctatta aaggccaact 1260
aatcttaaag gataagttta tcactcagtc agctgcagag attaagaaaa aacttcaaaa 1320
gtatgcctta ggcccagagc aaaacttaga aaccctactg aacttggcaa cctcagtttt 1380
ttataataga gatcaggaag agcaggggaa tgggacaaat gggataaaaa aaaaaaaaaa 1440
aggtgactgc tttagtcgtg gccctcaggc aaatggactt tggaggctcc agaaaaggga 1500
aaagctgagc aaattgaatg cctaacaggg cttgcttcta gtgtggtcta caaggacact 1560
ttaaaaaaga ttgtccaagt agaaacaagc tgcccccttg tccatgcccc ttatgtcaag 1620
ggaatcactg gaaggcccac tgccccagga gatgaaggtc ctctgagtca gaagccacta 1680
accagataat ccagcagcag gactgaggat gcccagggca agcgccagcc catgccatca 1740
ccctcacaga gccttgggta tgcttgacca ttga 1774

<210> 18
<211> 938
<212> ADN
<213> Homo sapiens

<400> 18
tgtaggaaga actcccttca ggacaggaca atagatggtt cctcccaggt gattaaggaa 60
aaaagacaca gtattcagta agtgataagg aaactcttgt agaagcagag ttagaaaaat 120
tgcctaataa ttggtctgct caaatgtgtg agttgtttgc actcagccaa atcttaaagt 180
acttacagaa tcaggaagca gccatctata ccaattctaa gttaatatgg actaaacgag 240
gttttattag tagcaaagaa aaattaaaat cccaaactta caaggttttc aactaaagtt 300
tgccaaaagt taacagtgta acatgtatta tcctactatc acacactctc aaaggatttc 360
tcagacagtt tgcaagaaat aacgtaatct atccttactc tacagtccca aatagactct 420
ttggtagcag tgactctcca aaactgccga ggtctagacc tcctcaatgc tgagaaagga 480
gaactctgca ccttcttagg ggaagagtgc tgtttttaca ctaaccagtc agggatagta 540
tgagatactg cctgacgttt acaggaaaag gcttctgaaa tcagacaacg cctttcaagc 600
tcttatacca acctctggag ttgggcaaca tggcttctcc ccttgctagg tcctgtggca 660
gccatcttgc tattacttgc cttcgggccc tgtattttta acctccttgt caaatttgtt 720
tcctctagga tcaaggccat caagctacag atggtcttac aaatggaacc ccaaatgagc 780
tcaactaaca acttctactg aggacacctg gactgaccca ctggcccttt cactggccta 840
aagagttccc ttctggagga cactacaact gcagggcccc gtcttcaccc ctatccagca 900
ggaagtagct agatcagtca ttgcccaatt cccaacag 938

<210> 19
<211> 1308
<212> ADN
<213> Homo sapiens

<400> 19
gatgcttgcc ccaggcaccc tcagtcctgt tgttggatca tctggtcggg ggcttctggc 60
ccaaagaacc tttgtcctct gaggcagtgc accttccagt gattgcctca gcattgtgga 120

catgggcaag ggggcagctt gtttctcact ggacaatctt ttttaaggtg tccttccaaa 180
ccacactggt aacaagccct accaggtgat tggcctgctc tattttctgt cctctctgaa 240
ccaccaaggt ttgtctgtct gagggtcatg actaaggctg tggcctttct ctgatcttgc 300
ttttcctttt tggcctgttc ctcttggtac ctattataga acactgaggt tgccaggttt 360
aacaatggct ccagattttg ttcagggcac agggctcatt ttggagcttt ctcctgatat 420
ctgcagctga ttgggtaata aacttatctt ttaggatcaa ttgactctca agagagttgg 480
gtgacagggg agtatatttc cttgaggcct cccatagccg ctctaggaag gcagaaggat 540
tttcttcctt tccctgagtt ataaaagaca tcattgaaca actcatggac tttttcccaa 600
ttctccgtag tccttctaga acacaggtca gcagatgttt acgactccag tccccatgat 660
ctgagtctag acaccagtgg ggatccatac tggggatggc ctgctgactg gtagggaatt 720
tgtccctttc tttggctgtc attctatcat ttacttgact aagataccaa gtatctccaa 780
attctcaggc tgcagctaaa gctgcattct tttcattaaa ggccagggtt tgatctaata 840
gcatgacatc tctccaagtg aggtcaaagg tttgccctag atccatagga catcagagaa 900
ggagaagggg acatacacct gagttagcca aattcccctc cctctacagc ttgaagggga 960
cataagcaat agcctgggga tttttgtggt cctttggaga tttctttgct tgtttccttc 1020
tgggtggggg agattagagg aggcttatca gtaataggaa ggggagctat agggaggcta 1080
ggatatgggg gtaagctgag aggtcatctt gtgggatgta aattgcaagc tttgcatagt 1140
tgtggatttt ccttacaatg aaaataaagc ttggacataa ggtatttcac tccatttgcc 1200
ttccctctta cagaaaaggt caagctgcag gatagtactg taatttatac ttccttcagg 1260
tggccatttc ttcccatcag agagagaata ctggggctgg gccatagt 1308

<210> 20
<211> 711
<212> ADN
<213> Homo sapiens

<400> 20
actgagagac aggactagct ggatttccta ggccgactaa gaatccctaa gcctagctgg 60
gaaggtgacc acgtccacct ttaaacacgg ggcttgcaac ttagctcaca cctgaccaat 120
cagagagctc actaaaatgc taattaggca aagacaggag gtaaagaaat agccaatcat 180
ctattgcctg agagcacagc aggagggaca acaatcggga tataaaccca ggcattcgag 240
ctggcaacag cagcccccct ttgggtccct tccctttgta tgggagctgt tttcatgcta 300
tttcactcta ttaaatcttg caactgcact cttctggtcc atgtttctta cggctcgagc 360
tgagcttttg ctcaccgtcc accactgctg tttgccacca ccgcagacct gccgctgact 420
cccatccctc tggatcctgc agggtgtccg ctgtgctcct gatccagcga ggcgcccatt 480
gccgctccca attgggctaa aggcttgcca ttgttcctgc acggctaagt gcctgggttt 540
gttctaattg agctgaacac tagtcactgg gttccatggt tctcttctgt gacccacggc 600
ttctaataga actataacac ttaccacatg gcccaagatt ccattccttg gaatccgtga 660
ggccaagaac tccaggtcag agaatacgag gcttgccacc atcttggaag c 711

<210> 21
<211> 711
<212> ADN
<213> Homo sapiens

<400> 21
actgagagac aggactagct ggatttccta ggctgactaa gaatccctaa gcctagctgg 60
gaaggtgacc acatccacct ttaaacacgg ggcttgcaac ttagctcaca cctgaccaat 120
cagagagctc actaaaatgc taattaggca aagacaggag gtaaagaaat agccaatcat 180
ctattgcctg agagcacagc aggagggaca atgatcggga tataaaccca agtcttcgag 240
ccggcaacgg caaccccctt tgggtcccct ccctttgtat gggagctctg ttttcatgct 300
atttcactct attaaatctt gcaactgcac tcttctggtc catgtttctt acggcttgag 360
ctgagctttc gctcgccatc caccactgct gtttgccgcc accgcagacc cgccgctgac 420
tcccatccct ctggatcatg cagggtgtcc gctgtgctcc tgatccagcg aggcacccat 480
tgccgctccc aatcgggcta aaggcttgcc attgttcctg catggctaag tgcctgggtt 540
catcctaatt gagctgaaca ctagtcactg ggttccatgg ttctcttctg tgacccacag 600
cttctaatag agctataaca ctcaccgcat ggcccaaggt tccattcctt gaatccataa 660
ggccaagaac cccaggtcag agaacacgag gcttgccacc atcttgggag c 711

<210> 22
<211> 2055
<212> ADN
<213> Homo sapiens

<220>

<221> CDS
<222> (1)..(2055)

<400> 22
ccc aag aca gcc aac tta gtt gca gac atc acc tcc tta gcc aaa tat     48
Pro Lys Thr Ala Asn Leu Val Ala Asp Ile Thr Ser Leu Ala Lys Tyr
1               5                   10                  15

caa caa gtt ctt aaa aca tta caa gga acc tat ccc tga gaa gag gga     96
Gln Gln Val Leu Lys Thr Leu Gln Gly Thr Tyr Pro     Glu Glu Gly
            20                  25                      30

aaa gaa cta ttc cac cct tgt gac atg gta tta gtc aag tcc ctt ccc     144
Lys Glu Leu Phe His Pro Cys Asp Met Val Leu Val Lys Ser Leu Pro
        35                  40                  45

tct aat tcc cca tcc cta gat aca tcc tgg gaa gga ccc tac cca gtc     192
Ser Asn Ser Pro Ser Leu Asp Thr Ser Trp Glu Gly Pro Tyr Pro Val
    50                  55                  60

att tta tct acc cca act gcg gtt aaa gtg gct gga gtg gag tct tgg     240
Ile Leu Ser Thr Pro Thr Ala Val Lys Val Ala Gly Val Glu Ser Trp
65                  70                  75                  80

ata cat cac act tga gtc aaa tcc tgg ata ctg cca aag gaa cct gaa     288
Ile His His Thr     Val Lys Ser Trp Ile Leu Pro Lys Glu Pro Glu
                85                  90                  95

aat cca gga gac aac gct agc tat tcc tgt gaa cct cta gag gat ttg     336
Asn Pro Gly Asp Asn Ala Ser Tyr Ser Cys Glu Pro Leu Glu Asp Leu
            100                 105                 110

cgc ctg ctc ttc aaa caa caa cca gga gga aag taa cta aaa tca taa     384
Arg Leu Leu Phe Lys Gln Gln Pro Gly Gly Lys     Leu Lys Ser
        115                 120                     125

atc ccc atg gcc ctc cct tat cat att ttt ctc ttt act gtt ctt tta     432
Ile Pro Met Ala Leu Pro Tyr His Ile Phe Leu Phe Thr Val Leu Leu
    130                 135                 140

ccc tct ttc act ctc act gca ccc cct cca tgc cgc tgt atg acc agt     480
Pro Ser Phe Thr Leu Thr Ala Pro Pro Pro Cys Arg Cys Met Thr Ser
145                 150                 155                 160

agc tcc cct tac caa gag ttt cta tgg aga atg cag cgt ccc gga aat     528
Ser Ser Pro Tyr Gln Glu Phe Leu Trp Arg Met Gln Arg Pro Gly Asn
                165                 170                 175

att gat gcc cca tcg tat agg agt ctt tct aag gga acc ccc acc ttc     576
Ile Asp Ala Pro Ser Tyr Arg Ser Leu Ser Lys Gly Thr Pro Thr Phe
            180                 185                 190

act gcc cac acc cat atg ccc cgc aac tgc tat cac tct gcc act ctt     624
Thr Ala His Thr His Met Pro Arg Asn Cys Tyr His Ser Ala Thr Leu
        195                 200                 205

tgc atg cat gca aat act cat tat tgg aca gga aaa atg att aat cct     672
Cys Met His Ala Asn Thr His Tyr Trp Thr Gly Lys Met Ile Asn Pro
    210                 215                 220

agt tgt cct gga gga ctt gga gtc act gtc tgt tgg act tac ttc acc     720
Ser Cys Pro Gly Gly Leu Gly Val Thr Val Cys Trp Thr Tyr Phe Thr
225                 230                 235                 240

caa act ggt atg tct gat ggg ggt gga gtt caa gat cag gca aga gaa     768
Gln Thr Gly Met Ser Asp Gly Gly Gly Val Gln Asp Gln Ala Arg Glu
                245                 250                 255

aaa cat gta aaa gaa gta atc tcc caa ctc acc cgg gta cat ggc acc    816
Lys His Val Lys Glu Val Ile Ser Gln Leu Thr Arg Val His Gly Thr
            260                 265                 270

tct agc ccc tac aaa gga cta gat ctc tca aaa cta cat gaa acc ctc    864
Ser Ser Pro Tyr Lys Gly Leu Asp Leu Ser Lys Leu His Glu Thr Leu
        275                 280                 285

cgt acc cat act cgc ctg gta agc cta ttt aat acc acc ctc act ggg    912
Arg Thr His Thr Arg Leu Val Ser Leu Phe Asn Thr Thr Leu Thr Gly
    290                 295                 300

ctc cat gag gtc tcg gcc caa aac cct act aac tgt tgg ata tgc ctc    960
Leu His Glu Val Ser Ala Gln Asn Pro Thr Asn Cys Trp Ile Cys Leu
305                 310                 315                 320

ccc ctg aac ttc agg cca tat gtt tca atc cct gta cct gaa caa tgg    1008
Pro Leu Asn Phe Arg Pro Tyr Val Ser Ile Pro Val Pro Glu Gln Trp
                325                 330                 335

aac aac ttc agc aca gaa ata aac acc act tcc gtt tta gta gga cct    1056
Asn Asn Phe Ser Thr Glu Ile Asn Thr Thr Ser Val Leu Val Gly Pro
            340                 345                 350

ctt gtt tcc aat ctg gaa ata acc cat acc tca aac ctc acc tgt gta    1104
Leu Val Ser Asn Leu Glu Ile Thr His Thr Ser Asn Leu Thr Cys Val
        355                 360                 365

aaa ttt agc aat act aca tac aca acc aac tcc caa tgc atc agg tgg    1152
Lys Phe Ser Asn Thr Thr Tyr Thr Thr Asn Ser Gln Cys Ile Arg Trp
    370                 375                 380

gta act cct ccc aca caa ata gtc tgc cta ccc tca gga ata ttt ttt    1200
Val Thr Pro Pro Thr Gln Ile Val Cys Leu Pro Ser Gly Ile Phe Phe
385                 390                 395                 400

gtc tgt ggt acc tca gcc tat cgt tgt ttg aat ggc tct tca gaa tct    1248
Val Cys Gly Thr Ser Ala Tyr Arg Cys Leu Asn Gly Ser Ser Glu Ser
                405                 410                 415

atg tgc ttc ctc tca ttc tta gtg ccc cct atg acc atc tac act gaa    1296
Met Cys Phe Leu Ser Phe Leu Val Pro Pro Met Thr Ile Tyr Thr Glu
            420                 425                 430

caa gat tta tac agt tat gtc ata tct aag ccc cgc aac aaa aga gta    1344
Gln Asp Leu Tyr Ser Tyr Val Ile Ser Lys Pro Arg Asn Lys Arg Val
        435                 440                 445

ccc att ctt cct ttt gtt ata gga gca gga gtg cta ggt gca cta ggt    1392
Pro Ile Leu Pro Phe Val Ile Gly Ala Gly Val Leu Gly Ala Leu Gly
    450                 455                 460

act ggc att ggc ggt atc aca acc tct act cag ttc tac tac aaa cta    1440
Thr Gly Ile Gly Gly Ile Thr Thr Ser Thr Gln Phe Tyr Tyr Lys Leu
465                 470                 475                 480

tct caa gaa cta aat ggg gac atg gaa cgg gtc gcc gac tcc ctg gtc    1488
Ser Gln Glu Leu Asn Gly Asp Met Glu Arg Val Ala Asp Ser Leu Val
                485                 490                 495

acc ttg caa gat caa ctt aac tcc cta gca gca gta gtc ctt caa aat    1536
Thr Leu Gln Asp Gln Leu Asn Ser Leu Ala Ala Val Val Leu Gln Asn
            500                 505                 510

cga aga gct tta gac ttg cta acc gct gaa aga ggg gga acc tgt tta    1584
Arg Arg Ala Leu Asp Leu Leu Thr Ala Glu Arg Gly Gly Thr Cys Leu
        515                 520                 525

ttt tta ggg gaa gaa tgc tgt tat tat gtt aat caa tcc gga atc gtc     1632
Phe Leu Gly Glu Glu Cys Cys Tyr Tyr Val Asn Gln Ser Gly Ile Val
    530                 535                 540

act gag aaa gtt aaa gaa att cga gat cga ata caa cgt aga gca gag     1680
Thr Glu Lys Val Lys Glu Ile Arg Asp Arg Ile Gln Arg Arg Ala Glu
545                 550                 555                 560

gag ctt cga aac act gga ccc tgg ggc ctc ctc agc caa tgg atg ccc     1728
Glu Leu Arg Asn Thr Gly Pro Trp Gly Leu Leu Ser Gln Trp Met Pro
                565                 570                 575

tgg att ctc ccc ttc tta gga cct cta gca gct ata ata ttg cta ctc     1776
Trp Ile Leu Pro Phe Leu Gly Pro Leu Ala Ala Ile Ile Leu Leu Leu
            580                 585                 590

ctc ttt gga ccc tgt atc ttt aac ctc ctt gtt aac ttt gtc tct tcc     1824
Leu Phe Gly Pro Cys Ile Phe Asn Leu Leu Val Asn Phe Val Ser Ser
        595                 600                 605

aga atc gaa gct gta aaa cta caa atg gag ccc aag atg cag tcc aag     1872
Arg Ile Glu Ala Val Lys Leu Gln Met Glu Pro Lys Met Gln Ser Lys
    610                 615                 620

act aag atc tac cgc aga ccc ctg gac cgg cct gct agc cca cga tct     1920
Thr Lys Ile Tyr Arg Arg Pro Leu Asp Arg Pro Ala Ser Pro Arg Ser
625                 630                 635                 640

gat gtt aat gac atc aaa ggc acc cct cct gag gaa atc tca gct gca     1968
Asp Val Asn Asp Ile Lys Gly Thr Pro Pro Glu Glu Ile Ser Ala Ala
                645                 650                 655

caa cct cta cta cgc ccc aat tca gca gga agc agt tag agc ggt cgt     2016
Gln Pro Leu Leu Arg Pro Asn Ser Ala Gly Ser Ser     Ser Gly Arg
            660                 665                 670

cgg cca acc tcc cca aca gca ctt agg ttt tcc tgt tga                 2055
Arg Pro Thr Ser Pro Thr Ala Leu Arg Phe Ser Cys
        675                 680                 685


<210> 23
<211> 28
<212> PRT
<213> Homo sapiens

<400> 23
Pro Lys Thr Ala Asn Leu Val Ala Asp Ile Thr Ser Leu Ala Lys Tyr
1               5                   10                  15

Gln Gln Val Leu Lys Thr Leu Gln Gly Thr Tyr Pro
            20                  25


<210> 24
<211> 55
<212> PRT
<213> Homo sapiens

<400> 24
Glu Glu Gly Lys Glu Leu Phe His Pro Cys Asp Met Val Leu Val Lys
1               5                   10                  15

Ser Leu Pro Ser Asn Ser Pro Ser Leu Asp Thr Ser Trp Glu Gly Pro
            20                  25                  30

Tyr Pro Val Ile Leu Ser Thr Pro Thr Ala Val Lys Val Ala Gly Val
        35                  40                  45

Glu Ser Trp Ile His His Thr
    50                  55


<210> 25
<211> 38
<212> PRT
<213> Homo sapiens

<400> 25
Val Lys Ser Trp Ile Leu Pro Lys Glu Pro Glu Asn Pro Gly Asp Asn
1               5                   10                  15

Ala Ser Tyr Ser Cys Glu Pro Leu Glu Asp Leu Arg Leu Leu Phe Lys
            20                  25                  30

Gln Gln Pro Gly Gly Lys
        35


<210> 26
<211> 540
<212> PRT
<213> Homo sapiens

<400> 26
Ile Pro Met Ala Leu Pro Tyr His Ile Phe Leu Phe Thr Val Leu Leu
1               5                   10                  15

Pro Ser Phe Thr Leu Thr Ala Pro Pro Pro Cys Arg Cys Met Thr Ser
            20                  25                  30

Ser Ser Pro Tyr Gln Glu Phe Leu Trp Arg Met Gln Arg Pro Gly Asn
        35                  40                  45

Ile Asp Ala Pro Ser Tyr Arg Ser Leu Ser Lys Gly Thr Pro Thr Phe
    50                  55                  60

Thr Ala His Thr His Met Pro Arg Asn Cys Tyr His Ser Ala Thr Leu
65                  70                  75                  80

Cys Met His Ala Asn Thr His Tyr Trp Thr Gly Lys Met Ile Asn Pro
                85                  90                  95

Ser Cys Pro Gly Gly Leu Gly Val Thr Val Cys Trp Thr Tyr Phe Thr
            100                 105                 110

Gln Thr Gly Met Ser Asp Gly Gly Gly Val Gln Asp Gln Ala Arg Glu
        115                 120                 125

Lys His Val Lys Glu Val Ile Ser Gln Leu Thr Arg Val His Gly Thr
    130                 135                 140

Ser Ser Pro Tyr Lys Gly Leu Asp Leu Ser Lys Leu His Glu Thr Leu
145                 150                 155                 160

Arg Thr His Thr Arg Leu Val Ser Leu Phe Asn Thr Thr Leu Thr Gly
                165                 170                 175

Leu His Glu Val Ser Ala Gln Asn Pro Thr Asn Cys Trp Ile Cys Leu
            180                 185                 190

Pro Leu Asn Phe Arg Pro Tyr Val Ser Ile Pro Val Pro Glu Gln Trp
        195                 200                 205

Asn Asn Phe Ser Thr Glu Ile Asn Thr Thr Ser Val Leu Val Gly Pro
    210                 215                 220

Leu Val Ser Asn Leu Glu Ile Thr His Thr Ser Asn Leu Thr Cys Val
225                 230                 235                 240

Lys Phe Ser Asn Thr Thr Tyr Thr Thr Asn Ser Gln Cys Ile Arg Trp
                245                 250                 255

Val Thr Pro Pro Thr Gln Ile Val Cys Leu Pro Ser Gly Ile Phe Phe
            260                 265                 270

Val Cys Gly Thr Ser Ala Tyr Arg Cys Leu Asn Gly Ser Ser Glu Ser
        275                 280                 285

Met Cys Phe Leu Ser Phe Leu Val Pro Pro Met Thr Ile Tyr Thr Glu
    290                 295                 300

Gln Asp Leu Tyr Ser Tyr Val Ile Ser Lys Pro Arg Asn Lys Arg Val
305                 310                 315                 320

Pro Ile Leu Pro Phe Val Ile Gly Ala Gly Val Leu Gly Ala Leu Gly
                325                 330                 335

Thr Gly Ile Gly Gly Ile Thr Thr Ser Thr Gln Phe Tyr Tyr Lys Leu
            340                 345                 350

Ser Gln Glu Leu Asn Gly Asp Met Glu Arg Val Ala Asp Ser Leu Val
        355                 360                 365

Thr Leu Gln Asp Gln Leu Asn Ser Leu Ala Ala Val Val Leu Gln Asn
    370                 375                 380

Arg Arg Ala Leu Asp Leu Leu Thr Ala Glu Arg Gly Gly Thr Cys Leu
385                 390                 395                 400

Phe Leu Gly Glu Glu Cys Cys Tyr Tyr Val Asn Gln Ser Gly Ile Val
                405                 410                 415

Thr Glu Lys Val Lys Glu Ile Arg Asp Arg Ile Gln Arg Arg Ala Glu
            420                 425                 430

Glu Leu Arg Asn Thr Gly Pro Trp Gly Leu Leu Ser Gln Trp Met Pro
        435                 440                 445

Trp Ile Leu Pro Phe Leu Gly Pro Leu Ala Ala Ile Ile Leu Leu Leu
    450                 455                 460

Leu Phe Gly Pro Cys Ile Phe Asn Leu Leu Val Asn Phe Val Ser Ser
465                 470                 475                 480

Arg Ile Glu Ala Val Lys Leu Gln Met Glu Pro Lys Met Gln Ser Lys
                485                 490                 495

Thr Lys Ile Tyr Arg Arg Pro Leu Asp Arg Pro Ala Ser Pro Arg Ser
            500                 505                 510

Asp Val Asn Asp Ile Lys Gly Thr Pro Pro Glu Glu Ile Ser Ala Ala
        515                 520                 525

Gln Pro Leu Leu Arg Pro Asn Ser Ala Gly Ser Ser
    530                 535                 540

<210> 27
<211> 15
<212> PRT
<213> Homo sapiens

<400> 27

Ser Gly Arg Arg Pro Thr Ser Pro Thr Ala Leu Arg Phe Ser Cys
1               5                   10                  15

<210> 28
<211> 1080
<212> ADN
<213> Homo sapiens

<220>
<221> CDS
<222> (1)..(1080)

<400> 28
acc tct ttt gta gaa aag gca aat gga gtg aag tgc cat aag tac aaa     48
Thr Ser Phe Val Glu Lys Ala Asn Gly Val Lys Cys His Lys Tyr Lys
1               5                   10                  15

ctt tct ttt cat taa gag aca act cac aat tat gta aaa agt gtg att     96
Leu Ser Phe His     Glu Thr Thr His Asn Tyr Val Lys Ser Val Ile
            20                  25                  30

tat gcc cta cag gaa gcc ttc aga gtc tac ctc cct atc cca gca tcc     144
Tyr Ala Leu Gln Glu Ala Phe Arg Val Tyr Leu Pro Ile Pro Ala Ser
        35                  40                  45

ccg act cct tcc cca act aat aag gac ccc cct tca acc caa atg gtc     192
Pro Thr Pro Ser Pro Thr Asn Lys Asp Pro Pro Ser Thr Gln Met Val
    50                  55                  60

caa aag gag ata gac aaa agg gta aac agt gaa cca aag agt gcc aat     240
Gln Lys Glu Ile Asp Lys Arg Val Asn Ser Glu Pro Lys Ser Ala Asn
65                  70                  75                  80

att ccc caa tta tga ccc ctc caa gca gtg gga gga aga gaa ttc ggc     288
Ile Pro Gln Leu     Pro Leu Gln Ala Val Gly Gly Arg Glu Phe Gly
                85                  90                  95

cca gcc aga gtg cat gtg cct ttt tct ctc cca gac tta aag caa ata     336
Pro Ala Arg Val His Val Pro Phe Ser Leu Pro Asp Leu Lys Gln Ile
            100                 105                 110

aaa aca gac tta ggt aaa ttc tca gat aac cct gat ggc tat att gat     384
Lys Thr Asp Leu Gly Lys Phe Ser Asp Asn Pro Asp Gly Tyr Ile Asp
        115                 120                 125

gtt tta caa ggg tta gga caa ttc ttt gat ctg aca tgg aga gat ata     432
Val Leu Gln Gly Leu Gly Gln Phe Phe Asp Leu Thr Trp Arg Asp Ile
    130                 135                 140

atg tca ctg cta aat cag aca cta acc cca aat gag aga agt gcc acc     480
Met Ser Leu Leu Asn Gln Thr Leu Thr Pro Asn Glu Arg Ser Ala Thr
145                 150                 155                 160

ata act gca gcc tga gag ttt ggc gat ctc tgg tat ctc agt cag gtc     528
Ile Thr Ala Ala     Glu Phe Gly Asp Leu Trp Tyr Leu Ser Gln Val
                165                 170                 175

aat gat agg atg aca aca gag gaa aga gaa tga ttc ccc aca ggc cag     576
Asn Asp Arg Met Thr Thr Glu Glu Arg Glu     Phe Pro Thr Gly Gln
            180                 185                 190

cag gca gtt ccc agt cta gac cct cat tgg gac aca gaa tca gaa cat     624
Gln Ala Val Pro Ser Leu Asp Pro His Trp Asp Thr Glu Ser Glu His
        195                 200                 205

gga gat tgg tgc tgc aga cat ttg cta act tgt gtg cta gaa gga cta     672

Gly Asp Trp Cys Cys Arg His Leu Leu Thr Cys Val Leu Glu Gly Leu
    210                 215                 220

agg aaa act agg aag aag tct atg aat tac tca atg atg tcc acc ata     720
Arg Lys Thr Arg Lys Lys Ser Met Asn Tyr Ser Met Met Ser Thr Ile
225                 230                 235                 240

aca cag gga agg gaa gaa aat cct act gcc ttt ctg gag aga cta agg     768
Thr Gln Gly Arg Glu Glu Asn Pro Thr Ala Phe Leu Glu Arg Leu Arg
                245                 250                 255

gag gca ttg agg aag cgt gcc tct ctg tca cct gac tct tct gaa ggc     816
Glu Ala Leu Arg Lys Arg Ala Ser Leu Ser Pro Asp Ser Ser Glu Gly
            260                 265                 270

caa cta atc tta aag cgt aag ttt atc act cag tca gct gca gac att     864
Gln Leu Ile Leu Lys Arg Lys Phe Ile Thr Gln Ser Ala Ala Asp Ile
        275                 280                 285

aga aaa aaa ctt caa aag tct gcc gta ggc ccg gag caa aac tta gaa     912
Arg Lys Lys Leu Gln Lys Ser Ala Val Gly Pro Glu Gln Asn Leu Glu
    290                 295                 300

acc cta ttg aac ttg gca acc tcg gtt ttt tat aat aga gat cag gag     960
Thr Leu Leu Asn Leu Ala Thr Ser Val Phe Tyr Asn Arg Asp Gln Glu
305                 310                 315                 320

gag cag gcg gaa cag gac aaa cgg gat taa aaa aaa ggc cac cgc ttt     1008
Glu Gln Ala Glu Gln Asp Lys Arg Asp     Lys Lys Gly His Arg Phe
                325                 330                 335

agt cat gac cct cag gca agt gga ctt tgg agg ctc tgg aaa agg gaa     1056
Ser His Asp Pro Gln Ala Ser Gly Leu Trp Arg Leu Trp Lys Arg Glu
            340                 345                 350

aag ctg ggc aaa ttg aat gcc taa                                     1080
Lys Leu Gly Lys Leu Asn Ala
        355                 360


<210> 29
<211> 20
<212> PRT
<213> Homo sapiens

<400> 29
Thr Ser Phe Val Glu Lys Ala Asn Gly Val Lys Cys His Lys Tyr Lys
1               5                   10                  15

Leu Ser Phe His
            20


<210> 30
<211> 63
<212> PRT
<213> Homo sapiens

<400> 30
Glu Thr Thr His Asn Tyr Val Lys Ser Val Ile Tyr Ala Leu Gln Glu
1               5                   10                  15

Ala Phe Arg Val Tyr Leu Pro Ile Pro Ala Ser Pro Thr Pro Ser Pro
            20                  25                  30

Thr Asn Lys Asp Pro Pro Ser Thr Gln Met Val Gln Lys Glu Ile Asp
        35                  40                  45

Lys Arg Val Asn Ser Glu Pro Lys Ser Ala Asn Ile Pro Gln Leu
50                  55                  60

<210> 31
<211> 79
<212> PRT
<213> Homo sapiens

<400> 31
Pro Leu Gln Ala Val Gly Gly Arg Glu Phe Gly Pro Ala Arg Val His
1               5                   10                  15

Val Pro Phe Ser Leu Pro Asp Leu Lys Gln Ile Lys Thr Asp Leu Gly
            20                  25                  30

Lys Phe Ser Asp Asn Pro Asp Gly Tyr Ile Asp Val Leu Gln Gly Leu
        35                  40                  45

Gly Gln Phe Phe Asp Leu Thr Trp Arg Asp Ile Met Ser Leu Leu Asn
    50                  55                  60

Gln Thr Leu Thr Pro Asn Glu Arg Ser Ala Thr Ile Thr Ala Ala
65                  70                  75

<210> 32
<211> 21
<212> PRT
<213> Homo sapiens

<400> 32
Glu Phe Gly Asp Leu Trp Tyr Leu Ser Gln Val Asn Asp Arg Met Thr
1               5                   10                  15

Thr Glu Glu Arg Glu
            20

<210> 33
<211> 142
<212> PRT
<213> Homo sapiens

<400> 33
Phe Pro Thr Gly Gln Gln Ala Val Pro Ser Leu Asp Pro His Trp Asp
1               5                   10                  15

Thr Glu Ser Glu His Gly Asp Trp Cys Cys Arg His Leu Leu Thr Cys
            20                  25                  30

Val Leu Glu Gly Leu Arg Lys Thr Arg Lys Lys Ser Met Asn Tyr Ser
        35                  40                  45

Met Met Ser Thr Ile Thr Gln Gly Arg Glu Glu Asn Pro Thr Ala Phe
    50                  55                  60

Leu Glu Arg Leu Arg Glu Ala Leu Arg Lys Arg Ala Ser Leu Ser Pro
65                  70                  75                  80

Asp Ser Ser Glu Gly Gln Leu Ile Leu Lys Arg Lys Phe Ile Thr Gln
                85                  90                  95

Ser Ala Ala Asp Ile Arg Lys Lys Leu Gln Lys Ser Ala Val Gly Pro
            100                 105                 110

Glu Gln Asn Leu Glu Thr Leu Leu Asn Leu Ala Thr Ser Val Phe Tyr
        115                 120                 125

Asn Arg Asp Gln Glu Glu Gln Ala Glu Gln Asp Lys Arg Asp
    130                 135                 140

<210> 34
<211> 29
<212> PRT
<213> Homo sapiens

<400> 34
Lys Lys Gly His Arg Phe Ser His Asp Pro Gln Ala Ser Gly Leu Trp
1               5                   10                  15

Arg Leu Trp Lys Arg Glu Lys Leu Gly Lys Leu Asn Ala
            20                  25

<210> 35
<211> 685
<212> PRT
<213> Homo sapiens

<400> 35
Pro Lys Thr Ala Asn Leu Val Ala Asp Ile Thr Ser Leu Ala Lys Tyr
1               5                   10                  15

Gln Gln Val Leu Lys Thr Leu Gln Gly Thr Tyr Pro Xaa Glu Glu Gly
            20                  25                  30

Lys Glu Leu Phe His Pro Cys Asp Met Val Leu Val Lys Ser Leu Pro
        35                  40                  45

Ser Asn Ser Pro Ser Leu Asp Thr Ser Trp Glu Gly Pro Tyr Pro Val
    50                  55                  60

Ile Leu Ser Thr Pro Thr Ala Val Lys Val Ala Gly Val Glu Ser Trp
65                  70                  75                  80

Ile His His Thr Xaa Val Lys Ser Trp Ile Leu Pro Lys Glu Pro Glu
                85                  90                  95

Asn Pro Gly Asp Asn Ala Ser Tyr Ser Cys Glu Pro Leu Glu Asp Leu
            100                 105                 110

Arg Leu Leu Phe Lys Gln Gln Pro Gly Gly Lys Xaa Leu Lys Ser Xaa
        115                 120                 125

Ile Pro Met Ala Leu Pro Tyr His Ile Phe Leu Phe Thr Val Leu Leu
    130                 135                 140

Pro Ser Phe Thr Leu Thr Ala Pro Pro Pro Cys Arg Cys Met Thr Ser
145                 150                 155                 160

Ser Ser Pro Tyr Gln Glu Phe Leu Trp Arg Met Gln Arg Pro Gly Asn
                165                 170                 175

Ile Asp Ala Pro Ser Tyr Arg Ser Leu Ser Lys Gly Thr Pro Thr Phe
            180                 185                 190

Thr Ala His Thr His Met Pro Arg Asn Cys Tyr His Ser Ala Thr Leu
        195                 200                 205

Cys Met His Ala Asn Thr His Tyr Trp Thr Gly Lys Met Ile Asn Pro
    210                 215                 220

Ser Cys Pro Gly Gly Leu Gly Val Thr Val Cys Trp Thr Tyr Phe Thr

225                 230                 235                 240

Gln Thr Gly Met Ser Asp Gly Gly Gly Val Gln Asp Gln Ala Arg Glu
                245                 250                 255

Lys His Val Lys Glu Val Ile Ser Gln Leu Thr Arg Val His Gly Thr
            260                 265                 270

Ser Ser Pro Tyr Lys Gly Leu Asp Leu Ser Lys Leu His Glu Thr Leu
        275                 280                 285

Arg Thr His Thr Arg Leu Val Ser Leu Phe Asn Thr Thr Leu Thr Gly
    290                 295                 300

Leu His Glu Val Ser Ala Gln Asn Pro Thr Asn Cys Trp Ile Cys Leu
305                 310                 315                 320

Pro Leu Asn Phe Arg Pro Tyr Val Ser Ile Pro Val Pro Glu Gln Trp
                325                 330                 335

Asn Asn Phe Ser Thr Glu Ile Asn Thr Thr Ser Val Leu Val Gly Pro
            340                 345                 350

Leu Val Ser Asn Leu Glu Ile Thr His Thr Ser Asn Leu Thr Cys Val
        355                 360                 365

Lys Phe Ser Asn Thr Thr Tyr Thr Thr Asn Ser Gln Cys Ile Arg Trp
    370                 375                 380

Val Thr Pro Pro Thr Gln Ile Val Cys Leu Pro Ser Gly Ile Phe Phe
385                 390                 395                 400

Val Cys Gly Thr Ser Ala Tyr Arg Cys Leu Asn Gly Ser Ser Glu Ser
                405                 410                 415

Met Cys Phe Leu Ser Phe Leu Val Pro Pro Met Thr Ile Tyr Thr Glu
            420                 425                 430

Gln Asp Leu Tyr Ser Tyr Val Ile Ser Lys Pro Arg Asn Lys Arg Val
        435                 440                 445

Pro Ile Leu Pro Phe Val Ile Gly Ala Gly Val Leu Gly Ala Leu Gly
    450                 455                 460

Thr Gly Ile Gly Gly Ile Thr Thr Ser Thr Gln Phe Tyr Tyr Lys Leu
465                 470                 475                 480

Ser Gln Glu Leu Asn Gly Asp Met Glu Arg Val Ala Asp Ser Leu Val
                485                 490                 495

Thr Leu Gln Asp Gln Leu Asn Ser Leu Ala Ala Val Val Leu Gln Asn
            500                 505                 510

Arg Arg Ala Leu Asp Leu Leu Thr Ala Glu Arg Gly Gly Thr Cys Leu
        515                 520                 525

Phe Leu Gly Glu Glu Cys Cys Tyr Tyr Val Asn Gln Ser Gly Ile Val
    530                 535                 540

Thr Glu Lys Val Lys Glu Ile Arg Asp Arg Ile Gln Arg Arg Ala Glu
545                 550                 555                 560

Glu Leu Arg Asn Thr Gly Pro Trp Gly Leu Leu Ser Gln Trp Met Pro
                565                 570                 575

Trp Ile Leu Pro Phe Leu Gly Pro Leu Ala Ala Ile Ile Leu Leu Leu
            580                 585                 590

Leu Phe Gly Pro Cys Ile Phe Asn Leu Leu Val Asn Phe Val Ser Ser
        595                 600                 605

Arg Ile Glu Ala Val Lys Leu Gln Met Glu Pro Lys Met Gln Ser Lys
    610                 615                 620

Thr Lys Ile Tyr Arg Arg Pro Leu Asp Arg Pro Ala Ser Pro Arg Ser
625                 630                 635                 640

Asp Val Asn Asp Ile Lys Gly Thr Pro Pro Glu Glu Ile Ser Ala Ala
                645                 650                 655

Gln Pro Leu Leu Arg Pro Asn Ser Ala Gly Ser Ser Xaa Ser Gly Arg
            660                 665                 670

Arg Pro Thr Ser Pro Thr Ala Leu Arg Phe Ser Cys Xaa
        675                 680                 685

<210> 36
<211> 360
<212> PRT
<213> Homo sapiens

<400> 36
Thr Ser Phe Val Glu Lys Ala Asn Gly Val Lys Cys His Lys Tyr Lys
1               5                   10                  15

Leu Ser Phe His Xaa Glu Thr Thr His Asn Tyr Val Lys Ser Val Ile
            20                  25                  30

Tyr Ala Leu Gln Glu Ala Phe Arg Val Tyr Leu Pro Ile Pro Ala Ser
        35                  40                  45

Pro Thr Pro Ser Pro Thr Asn Lys Asp Pro Pro Ser Thr Gln Met Val
    50                  55                  60

Gln Lys Glu Ile Asp Lys Arg Val Asn Ser Glu Pro Lys Ser Ala Asn
65                  70                  75                  80

Ile Pro Gln Leu Xaa Pro Leu Gln Ala Val Gly Gly Arg Glu Phe Gly
                85                  90                  95

Pro Ala Arg Val His Val Pro Phe Ser Leu Pro Asp Leu Lys Gln Ile
            100                 105                 110

Lys Thr Asp Leu Gly Lys Phe Ser Asp Asn Pro Asp Gly Tyr Ile Asp
        115                 120                 125

Val Leu Gln Gly Leu Gly Gln Phe Phe Asp Leu Thr Trp Arg Asp Ile
    130                 135                 140

Met Ser Leu Leu Asn Gln Thr Leu Thr Pro Asn Glu Arg Ser Ala Thr
145                 150                 155                 160

Ile Thr Ala Ala Xaa Glu Phe Gly Asp Leu Trp Tyr Leu Ser Gln Val
                165                 170                 175

Asn Asp Arg Met Thr Thr Glu Glu Arg Glu Xaa Phe Pro Thr Gly Gln
            180                 185                 190

Gln Ala Val Pro Ser Leu Asp Pro His Trp Asp Thr Glu Ser Glu His
        195                 200                 205

Gly Asp Trp Cys Cys Arg His Leu Leu Thr Cys Val Leu Glu Gly Leu
    210                 215                 220

Arg Lys Thr Arg Lys Lys Ser Met Asn Tyr Ser Met Met Ser Thr Ile
225                 230                 235                 240

Thr Gln Gly Arg Glu Glu Asn Pro Thr Ala Phe Leu Glu Arg Leu Arg
                245                 250                 255

Glu Ala Leu Arg Lys Arg Ala Ser Leu Ser Pro Asp Ser Ser Glu Gly
            260                 265                 270

Gln Leu Ile Leu Lys Arg Lys Phe Ile Thr Gln Ser Ala Ala Asp Ile
        275                 280                 285

Arg Lys Lys Leu Gln Lys Ser Ala Val Gly Pro Glu Gln Asn Leu Glu
    290                 295                 300

Thr Leu Leu Asn Leu Ala Thr Ser Val Phe Tyr Asn Arg Asp Gln Glu
305                 310                 315                 320

Glu Gln Ala Glu Gln Asp Lys Arg Asp Xaa Lys Lys Gly His Arg Phe
                325                 330                 335

Ser His Asp Pro Gln Ala Ser Gly Leu Trp Arg Leu Trp Lys Arg Glu
            340                 345                 350

Lys Leu Gly Lys Leu Asn Ala Xaa
        355                 360


<210> 37
<211> 26
<212> ADN
<213> Homo sapiens

<400> 37
ggaccataga ggacactcca ggacta                                          26


<210> 38
<211> 25
<212> ADN
<213> Homo sapiens

<400> 38
cctcagtcct gctgctggat catct                                           25


<210> 39
<211> 27
<212> ADN
<213> Homo sapiens

<400> 39
cctccaagca gtgggaggaa gagaatt                                         27


<210> 40
<211> 28
<212> ADN
<213> Homo sapiens

<400> 40
ccttccctgt gttattgtgg acatcatt                                        28


<210> 41
<211> 30
<212> ADN

<213> Homo sapiens

<400> 41
ggaagaagtc tatgaattat tcaatgatgt 30

<210> 42
<211> 27
<212> ADN
<213> Homo sapiens

<400> 42
gggacacaga atcagaacat ggagatt 27

<210> 43
<211> 27
<212> ADN
<213> Homo sapiens

<400> 43
gccttcagaa gagtcaggtg acagaga 27

<210> 44
<211> 25
<212> ADN
<213> Homo sapiens

<400> 44
gagcctccaa agtccacttg cctga 25

<210> 45
<211> 29
<212> ADN
<213> Homo sapiens

<400> 45
gatttcagta tctactagtc tgggtagat 29

<210> 46
<211> 27
<212> ADN
<213> Homo sapiens

<400> 46
ctaggaaatc cagctagtcc tgtctca 27

<210> 47
<211> 28
<212> ADN
<213> Homo sapiens

<400> 47
ccaagacagc caacttagtt gcagacat 28

<210> 48
<211> 28
<212> ADN
<213> Homo sapiens

<400> 48
ggacgctgca ttctccatag aaactctt 28

<210> 49
<211> 29
<212> ADN
<213> Homo sapiens

<400> 49
gcaatactac atacacaacc aactcccaa                                          29


<210> 50
<211> 26
<212> ADN
<213> Homo sapiens

<400> 50
gggggaggca tatccaacag ttagta                                             26


<210> 51
<211> 30
<212> ADN
<213> Homo sapiens

<400> 51
ccatctacac tgaacaagat ttatacactt                                         30


<210> 52
<211> 28
<212> ADN
<213> Homo sapiens

<400> 52
aatgccagta cctagtgcac ctagcact                                           28


<210> 53
<211> 31
<212> ADN
<213> Homo sapiens

<400> 53
cgaatacaac gtagagcaga ggagcttcga a                                       31


<210> 54
<211> 28
<212> ADN
<213> Homo sapiens

<400> 54
agcccaagat gcagtccaag actaagat                                           28


<210> 55
<211> 27
<212> ADN
<213> Homo sapiens

<400> 55
gcgtagtaga ggttgtgcag ctgagat                                            27


<210> 56
<211> 27
<212> ADN
<213> Homo sapiens

<400> 56
cccttaccaa gagtttctat ggagaat                                          27


<210> 57
<211> 27
<212> ADN
<213> Homo sapiens

<400> 57
accgctctaa ctgcttcctg ctgaatt                                          27


<210> 58
<211> 420
<212> PRT
<213> Homo sapiens

<400> 58
Thr Ser Phe Val Glu Lys Ala Asn Gly Val Lys Cys His Lys Tyr Lys
1               5                   10                  15

Leu Ser Phe His Xaa Glu Thr Thr His Asn Tyr Val Lys Ser Val Ile
            20                  25                  30

Tyr Ala Leu Gln Glu Ala Phe Arg Val Tyr Leu Pro Ile Leu Pro Ala
        35                  40                  45

Ser Pro Thr Pro Ser Pro Thr Asn Lys Asp Pro Pro Ser Thr Gln Met
    50                  55                  60

Val Gln Lys Glu Ile Asp Lys Arg Val Asn Ser Glu Pro Lys Ser Ala
65                  70                  75                  80

Asn Ile Pro Gln Leu Xaa Pro Leu Gln Ala Val Gly Gly Arg Glu Phe
                85                  90                  95

Gly Pro Ala Arg Val His Val Pro Phe Ser Leu Pro Asp Leu Lys Gln
            100                 105                 110

Ile Lys Thr Asp Leu Gly Lys Phe Ser Asp Asn Pro Asp Gly Tyr Ile
        115                 120                 125

Asp Val Leu Gln Gly Leu Gly Gln Phe Phe Asp Leu Thr Trp Arg Asp
    130                 135                 140

Ile Met Ser Leu Leu Asn Gln Thr Leu Thr Pro Asn Glu Arg Ser Ala
145                 150                 155                 160

Thr Ile Thr Ala Ala Xaa Glu Phe Gly Asp Leu Trp Tyr Leu Ser Gln
                165                 170                 175

Val Asn Asp Arg Met Thr Thr Glu Glu Arg Glu Xaa Phe Pro Thr Gly
            180                 185                 190

Gln Gln Ala Val Pro Ser Leu Asp Pro His Trp Asp Thr Glu Ser Glu
        195                 200                 205

His Gly Asp Trp Cys Cys Arg His Leu Leu Thr Cys Val Leu Glu Gly
    210                 215                 220

Leu Arg Lys Thr Arg Lys Lys Ser Met Asn Tyr Ser Met Met Ser Thr
225                 230                 235                 240

Ile Thr Gln Gly Arg Glu Glu Asn Pro Thr Ala Phe Leu Glu Arg Leu
                245                 250                 255

Arg Glu Ala Leu Arg Lys Arg Ala Ser Leu Ser Pro Asp Ser Ser Glu
            260                 265                 270

Gly Gln Leu Ile Leu Lys Arg Lys Phe Ile Thr Gln Ser Ala Ala Asp
        275                 280                 285

Ile Arg Lys Lys Leu Gln Lys Ser Ala Val Gly Pro Glu Gln Asn Leu
    290                 295                 300

Glu Thr Leu Leu Asn Leu Ala Thr Ser Val Phe Tyr Asn Arg Asp Gln
305                 310                 315                 320

Glu Glu Gln Ala Glu Gln Asp Lys Arg Asp Xaa Lys Lys Gly His Arg
                325                 330                 335

Phe Ser His Asp Pro Gln Ala Ser Gly Leu Trp Arg Leu Trp Lys Arg
            340                 345                 350

Glu Lys Leu Gly Lys Leu Asn Ala Xaa Xaa Gly Leu Leu Pro Val Arg
        355                 360                 365

Ser Thr Arg Thr Leu Xaa Lys Arg Leu Ser Lys Xaa Lys Xaa Ala Ala
    370                 375                 380

Pro Ser Ser Met Pro Leu Ile Ser Arg Glu Ser Leu Glu Gly Pro Leu
385                 390                 395                 400

Pro Gln Gly Thr Lys Val Leu Xaa Val Arg Ser His Xaa Pro Asp Ser
                405                 410                 415

Ser Ser Arg Thr
            420

<210> 59
<211> 32
<212> ADN
<213> Homo sapiens

<400> 59
taaactacaa atggttcttc aaatggagcc ca 32

<210> 60
<211> 32
<212> ADN
<213> Homo sapiens

<400> 60
gatgcagtcc aagatgcagt ccatgactaa ga 32

<210> 61
<211> 1740
<212> ADN
<213> Homo sapiens

<400> 61
   aggttggctg acaaccgctc ttaactgctt catgctgaat tggggcatag taggggtcgt 60
   gcagttgaga tttccttggg aggggtgcct tcaatgtcat caacattgga gcatgggcta 120
   gcaggccagt ccaggggtcc gcggtagatc ttagtcatgg actgcatctg gggctccatt 180
   tgaagaacca tttgtagttt tacagcttcg attctggaag agacaaacgt aacaaggagg 240
   ttaaagatac aaggattgaa atgtacggcc tgaagtgcag gggcatatga gtgtgggcgg 300
   tgcaagtggg gtttccttta gaaaaactcc gatacaatag ggcatcaata tttctaggaa 360
   gccacattct ccatagaagc tctcggtaag gggagctact ggtagtacag cagcatacag 420
   ggggtgcagt gagagtgaaa gggggtaaga gaacagtaaa aagaaaaata tgacaaggga 480
   gggccaagag gatctacgat tctagttact ttcctcacgg ttgtcgcctg aagagcaggc 540
   gcagatcctc tagaggttca caggaatagc tagcattgtc tgctggattt tcgggttcct 600
   ttggcagtat ccagggtttg gctcgagtgt gacttatcca agactccact ccagccactt 660
   aactgcggtt agggtagata aaatgactgg gtagggtcct tcccaggatg tgtgtaggga 720
   tggggaatta aaggggaagg gacttgacta ataccatgtc accagggtgg aataattcct 780
   ttccctcctc tcagggacag gttccctgta atgttttaag aactcgttga tatttggcta 840
   aggaggtgat gtctgcaact aagttggccg tctctcagtc aagcacaagg tcattggtta 900
   ggaagggctg tccatacagc atctcatatg gactaagtcc tgctttttgg ggacagtttc 960
   ggattcttag taaggctata ggcaacagag caggccatgc aaggtgggtt tcttgggtta 1020
   gcttttttag atgtcgtttg agtgtttcat tcattttctc aacttttcct gaggatcgtg 1080
   gcctccaggc acagtgtaag tgatattgta tacctaacgc ctgggatact ccctgcgtta 1140
   ctgcagcctt gaaattgggg ccattgtcac tctgtaaacc tcagggaagt ccgaatctgg 1200
   gaattatttc atgaattagt acttttatta cctcttgggc cttttctgtc ctacaaggga 1260
   aggcctccac ccaaccagtg aaagtaccca gattagtaga tactgaaatc tctgagattt 1320
   gggcatgtgg gtaaaatcta gttgctagtc ttctcctggg taatggcctg ttctttgttc 1380
   tcctgaagga gcttggcaat aaggcagggg attatttctt tggcacactt cacaggccct 1440
   gactatctgc ttgacagttt tgaaaaggcc tggtccagta aataatgatt tggccatctg 1500
   atgggtgctg tcaatgccta agtgaaaggt ctggtgaagg gttttaagta atttccattg 1560
   gttagctgca ggcaaaagta tttttctttt ggtggctggc catcctgagg agaggaaact 1620
   atgtcctcgt gagtttcccc attccatttc ttctgctgag tactggagct tggtttccca 1680
   gaggggatta ccccatacta ggggtccttc tgtaagcatt tctaatggag agtcctgcct 1740

<210> 62
<211> 7140
<212> ADN
<213> Homo sapiens

<400> 62
ttggtcttaa gaacacaaat gatatggctc caatgactgg aggaacacca gggtccttgg 60
tctcacgctg atttagataa aacgactgtc aggcctctga gcccaagcta agccatcctc 120
ccctgtgacc tgcacgtata catccagatg gcctgaagta accaaagaat cacaaaagca 180
gtgaaaatgg cctgttcctg ccttaactga tgacattcca ccattgtgat ttgttcctgc 240
cccatcttaa ctgagcgatt aaccttgtga aattccttct cctggctcaa aacctccccc 300
actgagcacc ttgtgacccc cgcccctgcc cctaagagaa aacccccttt gattataatt 360
ttccactacc cacccaaatc ctataaaatg gccccacccc tatctccctt cgctgactcc 420
tttttcggac tcagcccgcc tgcacccagg tgaaataaac agccttgttg ctcacacaaa 480
gcctgtttgg tggactctct tcacacggac gctcatgaca tttggtgcca aaacctggga 540
taggaggact ccttcaggag accagtcccc tgtccttgcc ctcactctgt gaggacatcc 600
acctacaacc ttgggtcctc agaccaacca gcccaaggaa cagctcacca atttcaaatc 660
aggtaagcag tcttttcact ctcttctcca gcctctcttg ctacccttca aactccctct 720
ctcactaccc ttcaatctcc ctgtccttcc aattccagtt ctttttcatc tctagtagag 780
acaaaggaga cacattttat ccatggaccc aaaactccag caccagtcac ggacttggga 840
agacagtctt cccttggtgt ttaatcactg cggggacgcc tgcctgatta ttcacccaca 900
ctccattggt gtctgatcac ggtggggaca cctgccttgg tcactcaccc acattccctt 960
ggtggtacgt caactgcaaa agcaggggac gcctgctttg gctgctcacc cacccccttc 1020
tctgtgtctc tacctttctc tttaaactta cctccttcac tatgggcaaa cttctgccct 1080
ccattccccc ttcttctccc ttagcctgtg ttcttaaaaa cctaaaacct cttcaactca 1140
cacctgacct aaaacctaaa tgccttattt tcttctgcaa cactgcgtgg ctgcagtaca 1200
aacttgataa tagctttaaa tggccagaat atggcacttt caatttctcc atcctacaag 1260
atctagataa tttttgtgga aaaatggaaa aatggtctga gatgcctgac gtccaggcat 1320
tcttttacac attggtccct ccctagtctc tgctcccaat gcgactcatc ccaaatcttt 1380
cttctttctc tcctgtctgt tccttcagtc tccaccccaa gctctgagtc ctttgaatcc 1440
tcctttgcta cagacccatc tgaactctcc cctcctcccc aggctgctcc tcaccaggcc 1500
gagccaggtc ccaattcttc ctcagcctct gctcccccac cctataatcc ttttatcacc 1560
tcctctcctc acactcagtc cggcttacag tttcgttctg tgactagccc tcccccatct 1620
gcccaacaat ttcctcttaa agaggtggct ggagctaaag gcatagtcaa ggttaatgct 1680
cctttttctt tatctgacct ctcccaaatc agttagcgtt tacgctcttt ttcatcaaat 1740
ataaaaaccc agccagttca tggcccatct ggcaacaacc cttacaggct ttacagccct 1800
agaccctgaa gggtcagaag gccgtcttat tctcaatatg cattttatta cccaatccgc 1860
tcccaacatt aaataaagct ccaaaaatta aattctggcc ctcaaacccc acaacaggac 1920
ttaattaacc tcacttcaag gtgtacaaga atagagtaga ggcagccaag tagcaacgta 1980
tttgagttgc aattccttgc ctcaactctg agagaaaccc cagccacatc tccagcaaac 2040
aagaacttca aaacacctga actgcagcag ccaggcgttc ctccaggacc acctccccca 2100
ggatcttgct tcaagtgccg gaaatctgac cattgggcca aggaatgcct gcagcccagg 2160
attcctccta agccacgtcc catttgtgca ggaccccact ggaaatcgga ctgtccaact 2220
cacccggcag ccaatcccag agcccctgga actctggccc aaggctctct gactgactcc 2280
ttcccagatc ttctcggctt agcagctgaa gactgacact gcccgatcac ttcagaagtc 2340
ccctggacca tcacggatac tgagcttcag gtaactctca cagtggaggc taagtccatc 2400

ccctgtttaa tcgatacagg ggctacccac tccacatcac cttcttttca agggcctgtt 2460
tccctttccc ccataactgt tgtgggtatt gacggccaag cttcaaaacc ccttaaaact 2520
cccccactct ggtgccaact tggacaacat tcttttatgc actctttttc agttatcctc 2580
acctgcccag ttcccttatt aggccgagac attttaacca aattatctgc ttccccgact 2640
attcctgggc tacagccaca tctccttgcc gcccttcttc ccaacccaaa gcctccttca 2700
tatcttcctc tcatatcccc ccaccttaac ccacaagtat gggacacctc tactccctcc 2760
ctggcaaccg atcacacgcc cattactatc ccattaaaac ctaatcaccc ttaccctgct 2820
caatgccagt atcccatacc acaacaggct ttaaagggat tgaagcctgt tatcacttgc 2880
ctgctacagc acgggcttct aaaacctata aactctccat acaattcccc catttacct 2940
gtctaaaaac cagataagtc ttacaggtta gttcagaatc tgcaccttat caaccaaatt 3000
gttttgccta tccaccctgt agcacccaac tcgtacactc ttttgtcctc aatgccttcc 3060
cccacaactc actattccgt tcttgatctt aaagatgctt ttttcactat tcccctgcac 3120
ccctcatccc agcctctctt tgcttttacc tggactgacc ctgacaccca tcagtcccag 3180
cagcttacct gggctgtact gccgcaaggc ttcagggaca gccctcatta cttcagccaa 3240
gctctttctc atgatttact ttctttccac ctctctgctt ctcaccttat tcaatatatt 3300
gatgaccttc tactttgtag cccctccttt aaatcttctc aacaagacac cctcctgctc 3360
cttcaacatt tgttctccaa aggatatcgg gtatccccct ccaaagctca aatttcttct 3420
ccatctgtta catacctcgg cataattctt catgaaaaca catgtgctct ccctgccaat 3480
tgcgtctcca actgatctct caaatcccaa cctcttctac aaaacaacaa ctcctttccc 3540
tcctaggcat ggttggatac ttttgccttt ggatacctgg ttttgccatc ctaacaaaat 3600
cattatataa actcacaaaa ggaaacctag ctgaccccat agattctaaa tcctttcccc 3660
actcctcttt ccattccttg aagacagctt tagagactgc tcccacacta gctctccctg 3720
tctcatccca acccttttca ttacacacag ccgaagtgca gggctgtgca gtcggaattc 3780
ttacacaagg accgggacca tgccctgtag cctttttgtc caaacaactt gaccttactg 3840
ttttaggctc gccatcatgt ctccatgcgg tagcttccgc tgccctaata cttttagagg 3900
ccctcaaaat cacaaactat gctcaactca ctctctacag ctctcacaac ttccaaaatc 3960
tattttcttt ctcacacctg acgcatatac tttctgctcc ccggctcctt cagctgtatt 4020
cactctttgt tgagtctccc acaattacca ttcttcctgg cccagacttc aatctggcct 4080
cccacattat tctggatacc acacctgacc ctgatgattg tatgtctctg atctacctga 4140
cattcacccc atttccccat atttccttct tttctgttcc tcatgttgat cacatttggt 4200
ttactgacgg cagttccacc aggcctgatc gccactcacc agcaaaggca ggctatgcta 4260
tagaatcttc cacatccatc attgaggcta ctgctctgcc cccctccact acctctcagc 4320
aagccgaact gattgcctta actcgggcct tcactcttgc aaagggacta cacgtcaata 4380
tttatactga ctctaaatat gccttccata tcttgcacca ccatgctgtt atatgggctg 4440
aaagaggttt cctcactacg caagggtcct ccatcattaa tgcctcttta ataaaaactc 4500
ttctcaaggc tgctttactt ccaaaggaag ctggagtcac acactgcaag ggccaccaaa 4560
aggcgtcaga tcccattact ctaggaaatg cttatgctga taaggtagct aaagaagcac 4620
ctagcgttcc aacttctgtc cctcatggcc agtttttctc cttcccatca gtcattccca 4680
cctactcccc cattgaaact tccgcctatc aatctcttct cacacaaggc aaatggttct 4740
tagaccaagg aaaatatctc cttccagcct cacaggccca ttctattctg tcatcatttc 4800
ataacctctt ccatgtaggt tacaagccac tagtccacct cttagaacct ctcatttcct 4860
tccatcgtgg aaacatatcc tcaaggaaat cacttctcag tgttccatct gctattctac 4920
taccccctcag ggattgttca ggccccctcc cctccctaca catcaagctc ggggatttgc 4980
ccctgcccag gactggcaaa ttgactttac tcacatgccc tgagtcagga aactaaaata 5040
cctcttggtc tgggtagaca ctgtcactgg atgggtagag gcctttccca cagggtctga 5100
gaaggccact gcagtcattt cttcccttct gtcagacata attccttggg ttggccttcc 5160
cacctctata cagtccaata acggagcagc ctttattagt caaatcacct gagcagtttt 5220
tcaggctctt ggtattcagt ggaaccttcg taccccttac tgtcctcaat cttcaggaaa 5280
ggtagaatgg actaatggtc ttttaaaaac acaccccacc aaactcagcc tccaacttaa 5340
aaaggaggat agagcccaaa aactcgcaac caagctagta attatgctga acccccttgg 5400
gcactctcta attggatgtc ttaggtcctc ccaaatctta gtcctttaat atctgttttt 5460
ctccttctct tattcggacc ttgtgtcttc cgtttagttt ttcaattcat acaaaaccgc 5520
atccaggcca tcaccaatcg ttctatacaa taaatgctcc ttctaacaac cccacaatat 5580
cgccccttac cacaaaatct tccttcagct taatctctcc cactctaggt tcccatgccg 5640
cccataatcc ctctcgaagc agccctgaga aacatagccc attatctctc cataccaccc 5700
ccaaaatttt tgctgcccca acacttcaac actattttac attatttttc ttattaatat 5760
aagaagacag caatgtcagg cctctgagcc caagccatca tatcccctgt gacctgcaca 5820
tatacatcca gatggcctga agtaactgaa gaatcacaaa agaagtgaaa atggcctgtt 5880
cctgccttaa ccgatgacat tccaccactg tgatttgttc ctgccccacc ttaactgagc 5940
aattaacctt gggaaattcc ttctcctggc tcaaaacctc ccccactgag caccttgtga 6000
cccctgcccc tccactaccc acccaaatcc tataaaatgg ccccacccca tctcccttag 6060
ctgactcctt ttttggactc agcccgcctg cacccaggtg aaataaacag ccttgttgct 6120
cacacaaagc ctgtttggtg gactctcttc acagggacgg gggtgacaac aacacggaca 6180
cacatggagt ggttttaagg agcagagagt ttaatacgca aaaaagaagg aagaggctcc 6240
cctgtacaga cacagaggga gggggctcca agccgagaga aggaaacccc atgtgcagtg 6300
gaaaagtggt tgattatact gggaggctgg aggaggcggt gtctgatttg cacagggccc 6360
aggggattgg gttgaccagg tgtatcattc atgtaccccg caaaaaacct ggccctccca 6420
cctcagccct ttaatatgca aatgtgggtt gccatgatgt tctgaaaaca catgaattat 6480

ctggaggggg ccatgacact tggtacatgt gctgacaaga agagggtggg aatcgccatg 6540
gtggccatgt tgggtggacc tagtttttaa tagcctgcat ttgcatatca aagtttgctg 6600
gcctggctct ttaagctgtc ttttctgtta gaaaaggaat ggtttggaat gggtgagggt 6660
tgcttcttat tacaagaaaa tttccaaaaa cctttactct ttctagctgc caaaaaacta 6720
tttcttaata acttatgtat taccataatt aggcagcacc aaagatccct gcaggtcaga 6780
ccactgcaat taacatgctg gctttactgc tgattatggt agctgcatcc acctagcctc 6840
tcatattgca actgcctgac ctctgccacc ccacgagcca cttatcccca cttataatca 6900
gcccatttcg attgtaacat ctgccactta ttcccgacgt tgtggtatat cctatagatg 6960
aattcattca acatccattc caacaccacc tctcttgcct tcctatactc tctggagagt 7020
gaattactga gtcacatgat cttcactgca gtcatttgtg gctatgtgac atagttctgg 7080
acagtgaaca tagacagaag tccctggggc gggcttcctt tctgggatga gggcaaaacg 7140

<210> 63
<211> 44100
<212> ADN
<213> Homo sapiens

<400> 63
tgcctttatt tccgtaggct ggtcatatgg cgctagcact cacataaagc taccgaggag 60
agcgaatgaa accaaaatca ctttaccttc acagcacgag gccgtcgtcc ctctcgatat 120
ttggcccgtg tgtcgcatac cgccctctgg acgtggtgat caaataaact ccctagctcc 180
ccgccgctcg acgccatctt gcctactttg atcctcgcag ggaggacaac atccgcccta 240
ctgagctccc ttttatccaa taagagagcg ggatgagtta aggagtgcca ggattggctg 300
gagaatcgac agcgtcggcc atcgtttcct gcgtgcgaag atttgatgaa cgaggtgccg 360
cccccgagcg gctcggcgga gaggcgcggt gggtgacaga agctttcttg tcccacccac 420
tacaggctta cggcaggatg cgcagcgggg agagggggcg gggccgcagg gggcggggcc 480
gatcgatctc ctccggctcc gacgtcctcg gcctgccggg tcccgggtcc tttgcggcgc 540
tagggtgggc gaacccagag cgacgctccg ggacgatgtg gggcagcgat cgcctggcgg 600
gtgctggggg aggcggggcg gcagtgactg tggccttcac caacgctcgc gactgcttcc 660
tccacctgcc gcggcgtctc gtggcccagc tgcatctgct gcaggtaacc tgccggcccc 720
gagccacctg atcttcagcc tggggtcgga cgaggccgaa gcctctcagg gacgcggcgg 780
gacaccggct gccacccggg cgccgccgaa gcgcgcagag atcagggtcc ctcgacggca 840
gggcccttct gggtagtctc tggatcccac aagtccagtg cagccctggg ctcgtcttat 900
cccaggtctt ttcacttggt gaaactgaac ctagaaacgt cctaatattc taccactgtt 960
tttataaata ttccttattc caggctggaa aagctcctga gaagtggttt gtttttatta 1020
ttttaaaagg tgttttcctt gccagccatt tccagttaac ctgcgctgct gccgtccggg 1080
ccgcgagagc gggacgcaga gttgttggcg gagcccctgt cggttcccgg ggactaagca 1140
ccgcgtccca tgagcgggaa aggttaatac aatgatggtt ctgccctgcg tcgctgacgc 1200
ggaacacagc tgtagtgtgt taggaacaca taacgtagtt aagatcactt gaagctctgc 1260
gatcagtcgc ccttctggac gttgtggtta ggatgtttca cagttctaac cactggtgga 1320
gatacagcgt ccatattttc ataattaaaa atagaggcac atggtctcac gagtttgagt 1380
gtacttatgg gggcaaaagg acggcgtatt tgaaatcctc ataaatcctg gatgcatggt 1440
acccaccagt ggctaatcta tgcaatgaat agagtttgca ataatttcaa gcatcccttc 1500
tttccacttg agttacttcc ccatacctag gggaagatat ttttggtcca ctgaaaacat 1560
gagttcagca gaatcctcct atcatcgtcg ttattatttt ttaccactaa gtagacaatc 1620
ttttggtttt tgatgggctt tatggctaga gacaaatcag tcactgtcac caagttccag 1680
gtagaagttg gttcagtgct ctgtcagctt cgatgggatt tttcaacatg ttttcaaatc 1740
tgcacttaat agtaggaatg ctttcttaca gtaactctaa tttgatccta agatgtagtt 1800
gttaccttac attcatcact gtttaagaat ttagtggtct tgatctttgt tttaaatttt 1860
gagccttcgg gaagtactta taagaattaa ttcatgcata tctttttgaa atgtaaatgt 1920
ctttagccct ggaacaaatt gctgtttctg ttcagcccat attagcagaa taggtcaact 1980
ttactttcta attatcaatg taataagttt attactttat agattccata aatctataca 2040
tttattcctc gatgaattat ataaatttat agaatttatg ttttatagaa aatttggaaa 2100
gcatggaaaa ttattaacaa gaaaataagt tacccataat cccagaactt agaggtgact 2160
aatgttgaca gtttggatca aatcttccag ttttgtttct aatctttatt tttaacataa 2220
atgaggtcct gtatacacac gtacagtttt gtgtcctggt gtttttattt aatgttatta 2280
tgagtgtttt attttgttaa aaggtcatca ttttaagttg ttaattagta ttctagcaca 2340
aatttgccat aatttattta attgtttact atgattgacc atttagattg tacttaattt 2400
ttaggcatta gaagtgataa actatatttt aatcagacgt tgaaaataac acatctttgt 2460
ttagaaaaca tcattttatt tctggttgtc taggatagat tcccagaatt cttgggttag 2520
aggccataga taattatgaa agcagaaaga ttcacaagtt gggagttaat acttgaatta 2580
ctttatttgg ggtgaagcat tgagtgcata atacagatca tgcagtaatg ggaagaaggg 2640
ttggaacaat ggttttctgg cctatgtcag acttaccttg aagcttttaa gaatacagat 2700
gttctgatca accctcagac ctattaaatc agacctaaaa tcttagggaa taggctttag 2760
gcatctctaa ttttaaaaaa tttattcagg ctacttggat gcacaaaaga gttgagacct 2820
actgtcctag aatcatagaa ttttaatgac gatagagacc ttaagcatct aggtcgtttc 2880
tgtactttta catgtaagga aactggcatt cctaggccag taccattgcc atgcagctaa 2940

tttgccctct tgtctatagc tcactctgca tcacccaacc taccgttctc actgtttctt 3000
ctataaccaa tctccttccc acttctgttc tcttactcat gccattcttc cctcagtcat 3060
ttttcttcct tccatacaaa ttccatgtct ttaaaaagga ataatcctac ctcctccaca 3120
tagctttcca attctctgtt gcccacattt gtctcccttt caatacttct ctgttgtgtt 3180
atgtgacaca tcacatttga tatactctgt actgtgtttc aagtattgta ttctcttgtt 3240
tactcaagtc attatttcag gactgactac ccagtagatg ctttaagtca ggatttctca 3300
accttggcac tgttgacatt ttgagctgga taattttttg ttttgggggc tctcctgtac 3360
attttaagat gtttaacagc acccttggcc tctatccagt agacgcctgt actgcctccc 3420
cctatctgtg acaaccaaaa aggtcttcag acattgtcag atgtctactg aaggacaaaa 3480
tcacctctgg ttgagaacca ccgcttcaac taagttatct tctctgtact cagaacttga 3540
tgtgattgca gcagggggag aggattcata tacacagtga atgcaaacga acctaaatca 3600
ccattcggat atggccacac aattttcatt tcccttgtgt tagcaagaga taccctaggc 3660
tttggacctg attattccta aggcattctg atgtatggtt ttacctgcag atttcctggt 3720
aatactgata cctcagtttg ggtcaaagaa ggtcaattaa ttgattgatt tgatttgact 3780
cctggaaaag acgctccttt ctagctgtct ctttcttctc tttacctgaa tagccagggc 3840
tctgtggttc aagtgaagta ttttgacata aaaattaact tagaacattg gtctgcagag 3900
tttgctcaat ataactgagc acatattgtg gctttatgga gctggttact actttttgac 3960
caaataaata attagaagta tttttcctcc tcaataaggt tcattttttcc tttttcagt 4020
gagctggtag agtttccttt tttgatattt cagggcatct ttcatatttc catctcttaa 4080
gtttcttcat atgaagtaga atttatctgg attatgtatt gctgactctg atgaaaaccc 4140
atagaaagca tctggggctt gatcaccttc attcttgtaa tagctcacac ggttacagct 4200
gatatggtaa cttaagactt ttgattccaa atctaggcaa aatacactca gttgaaagaa 4260
tttgtcagcc agaacagttg gactgttctg tgaaaattgt gagaaaaatt acacaactaa 4320
gtgatacatg atgatggctt tcttaaatat aaaattgtaa taacatggtt aatttccagt 4380
acgttatatt gtcccagaag tggctccaac attgtttgaa atttgtctca tttaaagaaa 4440
cataagctgg ctatggtggc tcacgcctgt aatcccagca ctttgggagg ctgaggcagg 4500
cagatcacct gaggtcagga gttcgagacc agcctggcca acatggtaaa accccatctc 4560
tactaaaaat acaaaaatta gccgggcatt tggtgggggc ctgtaatccc agctacttgg 4620
gaggctgagg caggagaatt gcttgaatct gggaggtgga ggttgcagtg agccgagatt 4680
gtgccactgc cctccagcct gggtgacaga gtgagtctcc gtctcaagaa aaaaaaaaaa 4740
aaaagcaaga aacataaaga ctgggcatgt tggctcatgc ctgtaatccc agcactttga 4800
gagactgagg tgggaagatc acttgagccc aggaggttaa ggctgcagtg agccgtgatt 4860
ttgccactgt actcgagcct gggcaacaca gtgagatcct gtctcaggaa aaaaaaaatt 4920
gcatgtaaat gaatgaattt gatatttaat attttaaatt atgaaaactg ttctgtagag 4980
atgtagatct tgccatgttg cccaggctgg ctttgaactt ctgggctcaa acaatcctcc 5040
tgtctcagtc tcccaaagta taaagattac acatgtgagc cactgcacct ggcctaatat 5100
ttttaactta atgaatttat tttgatataa ataaattaat aacactgaag cttcctgata 5160
taataagtct ttttgtgtgt gtgacgggtt ctcactctgt tgcccagact ggagtgtaat 5220
ggcactatca tggctcactg tagcctcaac ctccctgact caagtgatcc tcccacctcg 5280
gcttcctgag tagatgggac cacaggcgta tgccaccaca cctggctgat ttttaaaatt 5340
tattattgat acatattaat aaaattattt ttattttaaa aatgatatat gtggctgggc 5400
atggtggctc atgcctgtaa tcccgacagt ttgggaggcc gaggtgggag gatcacttga 5460
gaccaggagc ttaagaccag cctaagcaac atagtgagat cccatctcta tagaaaaaaa 5520
aaatggctag gtgtggtggt gtatgcctat attcccagct actcaggaga ctgaggtgag 5580
aggattgcta gagcccagga gtttcaagtt acagtgacct atgattgtgc cagtgcactc 5640
cagcctgggc aacagagcaa aatcctgtct caaaaaaaaa aaaagttcga aaatgcttat 5700
gatgcaatat aagtagtgga aaaggatatt aaattgtgcc tatatgaaca caactatatg 5760
aaaaacttgc acatagagaa aaggattaac aagaaataga ccaaattgtt cacatggttg 5820
tcttgtttgt ggagagaata tcagtagttc atttgtttcc ttccaagttt atatgttttc 5880
cgaggtctct ataatgagtt tgtaattgtt taatcataga aaaccctttt ttggtccttg 5940
gccacaaact tacatgtttt aatgtaattg ctttttttaat gagaataaat gttatatttt 6000
gcttttttaa aacctatatt cccatagtta tatgagccct tacaattatt aagaggctgc 6060
ataatataac gtttctggaa gggtacagaa gaaacagcag taattacctc tgagaacaga 6120
gacatggctt cacattttac ccttttgtac gttttgtgct tttgccacat gcatttatta 6180
ttcttccaat aaataagtaa ataaatatgg attgtatact ccatctggtt ggtgtttcat 6240
aattctaaaa ttatattgct acatttttaa agatgatatg tgtttctact tattaacgta 6300
tatgttaaaa tagtaaattt atatcttatt taataatttc cctattgata gacatttaag 6360
acagtctcaa gtgttcacta tcatagaaaa tactgcacag atagcttttg ctatagtttc 6420
tttttttcttt gaatcgttaa ttgggaataa atgctcaaat agttatatgt ggctcaactg 6480
ctatttaagt ttattgactg actgctgcca ttttgaattc tgaaggggtt gattaaattt 6540
ataatgctgc cataagaata taagggtatt ggcttcatta gcatccacca gcattgggtg 6600
ttggaaatga ttatagattt ttaaatgcta caacaaatgt agataacaga gaactatcta 6660
tagaactctt tttggacatg tgaattgtaa taatagttta ttttcatgtg aatccagaaa 6720
aatgtatacg aaaacctttt ttcctctcat ttcttatatg aatagaatca agctatagaa 6780
gtggtctgga gtcaccagcc tgcattcttg agctgggtgg aaggcaggca ttttagtgat 6840
gggggacagg taagcacatg tgatggcaat aactttcttc taatatcaca taatatagca 6900
atagaaataa aattaaaagt ttagattttt tgttaaagga ggtgagatgt cacctaattt 6960
gtatgctatt atgtaactag tctaggatat tgaagctgac tatactctgt ttttaggtca 7020

ttatcttgta gtttaccata ctccctactt gcttcttatt ctactattta actcattttc 7080
cacatcccct aattttggtt tcatgaaatt atttttcctt ctgaattact aggttctact 7140
tactattatt aaactttatt tctgacatat tttataacct tccatggtct cacttgatta 7200
aaaataaaaa attcagctgg gtgcggtggc tcacacctat aatcccagca ctttgggagg 7260
ccaaggtggg cggataattt gaggtcagga gttggagacc agcctgccca acgtggtgaa 7320
accccccctc tctactaaaa attcaaaaat tagctgggca tggtggcagg tgcctgtaat 7380
cccagctact caggaggctg aggcaggaga attgcttgaa cctgggaggt ggaggttgca 7440
gtgagctgag attgcactgc tgcacttcag ctgggtgaca agagcgaaac aatgtcttga 7500
aaaaaaataa aaaataaaaa attctacaac acagggttat tatttttcca tttttgtttt 7560
cccttatgag tttaatatgt ttagattata aacctgaaag cttgaatacc tatgtctatc 7620
ttttgttttc ttatgtttat caagttattc ctttaaacat tttctaaact gtaagaataa 7680
tgtgaggctg ggctcaatgg cttatgcctg taatcccagt gctttgggag gccaaggtgg 7740
gaggaccact tgaggccacg agttcaagat tagcctggct aggcaacata gcaagaccct 7800
atctctataa aaaaattaaa aaaattagct gggcatggta gcaaatgctt gtagtcccag 7860
ctactcagca gactgaggta ggaggaatgc ttgagaccag gaatttgagt gacctatgat 7920
tatgcactcc agcccgggca atagcaagac cctatctctt aaaagaagaa gatgtagtaa 7980
taatacatat tcattataac tatttaccca ttgaaagtaa aaaatgagtt tttacctttt 8040
cccagtccca tcctcagaat ggggatctca gtagaccttt aggattggaa gaatgagatc 8100
attcatattt tctgcaatta ttaccccaca aaatatttca gatacctttc catgtattac 8160
aaacaatgtg catttaacat gtctctctct ttctctctct ctctgtgtgc gtcttcatga 8220
tcctctgttg cagccctgcc agtaagacac tatctcctga agaatcactg ataggaacag 8280
aaagtggact ggctaggcca ggagtcctta gcttcttagg gggcaggagc tgctttgtgc 8340
tttctcagaa tcagatatat atgtggactg aaacatttaa aaacagaata gccaagggtg 8400
ctatacgttt aaaacttata tagatggggc tacattgctc tctattacta atttcccatg 8460
acaatacacg agagtgccat gtctttttaa cttgttttga gcacagacta atcttgttta 8520
tgcatgtttt ttgatgagaa taggctactc atgagaaatc tgtaaaccta acactagtcc 8580
cttgcatact ctaaattgtt gctagaatct taaaatttta gcaccagacg gaccttagaa 8640
atcattaact ttggtgcttt gttctacaat acaaggagat ggaatatttt acccaggatt 8700
gcttagcagg ttacagttct gccctctgag tacccagcac ttccctgtgg gcaacatcaa 8760
cttcctgatt ttcaagtctt aattagtact ctgaagaatc ctacttgttt ttaactccca 8820
tttgctttga agtgacttta cctgattttt ttagatccct tattgcagca atgccactaa 8880
gaaactgagt ctctagcttc ttggtgggca ggagctgctt tgtgcttgct cagaatcatc 8940
cttttcagta agggagatat tgaagagaaa tctactgagg agtctggggg tgaggcactc 9000
agggaaatcc tgctccagtc cacaaaagca gagaggaagg gttggttacc tagagtattt 9060
aacatgcaga ggctttggat tttactcctt taatccttgg aaatgcctat ggaaggggaa 9120
aggaagtaag atggtgactc cagcttatag acatactagt gttacatata tttaaactat 9180
aataggaggg tattattagt tttacttaac tttcaactgt gaaggattat acttctcaat 9240
atttgtctcc agtgtctatt tcagtgtatt tttcactttt cttgaagcag catgtctgtt 9300
gcaaaacttc tagaaataat gagaatattt atatattaga tcaagccata acttgatgat 9360
atagtcattt cttcttatat tttttactta catttttaca ttttaatgat tactttcatt 9420
tttgaaaaac atgtcatgct gagatgtatt tttcttcatt ctgtaattag ttatgaaaca 9480
gtttttccta aaatgctgag tatatcaagt cttggctaag aataagtaat aaatatttgc 9540
cacatgaaag actacacata tagccaggtg cagtggcttg cacctgtttt cccagctacc 9600
caggaggctg aggcaggagg attgcttgag cccagggttt ccaggctgca gtgaactatg 9660
attgtaccac tctactccag aatgggtgac agagccaggc cccatctctc aaaacagaaa 9720
agaaagatta catagactac atatacaccc ccatccaaaa catacacaca catctactta 9780
acctaaaatg gtaagaagat aacttcttat tttctaatat atgacacaga aaagtttttt 9840
taaagtagtt ttaaattttt aattttttct aggtatttct caagccatgt tcccatgtgg 9900
tatcttgtca acaagttgag gtggaacccc tctcagcaga tgattgggag atactggtaa 9960
agaaaaccaa ataagaacta tctcatttaa ggttaaatta cttcacaata tcaatgtctt 10020
tagctttctc taagctttat tatatattct gagttggttt tgaattataa gaatgaattg 10080
gggccaggca cagtagctca tgcctatagt cccagcactt tgggaggcca aggcaggtgg 10140
attgcttgag tccaggagtt caagaccagg ctgggcaaca tggtgaaacc ccgtatctac 10200
taaaaataca aaaattagcc aggcatggta gtgcatgcca ttagtcccag tcacttggga 10260
ggctgaggca ggagaatcgc ttgagcccgt aaagtcaagg ctgcagtgag tcaggatctt 10320
gccattgtac tccagtctgg aaaacagagt gagaccttgt ctcaaataaa aaaagaatga 10380
attgatagag atctaatgta caacctgaca actataggta ataaaattgt attggggatt 10440
catgttaaat gagtagattt taactactct taccacaaaa acacaaaagt gggtaactgt 10500
gagatgatgt atatgttaat ttacttcact atagtaacca ttatactatc tatatgtagc 10560
tcataacacc atgtcgtgta tattaaatat gcacattaaa atttgttttt taaaaaaaga 10620
attgagattt tttttaacta gatatggagt ggacaaaatg taaagtgaat tgatcttttc 10680
gtctgttggt tctaggagct gcatgctgtt tcccttgaac aacatcttct agatcaaatt 10740
cgaatagttt ttccaaaagc catttttcct gtttgggttg atcaacaaac gtacatattt 10800
atccaaattg gtaggtgcta ttgtaatatt tgctgtcata ttctacacta tagcattgag 10860
tccaaagtag aaatgaatgt gcactaatga gctttatttt ctacacagtt gcactaatac 10920
cagctgcctc ttatggaagg ctggaaactg acaccaaact ccttattcag ccaaagacac 10980
gccgagccaa agagaataca ttttcaaaag ctgatgctga atataaaaaa cttcatagtt 11040
atggaagaga ccagaaagga atgatgaaag aacttcaaac caagcaactt cagtcaaata 11100

ctgtgggaat cactgaatct aatgaaaacg agtcagagat tccagttgac tcatcatcag 11160
tagcaagttt atggactatg ataggaagca tttttccttt tcaatctgag aagaaacaag 11220
agacatcttg gggtttaact gaaatcaatg cattcaaaaa tatgcagtca aaggttgttc 11280
ctctagacaa tattttcaga gtatgcaaat ctcaacctcc tagtatatat aacgcgtcag 11340
caacctctgt ttttcataaa cactgtgcca ttcatgtatt tccatgggac caggaatatt 11400
ttgatgtaga gcccagcttt actgtgacat atggaaagct agttaagcta ctttctccaa 11460
agcaacagca aagtaaaaca aaacaaaatg tgttatcacc tgaaaaagag aagcagatgt 11520
cagagccact agatcaaaaa aaaattaggt cagatcataa tgaagaagat gagaaggcct 11580
gtgtgctaca agtagtctgg aatggacttg aagaattgaa caatgccatc aaatatacca 11640
aaaatgtaga agttctccat cttgggaaag tctgggttag tataaatttt ataacttggg 11700
agaaatttta tgtggcttaa acatccccaa attatgaatt agaatagtat ttcatatata 11760
aattgaaaat caattaaaaa gaaacacagt gcctaaaggc acttggggga cacatttacg 11820
ctttgcagta aagtccttgt ttggataaag attgtatgtt ttctggccaa gtaagcttga 11880
ataggtacaa gcttagatag gttcaggcca gagaggtcaa aattacttgc ctgagattgc 11940
atagctagtg ttacaactag gattcaaacc caggcagatt gacttggggg ttcatcagga 12000
tggagtgccc tacaaagcct cccatcttta atgcttgcag atttgttccc cagttaccga 12060
aagcaacttg ttaatattag ggaaaagggc cagtgtaggg agagatccat ggcatgaggt 12120
aaccttcctg ctgcatgtgg tggcacctgg attggaatgc atccaggagc tgcttaccct 12180
gccggtgtct gctctttaat ttgtgtataa cggagaggaa gtagacaggg caactagtgc 12240
tccagcccct catcctggcc acaaatatta atgctacctt tatatgacat aagtcactag 12300
tccatttatt ggaacctaaa tttgaaccac tgtaaagtaa gacttcatag tgataaagag 12360
aggaacttgt taggaaagag aataaaatag aaagagaagg ttgtctcctt ttgtagattt 12420
tttttttttc tccaacagtt ttacctgtga cctttataca aataactgac aaagcattaa 12480
tctctttggc ctacatcatt ttcttttcta tttttttttt ccacaagatg gagtttcact 12540
cttcttgccc aagctggagt gcagtggcat gatctggctc actgcaacct ccgcctccca 12600
cgttcaagtg gttctcctgc ctcagcctcc tgagtagctg ggactacagg catgcaccac 12660
cacgcctggc taattttttg tatttttagt agaaactggg tttcaccatg ttagccagcc 12720
tggtctggaa ctcctgacct caggtgatct gcctgcctcg gcctcccaaa gtgctgggat 12780
tacaggcatg agccactgct cctggccggc ctacatcatt ttctaaagct ccagaccatt 12840
cttttctttt cttttctttt cttttctttt cttttctttt cttttctttt cttttttctc 12900
ttctcttctc ttctcttctc ttctcttctc ttctcttctc ttttcttttc tttttttgag 12960
ttagaagctt gctttgttgc ccaggctgga gtgcagtggc accacctcca ctcactacaa 13020
cctccacctc ccaggttcaa atgattctcc tgcctcagcc ttcagagtag ctgggactac 13080
aagtgtgcgc caccactcct ggctaatttt tgtattttta gtagggacga ggtttcacca 13140
tgttggccag gctagtcttg aactcctggt ctcaagtgat ccgcctgcct cagtctccca 13200
aggtgctggg attacaggcg tgagccactg tgcctggcct cagatcatta ttttctgtta 13260
gctttaaact gtccgttcag gagatcccac tgcatcctca aattcaaaat atctaacact 13320
gagcttatga tttagctggt tctgtcatta gatgggaata tccttttatt tccttgaaat 13380
tatatggtga gaacagggag aagtgctgat ggtaaagtcc tgtgattaag atagcaataa 13440
ggactccgcc cttcccactc cactgaaggt tgaagagcca tggacaatga gaagtcacag 13500
taggtgaaat caggtactaa aatggacttg gcttgagaga tcaaaattga tcacttggtg 13560
atacaactaa caaattcatg ttaacttgaa cctttattac cctgtgaagc atggtgatta 13620
aaaaaaaaca acaaacaaac aggaaacttg attgttaaat tctctttaag tcagaatatg 13680
taccttagag tttttattta tgcttttgtc taccattaat atgtctgcac ctgctcttta 13740
gaagttaata gagagtaaag tcgtctttat gtctttcagt gcttacttat atttgggaag 13800
ttgagaaaaa tttttaacat cattattgat atatatatat atatatatat atatatatat 13860
atatatatat atatatatat atagataatt tttttttttt tcttgagacg gagtctcact 13920
ctgtcgccca ggccggagtg tggtggcgat ctccactcaa tgcaagctct gcctcccagg 13980
ttcaagcgat tctcttgcct cagcctcccg agtagctagg atacaggctc ccaccaccac 14040
gcctggctaa tttttgtagt tttagtagag acgaggtttc accatattgg ccacgctggt 14100
ctcaaactcc tgaccttgtg atccgcccac ctcggcctcc caaagtgctg ggattacagg 14160
cgtgagccac tgcgcccggc tgaggtaaaa tttaaagtgt acaattcagt catttttagt 14220
atatttatac tagttgtaca gccatcacca caatctaagt ttagaacatt ttcattaggg 14280
ggtgggagaa attttactct gctttttaga ttaagtttct gtctggatct aatcatttaa 14340
tcagacaatc aggcagattg tctgtgatta gttttggcca ttccagcttc ttcattggtt 14400
gttaactttc acaaataaag gctgctcaaa gattagaaat aacatttaat ttgaatgtaa 14460
atgtgccata gtttaaaaga tgggtttggt gaatacagtc aaatacatac atttaaagct 14520
ctaattctga agattatgta aagaaaagga aagaaatgta gggagaggat tgaaatgttc 14580
atggtataac aatatctgaa catccatctg gtcacaccgt tggtatttga atgttttgtc 14640
ctcctcaaat tcatatgtcg aaatcccaac tcccaaggtg atcgtattag gaggtgtggt 14700
ctttgggaag tgattaggtc atgaaggtga agccttcatg aatgggattc gtgctcttat 14760
aaaagagaac tgtgagaaat aagtttctgt cgtttgttag ccacccagtt taggatattt 14820
tgatatagca gcctgcatgg actgagacaa ctatgagtta ttatgatagc ttctgttatt 14880
tcacctaaat tcatagaagc taatatatca atatttatgc tatgaaatat ttcttaacca 14940
agctttgaat atatttatat ttttgtttat ttttaaattt cagattccag atgacctgag 15000
gaagagacta aatatagaaa tgcatgccgt agtcaggata actccagtgg aagttacccc 15060
taaaattcca agatctctaa agttacaacc tagagagaat ttagtgagtt caaatatata 15120
tgttacatca aaattctttt acacgttttg taagatttct agttgcttta gctaagtaat 15180

aagaatgttg tattcctttt tgatacaaat ctttttttat tgtgttaaac tatatataac 15240
ataaaatatg ccatgttcgc catttttaag tgtataattc aaaggcatta attacattca 15300
taatattgta caaccatcac cactatctat atccagaact tttccatcac cccaaagaga 15360
aacttggtac ccattaaaca ataattcccc gtccactcct ttccccagtc cctggtaatc 15420
tctaatgtat attgtgtctc tatgaattta cttattctag atatttcata tataagtaga 15480
agtatgcatt tgtcttatgt atctgactta tttcatttaa cataatgttt tcaaggctca 15540
tctgtgttgt atgtatcaga atgttattcc ttttcatggc tgaatactat tccattgact 15600
gcatatacca catttgttta tccattcatc tgttgatgga cacttgggtt gtttccacat 15660
ttttggctgc tgtgaataat gctacagtga acattggtgt acaagtatct gtttgagttc 15720
ctcttttcag ctcctttggg atatacctag gaattatgtt taactttttg agaagctgag 15780
aaatctttaa taaatgataa cacaaatact tatatttgcc aatgcaaata tgaatatttt 15840
tggcttttaa gagattgatc attttgccac gtggttgtaa ttaaaaaaaa ttgtcccatg 15900
ttgtttcagt attaatattg tagcctaaaa gagtgctaga ctgttttact ttttactcag 15960
ttaattcttt ggatactggt agagtcagga aatgagatat tgaacttaaa gatctttgca 16020
ggtggggtcc agtggctcac acctgtaatc ctagcacttt gggaagctga ggtgggagga 16080
ttgcttgagg ccaagagttt gagaatagcc tgggcaacat agcaagaccc catctctaca 16140
aaaaaattaa aaaaaaaatt aagccaggcg tggtagctca cgcctgttat cccaacactt 16200
cgggaggctg agatgggtgg atcacttgag gtcaggagtt ggagaccagc ctggccaaca 16260
tggtgaaacc ccatctctac taaaaatacc aaaattatcg gggcgtggtg ctaatcctgt 16320
aatctcagct actcaggagg ctgaggcagg agaaccactt gaactgagga ggtggaagtt 16380
gcagtgagcc tagatctcac cactgcactc cagcctgggt aacagagcga gactctattt 16440
caaaaaaagt aaaaataaaa attagacaca tgtggtggca catgcctgta gtcctagcta 16500
ctcaggaggc tgactgaagt gggaggatct cttgagccca ggagttccac actgcagtga 16560
gctatgattg tgccactgca ctccagccta ggcaatatct caaaaaaaat ttttttaaat 16620
agattattag gccagacgtg gtggctcatg ccagtaatcc cagcactttg gaaggccaag 16680
gcaggcggat cacctgaggc caggagtttg agaccagcct ggccaacatg gtgaaacccc 16740
atgtctacca aaaatacaaa aattagctgc aatgtctata atcccagcta cttgggagcc 16800
tgaggcaagc gaatcgcttg aacccgggag gcagaggttg cagtgagtgg agactgcgcc 16860
actgcactcc agcctgggcg atacagcgag attctgtctc aaagaaaaag gaatttgttt 16920
tcctgtcttt atcgtagagg gaggaaaggg agaatggggt tggaatggtt attgagtgag 16980
ccacattatg gtagatgtat cactgggcat agagaaaagg agcatttaaa acttttccgc 17040
ctaacagatg tttcttcagg ctacactgca ctcattgtgc taactgtaat gtcaaatccc 17100
agacctgtgc ctatagaaca tgaacatcct tcattggatt tgtttggtca ggcttacact 17160
ttattaggaa gatcagatgt taaaataagg gtgttaaagt taagttcaga tatgaggata 17220
attcattact attccttttt ctggcagcct aaagacataa gtgaagaaga cataaaaact 17280
gtattttatt catggctaca gcagtctact accaccatgc ttcctttggt aatatcagag 17340
gaagaattta ttaagctgga aactaaagat ggtgagtaca tttgttattt tgactttttt 17400
ttctatttaa atagttgtac atttttaatt gttcttgcaa cctgtcatac ctgtgaacag 17460
tatgtgaata gtgaaatata attatgataa ttaaacagta gtttttatgt attgaaaaat 17520
atctttggcc gggtgcagtg gctcatgcct gtaatcccag cactttggga ggccgaggca 17580
ggcggatcac ttgaggccag gagttcgaga gcagcctgcc aacatggcgc aaccctatct 17640
atacaaaaaa atacaaaaat tagcctgaca tagtggtgta tgcctgtagt cccagctact 17700
tgggaggctg aggcagaagg atcacttgag cccaggaggt ctgtgttcct gccactgcac 17760
tccagcctgg gcagcagagt gagaccctgt tggggggaaa aaaaaaaaag tctttaactt 17820
aaataaattt gacatttaaa atcttaaatt atttcatctc tgtttcagta ctaactctgc 17880
atttattact ttctttttaa taggactgaa ggaattttct ctgagtatag ttcattcttg 17940
ggaaaaagaa aaagataaaa atatttttct gttgagtccc aatttgctgc agaagactac 18000
aatacaagta atagcatgtt attgaatatt taataaaata ctatttgtta catatgattg 18060
ataataaagt atgaagttcc ttgtaacacc ttgcattgtg aagtgtatta aaaacctgct 18120
aagagtaagg aataacttga tttaaaatat tttattctgt aatctcttta aattatctgt 18180
acaaattatt gacttaacct aaatttaaaa atgaatgcct tagcacaatt aagttccaag 18240
aatagagttg atcatgttaa ctggtaaatg gatcatgatt taaaattctt ctaggattga 18300
aacaaatgaa aacgtagttt taagggtttg attttttaaa ttcctatttt tacatgcaat 18360
tttactgcac aacccatctt attttgacag ttcttaaatt cgcaactctt cagaaatatt 18420
atcagatcac ttttctttgc ttccataagt ttttttatta ttatattatt attttttttt 18480
tttaaaagac ggtgtctcac tttgtcgccc aggctggagt gcagtggcat gatcatggct 18540
cactgcagcc tcgacctccc aggctcaggt gattctccca cctcagcctc ccaagtagct 18600
gggaccacag gcgaatgcca tgatgcctgg ctaatttttg tatgttttgt agagataggg 18660
tttcaccatg ttgcccagaa ttgtcttgaa ctcctgggtt caagcagttg ttctgccttg 18720
cccacccaaa gttgtgggat tacaagtgtg agccactgcg cccagctatt ctagaagtat 18780
tttaagagtc atcttttttt tttttttgag atggagtctc actctgtcac ccaggctgga 18840
gtgcagtggc acactctcgg ctcactgcaa cctccacctc ctgggttcaa gtgattctcc 18900
tgcctcagct tccctagtag ctaggattac aggcgcatgc caccatgccc tgctattttt 18960
tgtagtttta gtagagacga gatttcacca tgttggccag gctgctcttg aactcctgac 19020
ctcaagtgat ctgccctcct cagcctccca aagtgctggg attctaagtg taaaccacca 19080
cacccagcca agagtggtct ttttacaata ttattttttg attaggacat tcattcttgt 19140
cataaaattg aagatactct agtcatttag aatttcattg ttttggaact agacattgtt 19200
tctttatttt tgaaatgtta ttgaaggaat accatttgga gaagatacaa atgtaagaat 19260

tgtgaaaagg ataattgtga cacaaatcaa aattatagat aaaaatatac ctgtaaaatg 19320
tattaaggca ataacattct ttctgcttgt tgaccataaa tatttatatt ccctggatgg 19380
gtacattgtt attgtcaagg gtgtttaaat aatgatcttg catgcataat ttattctctc 19440
tggtataaca gaatcagcaa tttagttttc tgggacccga gaaaaacatg caaaagacat 19500
actttgaaat gtaaaactga tttttccttg caactgtagg tccttctaga tcctatggta 19560
aaagaagaaa acagtgagga aattgacttt attcttcctt ttttaaagct gagctctttg 19620
gggtaagaag ttatggccaa actagcatgt tagacatgtt tttaacacta tatctggcag 19680
agttttcaat gtaaatatta aagtagatgt taatgtcaat aagtgatctt aataatgcat 19740
cagtagatat tttttcaagg attgtctcta tcttcacgcc tagcttataa tttgccttgt 19800
cgtctttttt tttttctctt tatttttatg tttttatcca tccctggtgg taggggataa 19860
ccttgtcttc ttcgataaca agaagtctga agcttattag aaattttact ttgagaattg 19920
atcgatgaga agaaagcaac tagatatcac gtggatcata tatgcttgaa taaaacaata 19980
attcttagaa caaataaata cattttaaaa gttaaagcca aaaacattag ttgaatgttt 20040
aaaaatattt caaattaagt tattccttca ctgtcttgta ttactgtaat aatttggatt 20100
atttgtgttt ttctcaactt ttaaaacaaa tatttaaaaa attcctcttt tgattaagta 20160
gggctagata aaatataaaa aatatttttt aaactcctct taatttccat atttcttata 20220
taatatgaga atctcttata aacactacct cttagaagtc tccacagaag ctttggtaga 20280
tgtagtagta gggatttgat ttcttagaat ggtataatct gtaaatgttt tagtaaaagg 20340
attaaacgat aaagtcaaaa tgtttatagc acagtgttta ttaatataaa ataaaatctc 20400
tttttttttt tttgagatgg actctcactt tgtcactcag gctggagtgc agtgttgcaa 20460
tctcagctca ttgcaacctc cgcctcctgg gttcaagcaa tccttccgca tcagcctcct 20520
aagtagctgg gattacaagc atgcaccacc acacctgcct aatttttgt atttttagta 20580
gagatggggt ttcaccatgt tggccaggct ggtctcaagt gatccgcctg cctcagcctc 20640
ccaaagtgct gggattacag gcgtgaacca ctgtgcccag cataaagtaa aatctcttca 20700
gactctcatg tgatcatgta aagtggcagg cagtcacagt caagaagtag tttaaagttc 20760
atgtttgtaa aatataatct acagattgat actggatttc ataggtaatg tttaagagaa 20820
aataagtttt tagttatcct cagtacttca aaagcaccca tttatgatta tgttgattac 20880
taaactaaat catttggggg ctagaggtgt ttttttatgt gttaagattc cttaaggagt 20940
tctattaggg caaaactttt agtaactgca tattttaaaa gtaataaaac taattttaaa 21000
agcttggagg ctgggcgcgg tggctcacac ctgtaattcc agcactttgg gaggccaagg 21060
cgggtggatc acttgaggtc aggagtttga gacgagcctg agcaacatgg tgaaaccttg 21120
tctctactaa aaatacagaa attagccagg tgtggtggtg ggcacctgta atcccagcta 21180
ctcgggaggc taaggcagga gaattgctcg aacttgggag gcagaggttg cagtgagccg 21240
agatcatgcc actgcactcc agcctgggtg acagagcaag actccgtctc aaaaaaaaaa 21300
aaaaaaaaaa gcttgaagtc agattcgaca ttaatcagta tactttctct caagtagggg 21360
acaatttcta agattttagt cttttaaaat ttattaacta gtctgagcat ggtggcttgt 21420
gtctataatc ccagcacttt gtggggccga ggcagatgga tcacttgagc ccaggagttg 21480
gagactagcc tgggcaacat ggcaaaaccc cgtctctaca acaaatgcac acacaaaaaa 21540
cccaatcagc tgggtgtggt gttacactcc tgaagtccca gctactcggg aggctgaggc 21600
aggaggatca cctttgccag ggcgtttgag gctgcaggga gctgggttca caccactgcg 21660
ctccagcctg gatgacacag caagcccctt tctcaaaaaa aaaaagataa aaaattaaat 21720
taaattaatt aactacactg ggaaggcaaa attcagcatt tttttatagc taaattttat 21780
cctgcttcag tcttttatca tgtaactatg tatatttttt acagaggagt gaattcctta 21840
ggcgtatcct ccttggagca catcactcac agcctcctgg gacgcccttt gtctcggcag 21900
ctgatgtctc ttgttgcagg acttaggaat ggagctcttt tactcacagg aggaaaggta 21960
agtggttaag gtgtgttcat ttttctgtaa catttaataa cttttcattt atctttcttt 22020
gggttttgac catctattat atagggtggg ttttgaccat ctattatata gggtttatac 22080
gacatatgga aagcattcat ttattcacta atatttctgt gtgtctgctt ttaggtgttg 22140
ggggagtgat gacgaataag actgatgttc tccatgccct ttttctgtgt cagttgatac 22200
aattatatgg tttttctttt ttaggctatt aggtgttgat agggttgagt aacttacaaa 22260
tgttgaacca gccttgcata cctgtgataa ataccacgta gttgtggtgt atcattcttt 22320
ctacattgct gagttttatc tgctaatgtt ctgttgagct tttgtccatt taagtttgaa 22380
agtgattagt ttgcagtttt ctgtttttgt gttgtctttg tctggttttg ctatccgtgt 22440
aaatctggcc tcataaaatg agatgggaag tattctctcc tcttcttttg tttttttgga 22500
agaggttgta taaaattgag gctgaatctt ggtggttgcc acaatgacag gaactatttc 22560
tgtgactgaa tatattggga attcctataa agcaattatt ttctagggaa gtggaaaatc 22620
aactttagcc aaagcaatct gtaaagaagc atttgacaaa ctggatgccc atgtggagag 22680
agttgactgt aaagctttac gaggtatgag tatggtaaca ctctatataa atcccttttt 22740
cattagaaag acaggaatgt tatacataat gctgtcaatc taataaatac acatatcatc 22800
tagtctttaa cttttctgtt tatcatttag tcattaaaat ttctttggct ttctaatgtt 22860
tttgataaaa tttctaaaac tctccatatt taatggaggc ctattttttt ttctagccag 22920
aactttttgt agactacatt tctggaagtg ctcactgaca ccactctgaa aaattagtac 22980
ttagaatata ctctaattgg tataaatgat ctctgaattg ctatggaaaa ctgggagaat 23040
ggttgcttca ggggagagaa agtaggaggc tgtggacagc aatgaggaga attacagttc 23100
accatataac acttttgtac ttttaaagtc cttaacattt acattattat ctattcaatt 23160
aaaaaatatt gggaagattt tactttgaac agttaatttt tcccccatgg gtaccgctgt 23220
catatagttc caactaatca tgaacttgtg tatttcctgt tctttgtaaa tttaaacttt 23280
gtaactcacc aggaagtttg aagccaaatt tgtgtttcaa atatagcaac tccaggatct 23340

ctaggcagat gcatttgcat ttgattttaa atgaatcttg atcccttact ctcacttatg 23400
ttttcccaca tcctactttt tttattttgt tgtaagccat ctaaaattct caatgggatg 23460
aaactgggta taaatgaata catgcataca ggaattatag tagcatattc cttttctttt 23520
ttcttttttt tttttttgga gacagagtct tgctctgtag cccaggctgg agtgcagtgg 23580
tgcgatctcg gctcactata gcctccacct cccaggttca agcaattctc gtgcctcaac 23640
ctcccgagta attgggacta caggtgcatg ccaccacacc tggctaattt ttgtattttt 23700
tagtagagat ggggtttcac catgttggcc aggctgatct caaactcctg acctcaaagt 23760
gatctgcctg ccttggtttc ccaaagtgct gggattacta gcataagcca ctgcacctgg 23820
cctccttttc tgagttttat aaaatttgat actttactgc acgctttgag actgtattaa 23880
ttgaaccatg ttgatgaaca agtttttgtg atgggtatat taataaaata tagatcaaat 23940
ttttatagtt aaatcaatat cgagcttttc tagtgctttc aaaaggacaa cctgaatttt 24000
cccagcactg aaatgatact gaaaccattt catatcttct gtattaagga aaaaggcttg 24060
aaaacataca aaaaacccta gaggtggctt tctcagaggc agtgtggatg cagccatctg 24120
ttgtcctgct ggatgacctt gacctcattg ctggactgcc tgctgtcccg gaacatgagc 24180
acagtcctga tgcggtgcag agccagcggc ttgctcatgg taaatgcatc caccactggc 24240
ttaaggtctt gttcttttgt cagtcagcat ttttagtctt aacaataaat ctactctctt 24300
cagagaataa tatatgtgtt atgttaagtg ttgtgtttga ggcccctgat ggcattctac 24360
agttgtccta tagactgtaa tagcaaaatt ggtagagtaa aaacagtgtg aaaattctgc 24420
aacttcatgg ttagtccttt agggtttttc attctccctt acttattgtt taatttacag 24480
atttactctt ttgttcattt gacaaatatt tgtcaaatgc ttgtgcacag tctgtattct 24540
caaattctag gagaaaaaga agggtgaaca gtattagcgc agaacgatac taataatgat 24600
ggctactgtg tatgagtagc cagccctttc ttggctttct tggattgctt tgtattctac 24660
atgaagatat tccctgggct ttacaggtca ataaatggaa attcagagag attaatttga 24720
ccagggtgac caacaaggag atgacagcat acactatgcg agaagtatac acagagtagt 24780
gtaggagcat ataacctaaa ctgggggtga ggtgggataa ggagttatca gggaaggctt 24840
tttggaggag ttgacaactg agccgagttt tgatggaaga gtagaaatta gcatgaacca 24900
atttcatgct aataaagaag caaaggaagc gtggtctaca ggcaaaagca cagaggtaca 24960
ggaagtaatg atatgttggg gaataccctg ttgactggag cttagagtgc aaggagagga 25020
gtgctaggga ggtgaggttg gagggtttgg cagcattgac ttgcttcaag gttcttaaga 25080
gctgaaatag atataaaatg caactaagag tggcttggat tattattacc tagtgtgtta 25140
atctcaaatt ttgaaatcta tagcatctat aggactggtg ttactaatct tacactcgat 25200
ctgttactgt tcttatacta gatctattag tccagtgttt aagggagtgg tgcagatttc 25260
taggtcagga caggactcag atgtacatta ttaatgccta tttcagttct gaccttctca 25320
tatgaaacct tataagacct ggggtaggaa gagattgttc tggaagtcat aggaatatga 25380
actgtatttt gtttaacaaa caatacagta tggaaattta tcacccttcc agaatattta 25440
tttcagagac aaatttttat cattcgttca tttatttcat aagatccacg agtagggaac 25500
ctcactagac attgctctga gtatatggtc tgagtttgca gtacctcttg tgtctccatt 25560
agatttatta ggtcctcaat agataaatca gggaataact agatggattc atttttaaa 25620
gacatgaaag agcgatacca tacatactgc accttaaagg tcaaccttag agtatcatta 25680
tttttaatga atgtataatt tttaaatttc atgtttactt ttcctaagct tttgcactat 25740
attgcttaat tccagctttg aatgatatga taaaagagtt tatctccatg ggaagtttgg 25800
ttgcactgat tgccacaagt cagtctcagc aatctctaca tcctttactt gtttctgctc 25860
aaggagttca catatttcag tgcgtccaac acattcagcc tcctaatcag gtaatacact 25920
acttgtaagg attattgaat tatgtccctt ttatagaaat tatttttcaa ttttattagt 25980
aattcgtggc tttaaattta tgcttctctt aatgatttta aggatatgta agtcaacatt 26040
tggtgcatat tgtgctagag gcataaatta taatttatag ccacctgaaa tgttagtatg 26100
cgctttccaa gaaaatgact tttttgaaaa tggtatttct ttgaatgaga aagaacagag 26160
agaaatagat agatggcttt taaacacttc attaattaaa cttttttttt ccaccatcac 26220
ataatggcac ttagtcccct ttgggaactc atgagggttt tagtggtagt gagctgaaag 26280
aaatatgttc caggactggc aaacatattc taaattcttt aaaattttca cctagcatct 26340
accctaaata ttcagaccct gtgctagtta actgctattg aagaacaaag gtattatatc 26400
tattattaag gataatagaa tggtatttga gatattggtc attgaatatg aatatgtttt 26460
gagaaataag ttttatagga accaaaaaaa aattcttaaa ggaaccatat attactaaaa 26520
atgcttctta ttggagaaag aaatgacaat catttattaa tgtgattttt tcacaacttt 26580
attaagatat aatttaagta caacaaactc acataaagtg tacaatttga tcagttttaa 26640
catatgtaga tgccatgaaa ccatcaccac aattaaggaa acaaacattt tcatcactcc 26700
agaagtctcc tagccctttt actacccatt cctcccctgc tccatcccca gacaactacc 26760
aatttgcttt ctgtcactat agatttgtca acctgatttt ctccaaatat acattcaaaa 26820
atatacagtt gaatacaatt ggaaattcga attttgtgtt tttttcttta ggaacaaaga 26880
tgtgaaattc tgtgtaatgt aataaaaaat aaattggact gtgatataaa caagttcacc 26940
gatcttgacc tgcagcatgt agctaaagaa actggcgggt ttgtggctag agattttaca 27000
gtacttgtgg atcgagccat acattctcga ctctctcgtc agagtatatc caccagagaa 27060
agtatgtttt actattaaaa cctgaacttg gaatcttctt tctattgtgg agaaatgtaa 27120
ttgtagtaag acaagaatta aatatattcc attgtagtat ttgaataagc agttatttga 27180
gtagaaaatt agtgtttcca gctaagatga tggcatattt tgaaaattca tatagtgaat 27240
ataactagta aaagaagttt tgtttatttt taaacagaat tagttttaac aacattggac 27300
ttccaaaagg ctctccgcgg atttcttcct gcgtctttgc gaagtgtcaa cctgcataaa 27360
cctagagacc tgggttggga caagattggt gggttacatg aagttaggca gatactcatg 27420

gatactatcc agttacctgc caaggtatgt ttaaaaaaag aaaaagtgaa tacttactcc 27480
cagaagaacc actgtattat tggctttggc tttatgtgtc agcttgccca atctccgtgt 27540
gagtcaacaa gtgtttactg agttaccaaa taaatgtctt aacactattt taggtacttt 27600
aacaaatttt aattttatta attaattttt tattagaatt gagacctcac tctgtcatct 27660
aggctggagt acactcacag ctcactgcaa cctcaaactc ctgggctcaa gcaatcctcc 27720
tgcctcagcc tccccagtag ctagaactac aggcatgaac caccatgccc ggccaactct 27780
ttaattttct tagagacgga gtcttgctat gttgcccagg cagacagatt ttaatgtgta 27840
tgatgcagtc tttgatgata agaaacttat aatggaaagc tgaggtgata gttacagtaa 27900
atacattttg atgtataatt ctgtttgctt taatcattca aattgtagta aagcaagatg 27960
aactgtctgc tgggatttga gcagaaatgg ataggaataa actaggaggt agaagagtta 28020
tcaaggttca caggactgat gggtgaagct agatttccag acccgggatg tcagtccttg 28080
aaaagcagac ttggcaggca tagacgaggc agatagcagg ataaaggaga caaatgtaga 28140
ttgttcttca gaagatcaga tggtagagtc taggaggtag tgtgttttaa tcagagatct 28200
gagaggcaaa gatcattgca tgagatcagg gacccatgca aaggagtgag aaaaaaaact 28260
gggttaagga gcctgctgca tggcaactcc tgggaacagt ggccactggg gcctgggaca 28320
tgttgattgc agcccaggac tgttaaaacc agtgtgagag aacatgggta tggaagtact 28380
agctagcagg atcatgaccc cgatgctggg atggggcatc aagcattagt acatggagat 28440
tcagtacatc cagatgcagt acatggagac tatatgcgta actgctgact ttgggcttct 28500
ttcagattgg agcagaggta gaggtgagtg ggaatattct caatagaggg aactaaatag 28560
gcatacctaa taaaggagac caggatattg cagacagtag cctcatgttt ggctcacctg 28620
ttcaaaaagt tctcttgttc ttgagcagtg gtgccttaaa aggtaacttg agaagcagtc 28680
gattatttgt tcagcctgga gactcttggg atattttact atctttgatt gaatagattt 28740
aaatgtacac agctctcata acttgcccca tgaagcatat ccatgaaagg cactatactt 28800
gttaaaagat tggtttgtac tttttaaatg tagtactttt aataaaacag gaaaaataga 28860
agttctgatg cagttatatg cattttatat agaatgtgtt cttaattgga aaaaatttgt 28920
cgtagttcct ttgagttcat ttacagtttt tagtaggaat tgtattttct actgttgtac 28980
ttgctgttac taaagaaaga tggtcgtgat taccatctga attttttttc tatacattga 29040
tctttagctg ctacttagtc atttctgttt agacttgagc tctttttcat attttttttt 29100
tttgtttctc agtatccaga attatttgca aacttgccca tacgacaaag aacaggaata 29160
ctgttgtatg gtccgcctgg aacaggaaaa accttactag ctggggtaat tgcacgagag 29220
agtagaatga attttataag tgtcaaggta tgttgtctac ttatcttctt tttttattta 29280
ggtaaaatta acataaatgc agttagccat ttcaaagtgt aaattcactg gcatttagtg 29340
cattcacaat gctatgcaac caccacctct ctctaatttc aaaacttttt cattccactc 29400
ctcctcttgc ttatcccctg gcaaccattc atctgctttt tgtctctatg gatttgcctt 29460
ttctgtatat ttcatataaa acaaatcatg caatatgtga cctttttttgt ctggcttctt 29520
tcacttatgt aatgttttca tggttcatcc aggtagtagc atgtatcagt acttcattcc 29580
tttgcatgac tgaataatgt taccatactt tgtttatcca cttatcagtg gtgaacattt 29640
gaattgtttc taccttttga ctattatgaa taatgttgct gtaaatattc atgcacaaat 29700
ttctccacgg atatgttttc atttctcttg ggtataaact gaggagtaga attcttgggt 29760
cttagggtaa ttctctaact tttcaaagaa ccaccaaact gtctttcaca ccaactgcac 29820
cattcccact agcagtgtgg ggggttcctg attctccaca tctttaccaa caccattatg 29880
tttctcaatt gtgggctagt ctcacatttg gaaagctagt gggagcagcg atccatctat 29940
taaaagttgt atgaaattga gtaatgagcc acctctctct tgtagggctt attatgttct 30000
tgcttaaggc aatcttcatg cattgtgaac agaattatac ataaatgctc agataaaagg 30060
gcaaaccatt cttaaaggga gtagacaact agaggcagga gaccatactg aggcaggaag 30120
ctggggtttt tatggttctg ttacttttga ctatatctca ccattgcttt tgtcaaagtg 30180
agactaggtc taagtttttt tcaggtataa ggtgagtgtg gtaattaagg ggcatgctag 30240
cagatcattt tgggtaatgc ttcacagtcc accactggtg tgtcattgtg gtcgcagatc 30300
cagtatctta gctgtgtaat ttcagacatc agcaatatta gtttaacaaa gggcaattag 30360
attccaagac aaaggaatcg tgtattattc tagccttatt caaacttgat ttataaatca 30420
gtttagtaat ttatttattt gtttctgtat ttatttttat ttctttgaga tggagtctca 30480
ctctattggc caggctggag tgtagtgatg caatcttggc ttactgcaac ctctgcctcc 30540
tgggttcaag ctattctcct gcctcagcct cccgagtagc tgggattaca ggctaatttt 30600
tgtattttta gtagagatgg ggtttcacca tgttggccag gctggtcttg aactcctgac 30660
ctcgagtgat ctgcccgcct tggcctccca aagttctggg attacagacg tgagctaccg 30720
tgcccagctc agtttagtaa tgtataactg ggttttaccc agttgtaaat tactcttttg 30780
tcgtgttttt ttgagaactg gcaatgacgg agaaactaaa agtgccaggc tgttgccttg 30840
ttcctgttat tttgccttag tttttttttt tttttttttt ttctctgaga ctgagtcttg 30900
ttgtgttacc aggctagagt ggagtggcat gatctcggct cactgcaacc tctgcctcct 30960
gggttcaagt gattcctgcc tcagcctccc gagtagctgg gattacaggc gcctgccacc 31020
gcacccggtg aatttttgta tttttagtag agacgggatt ttaccatgtt ggccaggctg 31080
gcctcgacct cctgacctca tgatccacca gcttcggcct cccaaagtgc tgggattaca 31140
ggcgagaacc accgtgcccg gtcttgcctt agttatttct tgttccctcc tctagtccta 31200
tagttctctg actgtattga ggaaatgtaa ttaaatatta ttatgttaat agatatttat 31260
gtggttgaat attagaaatt ccttattttg gtcacatatc ctgatcagta gttggtcttc 31320
tggagatagt gatttttcac tagagatgac tttaggacct attcaggttt tttttaagat 31380
cccaatttaa ggaaagacta ttctcattat tgattttgct atatgcaggg aaatttattt 31440
cgaaaggttt ttcagttggc ttttagggaa gattatatat tctctttttt tttttttggc 31500

cttttcccac atgttctaaa aatgatatat tctttaactc ctatgaaaat acattgtttc 31560
agtaattgaa gatgctgatt aaagtcatat ctctacacat tttttaaaat ttgagataga 31620
tgggactttg tcccttctta caccattcac ttattcactt ggaaaaacta ttatccaata 31680
cttatgtggc agacactgtt tctggcacaa gggattcagc agtgaacaaa actgcctttt 31740
tggagtttac attctactag tggaaagcga caacaagcag atagacacat tcagtatata 31800
attcactgtc agatggtggt ggtaagtcct atgtaggaag aaaagcaggg taaggaggct 31860
tggagtaact ggagtgagtc atagatggac ttgtcaggaa agggtttctg aagaggtggt 31920
atttgggcag agatctaaat aaaatgaagc aacaagccat gagaatatcc gggggaaaat 31980
gttctgggca gaagcatcaa gcatagaact tgtggtatga tatttattct agcacacatt 32040
aattttaaaa atgtataaaa gacatccatt taatcatatt aaagatttcc atgattcatt 32100
tagacttagt cagaaaccaa atttatattt tctttttaaa taattttatc tcaactctta 32160
ttttacccaa taggggccag agttactcag caaatacatt ggagcaagtg aacaagctgt 32220
tcgggatatt tttattaggt tggtagccta tgaatgtttt taaagtaact gactctgtta 32280
ttatttatca atcagtgctt tttttggtct tgtttttttga agaactgata tttgaaacct 32340
gtggtttatg tgaattatta ataagctaga ggacgtggat tctctatttc atcaaataat 32400
acaaaacatt ttagatatta aattttggaa attatttggt tttgttttac aatagaaata 32460
ctcctcaaag tggaatcgaa gtggttattc aaagaaatct cagagtagat tcttatatga 32520
agcaaataat tgcccctaat ttatctctaa atttgtaag ttctaaattc tttttcccc 32580
cagtttctaa tttatctctt ataagtcaag agtccatctg gccaatttaa tttcagtgag 32640
tgtaactatt ttgcatatat taaaaaactg tatatgaata cagaagatgg tatttaagga 32700
tgaaaataat tattcaaatg tgatagcatt atgggagtt ttaaaataaa agttactgtt 32760
ttattcttcc aaaaatttta ttataaagta tacagttaag agaatataca taaaatacat 32820
atgcagctta aggaagaata ataaaatgaa tacttcatgt attcaccacc gagtttacca 32880
ggaaaaagca taaacaaaat aaacctcttc cacgtaattc ctgggttaaa gagaagttat 32940
agtggaaaat atttgggagc aaacgataat gaaaatacta tccattaaaa ttgttagatg 33000
ttgcaaaact gatttcaagg aaaatttata gtgttaaatg tttagaaaag aaaaaaggtt 33060
agaagttaac cacttatgta tctatctcat gaaattagga aaattataga tataaactaa 33120
aaaatatgtt aaaagggaaa taataaagat aagaatgaag tttaatgaaa cacaaaacag 33180
agaagctcac aaagccaaga tttatttttt gaacaccgag tacaattgac aaatctctaa 33240
caagtttgat taagaaaaaa gaaagcatga ataaacaatt ttagggataa aaagggaaac 33300
atcgctaaag atatcccaga aatgtaaaag ataataaggg aatattatga aaatattcat 33360
gccaatacat ttgaaaactt aggtgacata gacaaaaaca aaattgacca aaattgagca 33420
aaaaagaaac aaaatctgag tagtcctgta acttagtaaa aattgagtta gaaaagttaa 33480
agaagtcttt acacaaatca aacatcagac tcagttttct aggagagttt tgccaaacat 33540
tcaagtagca gataattctg gtctattttt ggccccagaa gatatatttt acttgccatg 33600
catttaatga gatagctgtt gatttttttc aatcaccgtg acaggtgttt tatattaggt 33660
gttattcgcc agacatctag tccacctgtt gccagatatg gaattaatat tcacttattt 33720
tgaattaaaa tttgttaata aattaataaa acaaagtcaa agttcaaatt attaaaaaag 33780
taaaagaaat aaaatatatt ttatagagag cccttacaaa acagtaccaa cataatgagc 33840
tttccaaatt ttgaatgggc aaaataaatg aataggcatt tcacaaaaga aggaagggtg 33900
gccaataagt atatattaat ataaaaatgg ttacttgtaa taggaatcaa aagtgtttga 33960
cttattgact aagagtcagt ttttgttttg atccctgtta gtctatccag aaggcatggg 34020
tcttaataaa caccttgacc tcaacagttt actgaataca agggtaattt catatgcctt 34080
gccttcttta agggtttgtt gtaaagatta aaataaatac ataaatatat ataaatacat 34140
ttatatgtat ttatatgtaa ttacatacaa cttgccttct ttaagggttt gttgtaaaaa 34200
ttaaaagaag tatataaata tatataaata cataaaataa atacattcat atatgtatat 34260
gaaatcactt tgccaactat gaagcctgat tcaaatatga aatgttgttt gtttttccca 34320
gagcacaggc tgcaaagccc tgcattcttt tctttgatga atttgaatcc attgctcctc 34380
ggcggggtca tgataataca ggagttacag accgagtagt taaccagttg ctgactcagt 34440
tggatggagt agaaggctta cagggtaata attataaata cagaaataga atgttataac 34500
aaaatgtcat catgtcatca gattttggta aaaaaatgtt ctttttttcct ctaggtgttt 34560
atgtattggc tgctactagt cgccctgact tgattgaccc tgccctgctt aggcctggtc 34620
gactagataa atgtgtatac tgtcctcctc ctgatcaggt gacaatttca tatttagagt 34680
ccaaaaccca acaaatgcta cactctttcc ttgtgagctt tacttctgcc aggtaatggc 34740
aattgtcctt agaagaccag ctttcttagg gaaaagcttt agccactgtt tgctcaaagc 34800
ataaaaagat tctgaattag atgcaaagcc tttttttggc ccagtgcaag tctgaaaact 34860
ttgtaatcct tctgtgttgg ctgattgggg aaaaaaaaat gcaagaaacc taatgtatta 34920
tattttcaca ttatcttctg ttcaaagatt acatacttcc attatcctgt caaaaaaaaa 34980
actctgatac agaatcaagc atgtgaatcg taagcatgta agcaggtttc atagagataa 35040
tttttcaact cttccttgtc ctgtgttgtt ccaactctta ttctccaatt tagaagcaaa 35100
caaataaatg aatgaaagaa cagatagaca aatgaatagt caaaggtata aagtatctgt 35160
atatatgtta catgtagcta ttatttaaat tatttagatt ttccttttga aataccttct 35220
tggcacactt gcctaaatct agaaaataag cactgtgtga ataagaaatt atttacactg 35280
aatattttgt aggtttttgg gtttttgttt ttcagacaag gtctcacttt gtcacccagg 35340
ctggagtaca ctggtacgat cacaactcac tgcagcctct atggcccagg ctcaagcaat 35400
ctccccacct cagcctcccg agtagctggg accacaggca cacgctacca tgcccagata 35460
attttattat taatttttgt atagagatgg ggtctccctg tgttgcccag gctttcttga 35520
actccagggc tcaagtgatc ctcccacctc aacctcccaa agtgttggga ttacaggcgt 35580

gagccaccat gcccagcctt aagagtgttt gattttcatt cattttccta tatatattat 35640
ttctgttggg gaaaaaattc caaggaagat aaatagtagg ctgttggtac atttctcaac 35700
ttacttataa agctttttag atatataagg ttaatttatg aagaaaatca taagatacac 35760
aatttaagat aatattttta attttatttt ttatttgtta aataaatttt tctcctttca 35820
ggtgtcacgt cttgaaattt taaatgtcct cagtgactct ctacctctgg cagatgatgt 35880
tgaccttcag catgtagcat cagtaactga ctcctttact ggagctgatc tgaaagcttt 35940
actttacaat gcccaattgg aggccttaca tggaatgctg ctctcgagtg gactccaggc 36000
aagttatatg aggaagttgt tatgacattt tatgagtgat aaaagaagta caatgtcaaa 36060
atttccacct taaaaaatgc tattttttaa acaactttgg taaaactgta tagaaacata 36120
aatttacctt tagttgaatg ttccatagtt ggaatatggg ttttgcagag aatttataat 36180
tatgaagttt gatgtctgtt tctttaacat taccttaata ttggcaaaaa catgttggtg 36240
tttgcaagga tattatttaa attgggatac catgaattaa atactacaaa caaaaataat 36300
tagagttttt tgtttgtttg tactttaact tttaaaaaat aatcagttaa agttgttgtt 36360
ttgaagctca cattgttcca atctggccaa taggagcccc ttttgtatgg ctcctgtatc 36420
tttatgacat gtcctcatca ttcttgaatc acttcctcac ttccagatac agtaagttat 36480
tcttggccag gtgcagtggt tcacgcctgt aatcccagca ctttggcagg ccaaggcagg 36540
aggatcattt gggcctagtt tgagaccaaa tcatggttgc acaaactgta cccactatgg 36600
acaacagagt gggatcttgt ctctgtgaaa aatttaaaaa ttagctgggc atggtggcac 36660
atacctgtag tcctagcttc ttgggagagg ctgtggcagg aggatcgctt gagtaaatcc 36720
aggatgcagt gagccatgct tgtgccactg cactccagca tggatgacag aatgagaccc 36780
tgcccccaaa aaagaaaaat attcttggtt tatcttgtac tttctgtatc ccagccctag 36840
catcagcctt ttctctaaag acagtattat gattttaata tttacagtag atatttgaac 36900
tgttacatta tagactttac catatatttt ctaggaagga ttattctatt actcttcttt 36960
accacatttg tttggaatgt ctacagaacc tacagtttct aaatcagaaa ctccctaggt 37020
ttttgctatt ttggcaagcc attgaagttc ttccctctcc ctttactacc agaaaggtgt 37080
gtatttgtag agctctctat aatgagaaag cactctataa catggttgat tcatcatttt 37140
ggagtagaaa agtatgaatg gaaagtcaga gacataaaaa taaagcccag aggtctgagt 37200
cttagcttca ttacagactt tcttggggga tggttggtaa attatctaca cattctatct 37260
tgtctttata attttaatag ttaaattttt accatgtgcc tcaaaaccgt tagagaatta 37320
atgagctctt tgaaaaatgc ttctaagttt cttgtattgc tctaatagaa tgctatctat 37380
gttattattt atttctgaga ctaaaattgt ttacatcttt aaactggttg tccttttgtg 37440
tattttagga tggaagttcc agctctgata gtgacctaag tctgtcttca atggtctttc 37500
ttaaccatag cagtggctct gacgattcag ctggagatgg agaatgtggc ttagatcagt 37560
cccttgtttc tttagagatg tccgagatcc ttccagatga atcaaaattc aatatgtacc 37620
ggctctactt tggaagctct tatgaatcag aacttggaaa tggaacctct tctgatttgg 37680
tatcttgtgc agtcatcatt atacagttct gaaatataaa gctatatgtt ggtgtaaagt 37740
tgcagtgatt tctctcctaa ccagccccac atattcttcc tggttggttg gttcttcagt 37800
aaaatagtct tgtttcttgc ttacactaat tggtaatttg cattccttgt taagattttc 37860
aagacagggc tgggagcaag gaaccaaagt agcgcgtggt tgtgattacc tttggtttct 37920
ttgaggtttc tcttacctag tggctttaaa acatctttag gagcagttcc attttatagt 37980
aaacttaaat tctgttatca tgaacagttg aggataatga ataatttgat acaataatgt 38040
aagaaattcc tgaaaacaaa gtgttatctg tgatactttt gctgcatagt aagcacaatg 38100
aagtgtactg ataatgtttc aacaggaaag tgttttgatt aaatgtgggc agtatcactg 38160
ttctactagc attcaacatc tcttctaaaa attaatagtg gttcactgta attttattgg 38220
tacatgtaac atctgtacat gtgtttggtt atctatatgt ttcctggttt tttgtacatt 38280
tgctttatta atttaggctt tttttttttt ttttttttga gacagtctca ctctatcatc 38340
cagactagag tgcagtggca caattatggc tcactgcagc cttgacctcc tgggcttagg 38400
tgattcttcc acctcagcct cctgagtagc tgggactaca ggcacatgcc accatgccca 38460
gctaattttt gtatgttttg tagagacgag gtttcaccat attgcccagg ctggtctcaa 38520
actcctgggc tcaagctatc tgcgtgcctt gacctcccaa agtgctagga ttacaggtgt 38580
gagccactat gcctagccta actcagactt taaaaatata aaagcaattc atttttattc 38640
ccaagaacag taaggtggtg gtttaatttt agtctttaat tctgttttta atttattcta 38700
tttagaaatg tcccagaaac ttagtataac tttactttct gaaaatgaag aaacctgtcc 38760
ttgggcatta gtgtgttgga tttaagcaac aaagttaaaa aaacctaccc tgtgttatgg 38820
caattttcac ttgatggtgg ttctataaca caggtatcag tgaaccttta taaaagatga 38880
acaacttttc agcttgctta atttcagtta attaacatgt atacttatct atgttaatgt 38940
tttattgctt aaaatgttta atttttatat ttggtaaaca gatagttttt tctctccccc 39000
tcttccttcc atctttcatt actacaattt accatgcaga gctcacaatg tctctctgca 39060
ccaagctcca tgactcagga tttgcctgga gttcctggga aagaccagtt gttttcacag 39120
cctccagtgt taaggacagc ttcacaagag ggttgccaag aacttacaca agaacaaaga 39180
gatcaactga gggcagatat cagtattatc aaaggcagat accggagcca aagtggagta 39240
tggctttttc cccctcatta taattgttaa aacttcttaa aaattgtttc acccttttga 39300
tatatatttc tttgacttat aaacgagcta tatttataaa caagggacca gaacacatta 39360
actcagtcat ggttatgtgc ttccttgctt tcaatgtttc attatcttat aaggaagaga 39420
acgtatggtc tcttgaaaaa actgacaata agaagtaaca actggactac cacatttttt 39480
tttacatcct taatttaact cttcgtcaat ttcttttttt acttaaggag gacgaatcca 39540
tgaaccaacc aggaccaatc aaaaccagac tggctattag tcagtcacat ttaatgactg 39600
cacttggtca cacaagacca tccattagtg aagatgactg gaagaatttt gctgagctgt 39660

aagtaacaga ttctgttttg gaagtacagc tactattaca agtgacatag tattacactt 39720
aaacctttaa agttcgtgtt taaaataaaa atattttgaa tatttaaaag ctaattcaaa 39780
aaatatgtgt cgtagctatg cattaaaaaa ccccaaaatg tcagaagtac agaagtcaaa 39840
attgagtttt cattaaccag ttcatttgat tatatttgaa ttattcataa tggactcatt 39900
taattttagt aactttgggc tgggtgctgt ggctcatgcc tgtaatccca gctctttggg 39960
aggccaaggc aggtggatca cctgaggtca ggagttcgag gcaagcctaa ccaacacggg 40020
gaaaccccat ctctactaaa aatacaaaaa ttagccaggt gtggtggcat gtgcctgtag 40080
tcccagctac ttgggaggct gagacaggag aattgcttga acccaggagg tggaggttgc 40140
agtgagccga gattgcacca ctgcactcca tccagcctgg gccacagagc gagactgtgt 40200
ctcaaaaaaa aaaaaaaaaa atttagtaac ttcgaagaaa taagaaggaa aattaaaagt 40260
tgaaagtgat tctaatgtat agtttataaa attttgttat aaaaatacct gttttgcctt 40320
caaaataatt tatattaata ttttattgac ctcaagaaca tttaaataca ttcagattta 40380
ttcatttgtg gaccacattt gttatacatt ggatttaaag gatccttgca attgagttta 40440
tggccaccta tgcatctgag acccatggac tgggaaccat tctaggtcaa tgattcagtg 40500
tgattcaatt taagagatgt ttattcctgg tctttagaag ctgctacctt ttgttatcta 40560
attttgcagt actttgaagt atgtatgtat gtgtacatac gttagtgcta tgtatttatt 40620
aaagaagaat cagaaaacag aggtaaggaa aaataaggaa acaaatttct gttaagccca 40680
ccacctccca aagcatattt gtttatatgc ttatatatgt tttcctatta tggtaagaac 40740
agtctgtaca tattgctata tagcagtccc cctttatcca catacatcct gaaaattgtt 40800
ttacatttta aatgttaact actttattgt ttttaaatgt cattttatag tgtagctatg 40860
ccacaatatc caatttttag acatttaaat tgctcccagg caatgtggta atgaacattc 40920
ttgcagctga atatatgcac atatctaatt gtttcactag gatagaggtg gaattgtata 40980
acagggagct cacatttttt aaggcttttg aaatgtattg ccaaattgcc tgccagatat 41040
actgcaccat cactaacatt gtgtgttgca gtatttttct aaacttggcc cttttgattt 41100
tagaaaaatg atatcaataa tttacatttc tttgattaaa gtgtagaagt tataattttt 41160
catattattc attgtcattt gtattttatc ttttctaact tgtctcttca tcccctttgc 41220
tccgttttct attggagtgc aactttattt gtaagaattc tttttaattt ctgtgactgg 41280
aatttttttt tctagtttgt tatttcccgt tcatttctta aaatataatt gtgtttgcca 41340
acaatccatt atcttttgtt ttgtaatggt agtatttata catattaaat tatctctttc 41400
tttttttcaga tatgaaagct ttcaaaatcc aaagaggaga aaaaatcaaa gtggaacaat 41460
gtttcgacct ggacagaaag taactttagc ataaaatata cttctttttg atttggttct 41520
gttaagtttt ttgatggctt ttccatatgt tgtaacagga aaaaaatggt gtctatgaat 41580
ttcttcttaa tttaacaaat ttggttaatt tataaaatca cagattggta aatgctataa 41640
ttatgtaatg atcaggattg agattaatac tgtagtataa attgggacat tataacagat 41700
tccatatttt atttcctaaa atctaaattc agtctttaat gaaataatat tagccaaatg 41760
gtggaactaa tttatttctt ttgaggaaaa gataataaag aatgtaatta aatttaaatt 41820
tcttggaatt cccagttgta tattcatcac ctttgtagca tttgacaaat tttatgctta 41880
gcagcttctt cactgttttg aaataaaata tcctattacc tactgataca attatctgtt 41940
ctttgtatat caaaaaatgt gaaatttaca cataattcaa atacatttaa ttatccgctc 42000
aaccagaaat gaaatcacat ccctctacta tactacatcc agctccaagc ccaagatatt 42060
taaatgacat ccattcctct cctagttcca gttatgattt tatcttgata ttctctcata 42120
tatgaactaa attataaagt tagccaccat caatacaatc tgcgtatcta atatcttaac 42180
tatatagtaa tggggtaagg gaacagcaaa aaggagaaca ttaattaaaa tatacaagta 42240
agcctgggca acatagtgag accccatctc ttaaaaaaaa aattagccat gcatgatggt 42300
atgcctctag tcccagctac ttgggaggct gaggtaggag gatcacttgc tcccaggagg 42360
ttcaaggttc taaaccagca aagctcagaa tcccagggga tagaaacaaa gacttagtgg 42420
atcactagta ttaaactgag acacgtcacc ctgcattgca ctttgtttct cagttctttg 42480
atgaaatcac tgagctgaca tacctgccct cttttcacca taaagtgagt ttcatgatca 42540
gaagcaatgt ctatgggata gcctaacaaa caatgtaaaa accatttagt aagttcatga 42600
agggtggtgg tggtaaaaat ttggagaaca tacaaaacaa atacaattcc aaggtgtgtc 42660
ccctccagga aggacaaatt gctgcctgct ctgtgataga agaggatcag atgtaatcaa 42720
cctgccgtca gacttgggct gttctctcct gggtgtggac ttgcctggtt ggtcactgct 42780
gctgacaagt aggctgtcaa tatagctggg ttgtcatgtc agctgtggtg agggggaagt 42840
ccacattgtg gaggccacat ccctgcactc ttggccaatt tgaccatgaa tcttaagcac 42900
tggggtggct ggaaaagaca gccgattgac atccatacag aggtcatctt gaccacttga 42960
ttagtataag cactgaaggc ttttaactga gcattcacat aggacacaaa tattctgatt 43020
ctttgggccc attccaagaa ctctgggcat acttttcctc cagacctcat acccagttgt 43080
gttctttcca aatttctggt catctggtta tgttattagc cactatctgt gaatcagcat 43140
agatttttat atcagacatc tctacctcct gacagaatgg aggagatatg ttacttaaca 43200
attctgttcc cttggaagat ttcctgtctc cactgtttgt aagggctact ccctcaatgt 43260
agcagtaatg ctttcactct gatgggaagt cacagtggaa ttctgggtct ccaagaatta 43320
gtgttagtgc atacacagtg tctgataatc cccagagtgt ctggtgccct tggatcctgt 43380
gaagaaggct tggagaaaag aagattcatg gcaagaactt gtgatgtgat gacaggcct 43440
tttctctggc tcttcattct tagtctgacc taggtgtgag aattaggtca ggggccatga 43500
ctatattgtg gtgactcaaa ccaggccttt gtttactaac tgggagattt ttacattgta 43560
agaatcaagt aggatctttg cccatgtatt ttggtcttaa gaacacaaat gatatggctc 43620
caatgactgg aggaacacca gggtccttgg tctcacgctg atttagataa aacgactgtc 43680
aggcctctga gcccaagcta agccatcctc ccctgtgacc tgcacgtata catccagatg 43740

gcctgaagta accaaagaat cacaaaagca gtgaaaatgg cctgttcctg ccttaactga 43800
tgacattcca ccattgtgat ttgttcctgc cccatcttaa ctgagcgatt aaccttgtga 43860
aattccttct cctggctcaa aacctccccc actgagcacc ttgtgacccc cgcccctgcc 43920
cctaagagaa aacccccttt gattataatt ttccactacc cacccaaatc ctataaaatg 43980
gccccacccc tatctccctt cgctgactcc tttttcggac tcagcccgcc tgcacccagg 44040
tgaaataaac agccttgttg ctcacacaaa gcctgtttgg tggactctct tcacacggac 44100

<210> 64
<211> 16869
<212> ADN
<213> Homo sapiens

<400> 64
aagctttagt agagatctca aaaatggttg gatggtagca aattactaag aactctcaaa 60
gtttctaaag ccttagtttc agcttgctag aaaacctatg ttgagtatta tggctagttc 120
catagttgag ttgggaaatg tctttgagga gacacttttt cactttgtat tcatctgtac 180
attttctgtt acttgcattc tgtcatgctc aggctattag agcaggtaca tttttataac 240
tggaatgttt atgtgtagtg aagctctgag aggactttgc attagatctc agcagcataa 300
tcagaaggtt gtcctttgtc tcagcaattt ttaagctaat agtagcagaa attgcagtgg 360
aaatagactg ctttgccaca acattcagaa aatcatttat ctttttattg cagttcttgt 420
caccaaacaa tacattttag tacttctcaa attgcagaac tctcataggg ctgggaaaat 480
gcctgtagac acatacatac tatgaatgtg ctaatgtttt ttgtattttc atagcccatc 540
aaagctcctg agtcagtttc cactataatc actgcagaat caatcttcta caaggtaagc 600
ttttgtagag ttactgaagg aagagttggg cctagtgggt aatgtgccac taaaatgttg 660
gattagtcta aaggtctctg ctactcttta tttgtataag gtgtgattat actttttgtt 720
cccttcttag ctgttttccc ccataagtgg ctgttattaa aacatctcat ctagagctga 780
agtgggagga gaaagtgcct actgacacat gatgtgagga tcttaagtat ttttttttag 840
tgtagattgt aggaattatt cttaaaatgc tgattgtata gtgtggagcc atggaagact 900
gagccgttag tgcgatggca ttgaagaatg agaaggacag agacaggatt tggactagta 960
gaggttgtcg actgtggtgt caaatgggta gagtaggccc agagattcta aaatgccttt 1020
aagtggagtt gagctgagta agggcagtag tgaggattaa cacctactag aaattcatag 1080
tgagaggaat tccaagatgt tttgataaaa gaatgaggag gtcaggtttc ccagggccaa 1140
agtccatgaa catctgatac ctcagtgaga gaagtgacag attgttgtgt ttaaaccaga 1200
agtcttagga aaggaattag aacatagacc cccaaggctc ggcaggcctg gcacggcaca 1260
ggcagcaacc attgaaggct atttggtgtt tcgggatctg aactgtcatt taggggacag 1320
tggtgtgagt tagtacttta tacttgaccc aggtggactg agaaactcaa gtgatgatgc 1380
ccttaagtat actttttttt aagcccacaa tctatatagt cgaagtctgt tcctcccaac 1440
aggggtacac tggcattcct cagcagggct gggaaaaacc aacaacaaaa aaagtctgta 1500
cacaggcaaa catctctctt atttttccaa catttaatac attgttaata aaatatctaa 1560
agtttagcaa acagttgctg tgtatcagtg gctgagcatt ttgcatgctt tatttcattc 1620
agttcactct atgaggtgga tactactatc cccattttct agatgagaac attgaggcac 1680
agcgaggtta attaacttgt ccaagatcac atagccaaca agtcatggag tgaggcagtc 1740
tcatgccaga gcttaagcct agagcatagt tcctggctct acagctttag caagtgactg 1800
gctatgtgac gaggaccaac ctctctaatg tctcatctgt aaaataggaa ttgtaaatag 1860
ttactacctc agtgggtcaa atgaaatcat atgtgttaag cacttagcag agtaagcact 1920
caatgaatag taggagttat cacatcttcg tatttgtgca ttaccttcac agtttacaga 1980
ttaaggccag aagcaacttg ttgagctacg ggtttagtgt actaacagtt tccatgtgtg 2040
tctccatgga agggtgtgtg ggacctgtta ttgtgactgt ctgtactttc gtattgttgt 2100
ctgccaccca tgtttattaa atgataagga caataatgca acaaagtagt caagtaatgt 2160
tgcaaatgcc cagtattgta gtggctatca cagcagtgcc actggcaggc agcaccatgg 2220
tggcaagttc aagaggtcac tgccagccac tgagctagag cccagatcag gcatgcaaga 2280
ggagcctgag tgggagccac tggggatcac ggccaagagt gtgaccaccc aagacccaga 2340
atggctgagt ggcctccctg gagcatggca gtggcagaac aactccatga actcagatct 2400
ggtgatgcct aaactagtgc tgttctcgtg tggacccctt ttctctacca gaaaccttga 2460
atcctctcag caaatgagga gactactcag atcagtgact tagtcctgtt tggtgttata 2520
tatgtgtaca caacacagca catattaata aatacctact atgtgccagg cactgcctac 2580
cactggaatc tttcactaag acattgtttt tactttgcat ttctgccttt acactatgaa 2640
agtagatgtt ttggattcat attcattcag catacatttg aatatgctgt gttatgcata 2700
gtaagcctat gataagcaag tattctcatt tagaatttgg gaatattgat tatacatgtg 2760
gacaaacaaa ccataaatgc aaactattta tatgataaat aactttggac tgatggctgg 2820
gaggaaggac cagctattga tgggtaggaa ctagcaagta gcggactgtg gcctgcatag 2880
accagaccca tccgtagtga tccagatgaa acagccaccc tcagacactt ggataaaggg 2940
tccaccagga aaaaactcct ggcctatcag gtgctatgtt acagttcagt tactggaagt 3000
atttcctcaa aagtgttttt atggttgagg tacacattcc tacagcttta cctgctgcca 3060
agtccctgtt tcaagggaag cagcaatgaa ttacactgtt cccgtagtca aggacagtat 3120
atcttaccaa gaactatacc cacttaagga ggtgctggat gtcataaaga tttggatcaa 3180
ccattatggg tgttcagagg agagattatt tccagctcaa gacccaggga agaggacata 3240

ggatggatac cagagtcata gggaggattt aacacaggac atgtacacat tagttagttg 3300
ggtataaagt ggaacagaaa tgaatgagac acaaagcctt gaatgccaga aatactagta 3360
gtcctgttgt ggaaggatat aaaactcaac tgggagtgga agagaaaggc agcagtgagt 3420
ctaggagatg tacagtaggt tgaggtaaac atatcctgaa gactataatc caaagattat 3480
ttttggtttg aatttgtttt ggtttgaatt catggtatct attttctttg agtggatggt 3540
tggggagggt ggcatgtaga atgcattctt accaaatcag catgattttc aagacagtac 3600
agagaaaaga ctgctgagct gatgtaggag ctttggctgc agtctctatg gctttcagca 3660
agccgtttaa ccttactact gcttcatgac tgtggctaac aaagtaggga tagtacggag 3720
cacagaggat ttttagggcg gtgaaactat taatactctc tttgtatgat actataatgg 3780
tgggtacatg tcattataca tttgcccaac cccacagaat acacagcacc aagagtgaac 3840
cctaatgtga actctggtct ttgatgatgc tatgtcagtg tacgttcatc cgtgtaacaa 3900
gtgtaccact ctagtggtgg gaggggttat tgataatagg ggaggatgtg catgtgtggg 3960
ggcaggaagt atatgggaaa tctctctact tctgctcaat tttgctgtaa acctaaaacc 4020
tctgtaaaaa ataaagtcta tttttttaaa agtggggatg gtattacggc aatataaaat 4080
caaaatactt tatgaacaaa tcttttctcc agatgtaaac tgtcatatat gcaccctcgt 4140
atgtgtatgt ataattttca ttcaaacgtg aaacaacttt agaattggca ccaaacatat 4200
aaacactgat acattagact atctcgaaca ccttttactg accactttga aaacttgctt 4260
acctattaag gttcattcat agctgtgatg ttctattttt attttcaatg tgggattatc 4320
ttctgtttcc cccagggagt atattaccaa attggtgatg ttgtttctgt gattgatgaa 4380
caagatggaa agccctacta tgctcaaatc agaggtttta tccaggacca gtattgcgag 4440
aagagtgcag cactgacgtg gctcattcct accctctcta gccccagaga ccaatttgat 4500
cccgcctcct atatcatagg taagtttgac aaatggcaca ggtttttttt taacttagtt 4560
aactctccaa tattatgtaa aagagtgtgt tagtcagctt gggctgtcag gacaaaatat 4620
cacagactga gtggcttaaa caacagaaag tcactttctc acagttgtgg aggctgaagt 4680
ccaacatcaa ggtgctggca acacggattt ctggggaggc ttttcttcct ggcatataga 4740
tggtcacctt cttgctgtgt cctcacatgg cctttcatgg agtgagagct ctttggtgta 4800
tcttcttata aggacaccat ttctgtcaga tgagggcccc acccttatgg tttcatttaa 4860
ccttaattgc ctccctaaag gtctcatctc caagtaccat cacattgggg attagggctt 4920
caacatataa atttggaggg tggcgggggg ggatgcaatt cagtccataa caaaaaaagc 4980
atgagtatta ttaagtacaa aaaaattaga gagctttata gaaaatatga ggcattttat 5040
gtagctggag tgtgagtgct atcagttatt ttgagttaga gcaatgtgca tctactaaga 5100
agtggtatgg ataagatttt tttggagtga cccagggtta aactgtacta caagaatgta 5160
ttgctcagga actaggttat ttaggttact tatttataca aacctattca aaaataattt 5220
aggaaagaac tatcccagtt atcccatact tgcaaattct caatatgtgt gcctctgcat 5280
gctacacatg tcatcttagg cctttatagt ataaaggctg atagttgaaa tggcagctgc 5340
tgtgcttttg ttaatttcaa agctgccaaa acagttgtga gatagactca caagaattta 5400
ctgattaata caatttttaa agttttcaga tttttacagt tacttcagac tttttatctt 5460
tctgcagtga gcatgcatca ttacttttgc atcctgagaa caagcataag tgtgtttttg 5520
gagagaactc cagggacaaa taatatacca ctgttattct cacctatatg tcaagtttga 5580
tacattacca aacaattcta gccttctgct tataagtata tagaattttt atttacctta 5640
tctatggatc aggatctcag cagaggcagt gatgtatcag aatcaccttc gggattcctc 5700
tactgcctcc tctttctaat ccccagattc tgatatgcat ccttgtccta cagcgaggca 5760
gcatggcatg aggtcagaac accagttctg gagccagact gtctaggttc acagcctgcc 5820
atttaccggc catgtgactt tggcaagttt cttagtctct cttgcctcac tttcctcata 5880
tgtaaaatgg gaataataat agtgcctacc tcagaaggtt gatgtgagga atgaaggtat 5940
tgatacatgt aaacttagag cagtgtgggt acaaaataaa catgatgcaa gtgttcaatc 6000
actgtttttg ggagaatgcc atattcttta agccgttaaa gaagaaaaaa tgattaagaa 6060
taatttcaaa gtaatgcatg tttcaagggc taatgccagg ttgctcccag agtggtctct 6120
cccagtgtct agaaatttta acatcttatg aaaatgatat atatggtcaa aaatgtattt 6180
aacctttccc ttggctgcct tccagggcca gaggaagatc ttccaaggaa gatggaatac 6240
ttggaatttg tttgtcatgc accttctgag tatttcaagt cacggtcatc accatttccc 6300
acagttccca ccagaccaga gaagggctac atatggactc atgttgggcc tactcctgca 6360
ataacaatta aggaatcagt tgccaaccat ttgtagttca caaattaaaa ctgggtttcc 6420
aggcctggtg tggtggctca cgcctgtagc cccagctatt gcaccactgc tctccaagct 6480
gggcaatgga gtcagattct ctttcttaaa aaaccacaaa aaaactggat ttccagttct 6540
ctaatattct tagtaccaca agatatgtca taggtatctt taaatgaaat tcttagctgg 6600
aaaagtgact aaaaagtttt tctcctgcta cctagtaata aacaaatcat tgtttattac 6660
tggtcactta gaaaattaaa agggataggg ccaggcacag tggcttatgc ctgtaattgc 6720
agcactttta gaggccgagg caggcggatc acctgaggtc gggaagtgga tcgcctgagg 6780
tcaggagttc gagaccagcc tggccaacat ggcgaaaccc cgtcgctact aaaaatacaa 6840
aaattagcca ggtgtggtgg catgtgcctg taatcccagc tatttgggag gctgaggcag 6900
gagaatcgcc taaacccagg aggtggaggt tgtagtgagc caagattgca ccgctgtgct 6960
ccagcctggg caacagagtg agactcttgt ctcggaaaaa aaaaaaaaaa aaaaaggctg 7020
ggcacagtgg ctcacgcctt taatcccagc actttgggag gctgaggcag atggatcgcc 7080
tgaggttggg agttcgagac cagcctggcc agcatggtga aaccctgtct ctactaaaaa 7140
tacaaaaatt agccaggtgt ggtggcgcac acctgtagtc ccagctactc gggaggctga 7200
ggcaggagaa ttggttgaac ccaggaggcg gaggttgcag tgagcagaga tcgtgccact 7260
gcactccagc ctgggtggac agagcaagac tccgtctcaa agaaacaaac aaaaaattaa 7320

aagggataga atataatgaa atatattttg aacttaaatt atattctata tgtgtatctt 7380
cctaggcaaa agctgtaatt tccagagaga ccattaggaa caggtagtat ctatttttct 7440
ccattattta tttctagaaa ctcataaaat ggattgtatt tttctataag aacaaaatat 7500
taattaaggt atagatgact gaccaagggc ttaatcaaat aaaatgacta acagcatcta 7560
tcataaagcc acacaagcct tatgttctca tctcaaaaat gctgtgacag ctttttggct 7620
gctttaacca taagaaaaat gattggtgga tgattttatt agcccaggct tttaaaaact 7680
ttcatctagg ccacgtgcgg tggctcatgc ctgtaatccc ggcactttgg gaggcctgag 7740
tggatggatc acttgaggtc aggagttcag gaccagcctg gccaacatga tgaaaccctg 7800
tctctactaa atatacaaaa attagttggg tgttatggtg catgcctgta atcccagcta 7860
ctcgggaggc tgaggcagga gaattgcttg aactcgggag gtggagattg cagtaagccg 7920
agatcgtgcc actgcactcc agcctgggtg atagagcaag actgtctcaa aaaagaaaaa 7980
aaagaaaaaa ttttaattta atccttctgt agaaacaggc attcagaacc attccattga 8040
tcttaataaa gctgctcttt actgtttcta gtcaaaaatg agacttcgat caaaccataa 8100
gattttatac tgcagatagt cagcttcacc aaagccgcag aggaaacatg tcgagatcag 8160
gcttcctgct tgatagtctc ttgactacca ttaaaacgaa tattgggagg tcatgaaagt 8220
cattggtagg ccattagcat tgatatcttt aaaacatcta ccctaaacca tctgctatgg 8280
acccataata agaggcctgt tgtatatgaa attgtctaga attcaggtgc aggtctttgc 8340
cggttaagta agggagcaac acgtaaaatg ggagaggagt ggggtgtact cacttgcctc 8400
ctcttttgtc ctgatttaac cagcattttt caaccctggg aaaatttgca gaatctaagt 8460
tgattgtaat gattttgagc tgcagcagct ttaactctta cccttttcc acatagttat 8520
ggtgtttgag ttggaaagaa acaactatag gtagctacac gtacataatt atctctttat 8580
tcacaaaggg tatagtaaaa ttgattgtaa ataactttct aagtgccaat attcaaaact 8640
tttggattaa aatgtatttt tcaccgtgca tttactttgg atgtatttat ttcatttaaa 8700
caatttaaat ggggctcttt aaccaaaaat ggtatttaaa accaaaacag tatcgtactt 8760
agaatttgga gtagaggccg ggcacagtgg ctcacgcctg taatcccagc actttggaag 8820
gctgaggcag gcggatcacc tgaggtcagg agttcgagac cagcctggtc aacatgaaac 8880
cccgtctcta ctaaaaatac aaaaattagc tgggcgtggt ggcgtgcgcc tataatccca 8940
gctagtctac tcgggaggct gaggcaggag aatcgctgga actcaggagg cagagactgc 9000
agtgagccga gatcgcgcca ctgcactcca gtctgggtga cggcatgact ccatctccaa 9060
aaaaaaaaaa aaaagatttt ggagtagatt catcattaat aagtaacaga ttttaggaaa 9120
atcaaaaaat ggctaataaa atgaacacaa tgtaaaacat ttattaaaat gtagactttt 9180
aaaaatctat aaattgatca tctgtttata aattggcaga tggttgtgta ccatctttta 9240
aaataaagat tgaatttcac ccagtgtgat ggttcccatt gcttatattt ctcctgctga 9300
ggccggacct gatatggccc tggtctgtgt tcccagcctt gtttcctcat taccactaaa 9360
atctttcccc tgtatgcccg cccaattttt ctggctctga gtccttgttc atactgttct 9420
ctccaattct accttccaaa ggcctttctt aacaccttcg gattctttct ttgagaactt 9480
tccagattcc catgcctttt tggaatcaat ctctatccta ttgtcatcac atttaagttt 9540
ctacttccat catcctcact cctatccctt tggtcctggg atgacaggga tgctgtgttt 9600
tatttactca tctttgtaac ttccacataa cctaaccccg gttcttgctt atgggagatg 9660
ctgattgtag ggtctgagtt agatactgtt aactaaaatg cttgttgata ttttagttat 9720
taattcatat taactttggc tgaaactttt aaattctatt gtgaatagtc aagtaaaatt 9780
tagattgtta cattctgggt tagtattaga ttgtttttaa gattgtttta aacaagatgt 9840
ttttaagatg agttttaaat agttctctta acacaaataa agcttaatat gagtatttga 9900
aggaaattat cccaaaccat tccagttcct ggctgtgaaa ggcttttcca ggcctaataa 9960
gttttccact tcagccgtaa gtaggtgaaa tcaaatgaac aatagaggga aatgtattta 10020
tttgctttat acacatgcat gtgtgttgtg tctacatata aacattgcac acgcttagaa 10080
tgaagtttct gtcatgccca gaaaagggag aggcattttt gtggattttg tctggctgcc 10140
ctggggatgt ttgaagaact gtgctgttta cttcatacca ggtgtgtgag ccataccttt 10200
ggtaggaggg tatacctcct acacccaaga aatataagcc aggagaaggt ctgtgccaag 10260
agaaggaacc caaatgaccc acaagaggtg ggccattaat tattgggtca gatgcataaa 10320
tgcacagtaa tttatttaag cacctcttaa tggtgaccca caaggaagat tgctcgtagt 10380
agcggaaagg ttcacaataa ataagagaaa aaagcagaat gtagaactgt atgatagcaa 10440
ttctgcaaac aagaagcatc ttttataaaa gatggaagga gcccaggcac agtagctcat 10500
gcctgtaatc ccagcacttt aagaggctga ggtggaggat cacttgagct gcagtgaccc 10560
atgattgtgc caccactcca gcctgggtga tagaagtgag accttctctc aaaaaaaaaa 10620
aaaaaaaaaa aaagacggaa attcctccag aattttaaca tgtcaacaga ggtttctgc 10680
agctactttt ttcagcttta tacttcgcag tattttccaa attttctcta acaagcagta 10740
ttttccaaat tttttacaat aagcacacac acacacacac gtttgtttgc ataagtgccc 10800
aactggtggt gaacaaccgc tggcttttag tctatacata tctagaatat tttataaata 10860
gtagttctta aacccttgaa agggagtgaa tgaccagctg agaaaataaa gtcagtgatt 10920
tcattatttt cctatattca catcatgatt ctaggaaaga acttgggagt gacttccttc 10980
agcttcagcc actcctgggc caggcgcatg cttagctctg tggtaaaggt caccagcttc 11040
ttctgcaggg tgcctgtatc atctgaattg gaggtttggc gagggtaaga gactgatgta 11100
ggttcaagtt tttctttcct gtcctccact tgaaatctgt cttcccttcc agactgcctg 11160
cgctgctgac ttaaggcccc aacaccaaac acagaagcaa cagccttaca cagagtgttc 11220
agcaagctcc aacaattgtg taaggtaaag tttcctttat agattccttt tctatatcgc 11280
tcctagtggt tctgtttctc tgatcgaatt ctggctgata acagttgctg agactctgaa 11340
agagaaggca aggaactact gtttctcatt ataaactgtt tagaattatt tggccatctt 11400

tttgctatga atatgtagtg ctttgataca ttttttaaat caaaaagtaa tgaaagagat 11460
cacataggga aagatagatt ggattatttt taaagtttat atactaaatt gaaaagcaaa 11520
gaataaaatg ggagaaacag ctccctcatg tggctgttgg caggaagctt ccattcctct 11580
ctgtgggcct ccacaggttt gctcacagca aatggtccgt gacagaaaga cgcaagggca 11640
gttgcaccca agatggaagc caccatcttt tctataacct aatctgaaag aagggacata 11700
ccagcacttc tgccatatgc tgttgggtca cacagaccaa ctctggtaca gtgtgaacac 11760
aggaccacac aagggcgtga attccaaggg cagagaccac tagggaccac ctcagaggca 11820
cagagggaca ccctatccag ctggtggcca atgtaaatta acatagcttt ttagaatagc 11880
aatatgtatc tataatctta aaagtattaa aagtacttct tgatccagta atttcatttc 11940
taagaatcca tgctaagagg atttaaaatg tggaccaaaa aatgggtata aaaagaagtt 12000
gttaacagta tttaaagttg tgaaaaacca gaaacaatct aaaggtccaa caataggaaa 12060
atgaattttg atatttttct aatagaattt tatgctgtca tcagaaatac catttacaaa 12120
taatttttaa taacgcaaaa aaaagtttat aaaatgttta gtgtaaaacc tggacacaac 12180
tacataatga ttctgatttt gtaaaaaaaa aaaacaaaaa cacacacata tacacatgca 12240
tacatatgca tataaagaaa actggaacaa acaaaataac aagcatagtt ggaattacag 12300
tcattttaat attctttatg cttttaaaaa ttttgaagtt tgtattacta gcatccacta 12360
cttacgtagt caggaaaaaa atacaacttt aaaatagata tttaggtcca aagatggtaa 12420
tctaaatggt gttacaggct gaatgtgtgc ctgatcccca tgccccaagt tcatatgtta 12480
aagccctggc ccccaaggca atggtattag gggagtaggg cctttgggag gtaatcagat 12540
ttctacgagg tcatgagggt ggagcccgca tagtggaatt agtgtccttt taggaagagg 12600
agaacagacc aaagccttcc tttctctcct cactatgtaa gaagacagcc agaaggtggc 12660
cacagccagg aagagagctc tcaccagaac ccaaatctgc tagcaccttg ctcttgggtt 12720
ctcagcatcc agaactgtga gaaatgaatg tgtgttgttt aaaccactca ggctacggta 12780
ttttgttgca gcagcccaag ctgacagaga tagaaacaac acaaggaccc atcagcagac 12840
gaatggatga tcaaaacgtg gtgaggtcgt gcagtgggat attattcagc cgtagaagga 12900
atgaaattct gatacatgct ataatgatga accttgaaaa catgttaatg gaaataagcc 12960
aaacttaaaa ggacaaatat tgtataattc cacttatatg agttagttac ctagaatagg 13020
caaattatgt catagataca gaacattaga ggttaccagg gttgtgggaa gaggggtatt 13080
gtgggtacaa attttcggtt tggagtgatt ttgaaaaaat tctggaaatg ggtagtgaca 13140
gtagtcaaca tgatgaatgt acttaatgac actaaattgt acacttaaaa atggttaata 13200
ctgggctggc gcagtggctc atggctgtaa atcccagaac tttgggaggc caagacaggc 13260
ggatcatgag gtcaggagat tgagaccatt ctggctaaca tggtgaaacc ctgtctctac 13320
taaaaaataa aaacaaataa aaaaaaaatt agccgggcat ggtggcaggc acctgtagtc 13380
ccagctactc gggaggctga ggcaggagaa tggtgtgacc tgggagtcgg agcttgcagt 13440
gagctgagat cgcgccactg cactccagcc tgggcaacag agccagattc cgtctcaaaa 13500
aaaaaaaaaa aaaggttgat acctgggtgc ggtggctcat gcctgtaatt tcagcacttt 13560
gggaggccaa ggcaggcaga tcagttgagg tcaagagtta aggaccagcc tggccaacgt 13620
ggcgaaaccc catctctatt aaaaatacaa aaattagtcg agtgtggtgg tgggtgcctg 13680
tagtcccagc tgctgggagg atgaggccta ggaattgctt gaacccagga ggcagaggtt 13740
gcagtgagtt gagattgcgc cactgcactc cagcctgggg gacagagcga gacttagtct 13800
caaaaaaaag gttaaaattg taagttttgt tatgcatatt ttaccataat ctttaaaaaa 13860
tagatatata ggagataaag tcaacagaat ttaataacca gttgtaaata gagactgagt 13920
gaggaggatg aattaaggaa gacattgagt acaacttttt ggtaggtgaa aaactcttaa 13980
aaaaatacgt gggcaaagat cctacttgat tcttataatt taaaaatctc ccagttagta 14040
aacaaggcta ggtggagatt tgcatgtgat gtgaggtgtg tgttctgttt tgtaatgtga 14100
ggactgtgag ccatctcctg gacttgaata tccattagat aattgaaaat acggatttga 14160
gaactcagga gacgtgcaat gcagtaacaa aactctgcac ctagttgatt tctgtctcct 14220
aatttaatgc ttttatggga caaactgtta ggcaggtggg caagatggac agccatattt 14280
ttgtgggttt ctggcctgtg ggccagcctc agtgctcact ctgaggtcat gtccaaactt 14340
agaacacatt caggcctacc acagtcaagg ctccctttct caactctagt cctctgcaca 14400
aatatccgaa gcctagaaat aataatcatc tgtccttgtg tcttgcatta tgaaagccta 14460
ggaaagggcc ttgggaatta agaagaatgg aaaaactggt ctaactgctg catgcttcag 14520
cttgcagggg aatcactgaa atggggacag gccataaaag gacaaccaga agagtggctt 14580
cagcaaaggc atcgtttttc agagcaagct agagaatcct gccagcgtcc tcaggcaggg 14640
cccctgggca cagaggttag gcaagggagt gtcccagcat gttgatgccc tgagcatcag 14700
aataatgcca tagaggagct tccaaagagt tcatttcagg ttttgtaagc cgaacatttc 14760
taggcaaata aaatttgatt ttgtgaataa agcttgtttc ttcaactcca gtgcagattc 14820
tcatagattg atagtggctt gtgatccaga taaagaaaac aatttttcaa agattcatat 14880
tctttgtaga tgtacggatt tagagaccat ctaatctaac tccctcattc tacagatagg 14940
aaaaatgagg cctaaagaag ttaagaaaat accatggaaa tgtcactgct gaactgccat 15000
acgtaggatc cgaaagaaat tgggtaaatg ctactgtgag aaatacagta ctaggtccaa 15060
agaatctaat acaaattaaa aatctaaatg ttatttctaa agcatccctg cacatggctg 15120
aacttacata gtttcatttt ctttcttttc tgttgaagaa gaggcaattg gctgggtgca 15180
gtggctcatg cctgtaatcc tggcactttg agaggccgag gcgggtggat cacctgaggt 15240
caggagtttg agaccagcct ggccaacatg gtgaaacccc atctctacta aaaatacaaa 15300
aattagctgg ctgtggtggc cgctgcctgt aatcccagct actccagagg ctgaggcagg 15360
agaattactt gaatctggga ggtggaggtt gcagtgagcc aagatcacgc cattgcactc 15420
tagcctggat gacaagaggg aaactccatc tcaaaaaaaa aaagaaaaaa agcaatcact 15480

aacctgtgtt gtttattaaa catgacagac tggcatgaag taattaccaa actgtaaaca 15540
aaaaagctac aatctgccag gcatggtggc tcatgcctgt aatcccccac cttgggaggc 15600
caggttgggg gatcacctga ggcctggagt tcaagactag cctggtcaac atggtgaaac 15660
ctcgtctcta ctaaaaatac aaaaattagc ccggcgtggt ggcacatccc tgtaatccca 15720
gttactcagg aggctgaggc aggagaatca cttgaacctg ggcagtgggg aggttgcagt 15780
gagccaagat cgcaccgttg tactccagtc tgggccgaca gagtgagact cggtctcaaa 15840
aaaaagaaaa aagaaaagct acaaccttaa tctcaacttc tcataacatc atctctactt 15900
ctgattagaa gagtggaagt ggggaggttt attacaaaaa gactgttata ccttacacac 15960
ttctccccat gaatagtgaa ggtgtgagtg aaaaagacag caattttatt tttttttgga 16020
aacaggttct tgcactgtca cccgggctgg agtgcactgt tgtgatcact gctcactgca 16080
gcctccacct cccaggctca agtgatcctc ctacctcagc ctcctgagta gctgggacca 16140
cagttgtgca ctaccatgcc cagctatttt tttttaagag atggggtctc actatattgc 16200
ttaggctagt tctcaaactc ctggcctcaa gcagtcctcc gaccttggcc tcccaaaggg 16260
ttgtgattac aggcataagc caccacaccc agccagcagt tttagaataa agggtgaagg 16320
tgctgttggg gaaatataat ttaaaaaaca aaatcttctc tcaacccaga aatcctctcc 16380
atgaaggcag tagagaaaga taagctttat tattgaataa aaattaaatg agaatgtgat 16440
gcacatcaca ggcactttgc taagagatca caaagacaga aggaaatttc accattttgt 16500
acagccaagc aggtacagcc cattacatgt atgttttcga gataaatagt cctcaactaa 16560
gagaacttga cagcaccact ggtcacacag ttcattctaa ctttacctga taattgatgt 16620
gaccacttgt gttatctaag atatcaactt ttcgggggtg ggggagtgtg gaaacaggag 16680
ttacttttat agcttggtgc aaggtactca ttaagattag gctgttaccc tcccacagaa 16740
actggaagat aggtatgcta tctggtaatg tttacatttc ccagatcctt gagaaagaca 16800
ttcctaggtc ataaagctga caaaaggctg attcagtttt taaatatata tatctgtata 16860
tgtatttca 16869

<210> 65
<211> 15000
<212> ADN
<213> Homo sapiens

<400> 65
gatctcttga tcccaggagg tcaaggctgc aatgagctaa gatcaagcca ctgcattcca 60
gcctgagtga tagtgggaga ccttgtcttt aaaacacaca cacacacaca cacacacacg 120
agggcctttg accactcttg agtagaagac tcgagaagaa caaagtagaa ggccagagaa 180
gaacaaagtt acttgaaaga tctcttatta aagagaatgt acaagctatg aaaaaaaaaa 240
aacacacaca cacacacaaa cctcatctgg aatgaaaaaa acataatgca tttggtttct 300
ggttccttag gctgttatgg aacaaccaaa gaacattatt ttggtttctg aggtcagaac 360
tattttattc ccctcaagca cactatgctt atggtttgag ggagaatgag aaataggaaa 420
ctaggaacag gctgaaatgg tctaatcttg accatctaat tctgcagtgt cttattctca 480
ttctaaaaga gaatggttat attcgctgtt ctagcataaa aagtaatgat aaaaataaaa 540
gatcccgtat taccagacaa taatccccta gactgtttta atgcttggtt gagtatttgc 600
ttatgatctc agactttaaa agatggtctc cccctatggt gaagcttgtt aattatgtag 660
gcatcattaa tgtctgttta cttatcaaaa ttttatcatt gttagttgta ttactacttg 720
acagtccaat ttatttaatt gaaaagattg gttaacattt tatagtcaaa gtaattgttt 780
cctgtgtttt ttcctgttta ggttattgga gtgatgagta aagaatacat accaaagggc 840
acacgttttg gacccctaat aggtgaaatc tacaccaatg acacagttcc taagaacgcc 900
aacaggaaat atttttggag ggtaagtaag ggaaatttct tcagacccat taaatgttag 960
gaaaaaatgg agctaaaaga gctgggtggc tcacctttct catcctgtgc tgagaaatgc 1020
tggggctcac ccataagtat ccagcatccc catggacaca gggaattctg aacaaatgtg 1080
atgaaaccga tgaaatgtct ggcctgtagg tggttagtga tggagatacg ggctatatgt 1140
gaatcttgat ttttgcaatt cattagagct ttgtaatgaa aggaaacagt ttgttgcttg 1200
ctttaaggat aggttcattt gcatttctcc gcaaggaagt agtaatgagt taccaagcct 1260
tagatttcac cccttttga tttcttgctg acttaacttt aattgaatgg aagagttatc 1320
acaaatgaat tatctttttg gttttttttt ttttgagatg gagtctcact ctgtcaccag 1380
gctggagtgc aatggcatga tctcggctca ctgcaacctc cgcctcccag gttcaagcaa 1440
ttgtcctgcc tcagcctccc gagtagctgg gactaaggtg cgcgccacca tgcccagtta 1500
atttttgtat ttttagtaga gacggggttc cactatgttg gccatgatgg tctcgatctc 1560
tggacctcgt gatccgccca ccttggcctc ccaaagtgct ggaattacag gcaagagcca 1620
ccgcgcccag ccaggaatga caaatgaatt accttataag taaatgccat taaggaagga 1680
tagctggaag atgggttgag gggaatggag gaccacagaa ctagtcctat ttaaatacat 1740
gtgcatggta aaatgattcc atttgacaat aggttaatta tctcatagca taaggaaaat 1800
gcttaacagt catatgcaag atgataagct ttcctatagc atccaaccaa aagatctagc 1860
cagtacaatt tcctttgcta tattagggtt agaaaggccc ccagaggtga accaattaga 1920
tggaatcctt gaataaaaca ctggattagc agtgaacaga aaaaagtcag attgctttcc 1980
ttcttcccat agatgtctca gggatattta gtttcctcag aagataaaga atttagtaag 2040
cgtttttttg tgcatactta catgaaatgt acattatttg aattctttaa aaagaaacag 2100
ctgcatgata acaaaaattg tgttatgctt gctttagctg gtatttttgc ctagaacgat 2160

tatatcgttc ggacaagaag ctattcctaa gaaacaatat ttttaatcca ggaagttttt 2220
catttttaga aatttatctt actatttccc aagcaaaaga gggtagttac agattcacta 2280
agaatcatgt gctcacaatt tttatttaat aattattcct ccttaaaata tattaatcac 2340
ctgacttaca atggtggaac catgagtgca tttttgcctt tattgtcaat aacgtcttct 2400
cagaagtgag ccacaaaggt gcatagttct tggagttaaa ggtctgaatt aagacaatcc 2460
agcataagtc tcattaatgt gtgattattt tgagaaaagg caagaagtac ctaagaatct 2520
ccccctcact gtccagttcc ctgtttcatt taaagattca ctgtaagtaa ctgaaaggct 2580
ttccttggga ggatttattt gaatcagtct ttcacatgca aaggatattg tagaacatct 2640
cgtttttgct ggcaggaata tgaacatctg ttgtgaggaa agaaaaagtt tcatgcaaat 2700
tacactgcca aagaagggat gttcaagttg agaaaccagt gacatttctt gtaactgtac 2760
tatgaatcag cgcattttaa tcttctagat aatatatgga agtgcaggaa ggtggtagga 2820
aacggtgttc attttacata tgcgttattt tattctgtgt gagtgacttc atggcaccga 2880
cattgctgtt tttaaatgag gatacagtaa attgcagtcc gaggaaggct aactggaatc 2940
aacatacccg tagctttaga aagcagtttc cgcaccagcg aagagtacaa gagcgatgga 3000
accccatgtt cctggaagtt tgcacatcag agtaaacaaa cttgaaaacc cctcttgata 3060
gcagaattca cccagccttg ttccattttc tcttaacaaa acacaccgca aaagctctca 3120
caagctgctt tgatgaagcc acatgtattt cccccttcac aatttacagg aagttactct 3180
taaaagaaag tgattctggt gtttaccgcc tgtgttaaag ggacagagtt cctttttatt 3240
tctgataacg tttgagcgaa atacagaaac tatctgtaga ctagcatagt cggtacgtga 3300
gtaaggaaaa gcaataacct gctgtccggt gagcacaaaa ttcctgctac gaacagtgcc 3360
ttactgctgc ttggagactg caagtcgcag atcacactag gtattgactg attgtataag 3420
gaaatttctt aaagtctaaa gtaaaggtgg tacctcctaa aaagagggga agagagaaaa 3480
ctttgtgtgg aaggataagg agtgtgttta tagtttcagt aagagtgtac gttttaattt 3540
ttcttcttcc tctgcctctt tgccaagtag cctgagtgca tctgttatcc agaagtagta 3600
ttactctagg acaaacttca aattcttcat tctgcgttgc ctttaaggaa caacatactt 3660
tcttcctgtt ctttttccaa aaacacacgc ctatggctct gtgtgtggtg ttttagccag 3720
cctcctccca gataaggggt tcccttccct cctttgcatt gaaaggaaag tgcaagtctg 3780
gacatgttta tcaagaggaa aagtgacttc tcagtaatag actgtcaaat tcgggctgct 3840
gcccgagtgt tcgctttgtt atggcaggtg aagttcacct ttgccccacc cagtgtttcc 3900
acaaaaaggc aaggttccaa gtattcatat gaacaagtgt tactttagga cttggagggt 3960
tgggggtgga ggatgtttgc atagttgaag ccttgggcgg gggtgtagga aacggcgagt 4020
acagaggcca tagaaaaagc taagactcag tttgacgtcg tcagccggct tggtcttcta 4080
cccagtgact caaagcacta aaagtcagca taatcggaac tgaagtcagt agcatcgccc 4140
atttgccatt cactgcagta gcaaaagtag tactctgtgg tgggttaatc ggtttgaggc 4200
agctccttaa atgaacattt gtgtttcatt tttctgttat tttcccgaac atgaaaagac 4260
gataaaactg aaatggaaaa ggtaactgac aaaagtgtgc cttacctgtt tccgccctga 4320
tttctgctga ttcaagacta ttctggctaa actgattgga ttctttttct aactaggcag 4380
taggggatca gaaatcacac acggtaccgg ctgtgtttat tctgagaggt gctggggagc 4440
tttgggtctg acttcctttt acatgcctgt cttctctttt ggacagatct attccagagg 4500
ggagcttcac cacttcattg acggctttaa tgaagagaaa agcaactgga tgcgctatgt 4560
gaatccagca cactctcccc gggagcaaaa cctggctgcg tgtcagaacg ggatgaacat 4620
ctacttctac accattaagc ccatccctgc caaccaggaa cttcttgtgt ggtattgtcg 4680
ggactttgca gaaaggcttc actaccctta tcccggagag ctgacaatga tgaatctcag 4740
taagtggatt acagaacaaa aaaataaaaa atgccagtaa tgtcggttct gcccctttga 4800
actaataaca tgttgtttaa ttatacggct ttgtcatgtg ttggatgaag taggtggctt 4860
aagctaggga ctaggaagag gaaaaacatt ttttgagtcc ctattaacta ttaggaaact 4920
tgatcattta aaagtatata tatatatgag gagctacctt gagttttgaa ttcaggatgt 4980
tacaggaaga aatatatgtc caattctaat ttatccaaaa gcagttggga gaattacagg 5040
gattggtcca gacatgctgc gtatgcaagg tatagccctc atctgtggta ctttggcagg 5100
gcttagactg catcaaaata tttatagatg tacatttgag tgtacagtta ggatctgatg 5160
tggaacattg taagatcatt gctagaaaaa ctttgtcata atttttcaat attattctaa 5220
gtgaataacc gtaaagattt tacatcttag cttccttcct tacagtaaaa aaactatctg 5280
atctcttgat cagtattata gtagccacct atcactttat cttaacaaat tctcaattcc 5340
ttaggtttat gtgcttttac ttcttttatt tgattaaaat tgctgtcatg acctctctct 5400
gcagagggct gcatcatttt ggtcattctc aagtgatctc tttgagcaat ttaagaattg 5460
ccataagatt ctaacctctg ctgtaactat ggttgtgtgt tcttggttag accactaaat 5520
cttattagca gttttaaaaa ttattccttt tggtttagaa gttaagacta aatgctgaag 5580
tttttgtaac ttttggtttt gatatcattt caaacttaag aaaacatttg aagaaaagga 5640
caaagaattt ccacttaccc tttacccagg tttaccagtt attgataagt atatccattt 5700
gctttaccag aaggctaact tgttttagtt ctcattttca cctttgagac atttggaata 5760
aatatcaatg ttaacataaa ttggaatttt gactttgatt ttaggaccaa tgaacaagcc 5820
aagtacttac cctagtcata tataatccaa ctgtatggtt atttggtatt cattccacac 5880
ttcattttac ttgatctccc ttaagattgc aagattgtgt ttgcagtttt tctgaaaatc 5940
tggggctata aaagcatcag gacctccccc gtaggggagg tcgtgtgttt ggggtcctta 6000
cacaacaggt tacccttgag cttcaggaaa agaactggct ctcagttccc cagttccagc 6060
ttaatgggtc taattaggtc ctgaccaaaa aggtggcagt tcttttccct catgtctctt 6120
cagcgctccc cgagactctg gagactctgt catatcccta gggctgagcc tcccaggaac 6180
cattcggctg ttgtggcatc tgtgtatgcc atgcccagtg ctgaggacct agtaacaaac 6240

gacaaatgca caggcacagt ggcattttg tggaactcgt attccagctg tgcgtctcag 6300
aagaagcgca cagctccctc ctggctttct taacatagtg agccacttcc acttaagggt 6360
ctccttacat tccttgagtt taatcattca tggattcaga ggaaagtctt ttgatttttg 6420
cttttcttta aacagttcat ttgaggtgac ctaccccagt gactttgcac caaccaccaa 6480
gaaacttttt tgcatgcttc ccgcaccctg tgccaatcaa gggaagggtt taaaggcctg 6540
gcgtttttat tcctcaaaga aaggttttgc acagtatttt aaggttcaag tgcttctact 6600
ttgtgttcag aagcaactgt catatatact gtgaaatgac accttttatt tatccctttt 6660
tatttatgca gtatgtcccc ttttattttg gcagaatttt ttctaaatgg tggtttaaca 6720
ttttcaagca catttcattg tccaatattc atagtaaaga atgagagtta acaataacca 6780
gtcacattaa aacaagattc ctgctgccag ttgtgaaacc ggttgtctta ggcgtggcag 6840
ctgatgattg agactgtgat caggaaaatt tccactattt catcaggcct aataggtaga 6900
ttgtgtctcc aaatgaactg tgttgggttt ccatgcttaa agcacaatag aggtggtgca 6960
agaatctcca tgagggctta aatggcagtg atggttcagg cggtagagtt tggagaagaa 7020
gggatttgaa acaaaccaaa ggaaagaaaa gtaagtagcc agaaatcaca aaatggcatt 7080
tttctaaaaa caaaggaaaa ggaataaaag aactaataag tttgaaaccc ctacccctcc 7140
caaatttggc aggggggag gtattttttt tctatctatc taactaaccc atctagaaaa 7200
cagttgacca aattatagac ttctaaatgt taatctgctt tctcagtttc agttgaaaag 7260
agactttgtt ttgcctactg cagaacttct aggttctttc ttatagtctt ggggttctta 7320
ttatagatcg aaaatgtgag tcggcataat taagccattc ggagtcttca gaagcagttc 7380
actcttgaaa tgactccgtc cgcctacagc catttaagat ttcagaacaa aaacagatct 7440
tgattttctt tttcatgtta actcaagctg ttgctgagtg ggagagtcag aaatgacacc 7500
agctccactg attactcagc tgctgaagga tgatttttta aaatgcacct ttactgtata 7560
tggacttcct aatttccacc tgtagagcat cttagggagg ctaacatgtc actctggatg 7620
ttcttttaga ataagatgca aatctatttt tctgaaggca ttagagatag caaacattta 7680
ttgtgagttt actatatact aggcactgtg ctaagtgttt tgcatagaaa gtttaaaatt 7740
ctggcttttt tgttggccca atcataagtt tcatatcagt tcaacattca aattatatta 7800
aggtacttaa gaagaatccc tggctaaatg tgaggggcag tgccacagat ggactgaaac 7860
tttatgctta ttgcacattt atgctattat tatttgttga attatagaac caagggagtg 7920
tggaagccac tggaaaaaat atgagactta gatacataat ttgagtaaaa atggctcaaa 7980
gtcatgaggg taaagttttt tgtatttcca ttttattcga gcggcatcgt ttttaaaaat 8040
cattatgaat ttgaccctat atagatgttt ccaaataatt ctttttcacc ttcataaaat 8100
tccttcctgt ggctgtgaga tgccttgcct atcagttttc aagcttagtt gtctttctca 8160
tcctttacca ttttagcttt aaaaaacaaa agtgacaatt agaacttcct gcctgctggg 8220
cctcactgaa agaccgatat tggcctgata aggagatatt tattttgttt tagtggcttc 8280
agaaatccct ctccctcagc aagctttcca tcacggcccc cccgtcagca tcttccctga 8340
tagcgttctt ctctgtgttt attctggggc ttcaggctcg cccaggagga actgataacc 8400
gctggcagga gataacattc tctaaggggc tctcaaattg gaatcgaatc cctcaagcca 8460
gtcagcctag agaatacatt taaagggttc agttctggag tttcacagag ttcatttcta 8520
gacctatcag atagcaagtg tggagttctt tctcaactaa attcaagcag agacattttt 8580
tagacgatga aggatatttg cacaaaggct tcagcatgat cccccaaacc tgctgcctct 8640
gaaggcatct ccacacattg acagccaatg ccttcagtgc gttcctaggg caggtgtcct 8700
ggcttgagtg actgtcctcc aataatcaga gctcaaacta aacatcgtat gttttacttt 8760
tggtttccag gcaaggctga gcagggaatt ttcagttttc cctgcccaga tgggtgtttt 8820
ttcctgaagg catcatttat tgtgtagcga ggagacaggg ctggctgtgg cagggatagt 8880
ctagaactgt cctcattgct gctgttccta aatagtatct ttaccaagta ataacgtgcc 8940
gtctttggga ataagtgctt tcctcttagc ctgttctgtt ttcttgggtg cgctaagtaa 9000
ttgaactggc tcaggaagta cctattgtgg tttggcagag gtgactgtca cgccttgtga 9060
ctccaggggc cagcactgct gggatcctgg ctagaccaga cagagccttg gtgaagtgct 9120
taggctgtct gcacatcgcg aggaaggtgg tattcacttc gctaagctcc ttggcatagg 9180
cagtttgaac agggctttat caaattcgta ttcaacaaga gtagaagcga aaattgatga 9240
ctgtgtatta cttgaaatga gtcttaatct ttcacattta gttctcaggg tatgctgatt 9300
tcctttaggt aaaccatgaa catcagaaag acttttatta acctatgaca gggtccccac 9360
cccagtattt ttccactcca ttaaaatgga agtttttttt ttttttttct tttttgagac 9420
agagttttgc tcttgttgcc cagtctggag tgcaatggca caatctcggc tcaccacaac 9480
ctccacctcc cagattcaag cgattcttct gcctcagcct cccaagtagc tgggattaca 9540
ggtgtgcgcc accacgccca gctaattttg tatttttagt agagatgggg tttctccatg 9600
ttggtcaggc tggtctcgaa cttccgacct caggtgatcc gcccacctcg gcctcccaaa 9660
gtgctgggat tacaggcaag agccactgca tccagcttag gctatcttac tccagcctaa 9720
acagcaattt tctatcataa ggtctgtact aatgaaaaca gaatcaccca aggctgctgt 9780
ttgttctgtc tgtgctgcca ttgtccgcat tttgctgagg aggaaacgga actgcacttt 9840
tgagtgagtg gcccagagcc ttctagaatg agagtgcgtt ggaagccaga tatgtggcga 9900
ttgtgtcgcc agctgttact caggttttct caagaaggag gagcaacttt ggcagttttg 9960
cttcagttct ctctagccct ctgtgtaatc gccccttttt ctttatttca gcacaaacac 10020
agagcagtct aaagcaaccg agcactgaga aaaatgaact ctgcccaaag aatgtcccaa 10080
agagagagta cagcgtgaaa gaaatcctaa aattggactc caacccctcc aaaggaaagg 10140
acctctaccg ttctaacatt tcacccctca catcagaaaa ggacctcgat gactttagaa 10200
gacgtgggag ccccgaaatg cccttctacc ctcgggtcgt ttaccccatc cgggcccctc 10260
tgccagaaga ctttttgaaa gcttccctgg cctacgggat cgagagaccc acgtacatca 10320

ctcgctcccc cattccatcc tccaccactc caagcccctc tgcaagaagc agccccgacc 10380
aaagcctcaa gagctccagc cctcacagca gccctgggaa tacggtgtcc cctgtgggcc 10440
ccggctctca agagcaccgg gactcctacg cttacttgaa cgcgtcctac ggcacggaag 10500
gtttgggctc ctaccctggc tacgcacccc tgccccacct cccgccagct ttcatcccct 10560
cgtacaacgc tcactacccc aagttcctct tgccccccta cggcatgaat tgtaatggcc 10620
tgagcgctgt gagcagcatg aatggcatca acaactttgg cctcttcccg aggctgtgcc 10680
ctgtctacag caatctcctc ggtgggggca gcctgcccca ccccatgctc aaccccactt 10740
ctctcccgag ctcgctgccc tcagatggag cccggaggtt gctccagccg gagcatccca 10800
gggaggtgct tgtcccggcg ccccacagtg ccttctcctt taccggggcc gccgccagca 10860
tgaaggacaa ggcctgtagc cccacaagcg ggtctcccac ggcgggaaca gccgccacgg 10920
cagaacatgt ggtgcagccc aaagctacct cagcagcgat ggcagccccc agcagcgacg 10980
aagccatgaa tctcattaaa aacaaaagaa acatgaccgg ctacaagacc cttccctacc 11040
cgctgaagaa gcagaacggc aagatcaagt acgaatgcaa cgtttgcgcc aagactttcg 11100
gccagctctc caatctgaag gtaggccttg agagagagca gtccaagggg ctgtgagtgc 11160
atgcttgtgt ttgtatttag cttgctttcc atggggtatc gattgcattt gcagtagtat 11220
gagcccccgg ttggggatag tgggtatgga ttccgcctgg cttttgccac ttctagctct 11280
ttgactttgg acaagtgact tcccttctcc tgattttctt ctgaataata aaaaaattag 11340
gggtttggac tagaagatta ggtgaaactc cctgctagcc tgtgattttt gtgcttttaa 11400
gaaaaacacc attctgaaaa catgaagatt tcttcttttt aagactgtct tgatgctttt 11460
cttaagatat ttgcatcaac acttgagtct tggagcagaa atgttaggtc tcagagccag 11520
cttgagagca gagctaacac atgtggcttc ttcccaggtc cacctgagag tgcacagtgg 11580
agaacggcct ttcaaatgtc agacttgcaa caagggcttt actcagctcg cccacctgca 11640
gaaacactac ctggtacaca cgggagaaaa gccacatgaa tgccaggtgc gcagtatttt 11700
ctgggtagac cttctgacct ttgtagaaaa tgtctgtgag tcaccctccc atgtcctata 11760
tagcccgtag ttaaagccaa caccagattc tgcgttgtcc catcctggac tgatggcact 11820
atggtccttc ccagtacttt gtatctgctg atgacttgag atggcacagc cagcttccag 11880
tgggtgggaa aatggtaggg gaaataaaca gcccctcgtg tgctgtgtgc ccacatcccc 11940
ccgtttgctt aataccacac tggaggtgcc acaaggaggc ttctcacctc ctaggttgct 12000
gggcgttggc cggtaagcct gcccctcccg ttggcaactc ttaatcttct ggccttcctg 12060
tctcccttcc ctgctgtctc tctcccctac actgtaggtc tgccacaaga gatttagcag 12120
caccagcaat ctcaagaccc acctgcgact ccattctgga gagaaaccat accaatgcaa 12180
ggtgtgccct gccaagttca cccagtttgt gcacctgaaa ctgcacaagc gtctgcacac 12240
ccgggagcgg ccccacaagt gctcccagtg ccacaagaac tacatccatc tctgtagcct 12300
caaggttcac ctgaaaggga actgcgctgc ggccccggcg cctgggctgc ccttggaaga 12360
tctgacccga atcaatgaag aaatcgagaa gtttgacatc agtgacaatg ctgaccggct 12420
cgaggacgtg gaggatgaca tcagtgtgat ctctgtagtg gagaaggaaa ttctggccgt 12480
ggtcagaaaa gagaaagaag aaactggcct gaaagtgtct ttgcaaagaa acatggggaa 12540
tggactcctc tcctcagggt gcagccttta tgagtcatca gatctacccc tcatgaagtt 12600
gcctcccagc aacccactac ctctggtacc tgtaaaggtc aaacaagaaa cagttgaacc 12660
aatggatcct taagattttc agaaaacact tattttgttt cttaagttat gacttggtga 12720
gtcagggtgc ctgtaggaag tggcttgtac ataatcccag ctctgcaaag ctctctcgac 12780
agcaaatggt ttcccctcac ctctggaatt aaagaaggaa ctccaaagtt actgaaatct 12840
cagggcatga acaaggcaaa ggccatatat atatatatat atatatctgt atacatatta 12900
tatatactta tttacacctg tgtctatata tttgcccctg tgtattttga atatttgtgt 12960
ggacatgttt gcatagcctt cccattacta agactattac ctagtcataa ttattttttc 13020
aatgataatc cttcataatt tattatacaa tttatcattc agaaagcaat aattaaaaaa 13080
gtttacaatg actggaaaga ttccttgtaa tttgagtata aatgtatttt tgtcttgtgg 13140
ccattctttg tagataattt ctgcacatct gtataagtac ctaagattta gttaaacaaa 13200
tatatgactt cagtcaacct ctctctctaa taatggtttg aaaatgaggt ttgggtaatt 13260
gccaatgttg gacagttgat gtgttcattc ctgggatcct atcatttgaa cagcattgta 13320
cataacttgg gggtatgtgt gcaggattac ccaagaataa cttaagtaga agaaacaaga 13380
aagggaatct tgtatatttt tgttgatagt tcatgttttt cccccagcca caattttacc 13440
ggaagggtga caggaaggct ttaccaacct gtctctccct ccaaaagagc agaatcctcc 13500
caccgccctg ccctccccac cgagtcctgt ggccattcag agcggccaca tgacttttgc 13560
atccattgta ttatcagaaa atgtgaagaa gaaaaaaatg ccatgtttta aaaccactgc 13620
gaaaatttcc ccaaagcata ggtggctttg tgtgtgtgcg atttgggggc ttgagtctgg 13680
gtggtgtttt gttgttggtt tttgttgctt tttttttttt ttttttttta atgtcaaaat 13740
tgcacaaaca tggtgctcta ccaggaagga ttcgaggtag ataggctcag gccacacttt 13800
aaaaacaaac acacaaacaa caaaaaacgg gtattctagt catcttgggg taaaagcggg 13860
taatgaacat tcctatcccc aacacatcaa ttgtattttt tctgtaaaac tcagattttc 13920
ctcagtattt gtgtttttac attttatggt taatttaatg gaagatgaaa gggcattgca 13980
aagttgttca acaacagtta cctcattgag tgtgtccagt agtgcaggaa atgatgtctt 14040
atctaatgat ttgcttctct agaggagaaa ccgagtaaat gtgctccagc aagatagact 14100
ttgtgttatt ctatctttta ttctgctaag cccaaagatt acatgttggt gttcaaagtg 14160
tagcaaaaaa tgatgtatat ttataaatct atttatacca ctatatcata tgtatatata 14220
tttataacca cttaaattgt gagccaagcc atgtaaaaga tctacttttt ctaaggcaa 14280
aaaaaaaaaa aaaaaaaaaa gaacactcct ttctgagact ttgcttaata cttggtgacc 14340
tcacaatcac gtcggtatga ttgggcaccc ttgcctactg taagagaccc taaaaccttg 14400

gtgcagtggt ggggaccaca aaacaaccag ggaggaagag atacatcatt ttttagtatt 14460
aaggaccatc taagacagct ctattttttt tttgccactt tatgattatg tggtcacacc 14520
caagtcacag aaataaaaaa ctgactttac cgctgcaatt tttctgtttt cctccttact 14580
aaatactgat acattactcc aatctatttt ataattatat ttgacatttt gttcacatca 14640
actaatgttc acctgtagaa gagaacaaat ttcgaataat ccagggaaac ccaagagcct 14700
tactggtctt ctgtaacttc caagactgac agctttttat gtatcagtgt ttgataaaca 14760
cagtccttaa ctgaaggtaa áccaaagcat cacgttgaca ttagaccaaa tacttttgat 14820
tcccaactac tcgtttgttc tttttctcct tttgtgcttt cccatagtga gaatttttat 14880
aaagacttct tgcttctctc accatccatc cttctctttt ctgcctctta catgtgaatg 14940
ttgagcccac aatcaacagt ggttttattt tttcctctac tcaaagttaa aactgaccaa 15000

<210> 66
<211> 46340
<212> ADN
<213> Homo sapiens

<400> 66
tattttactt cagtaacaga aaatgaaaga aatgttttaa tgttgctgat tgtattacct 60
tcaggatcaa tagcagaagg acaaacttct ttgaggagat ctcctagtgt gtgcaactgt 120
ccatctgcag ccacaggacg aaacagcttc tgaatgaaag gtctttcagt cgttgtctat 180
ttgaaaaagg aaaaaatgat tcaagcaatt aagtctttgt tgctgccaat tacaaattta 240
tatatcataa actttatgtt ggcattaggt gccttttgat acggtgttag cataattaca 300
caacatcaca gatgtggtat cactgtgaaa aatgtttaac atgataaatt caggtaaatc 360
taattctgag gaaacagaca aatccaaagt tgggtgggac attctaaaga taattggctg 420
ggacccttca aaaacttaaa gacattaaaa agcaaacaac acaaaaagat atcaacaaaa 480
gcatttttc tcagtatctc ttaaagagac taacaaagca aatacaaaac ataaaccatg 540
gctgaatact aaattgaaga aggacatttt ttagaaatcc aactatgaaa cacagttttg 600
ggataaatgg ggaaatacag aatggacaac tgataatatt attgagttaa tgtcaaattt 660
cttaggtaca ataaggacaa tccttatttt taagaaattc attgttcaag tgtttaggaa 720
agaagtgcca tgatatccaa aacttaatct tctttctctt tttttggaga cagagtctcg 780
ctctgccacc ccggctggag tgcagtggcg cgatctcagc tcactgcaac ctctactttc 840
caggttcaag tgattctcat ggctcagcct cccaagtagc tgggactaca ggagtgcgcc 900
accatgtcca gctaactttt tgtattttta ctagagatgg ggtttcacca tgttgcccag 960
gctggtctca aactcctgag ctcaggcaat ctgccggctt cggcctccca gagtgttagg 1020
gttacaggcg tgagccaacc gctcctggcc ccaaaactta accatctaat ggttgagaga 1080
gagacagaga gagagagaaa gagagagaca gagaatgtgt gtgtgtgtga agacaaagca 1140
aaaataaaaa aatattaact aatggtgatt ctaggtagag ggtgtatgat tttagtagtt 1200
tcattatttc aacttttcga taggtttcac aatttccaaa acagcagatc cagccatttc 1260
atctgacaaa aactgttagc agcactacat cgtaatttat tgctaataat ctcattgttt 1320
tactcttaaa attgtttcat ttactaaatt tccttagtga tgatggaggc tttatcatga 1380
cagagtacag aggctctgaa atgagccagt gtctatgaag agcaccactg tttgcaagat 1440
ctatgatctt gtacccagtt tcctttatct gttaatttgg gacattccat atctcttgag 1500
tttgttgtgg aaataaatga gcaactttgc caaccacaga gtaaataaat aaatgttaaa 1560
gagaataaaa gcatttttac ctcctctctc cctcttaacg gttatttcac tttaagatgg 1620
taaattttaa gctttctgag atgaaaaatc attaaaactt aacaagaaca gagaaatgcc 1680
atacatacat atttttgtt tgcttgtttc ctgagacaag gtttcactct gtcacccagg 1740
ttgaattgca gtggtgcaac ccccaagttg caatcctcca cctaagcctc cagagtagct 1800
gggactacag gtgtgagcca ccatgctcag ctaatttttt tacttttttg tagaaggggg 1860
tctcactatg ttgcccaggc tgcctcatat tttataagaa tatgacttca aacacttagg 1920
cattagcgac aaggttttgt ttttgtcttt taatgacaga ggtatacctc aacatatttg 1980
acacaactgt tagagatttg gtttaaaaag aaatagacat ggatgaagct ggaaactatc 2040
attctcagca aactaacaca ggaacagaaa accaaacacc tcatgttctc actcacaact 2100
gggagctgaa caacgagaac acatggacac aggcagggga acatcacaca ccaaggcctg 2160
tcggggagta gggggctagg ggagggatag cattaggaga aatacctaac gtagatgagg 2220
ggctgatggg tgcagcaaac caccatggca catgcatatc tatgtaacaa acctgcacat 2280
tctgcacatg tattccagaa cttaaagtat aatacaaaat gaaaaaataa ataaaaataa 2340
gtagaaaaaa taaacatgta agcatgtgag ctgcctttcc taattctatg tttatgtatt 2400
cactgaatac atagtatttt aaaatagtaa tccaataata tatttgagtg tttgtgacaa 2460
gtatgaaaat tgtaattttt aaaaaatctt gataatatgc attgaatatg atttaattca 2520
cttcactatt tgaactcttt agggattatt tttaaaaata tgattgatat cctttgatat 2580
gttttggctc tgtgtttcca tccaaatctc atctcaaatt gtaatcccca cccgtctagg 2640
gagggactgt aatccccatg tgtcgaggga gggaggtgat tgggtcatag ggtggtttt 2700
cctcatgttg ttctcgtgat actgagtgaa ttctcatgag atctgatggt tttaaaagtg 2760
gcagtttttc ctgcactctc atctctcttt cctgctggct tgtgaaggtg cctgcttccc 2820
tttctgccat gatttaagt ttcctgaggc cccacaagc catacggaac tgtgagtcaa 2880
ttaaaccttt tgcctttata aattatccag tctcagatat ttctttaaag cagagtgaaa 2940
acagactaat acattcttca atttaaaaag ccatactttc tcatacaagt tgaaaccaag 3000

aacaatatca tgcataatca agtgattaac tgtgtaaaga taataaggtt gaggagttca 3060
gagaagaaaa gaaatgaata gggaactgta gtgataattt aaaatagcca tccctcactc 3120
agggttttg atcttcaggc catgaagaag cttttaatgc tttttagcaa aggaagtaat 3180
gttggtgaaa ggctttttct gacgactaat ggaaagcagt gctatgtatg gtgacttggt 3240
tatgaaccaa aaccagaatg actggtgaga ggctgactga atacagcaag cttatgtgaa 3300
gacaactgga gctggtgcag tggaaaagga agacagcagg actgtaccca caactcaaag 3360
aaaaaagtca gaaggtacct cccgcagtcc aacctgaaaa caacaaagtc aaaggaatct 3420
tttcaagaat ttggagctct cattcatatc ctaattagtg tatgaaatgt gaggtggctt 3480
tgctataatg aaattacctg gaatatttct aacacaaaga aataataaat gcttgaggtg 3540
gtgaatatcc tcatttgatc attacacatt gcatgcttat agcaaaagat tacatgtacc 3600
ccataaataa ttgcaactat tatgtatcca taataattaa aactaaaaga ttaaaaatta 3660
cctgaaaaaa aatgctaaac aggaaaggcc aactagtctt ggttacatat taaaaaacag 3720
aaattcttct ctaacctcac tattggagaa atatcctgtt atttttatat atcttttttt 3780
tcaccctttc ccaaatctga gcaagtatta taaaggtata accttcaaca atcttttatg 3840
atgaggtatt tgcttactgg ggacaaagcc ccagtgctat tacatagtgt agctaaacgc 3900
tgtagaatgg taaaaacaag aaaatgctca gcaaagtgtt gtttctcatt taatgaaaat 3960
cttattttaa aacacaaaaa ctcaatatac cccaaccaaa aatctgatga acattttctg 4020
tttaatattt attatacagt acctttaaaa acgtaatatt cttattctta aaaatttagt 4080
gtgctagcaa atagcaatta agtacctaag tcaatcagga cgacaaaaaa atactcaatt 4140
tggggagtta gttacttcta tcatctgaat gcgtccctcc aaaattcatg ctgaaaccta 4200
ttcctcatca tggcagtatt aagaggtgaa gcctttgaga ggtaattagg tcatgagggc 4260
agagtcctca agaatgggat caatgctctt ataaaagagg ccccagggag cttgtaaggc 4320
ttttgcccct tctgccatgt tgggggggtg ggggtggggg cgcagcaacc agtgctaact 4380
ctgaagcaga gagcagccct caccagaaac cgaatctgtt gaagccttga tctctgactt 4440
cccagcctcc agaactgtga gaaataattt tctgttgttt ataaattacc cagtctaggc 4500
tgggcgtggt ggatcacctg aggtcaggag ttcaagacca gcctggccaa tatggtgaaa 4560
ccccatctct actaaaaata cagaaaatta gctgggcata gttgtgggcg cctgtaatcc 4620
cagctactca ggaggctgag gcaggagaat cacttgaacc cagaaggcag aggttgcagt 4680
gaatcaagat catgccattg aactccagcc tgggcaacaa gagggaaact gtctcaaaaa 4740
aaaaaaaaaa aagtacacac tctaacatat tttggtatag cagcccaaat ggaatggact 4800
aagacaatta cccttaaaat aaaagctccc atagagagat catgcattca agtacagagg 4860
ttcttaaggg caatgggaat ggaggacata ttcctgcaaa cttttcaaca gctctcatta 4920
gcccgatgtt agagctctgc aaagaagact aaattatact gagaaatatt tttaaatctc 4980
cacaaatagg aatgctgtaa acgttgattt agtatatata aaattagaca agactaacaa 5040
tatccaatgc aatctaaatc ttaggttgac agacaagaaa gccactgcaa acaggaatat 5100
accacaatac ctgatcttgc cacatatttg taaatatgca aagtatttca ataacttcca 5160
agaaacagta ttactctcat gagaaataac atgatgtaag tcacctttga aactgtcctt 5220
gttacttttt caaatgtatg ttagtcattt cttaacacca aatgaaatga aaaactgagg 5280
tggtaatggc tggctgctcc catctctcct ctactcatgt gccttcacca atacagcaat 5340
cattttttct tatatgggaa atttacagtg ttgatatagc tcagagatat attgaagaaa 5400
agcagaaaaa cgaaacttat aaacatttta ggaaacctta tgtattttct taaatagttc 5460
aagtgtaaaa cttagaattc ttataaataa tgtgtgttac agctatattg taaatggtgg 5520
ctcatgcctg taatcccagc acttcaggag accgaggtgg gaggagagct tgagcccatg 5580
agtttgagac tcacccgggc aacacagaga gacctcatct cttaaaaaaa aaagaaagaa 5640
agaaagaaat gaaatgcaaa gaaaaagtct ctatttcaaa tgtagccagt agagccaata 5700
ggttaaccaa tattaacatt aacgttgata aaacaagaaa tgatgattta ctataagctg 5760
aaaatcagac aatgtatgga ctttaagagt aacaggcacg atcatcacaa acttaaatca 5820
ggtttgagtc ctatgagtta tatacagtta catgatgcaa caaaagatgc cagccagttg 5880
ttaaagagta ttagattcgg ctgggggtgg tggctcatgc ctgtaattcc agcactttgg 5940
gaggccgagg agggaggatc acgaggtcgg gagtccgaga ccagcctggc caatatagtg 6000
aaacctgatc tctactaaaa atacaaaaac tagtcaggca tggtggcacg tgcctgtaat 6060
cccagctact cgggaggctg aggcaggaga attgcttgaa cccagggggc ggaggttgca 6120
gtgagccgaa atcgcgccac tgcactctag cctgggcaac agagcaagac tctgtctcaa 6180
aaaagagtat tagattcaag tcctgtttct gtcatttatt atggaaccat ggacacaact 6240
acctatcttt cctgaacctc agtttttca actgcaaaac aggaatatat acatatgtgt 6300
atatatacat ctgtgtaaac acatatgtgt atatatacat ctgtgtaaac acatatgtat 6360
atgtataaat ggagataata cctacattat agtttctgag ataataaaat gcacaacaca 6420
attctgacac ataacaattt gtaacttaaa acataccatc accagggcca ctagttttag 6480
aacactgtaa tgcatagtct aatttaatac tatgcaaact gtgttcactc aaggttttat 6540
ttcctttaa tttcattcat ttactcttca gttgtttgta agctaaaaag tccagaatca 6600
tgaaattcag aagtttacgt tttaatgttt ttctatatgg caaggaaaaa aaaaagggca 6660
aagtcatttt aacactactt tcaaaatcag cctagaactt aacactaaag gcatgaccca 6720
taaaagggaa tactaataaa tagacttaat taaaattaaa caacaacaac aacagctaag 6780
cttttgttct gcaaaagatc ctgtgaagag aatgaaaaca taagccgcag gctgggagaa 6840
aatatttgca aaccatattt ccgagaaagg tcttgtgtct ataatatata agaactccca 6900
aaattcaaca gtttttaaaa aaagcaaata atccaattag aaaatgggca aaagacatga 6960
acagacattt taccaaagag aatatatagg tggcaaataa gcatatgaaa acatatctca 7020
cacatcatta gccattaaag aaatgcaaat taaaaccaca atgtgatatc attacacacc 7080

taccaaaata tccaaaataa aaattagtgg taacaccaaa tgctggtgcg catgtggaaa 7140
aatagtcctt cacacactga tggtacaaat gcaaaacagt acagtccctc aggaaaggag 7200
tatggcagtt tcttacaaaa ctaaacatgc acttaccata tgaccaagta attatactct 7260
tgaatattcc cagaagtaaa aatgtcttct ccaaaaaact tatacatgaa cgttcatagc 7320
tgttttattc gtgagagtca aaaacagaaa gcaatcccag ggctacccat taaaacaggt 7380
gaatgcttat aaactgactg taataggtct gtcccacgga atactactca gcaataaaaa 7440
ggaacaaact actggtatat gcaacaactt ggatagatct caagggagtt atgttatgtg 7500
aaaaaagtca atctcaaaag gttacacact gcatgactcc actgatataa cattagtgaa 7560
atgacaaaaa ttttagaaat ggaaaacaaa ttagtagttg tcagaggtta gggaagaaat 7620
gcagtaaggt aggtggctgt ggctataaaa gggtagccta agagatcctt ctgttgaaac 7680
gggtatattt tgaatatagg gtgaatttac atatgtgata aagattgcat agaactaaat 7740
acacacacac agtatatgta aaactaagga aatctgagta aggtttgtgg attatattaa 7800
tacaatttcc tggttgtgat actgtactgt aattatgcaa gatgttagaa ttgggggaaa 7860
ctagatgaag ggtatgtaga tctttctgta ttatttctta caattgcatg tgaatctgta 7920
attatctcaa aataaaaatt tttttcaaaa tttcaaaaca actagtctag agctttgtta 7980
atcaaagttt tctctgagga cctgtagcat tttggttatc acctggatct tattaaaatg 8040
tagattctca ggctgcatat tggaattcct gaattggaat ccgcatttta acaagatttc 8100
caagtgattc atgtttaaag tttgagaagc actagtctac aacaatgact tttaaccttt 8160
caacctactc taacacactt gaaggccata acaaaattca catcaataac agttgctcgg 8220
ttggacagtg actctcaaca caaatgagtg aggaaaggtg gggactcaag actcaggtag 8280
caggaaaagc cccttaggtg atcctgatga aatgttttct ccatcctggc tgaaaaaccc 8340
agaacagtca attaaggctc aaaacaaaag taatgtttat aatactggag atctttaaaa 8400
ggcagataat atatactata acagagcaaa ggtaattatt acaatgtata aatcttataa 8460
gaaccaaaat cagaattaaa atcactaagc acataatgaa aatcctttaa aaagtataaa 8520
aatgaatgta gtctaagtaa atactaataa tggcagttat agtgagaaaa gctctagagt 8580
cttttactct tcatacttcc tagtcacaaa catctatttc caaaactgac ccttcgtatt 8640
tcaaataatt tatggcctgg tacagtaata agagcatgat atttaaagcc agtcagaaga 8700
cacatattct agctctggat ggcacttgat gacgatggat tcagcttatg gttccaatcc 8760
cagctctgtc aattagtacc tatatgaccc tagtcaaata cttaaacctt cttgtgttac 8820
ttgtgtgtca attgtatcat ctataaaatg aggatattaa cagtatatac ctcatagatt 8880
tttttgtgaa ggttatacaa ttaattcata taaagtattt agaacaatgt ctagcacagt 8940
gaattctcaa tgagtgttat aattgttctt tttaaatgtg acttgactct caacagaact 9000
ctactgaatt ctaatatgta ttctgtattg agctgtcaaa aaaaataagg attataataa 9060
catatactat tcttgtagtc aaccctgtta ctatgttatt actagtgtca gttttgttgt 9120
tttggtcata catattgttt tacatacatt aagaattatt agaaatgttg gtttattaaa 9180
aatgaccatt tatggctaga agggtatata tctggctcac tgactgtgga gtcaatgtcc 9240
ataaagagga ggaagaatgc catcagagta aaaggagatt ctattcactg aaacaaagtg 9300
ataaaaagct atgaaagaga aaaacataaa ataaccaaag gggtgaaact taacagatgc 9360
ccagtagatg cacaatgcac tgggttgtaa aacttaaaat ggccttaatt aaaagccaag 9420
cacggatgga ggtgctgggg gagtctccta cggacacagc aggcagaatg taacaatgac 9480
aaggggctca agtttattta aaaagagatt ggacaggccg ggcgtggtgg ctcacgcctg 9540
taatcccagc actttgggag gctgaggcgg gtggatcatg aggtcgggag ttcgaggcca 9600
gcctggccaa catggcgaaa cctcatctct actaaaaata aaaaaaatta gccgggagtg 9660
gtggcgtgca tctgtagtcc cagctactca ggaggctgag gcaggagaat cacttgaacc 9720
tgggaggcaa aggttgcagt gagctgagat catgtcactg cactccagcc tgggcaacag 9780
agtgagactg ctcaggatct cccaaagacc caaatccctg taaactgaat gcataatatc 9840
atttgctcca gtgaggctta gatggacatt ctagtcttct tggttgagct gaagaaacaa 9900
atattatatt gataatttat gtatgttgta tttttcaagg tatagcaaca agtttttatt 9960
catcagctac tttgtgtgtg tgctttgttt ttaagtcttt tgaaacagga tggtgattta 10020
ctacatttat aagtaaaatt tatttgattt acaagggttg cttaagtgta tcacaggatt 10080
tcacttgtta tatttgcagg tgcttaaaaa atcagctata ctaaactata actggaatta 10140
gcaaagttca tttattgatt aatcaagaat ataattagat ttgcctaact atataagtag 10200
tactatgtgt tatttaagaa ttaaatctag aaaagggatg gactctggaa atatcaagaa 10260
gtgaaaaaga ctgctctcat ttttgtacaa caattactaa atttctaagt agcattaatt 10320
gaactgaaaa ggcattttag aaaaactaga ttttacaatt tataactcta ataaaacaca 10380
actaactatg agtgtgcttg ttcatgccca aaagctacct tccaaaatta aaaaccctat 10440
tggatggctg ggtgcagagg ctcatgcctg taattccagc actttgggag gccaaggcgg 10500
gcggatcacc tgaggtcagg agttcgagat cagcctggcc aatatggtga acccgtctct 10560
aacaaaaata caaaaattag ccgggcgctg tggcgggtgc ttgtaatccc agctactcgg 10620
gaggctgagg caggagaatc acttgatcct gtgaggcgga ggttgcagtg agctgacacc 10680
gtcccactgc actccagcct gggcgagagc ccagagcgag actccgtata ttaaacaaaa 10740
caaaacaaaa ctcaaaaaac cctattggca attactaggg ccatcaaatc agtatatttt 10800
cacttgacac acaattttga gataatgaac cgaacttact atttttgaaa atattacata 10860
ataaatatta gtgaagcttc attgctgaaa tggtgacaaa gatgaatagc aataaaactt 10920
ttcttataga tctttagcaa aaacaaaaaa accccaagca tactatggta cattacttta 10980
gagaatcaag tagctgctag ttgagtaata gtggtaatag gcactacaat gatataaaca 11040
aattacaaca aagaatattg tttttatttc ctgtccatgt tttaaaaaag ctttggtttt 11100
acctatgttt aacaaaagca taggtacaac aacgactact actactaaca tataagtagc 11160

ctggatagaa ttatcttaat agtagtaccc aagtgcagga tctctaagta atgatcagaa 11220
ggcaggaata aattttatca gaaatcttca ttcattacat atttactatg catttaccag 11280
ggtatcacta tgctaatgga tacaaagata aataacatgc aaacaactgt aatacagtgt 11340
tatgtgataa cagaaatatg tacaaagcac tatgaaaaaa attacaaagc ttgagcacaa 11400
attttaactc tggacttact ggcatttaga gcaaaaccaa aacaatccta actggttaat 11460
ttcattttct aagagttgga agctatatca gtaggtacaa agtaaaatat gctaattgtg 11520
gtagaaagta aaatattaca acagtagaga atttcaaaag aagataaaaa taatggaggg 11580
aatatagaag gtcttcaagc ttccagcttg aaatacatat ttttttttaa atagagaaag 11640
agataaagtc atttgagtat tcagagggca gactgaatat aatggtactt ctgagaaatc 11700
agtggataag gagagaaaag tggactaaag gccatagcat atagagcttg gaatgtcaaa 11760
tgtagtggaa ataacaaagg tttggttgga atcccaactc ccaacaacgt actgtgtatc 11820
tagagcaaat tacatcaacc tttgggagta ctgtttctga atctgaaaaa tgaggaaaac 11880
ttatctttga acaattgatg tgataattaa atgagatata tgaaatatct aatgtaacaa 11940
gtgcttaaca atgactagtt cttttcattc ctctcttgaa ccattgtgaa acgtagaacc 12000
aagaaaggta acagtattta gttgttacag aacccattaa gagagaataa aaaataactg 12060
gtattctaac ttcagtttcc tttgaagtct tgttaatgag aataaatatt atgtggcaca 12120
aagaaaaaga aaacaggggt ttacacagga tatgctgcca gactttacca acaatgacac 12180
atgatatctg cttcaactgt cccatgcata tttggcttaa gatatattca tgcatatcaa 12240
attttacatc acatggtttt caaaagaaga ttcattaaaa ttagcttaag aatgtacaca 12300
atatacaata cctcattaaa taaaaagaac agaccatttc caaatgaatg cttttagagc 12360
tttacagtaa acagtctttt ggtggtagaa agagggggaa cagagagggg agtgggtggg 12420
agtctgtagc acttatcaga ctacttttat cctttatgta gagaaatagg agagttgaaa 12480
ataagcactt tctgtactta tgttgagagt ctgaagccca cttttaatag tcttgacaac 12540
actaaaaaat aataattaac atttgaaaag ctgtcattat tatagtcagg gacacttaat 12600
ctccaaagga gaagtttctt aattgatact atgattaaat aaaagcatcc atcagaatta 12660
tatccacaat ctggtttgga gtttatgttt tgtcttattt aaattgttat acttattata 12720
attctgtcta gacagtgcca aatgtacttt gtcatacaaa cacttgaggc aaattttctt 12780
caaataagcg caacactttg tttcctcttc gtatcctttg actgaataac gtgtggtaca 12840
gagaagtaat acttcccttt cttgggatcg agatcaattt gatgcttgtt ataagcccat 12900
ttacagaaca aatggtattg cttttaaatt tttatatgaa cttatcagta gactagccaa 12960
aaaagaagct tcatataaaa gtgctaggat tgatattctt agtaataatt aggtaaattc 13020
tctaaaattt tctcccaaaa gatctgaaaa atcataccaa gggaagtata gtttaaattt 13080
cattatatat aatagcttta aaatatcttt gctaattcta cccaaagcca cactaaaaag 13140
actaatacaa aaagaatgta attaataaac tattttcctc tgaagaatca aagggcactt 13200
ctgcatatga acatgtttta tccttttggt gtacttacat aaaataatta agaaacactt 13260
ttaattagta taaacaaaga aatcaaaata gcaagaagaa atgtctgagt aaaagcagct 13320
gtgctgacct caaaagtgaa attctgttct cttgatgccc agttaagtgt ctaacccagg 13380
gaaaagtgat tctaaacctg ggctaggagc tagtggagct cttcaaacag tctcacctac 13440
cctcacccct caaggaatgg tctatgggtt ctgtggtgaa cgctaaagtt tataacatgg 13500
gaatatttat tattttgttt ctaacacaaa taatttttaa aaatttattc tactaaagta 13560
acatcaaagg gaaatttcat aaaaattctt ttgaaatttt tagaagtagc aaataaaggc 13620
aagtgataaa tattttacag atttcaccac ttacgtaatc tgatcaacaa attttaaaaa 13680
catagcactt gaatactatt aaaaatatat taaaaaggta acatagtaaa actataaaat 13740
tctttaaaaa aaatataaga ggaaaccttc gtgaccttgg attaggaaat ggtttcttac 13800
atacggcaac ctaaaaatac aagcaaccaa agaaaaaaac agacaaactg gacttcatca 13860
aagttaaaaa cttttgttct tcaaatgaca tcatcaagaa aataaatccc acagaatggg 13920
acaaaatatt tgcaaaccat atctgataag agaccactat tcagaatatg taaagaattt 13980
gtaaaactta taaataaaaa gttaaagaag tcaattttaa aatgagcaaa ggatctgaag 14040
acaattctcc taagaaatac gaatggctag ttaaatgcat gaaaagatgt ttagcatcac 14100
tggtcattag gaaagagcaa aaaccaaaat gatatactcc ttcataccca ctaagactgc 14160
tgtaattaaa actatagaaa ataagcgttg gcaaggatgt ggacaaattg gaaccctcct 14220
catacactga tggtagaaat gtaaaatggt gcagatgctt tggaaaacag tctgacaata 14280
ccccaaaggt ttaaacgtgg aattaccatg caacccagca attctactcc taagtatcta 14340
cccaagagaa atgaaaatat atgttcacca aaacatttgt acataaatat taactgcagc 14400
ttttattcat aatagccaaa aagtggagac aatccacatg tctatcaatt ggtgaattga 14460
taaacaaaat gtggtatctt catacaacta ttactgggcc ataaaaagaa tgatgtattg 14520
atacatgcta caaaatgaat gaaccttaaa aacaatatgc aagcaaaaga aaccagacac 14580
aaaaggccat atattacatg atgctaatta cataaaatgt ccagaaggga gaaataaatt 14640
agtagttgcc aagggctgga gggaggggga atgatataag tgactgccaa tgggcatggg 14700
gtttcttttt agggtgatga aaatgttctg aaattttatc acgggaatgg ttgcacaact 14760
ctgtgtaact tagaattcag tgactcctaa aaccaatgaa tagcatgctt taaaaggtga 14820
cctttgctga gcatagtggc tatagtccta gctacttggg aagctgaggc aagaggatca 14880
cttgagccag gagttccagg ctgtactgca ctatgatcat acctgtaaat agccaccata 14940
cacaccagcc tgggcaacac agaccatgtc tctaaataaa taaacaaata aataaataaa 15000
agggtgacct ctgtagtatt gagattatac ttcaagtaag ctgttattaa aaaaaaaaaa 15060
gttatcatat gggtggcagg ggaaatcatt ctgggatgat ggctaacttc atcagtattt 15120
gatttatacc tatgcatcat accttatgtt tgttttatgc atttgtgggg tttttaaaa 15180
aaattatatt tcataaaaac aaattttaaa aaaattaaag tcaagaaccc caaaacaaca 15240

aagatcagag atacatttct accttatcaa ttcagaaaaa ttacaagttt ttttcttaaa 15300
aattgtatag catcatggtg attttaagtt acctgtagga atttaaataa ctttgtctta 15360
actgttcacc aaaactcatt taatattcat gttctgatac tgaaaatgaa gctgaaaagt 15420
tttgaaatta caatatgcta gtttaaaaag gtttactaaa atacataatt tcattataag 15480
gagtaatatg aaataaaagt atcaaatatg ggaccattaa aatgtccttt actaacaaat 15540
tgctacccac attgtggact cactgcgtcc actgtttgcg agcttttcca gaacgctcgc 15600
caccagttag ggtagccaag aactcctcat cttcactttc ttcctcacta gcttggaacc 15660
tctggattcc cacccacact gctgtgacct gaatggggaa gagaaacgcc atagtaaggg 15720
aactcttcct tttatagatt tctgaattag aatctggcat tacaaaagaa caatgttata 15780
aatccaggtc agagtttata gttctatttc actattactt atatggcttg tcctaggaac 15840
ttaactatta tttacaatgt aagtacctat ttccacaaaa aaattcaaaa ttttggaata 15900
caatatctga agagagaatg gtctattgaa tccaaagtag gctgatacat cccaacagta 15960
tttcagattg agataataat aataccacca attcatcaag tcaaattata tgcttatttt 16020
ccacaatgga agttttaaaa tagtataaac attttaatat atagcaggct taacttatga 16080
ttattaaaca gggttctaag aaaatagtat acatcaaata ttaatgtgct tcttgtataa 16140
tttaggtgac aatttatcca tctgagaaat gcaaaagaga ctttggtaag gggttgagta 16200
aggagcattc tgtgtcaaag aattcactag caaaagaggg tatactgtag ttacaagcta 16260
taatcactgt acttatttta aatccctctt cagaaccagg tcttaaaaga tgataaacat 16320
ggcctcatga ataactatca accaaactat agaaaagagt gcaagagtgt ggtgttctaa 16380
cttaaaatat ggtgttttat tcaaataatt ttatttaagg ctccaaaagc agcagcctca 16440
ttccccagaa atcatagtta aatgaaatct tccttactaa aggaaaaatg aatcacaata 16500
tttaacgtga acattttaaa aacactctaa agcaacaaaa ctattcaatt gtatgtgata 16560
tggcttagaa aggcatgtag gtaaaaagga ctaaaaactc taataatggt tgggccaaaa 16620
gtaaatttgt tagttctact ccattaagca ttcctcaagc agtgtaaaaa tcagagttca 16680
agttacactt tgatgtgtag atcctttgaa agccactcta ccctgtttta tatgaagcat 16740
ccgcagctaa aatgaacacc tagtgaagag tatgaatgct gcaatacata agcagacgtc 16800
agaattgtcc caagctgatt ctaagttact ttaaacatgt atgcagagtc agaatatgac 16860
ttacttctta gaagtaacag ataattacct ttggcataat gaaaaaaact ttaaatgtaa 16920
gttaatacag gtattttccc tttagcaaag ctttgctttt aaaagaaaac ttcaaaactt 16980
aaattaaaat aggaaatgct ctactatgta gtaaaaatac tttttagatt actgaagcaa 17040
agaaaaggaa ggattctatg agggaggaaa agtgggagaa aaatgtaaag aaaaaaagga 17100
agaaggaaag aaaagagaaa aggaggaaag aacacaagga cagaaaggcc tattgaaata 17160
tattatttct ttcaaatttt aaacgagcag aataaattct tttgttttat aactatgaaa 17220
taatctatgt tcctcttatc tatgcttgga aaatttagac aaaatgttaa gagtaagtac 17280
tacattggat ttccgggtct tcagctctga aaacaagctg tttcttaaca tacgtcaatt 17340
ttctatattt catgtcattt ctatttgcaa atgttataaa gttcaatatg atgtaaaaca 17400
tggttaaatg aagttcaaaa ataagtataa catacattag tttggctatt ccaaatttca 17460
tgcacattaa ctcagccaca catctaacac agtcagccct ccctatccag gggttctgca 17520
tctgcagatt caactaacca tgggtcgaaa atgtttttgt accaaacatg tacaggcttt 17580
ttttcttgtt atcattccct aactacagta taacaactat tttcacagtg tgtacatgtg 17640
tatgaaatat tataagtaat ctacagataa tttaaagtat acaagagggt atgcataggt 17700
tatatgcaaa tactacacca ttttatatca gactctcaaa catcagtaga atttggtaac 17760
ccagggaggt cctggaacta atcacccaga ggtatcgaca gatggctata tataaatcac 17820
tcagtgaatt caggattcac attatttcac aactagtata attttatgtt gttcacataa 17880
ttgtgtcaca acatatacat gcagacaggt gactttcatg aaaagattac acccaagata 17940
gacatatggt ctactcaaat acggtttcca aatgtgtatc caatcttgtt taattataat 18000
caaactcacc attccattga taagcgacct ctaccaacct gcttatcccc tccaagcaat 18060
ataacagtgg ttctctgaac caatattgac cctcctttaa attgatagcc tttttttaaa 18120
aagctaacca ttgagaagta catactgttg aagacagaac atattctgta aaatgctccc 18180
aagatatcaa agtcagatga tacaactgaa tgtttatgct agattatatt tctaagctga 18240
gaattacatt ttaatatacc ataagcaatc tgcaaaagaa gcaacttgcc taaagatttc 18300
aggagtttca agtatgcata tgtcaatatc tgtatcaata tgtaatatca atataatcaa 18360
tgcacacaac aatacgtaac tgtacttata tcatctcctt agcactaatt attacaaaca 18420
atctgcatgc actgcaaagc aaaagtataa tataaaatcc caaaaaacct tgaaaattta 18480
ataaaaccaa aaaacaggca tcacacacaa gaactgaggc gtatacttca ttaatgagta 18540
tgatatcctg atatgaaatg tcaaacaaaa ttacccaggc tcaggttaga aataaagata 18600
ggacattagt ctttgtattt ttaaattgat tttttcttct aatattcctt aatgataacc 18660
ctatatatta cctacttaaa attattagca aatagttatt ttaaaagtat gagtaattag 18720
accaaaagca actctcatat ttacccaaaa gaaggaacca ctaccaagaa tcaaagccta 18780
gtaattctgt tcttaacaga caggtgttgt gtattctggc atgttacatg aaaatcactt 18840
atgagaagaa cagaaaaaaa aattagaagg tagttttcac tatggaaata ggtaagtgat 18900
taagcagatt ttcttacacc atgaaattgt cagcagactc aataatcacc ctaaggggca 18960
tcattctgga tgccgacatt ctctatgatg gaaagggact gaaagtaaaa tgcactaatg 19020
acataaagaa accaatatcc aatagtaaag ttgaagaaat aaacattctt tggacaggaa 19080
ctaagctgaa gtttgcaact accaagaatg tattatgcca gcagtaaatt aggaaactaa 19140
agcccatgtc aaccaatgaa aaatgggagg actgaaatca atcattaaag cagcagcaag 19200
gttctaacta ttctaaggta taggctacct ctggcgtata ttatcagagt tgacaattct 19260
tccaagaaat tctaacatca actgtaatct gaggtccttt aaaaaataat ataaaccagg 19320

cagtagactt acattttgta atattttctt ctaagagctg tacattaaga ttttatttgt 19380
gatataaata ctatcaaata attagctata gaacagctct attttcaaca gttataacat 19440
tttaagccat ctcacattta acctaaactt ttatcaaatg tcaaaactga ggccgggtac 19500
ggtggctaac acctgtagtc ccagcacttt gggaggccaa gatgggcgga tcacttgagc 19560
ccaggaattc gagaccaacc tgggcaacat ggtgaaaccc catctctata aaaaatacaa 19620
aaattagctg cgcctggtgg tgtgcgcctg tagtcccagc tactagagag gctgagggag 19680
gagaatcacc agggcctggg agatcaaagc tgcagtgagc tgagatcgtg ccactgcact 19740
ccaccctggg tgacagagtg agaccctgtc tcaaaaaaaa aaaaaaaaag aaagaaagaa 19800
aaaaaaatca aaactgatca cttgaggtcc aacttatgtt tactatatct acttatattc 19860
ccaaagacat cttaaggaga gatgaaatca taaaaaggtg aggatgagaa agaaaatagt 19920
aagtcagtaa ggtcaatttt tacatatatt aggctagcat aataaaaata tgagtgtctt 19980
attattattt ttttttgaga cagagtcttg ctctgttgcc caggctggag tgcagtggtg 20040
caatcatggc ttactgcaat gtctgccttc caggttcaag caatccttgt gcctcagcct 20100
cctgagtagc tgggattaca ggtgtgcgtc accctgccca gctaattttt gtattttcag 20160
tagagacagg gtttcaccac gttaaaccat gagtttggcc aggatggtct caaactccca 20220
aagtgctagg attacatgcg tgagccactg cgtctggcct aaagtgtctt attataacca 20280
agaatttatt tgtggagaga ggtaaagaaa actcattttt agtgaaataa ttaaaactgc 20340
atcattcaca atctatcttt caaaatgagg tattaactat tttggcttct aaaattaccc 20400
catatactac atgcatgagc atgggaattg aagttatttt attcctaagt ttgagacttc 20460
atgttttaat gtgatcacta aaaatttcct aattgatgat taggaaaata actttctgta 20520
aaattccaga attttagctg tttcaatctc ttcatattaa ggggagaaca ttatgttttt 20580
actttctgtg catgcacttt ctttattaga agaaaatgga ctgagggcag taagcaaccg 20640
aaaaggaaga gtaataagaa gcctgatgtg tgtgaaaact ggagaacagt ctcaaatcat 20700
aaaaagttat gacagaagag gcataaaaaa taaaagtaat gaacttaata tatgaaaggt 20760
aataatgatt aagagcatag gctataaagc cagactggac tccctggatt caaatcctgg 20820
ctcttctaat tactaggtag gtaaccctga gcaagtttca atgaccaatc tttttctcaa 20880
ttacctcagg tatataaagg ggacagtaac agcatttaac ccagaggaca ataaggatta 20940
aataaataca tgtaaaataa tttaaaacag tacctggtat tcaataaagc gcaataaatg 21000
ttagctgcta ttattattca tctaaacttt actttcatta ccagcaatat tttttaatct 21060
taaaaatatt gaataaaaca atgacctagc ttagtaaata aattcataat gagaaaatgt 21120
tgatttcatt taataataac tttagtagtt tgggataaca ctttgcatat tttaatttcc 21180
ccagctataa ataactcaaa taatttgcca tcagatgatc tgttattttg aagttaacaa 21240
ataaagcatt tcctaaaaaa gttctaatac ataacttttg ctctcatctt atgttttaaa 21300
aacaaaatgg caaatcatct gcatcaaata gttcctactc ttataacatg acaattgttt 21360
taaaatatat ctgctggaaa aagcaactga agtcctagaa aatagaaatg taattttaaa 21420
ctattccaat aaagctggag gaggaagggg aaaaacatat ctgccaaata agcttataat 21480
taatagttgt tttcagtttt caaaaatcca cataggaagc aatttaagcc taaattgcct 21540
aagtctcaat ctcagcgtag tagatagctt agggcaatca aaacttgctg tgttgggctg 21600
ccccctacag gactcaattt acctatttct tttaaaaggt gtgtaagtag gaaatatgat 21660
tcaagtttta cattaacaat attaatgcta aagcagatga ttatcattca cgcattcact 21720
ataggaggaa acagtctctg agaaccatct atagagatac agagagaaat gaaacaatcc 21780
ttgtccttga ggaattaata gtttactgct tacagagaaa ctacatacat ggtgaaatat 21840
ttaaaaatag ctcatgatat cctctatgat attatgtttg ctatagaaaa agaacaaggc 21900
tgaagatcta agatccaagt tctactgttg gctctgccat caaacaataa gctaaacaat 21960
gtacaagtca gttttgggga agctgtctta ttcccaaaat gaggaggtta aattagttaa 22020
ttcttccagc ctctatggct ctaatattcc acagttacat ttgtcaaaac aaaaggtaga 22080
aggaaatgtt tcaaaaacag acttcgcaga aagaacatct atatgatatg aagggctggg 22140
gcatatgtga agaaatcaag gaagacttct tgaggaaggt gacatctgaa gtaactttag 22200
aagcactctg ggagccaagg ctattcccag gagttaacag agtcagataa taaaagatca 22260
aagatgttta ggggaatagc atgcagtgtt atttggttgc agtctagcta tattttagga 22320
aacatcaaat taatatcagt ataaaactca acagaatgga gggagaaaaa gcaggtagaa 22380
aaatctaaga accactaaaa tagttcatct agaagataaa ggacccatga gctaaatcag 22440
tgcaaatggc aagaagggaa taaatgaaga cagttctggt ccattagaac tgcaactcaa 22500
caaaagtgat caaaagagtt attccaaagt attgacctgg taacttgaag aaaagtaaag 22560
aaagaggaaa ctggacactg aaacagaaga agtagattat gtatttggta gtgaatggaa 22620
gtagattggt gggaccagtt agaacctcac agagaagaac tatgttaaga ccagaaatac 22680
ggccaggtgc ggtggctcat gcctgtaatc ccagcacttt gggaggcctg ggtgggcgga 22740
tcacctgagg tcaggagttc aagaccagcc tgacaaagat ggagaaaccc tgtctcccct 22800
gtctgtacta atacaaaatt agccaggtgt ggtggtgcat gcctgtaatc ccagctactc 22860
aggaggctga ggtaggagaa tcgcttgaac ccgggaggcg gaggttgcag tgagctgaga 22920
tcgcaccatt gcactccagg ctgggcaaaa agagcgaaac tcttgtctca aaaaacaaac 22980
aaacaaaaca aaacaaaaca cagaaataca tcaattaaaa aagtgagcta ttcaccagat 23040
atgttccact ggtcataaaa caaaagaata caggaggcat gacaagccat catcattgct 23100
gttaaaataa ctcacagcaa aattataatg atttaagtca ataacatcta ataattccag 23160
ctatagtgtg caatttaatt tattatgtgc caggcacaat agtttattaa aggtattacc 23220
tctaattttc acaataaccc tattttacag attataaaat ggaggcccag agatgtaagg 23280
tgaacgagcc aaatcaccta gttacctgga atataaactc agaactgcct aaatcaaaag 23340
ctctcaatct taaccacatg ctatactgat gcatgtcaaa gattcaattc attcagattt 23400

ttcaaggtta tcggaaaacc tatgtagata aaaatttcca aaataatcaa ggatatgtaa 23460
cttttacaga aagcaatcac tgatcatcta ttgcaatact catgttctta agcaatatac 23520
tgagttgaaa tttttatatt ttataaataa ttagaaagaa tacatttttt aaaactttaa 23580
aaaacacctc agtttttatt ctcttcccca aatttcaaca aaatccattt atccaaactt 23640
gaggttgaat cattaaagtg gtgatatcat cagtaatagc agagtgagga ccctgaatat 23700
actctcctcc ataaaagcaa caagaacaca aaaattctca aaatgaactt tttctgaaat 23760
ctttcaaaag ccccactctc agaaaactgt cattatttga tctgccagtt ccctagaaaa 23820
acctccctca taggacatta tttgacttga ctcagagctc actcagtgca aacaatttta 23880
tcaccaggag agtttgtgga aaatcagtgg caattgttaa acatcacatc tgccatgaga 23940
tagcaataac agatggaaca aacaagctaa ccaaaaaatt aaaagaaaaa cctgggaaat 24000
aagaaatcca aagggggtct gaaaagttct aacatatttc tgataatcca gaaagccata 24060
cacatgtata gagctgtgta cacgctcaaa aaacatctac gaaggcccta aactctcacc 24120
tatgggaaac cctgaggctc tgtacaagaa gaaagtaaaa tccagttata aattgcttgc 24180
cgtatcattg aaggcaatgc cccaacattc acacataggc ccctggcaaa gattggaaga 24240
tactctagtt ctaggcattc aagaaaatct cttctaatca tcagatgatc actaaactca 24300
ccaagcagta actttagggg cctgtgtgat aaaaaataaa aacctgaaag aattagttca 24360
ggaaagaaac taaacaagca acagcaacaa caaaaacaga ccttgggaaa gggggaagc 24420
atctggtttc cagagttatt ctgttatact atataaaata ttcaggtctc aacaacaaca 24480
aaattacaaa gacatgcaaa gaaacaagta taagccacaa actgggggga aaaagcagca 24540
gaaactggcc ctgaaaaaga ccagatgctg gacttactgg acaaagactt taagagagtt 24600
attttaaata tgcgcaaaga actaaaaaaa agtttatcta aagaactaca ggaaagtatc 24660
agaacaatat ttctgatcct tcagaagaac cactttttgt cactacagat tagttctgtc 24720
tggtctagaa cttcttaaaa acagaatcat agagtatatt ctctttatat cagctctttt 24780
tactcaacac aatgttgtgt gagatttatc catgttgttg catgtatcat tcccaaacag 24840
aaatagaaat tatagagata aataggagtt acaaaaaagt accaaacaaa aattctggag 24900
ttgaaaagca caaaaactga attaacttga ggggctcaac agctgatttg ggcagccaga 24960
agaatgaatc agcaaatcta aagataggtc aattgcgaga aagagaggga agaaggaagg 25020
aaggaaggaa aggaggctca gagacccaag agacaccatc aggcatacca atatacatat 25080
aatgagaggc ccagaagaag atgcagaaaa agggtcagag tatctgaaaa aataatggcc 25140
ctaaacttcc cgaacttgac cccaaaaatt aatctacaca tccaagaaga taaacaaact 25200
aaaaagaata aaatcaaagc gatccacacc taggtacatc ataatcaaat gactgaaata 25260
taaagagaga ctctcaaaac aggcaaggga cttatgtaca aaacatcttc agattaataa 25320
caaatttctc atcagaaatg atgttgtcaa taggcaatca gatgacataa tcaaagcact 25380
gaaagaagta gaatgtctgg gacctggaat gctggtggac acctgtaatc tcagtatttt 25440
gggtggccaa ggtgggagga tcacttgagg caaggagttg aagaccagcc tgggcagcag 25500
aaagaggctc tgtctctaca aagaataaaa agattggctg aatgtggtgg tgtggacctg 25560
tagtcccagc tactcaggcg gctaaggtgg aaagatcgct tgagcccagg agttggaggc 25620
tgcagtgagc tatgactgtg ccactgcact cttgcagtgg agaccctgtc tctataaaga 25680
aaaaatgtca accaaaaact acatgcagaa aaactgcact tcaagaaatg atcagtacct 25740
tgaagctctg aaggtgctta agactgtaga tcaataccat agaaaataat ttagtattta 25800
ggaatgtaag aaaattaaga cagccttgtt tgataactac acataatact gtaactgttc 25860
ttgcactgtt ctggttattg tcaagctatg agcacaaact gatgactgaa atacagaata 25920
cagaacagga tataaaatct tatcaggtaa agttaggcaa gcaattacta gttgtaattc 25980
aacttgaagg agaaggaata aggaaccaac tcaaaccagg cagcaatgaa ttgtaaaaaa 26040
gcttaaggta aaacaaacag ggaaataaaa caactcagaa cctaagcata tcgtaagaac 26100
ctaatctaac aaggaggggc ttaaactgat tattttacag cttgggtgca attatcccac 26160
aaaaaacttt caggagtttc accagtccat aaactatttg gttattagaa aatagcttta 26220
ttgggctacc ctctttgggt cccctccctt tgtatgggag ctctgttttc actctattaa 26280
atcttgcaac tgcactcttc tggtccgtgt ttgttacggc tcgagctgag ctttcactct 26340
ccatccacca ctgctgtttg ccgccatcgc aggcctgcca ctgacttcca tccctctgga 26400
tctagcaggg tgtccgttgt gctcctgatc cagtgagacg cccattgccg atcccgactg 26460
ggctaaagac ttgccattgt tcctacgcgg ctaagtgccc gggttcatcc taattgagct 26520
gaacactagt cactgggttc cacggttctc ttctgtgacc cgtggcttct aatagagcta 26580
taacactcac cgcgtggccc aagattccat ttattggaat ccatgaggcc aagaacccca 26640
ggtcagagaa cacgaggctt gccatcatct tagaagcagc ccgccaccat cttcggagtt 26700
ctgggagcaa ggaccccctg gtaacaattt ggcgaccaca aagggacctg aacccgcaac 26760
catgaaggga tctccaaagc ggtaatattg gaccactttt gcttgctact ctggcctatc 26820
ccttagaatt ggaggaaaat actgggcacc tgtcggccgg ttaaaaacga ttagcatggc 26880
cgccagactt tagactcagg tatgaggcta tctggggaag ggctttctaa caaccctcaa 26940
cccttctggg ttgggaacct tggtctgcct ggagccagct tccactttca attttcctgg 27000
ggaagccaag ggctgactag aggcagaaag ctgtcgtccc gaactcccgg cattagccgg 27060
ttgagatcat gtcgcagcca gaagtctcta ctcaacagtc gcccatgcgt gcgctcctac 27120
cttcccttct gtcccacacc tcctgggtcc caaccacgac tttcttgaaa gtgtagcccc 27180
aaaattctcc ttacctctga atctacttcc tctgatccct gcctcctagg tactaatggt 27240
tgagactttc atttcctcta gcaagttgta tctccaaagg gatctaagga agctctatgc 27300
tgcgccctta ggcatctagg ctataaaccc agggagtctt gtccctggtg tccctcctga 27360
tttaggtata cagctctaga catgggcagt tatgtgggac ctgttcccca ccacccttgc 27420
cagggcccca agtttgtaaa tggctaagag aggaaacaga gagagacaga gagaaagaga 27480

cagtgagaga cagacagaga cagagagaga gagagacaga gaggagagag agagagacag 27540
ggaggacagg gagagagaca gagaggagag ggagagagac aaagaggaga aagaggcaga 27600
gagacaaaca gggagtcaga gaaagaaaga caaagataga aatagtaaaa aaaaacagtg 27660
tgccctattc ctttaaaagc cagggtaaat gtaaaaccta taattgataa ttgaaggtct 27720
tctccgcgac cctataacac tccaatacta ccttgttgtc agcgtaaaca agggcgtagc 27780
ctgaaaacac taagaccact gacaacccat agccttccta tcaaaaatcc ttaacatcca 27840
gtgacctgcg gatggcccaa atgcattcaa tctgtagcgg caactgcttt gctaacagaa 27900
aaaagtagaa aagtaacttt tagaggaaac ctcattgtga gcacacctca ccggttcaga 27960
attattctaa gtcaaaaaag caaaaaggta gcttattaac tcaaaaatat taaagtatgg 28020
ggctattctg tcagaaaaag gtaatttaac actaaccact gataattccc ttaaccctgc 28080
agatttcctt acaggggatt taaatcttaa ttaccataca aaggtccgac cagacctagg 28140
aggaactccc ttcaggacag gatgatagat ggttcctccc aaatgactga ggaaaaaacc 28200
acaatgggta ttcagtaatt gatagggaga ctcttgtgga agcagagtta gaaaaattgc 28260
ctaataattg gtctcctcaa atgtcagagc tgtttgcact cagccaagcc ttaacgtact 28320
taccgaatca aaaagactat ctcaatcctg actcaaaagc ttacttatac cctctctgaa 28380
acgaatttgc ctaagaactg ttgtttatgg gaatgcatct tgatggagca gctgggttgt 28440
tatgaaatac tcaggaactc agcctagctc taggactcac ccctgagcac aaaggcaatg 28500
ttgggcacgc tggtaaagga ccactagaat ccagcagccc ggaccccttt ctttgtgatc 28560
aagaaaggcg ggaaaagggg tgagggctgc tacatcagtg agcataacta atccgataag 28620
cagaggtcca tgggtggtta cacaccccgg aaaggaataa gcattaggac catagaggac 28680
gctctaggac taatgctcat cggaaaatga ctagtggtgc tggcatccct atgttctttt 28740
ttcagatagg aaacgttccc ctcaaggcaa aaacacccct aagatgtatt ctggagaatt 28800
gggaccaatt tgactctcag atgctaagaa aaaaaagaca tattcttctg cagtaccgcc 28860
tggcaacgat atactcttta agggggagaa acctggcatc ctgagggaag cataaattat 28920
aacaccatct tacagctaga cctcttttgt agaaaagaag gcaaatggtg tgaagtgtca 28980
tacgtacaaa ctttcttttc attaagagac aactcgcaat tatgtaaaaa gtgtgattta 29040
tgccctacag gaagccctca gagtctacct ccctacccca gcatccccca gactccttcc 29100
ccaaataata aggacccccc ttcaacccaa acggtccaaa aggagataga caaaggggta 29160
aacaactaac caaagaatgc caatattccc cgattatgcc ccctccaagc ggtgggagga 29220
gaattcggcc cagccagagt gcacgtacct ttttctctct cagactttaa attaaaatag 29280
acctaggtaa attctcagat aaccctaatg gctatattga tgttttacaa ggtttaggac 29340
aatcctttga tctgatatgg agagatataa tgttactgct aaatcagaca ctaaccccaa 29400
atgacagaag tgtcgccgta actgcagcct gagagtttgg cgatctctgg tatctcagtc 29460
aggtcaatga taggtcgaca acagaggaaa gagaacgatt ccccacaggc cagcaggcag 29520
ttcccagtgt agaccctcac tgggacacag aatcagaaca tggagattgg tgccgcagac 29580
atttgctaac ttgcgtgcta gaaggactaa ggaaaactag aaagaagcct gtgagttatt 29640
caatgatgtc cactataaca cagggaaagg aagaaaatcc taccgccttt ctggagtgac 29700
taacggaggc attgaggaag catacctctc tctgtcaact gactctactg aaggccaact 29760
aatcttaaag gataagttta tcactcagtc agctacagac attaggaaaa aacttcaaaa 29820
gtctgcctta ggcccggaac aaaacttaga aaccctattg aacttggcaa cctcagtttt 29880
ttataataga gatcaggatg agcaggcaga atgggacaaa tgggataaaa aaaaggccac 29940
cgctttagtc atggccctca ggcaagcgga ctttggaggc actggaaaag ggaaaagcta 30000
ggcaaatcaa atgcctaata gggtttgctt ccagtgcggt ctacaaggac actttaaaaa 30060
agattgtcca aatagaaata agccgccccc tcgtccatgc acctcgtgtc aagggaatca 30120
ctgtaaggcc cactgcccca ggggacgtag gtcctctgag tcagaagcca ctaaccagat 30180
gatccagcag caggactgag agtgcccggg gcaagcacca gcccatgcca tcaccctcac 30240
agagccctgg gtatgcttga ccattgacgg ccaggaggct aactgtctcc tggacactgg 30300
tgtggccttc tcagtcttat tttcctgtcc cagacaacgg tcctccagag ctgtcactat 30360
ccaaggggtc ctaggacagc cagtcactag atacttctcc cagccactaa gttgtgactg 30420
gggaacttca ctcttttcac atgcttttct aattatgcct gaaagcccaa ctcccttgtt 30480
agggagagac attctagcaa aagcaggggc cattatacac ctgaacatag gagaacaccc 30540
gtttgttgtc ccctgcttga ggaaggaatt aatcttgaag actgggcaac agaaggacaa 30600
tatggacgag caaagaatgc ccgtcctgtt caagttaaac taaaggattc tgcctccttt 30660
ccccaccaaa ggcagtaccc ccttagaccc gaggctcaac aaggactcca aaagattaag 30720
gacctaaaag cccaaggcct agtaaaagca tgcaatagcc cctacaataa tccaacttta 30780
ggagtacaga aacccagtgg acagtggagg ttagtgcaag atctcaggat tatcaatgag 30840
gtcactgtcc ctctatacct agctgtacct aacccttata ttctgctttc ccaaatacca 30900
gaggaagcag agtggtttac agacctggac cttaaggatg cctttttctg catccctgta 30960
catcctgact ctcaattctt atttgccttt gaagatcctt caaacccaat gtctcaactc 31020
acctggactg tttcacccca agggttcagg gatagccccc atctatttgg ccaggcatta 31080
gcccaagact tgagccggtt ctcatacctg ggcactcttg tcctttggta tgtggatgat 31140
ttttactttt agccgccagt tcagaaacct tgtgccatca agtcacccaa gtgctcttaa 31200
attttctcgc tacctgtggc tacaaggttt ccaaaccaaa ggctcagctc tgctcacagc 31260
aggttaaata cttagggcta aaattatcca aaggcaccag ggccctcagt gcctattctg 31320
gcttatcctc atcccaaaac cctaaagcaa ctaagaggat tccttgacat aacaggtttc 31380
tgccaaatat ggattcccag gtacggcgaa atagccagac cattatatac actaattaag 31440
gaaactcaga aagccaatac ccatttagta agatggacac ctgaagcaga agcggctttc 31500
caggccctaa agaaggccct aacccaagcc ccagtgttta gcttgccaac ggggcaagac 31560

ttttctttac atgtcacaga aaaaaacaga aatagctcta ggagtcctta cacaggtcga 31620
tgagcttgca acccatggca tacctgagta aggaaattga tgtagtggca aagggttggc 31680
ctcattgttt atgggtagtg gcggcagtag cagtcttagt atctgaagca gttaaaataa 31740
tacaaggaag agatctgtgt agacatctca taacgtgaac ggcatactca ctgctaaagg 31800
agacttgtgg ctgtcagaca accgtgagga aagtaactaa aatcgtaaat ccccatggcc 31860
ctcccttatc atatttttct ctttactgtt ctcttacccc ctttcactct cactgcaccc 31920
cctccatgct gctgtacaac cagcagctcc ccttaccaag agtttctatg aagaatgcgg 31980
cttcccagaa atattgatgc cccatcaaat aggagtttac ctaaaggaaa ctccaccttc 32040
actgcccaca cccatatgcc ccacaactgc tataactctg ccactctttg catgcatgca 32100
aatactcatt attggacagg gaaaatgatt aatcctagtt gtcctggaag acttggagcc 32160
actgtctgtc ggacttactt cacccatact ggtatgtctg agggggggtgg agttcaagat 32220
caggcaagag aaaaacatgt aaaggaagta acctcccaac tgacccgggt acatagcacc 32280
cctagcccct acaaaggact agatctctta aaactacatg aaaccctcca tacccatact 32340
tgcctggtaa gcctatttaa taccaccctc actgggctcc atgaggtctc ggcccaaaac 32400
cctactaact gttggatgtg cctccccctg tatttcaggc catgcatttc aatccctgta 32460
cctgaacaat ggaacaacta cagcacagaa ataaacacca cttccgtttt agtaggacct 32520
cttgtttcca atctggaaat aacccatacc tcaaacctca cctgtgtaaa atttagcaat 32580
actgtagaca caaccaactc ccaatgcatc aggtgggtaa ctcctcccac acgaatagtc 32640
tgcctaccct caggaatatt ttttgtctgt ggtaccttag cctatcgttg tttgaatggc 32700
tcttcagaat ctatgtgctt cctctcattc ttagtgcccc catgaccatt tacactgaac 32760
aagatttata caattatgtt gtacctaagc cccacaacaa aagagtactc attcttcctt 32820
ttgttatcgg agcaggagtg ctaggtggac taggttctgg cattggcggt accacaacct 32880
ctactcagtt ctactacaaa ctatctcaag aactcaatgg tgacatggaa tgggttgccg 32940
actccctggt caccttgcaa gatcaactta acttcctagc atcagtagtc cttcaaaatt 33000
gaagagcttt agacttgcta acctctgaaa gagggggaag ctgtttattt ttaggggaag 33060
aatgttgtta ttatgttatt ttagcggaag aatgttgtta ttatgttaat caatcctgaa 33120
ttgtcacaga gaaagttgaa gaaattcgag attgaataca acgtagaaca gaggagcttc 33180
aaaaacacca gaccctgggg cctcctcagc caatggatgc cctggattct ccccttctta 33240
ggatctctag cagctctaat attgatactc ctctttggac cctgtatctt taacctcctt 33300
gttaagtttg tctcttccag aatcaaagtt gtaaagctac aaatcgttct tcaaatggaa 33360
ccccagatga agtccatgac taagatctac cgtggacccc tggaccggcc tactagccca 33420
tgctccaatt gtaatgatat cgaacgcacc cctcccgagg aaatctcaac tgcacaaccc 33480
ctactatgcc ccaattccgc aggaagcagt tagactggtc gtcagccaac ctccccaaca 33540
gcacttgggt tttcctgttg agtgggggga ctgagagaca ggattagctg gatttcctag 33600
gccgactaag aatcccaaag cctagctggg aaggtgacca catccacctt taaacactgg 33660
gcttgcaact tagctcacac ccgaccaatc aggtagtaaa gagagctcac taaaatgcta 33720
attagacaaa aacaggaggt aaaaaaatag ccaatcatct atcgcctgag agcacagcgg 33780
gaaggacaat gatcgggata taaacccagg cattcaagcc ggcaacggct accttctttg 33840
ggtcccctcc ctttgtatgg gagctctctc tgtcttcact ctattaaata ttgcaactgc 33900
aaaaaaaaaa tagcttaatt gaagaataaa ttaatacaat aaaaggaata cattttaagt 33960
atacagttca aactgtaaca gtgttacagt ttcaagagga ccccttcaac aagatattgg 34020
gcatttccat catgccctaa aagttccttc ttgtccctta ctggttgggt ccatctctac 34080
tacaccctcc tgacctggcc cagaccttgg cctcagaaga atcatttttt tgtcactaca 34140
tattagtttt gtctgttcta gaacttctta aaaacagaat catagagtat gttctctttg 34200
tattggttct ttttactcaa tgtaatgttc tgtgacattt atccatatta ttgcatgtat 34260
tattcctttt aatcctgaat agtatgctgt tttaggaata taatgcaatt gtttattcat 34320
ttacctgttg acagatatct gagctattat gatggatatt atgaataatt ctgctatgaa 34380
cacttctgta caatgttttc tcggacatat attttcattt ttcttgagtg gagctgttag 34440
aactgttgga tcagaaagta agcatatgtt gaattttgaa agaaactggt aaactcttgt 34500
ctaaagtgat ttgtaccatt ttacactcct actaataatg tatgagagtt atatttgctc 34560
cacagccttt ttactacttt gttaatcttt ttagtactgt caaccttttt aatttatcca 34620
atctagggaa cgtgaagtag tatctcactg ttattttcat tttcctgatg agtaacaata 34680
tcgtgtatct tttcatgtgc ttattagcca ttcctatatc ttttgtgaaa tagttaactt 34740
aaatttgtaa ctaaaggtgc tttcctgagt ttcaggtagt aagcctattt ccctcaagtg 34800
aataaactac agtcttggaa tgaaaaatta aacacagtgg agacattttt tgtataagtt 34860
gttttactct gtgtatgtct ggtttgctta gtctattatt atatgcccca tgaaagcaaa 34920
cacagtgctt atttcactaa tgagtatcac tagcacatag aactgtgctt gcccaaagca 34980
tgaactcaat aaatatgtta atgtgtatgc atgcacatac atctacatgc atgtacatct 35040
atacacacat ataaacatat attaattttt agacccacaa atctaagaaa actaattctt 35100
gagcctctgg tttgaagaat tctcaaatta ttaacatatc tttatgttcc actccacatc 35160
cactgtacct gaaatagccc tactgttcta ctttggtaaa tcaggcaaat ttaatttttt 35220
aaataattaa gattccaact aattttaaaa tataatttga aagttaacaa tgaaatacat 35280
tacataaaaa gaaaatttta aataaaagca aaactaaacc caataagagg aaagaaagtt 35340
gggctgtatt tctttaatcc tttaaaattc aaatcacaca atgctccaat gaaatcttca 35400
ttaactgaac caaactatgc ccatgaaaga tctcatatgc aactgctaaa acctcaataa 35460
acatattcat cttcttgcaa aaaagatatt tctttataat atgcacatgc agtatatact 35520
attttgaggc agatttgtac tttagtcctt gttccattgc ttaccggctg gctgtccttt 35580
gtctggtcat tgacctccaa cttaaaaaat aatacttgcc ttgtctaccc cacagaagtg 35640

ttatgaaagt caaacaaggt agcataaagg tattttacaa gatataaagt gctataatac 35700
agattttaaa aatcactcta catcccataa tactttgttg tacaatttta gagcaatagt 35760
agaaaataac aattattgcc taattgaaaa tccagtcccg aattccataa aatgtatgat 35820
atgaacatta tagtacatca tattacgagc cccaaataat cactgcttat atagttggtt 35880
aggatttcct tagtttgttc atatagttta tatatttatg cagtccctat tttgtgagag 35940
gcattgtgag gagcataaag acataagcac agtacagagc cttagcttct ctacatttac 36000
taaagaagac ttcttcttgg gtatttaatc aatatttaaa gtattctggg aagaaatgaa 36060
attaacttca tagactgacc ttagattact atcattacaa aaagatgcct gagtgatctg 36120
tctttaacat accagtattt atcttataac tgttatattt acttgaatca gaagtgaagt 36180
ccttttaagc actaagcatc cattctatac tttcttgtct ttacatatga gatacaaatc 36240
atatttttaa aacttttatt tacttttatt ttttagagac ggagtcttgc tctgtagccc 36300
aggctggagt acagtggcat gatcttggct caccacaatc tccacctcca cttcccaggg 36360
ttcaagtgaa caaatcatac ttttaagcac agattctcaa catgtatcct agcatgctac 36420
tgccataact agggtgtgaa ttaagtatta aagacagctt accccaaata ttactgtaac 36480
atatatctct aaatgaaaaa gaacatatta acaactatac ttggatggga ttctgggagc 36540
taacccatcc ctctctcccc tttcctccaa attccatctc ctattaacac accagctctc 36600
ctgagctaag cagctcctgg ggttggggaa gggtgtacat ggagaaagct agaacctcta 36660
cagtgttttc ctctctggga ggaactagca ggcatacgaa cagaaaaagc tgaataaaag 36720
gctgaatcct ttctattcct gaggcagaca gagagaagac cagggaacaa agagacttcg 36780
accaagagcc ctgccaggta ttgatacctt tgatactgag aaaatatctg ggatatgaaa 36840
tacaaatgct aaataagtat ctttgaaata ggggtaaaag aataaagggt cttgatgagt 36900
aaaatgggta gtatttttta ataacctgat aatgagcttt aggaaaaggg aaggtcaacg 36960
ttatggaatg aaaacacaga ggtaccaaat ttaaaagcat aaaaaaaagt ggaggggggg 37020
aacccaataa cttcatcaaa ctagcaaata acttagtatc atttctaatt agaaacgcta 37080
gaaggaaatc acttagatct gataaagact aggctataat tctaactgat gaaacactta 37140
aactgtatca attaatacca gaaaacaaac acagaaaagt ctactagaac catcattatt 37200
cagcacagtc ttggtaatgc aatactataa tagcaatgca ataaagcaag aaaaaaaaaa 37260
gtttgtaaaa acacaatagg atgagatttt tgtttttcca atgccataaa taactagaaa 37320
tggaaacaaa ataaagaaaa acaaaatcta caaaacacct ggaaataaaa agaaaaatgg 37380
tctatttgaa gaaaacctta aaatctatgc agaacataaa acaaaatctg aataaaaaga 37440
aatatcatgt tcttgtctgg gaagacttaa tatcataaga aagtgaatta tatcaaaatt 37500
taaatcgaaa tttaatgtat ttccatctct aatcagacag gacactatgg ggaactgaat 37560
aagtgatttt aaaagtcatg gaaaattaat aactgagaat aaccatgaaa agtatgaaaa 37620
aaggagacaa atgaattgct ccaacagata tcagaacgct aaaattaaat aaaaatacta 37680
ctaggataag aaaatacata tactgatgta atgaataaag aatccagaat tagattccag 37740
taagtcaaac tactttacta taaaccaggg gtggcatatt catccagtgg gaaaaggaca 37800
gtaagaagtg agtaaactat ggcccactgg ccaaattgtg gcctctgcct atttttgcaa 37860
ataaagtttt actgggacaa agccaagcct atcatttgca aattgtctat aaatattttc 37920
atgttacaga atcacacagt ttcaacagag accatcttgt ctacaaagct gaaaatatct 37980
actatctggc ccttgaagaa agtttgccaa accttagttt atataataaa agatcagcta 38040
tctcatagac acctatctca cacaacacat tgtgggaaag gaccttcttt tttttttgag 38100
acggggtctt gctctgttga ccaggctgga ctgtagtggc atgatcatgg ctcactgcag 38160
cctcaacctc ccaggttcaa gtaatgctcc caccacagaa tcccaaacag ctgggagaga 38220
tgtgtgccac tacgcctggc taaggggcct ttttaacaga gaaagaaatc cacatactac 38280
taagaaaaag aagggcatat ttgatatata tttatatttt ttatatagat atcataaaaa 38340
tcaagatgaa ttatacagtt atattttgca atgtgtttga cggtaaaagt ttaatatcta 38400
taaaaattat tttataaaat atctttaata tatttataga tattataata taaaatatct 38460
ataaaattat tttataaaat aaaaagttaa gaagaaaaga taggcaaaac aaaatacagt 38520
gcaatttaca gaaaaccaag tccaaatggt caacaaagat aaaacagatt tataaactca 38580
ctaagtgtga gagaattatt agttaaagta aaaatatctc tctataccca caatactact 38640
aaaaatcaga gttataatgc cctattgctg gtggagatgt aaggggagaa gcatgctctc 38700
atatactgtt agtgaaaatt taaactaata catttttgaa aagtaagctg gcaatttttt 38760
ttttaatctc taccttttga tgcaaaaact catttttggg tacctattcc ataccttaaa 38820
aaaaatacat atgcttactg tagtactgtt tataatggta aaaactagaa aaaaagaaaa 38880
cttgatagtg aatactgaac aaattacagt gcatctacag attaaacata atgcagccat 38940
taaaaaagaa taaattaggc tgggtgcggt ggctcatgcc cgtaatccca gcactttggg 39000
aggccaaagc aggcggatca cttgaggcca ggagttcgag accagcctgg ccaacatggc 39060
aaaaccctgg ctctacaaaa aatacaaaaa ttagtcgggc atggtggtgg gcacctgtag 39120
tcccagctac tcaggaggct gaggcaggag aatcacttga gcctgggaga cagagattgc 39180
agtgagccaa gatcatgcca cagcattcca gtccaggtga cagaacgaga ctctgtctca 39240
acaaaaagaa caaattaaac cctacaactc atcaacaaaa atacccaaac ccaattcaaa 39300
aatgggcaaa ggacttgaat agacatttct tcaaggatga taaacaagca catgaaaaga 39360
tgcagagcac tattcattag tgattacatc ccacatgcat taggatggct agtatgaaga 39420
acagaaaata ataaatattg gtgaagatct gaaaaacaga aacctttgtg cactgttggt 39480
gggaatgtaa agtggtacag ctactacgga aaacagtatg gccattcctc aagaaaataa 39540
aaataaaatt atcttatgat aggaatatgc atttctgggt aaatacccca aataactgaa 39600
aacagggtgt acacccattt caacatttac atgtcaattc aactgggcca gaatacccag 39660
atatttgttc aaatattctt ctggatgctt ctatatatat gttttttggc tgaggttaac 39720

atttaaattg gtggattctg agtacagcag attaccatcc acaatgtagg tgggcctcat 39780
ctactcagtt gaaggtctta cagaaaaaga ctgacctccc ttgagcaaga aagaattcag 39840
gcaacagact gcctttggac tcaactgcaa ctcttccttg agtcaacagc ccatcccatc 39900
accctggctt ggtgagtcca gggtctgatg aggtaggctg cagactcaag gaagagctgc 39960
caaaaccagg aaagccaatt cattaaaata aatctctctc tacacaaaca cacacacaca 40020
ctaccaccac caccatgatg gttctgtttc tctggagaat gctaatacac ccctgttcat 40080
ggcagcatta ttcacaatag ccaaaaggtg gaagcaactc cagcagatga atggagaagc 40140
aaaatgtggt atgtatatac aatggaatat tattaagcct ttaaaaagtg gaaattatat 40200
ctatctatat ctatacacac atactcacac acacacacac acatttatag aagacagggt 40260
ttcaccatgt tgtcaaggct ggtctcgaac tcctgggctc aagcaaaccg cctgcctcag 40320
cttcccaaag tgctgagatt acatgtgtga gccaccacac ccagccaaaa aaaggacatt 40380
ctgacacata atacaatata gataaacaat gaggacatca tgatatgcga aataagcctg 40440
tcacaaaaag gcaattagtg tatgattcct cttgtatgag gtacctatgg atgtcaaatc 40500
cataaagtag aatggggaaa cagagagttg tttaatgggt atagagtttg ttttgcaaga 40560
agaaaagagt tttggagaat gaatgtacaa cagtgtgaac ataattaaca ctactgaaaa 40620
tggttaagat tataaatttt atgttacatt tattttacca tgattaaaaa ttaaaacaaa 40680
ataatattaa ggaaaaatac tataaataac aacaacaaaa aaaacacctc aagcaactta 40740
cattcacctg ggaaacagaa tacatcctat tctgctagag atatatctgc agttcaaaat 40800
ttattacaaa tgatgttgtg tatctttttg aaatgactga aaaactaaat taaaagcaat 40860
aatattcagt ttactaacca gtaagtcctt ctttcatggt tcctgacttt tctgtaagat 40920
gttattgcaa gatatctact aaaatggaaa acaactgaaa aggcaaaatt ataatttctt 40980
atcaacatcg ctaaaaccct ggaggggaag aatcctaaca aacatggcca taatttgcca 41040
catatttcta ctgtcctcac ttttcaaaat ccagaaatca acatttctgg aaacaaaaca 41100
gagtctaaaa tttggctcct tcttcagttt agaaggtgcc aagttaatcc ctgacatcct 41160
agtttccatt ttcaaaaatg tactttttct ctccccaaac cggtatctag attcttaaat 41220
atttttagca catagaagtt aaatagattt gcttaaccaa aatagccagt aaacctccca 41280
aaagaattaa aatattaatg gcgctttaat gatacaaatg aacaacttta cattcaatcg 41340
tcaatgggaa aggaagcaga attctgagga ttatgaaagt aaacaaaacg aagttcaaat 41400
tctactttat tttacttttt tgtaactaat gaacaacttc ttccaaagac aagtaggaaa 41460
tacaaaaatt agccaggcat ggcacatgcc tgtagtcctg gttacttgga aggctgaagt 41520
gggtggatcg cttgagccgg gaaggcagag gctgtagtga gctgagatca catcactgca 41580
ctcaagcctg ggtgacagag caagaccctc tctggggaaa aaaaaaaaaa aaataggctg 41640
ggcgcagtgg ctcacacttg taattccagc actttgggag gctgaggcag gtggttcacc 41700
tgaggtcagg agttctagac cagcctgacc aatatggtga aaccctgtct ctactaaaaa 41760
tacaaaaatt agccaggcat ggtggtgggc aattgtaatc ctagctactc gggaggctga 41820
ggcaggaaaa tcgcctgaac ccaagaggcg gaggtttcag tgagccgaga ttgcactagt 41880
gcactccagc ctgggcgaca gagcaagact tcatctcaaa ataaataaat aagtaagtaa 41940
ataaaattaa aaaatatata aaaataaaac aaagataagt aggaaccatc cttttttttt 42000
tttttttttt ttttttttaa agatagggtc tgtttctgat gcccaggctt gagtgtagtg 42060
gcatgatcat ggctcactgc aaccttgacc tctcaaatac aagtgactct cctacctcag 42120
cctcccaagt agctgggact acaggtgctt accaccccat ccggctcatt taaaaaaatt 42180
tttttgtaga ggtggggtct cactatgttg tatccaggct ggtctcattt taactttatt 42240
agaaaacaag cattgtttta tcagcttctt gtttttttaa aactaaaaat aacactgcta 42300
ggttgtttct atgaagattc tctaaattta tttataacct taagaataac atgtagaaca 42360
aagtagatga ctgaatgatc tttgttgaat aaatatgaat ggatattcaa ataattaaaa 42420
atctcttaag atctcccatt ctttacagga tacagagaaa actcgttaat atggcctgac 42480
ttttaccttt gcagccttat ccaaactctg tggtcaagac aaacaggttg tccttatact 42540
tacaacgtcc ccctttgcct acaaagctct tctcatgact ctttgcctat cttaagttca 42600
cctatctgtc aaatctctgg gaatgcaaca tttcctcaag gtagccttct ctcctcccaa 42660
actagaacaa attcttcctg ggcattaggg tttttattgc actgtatgtc tcttcttcac 42720
agcaatcaca gttccaatgt tatatttgta ttcttagttg atttgtttct ttccaccttt 42780
agactataac cttctaaggg gtcacacata atatcgatca tcagttgtat cccttgtgca 42840
tagcacaggg catggcaggc aaatatgtgt gtaaataaac ttgttgaatg aatcaatgag 42900
acacactttt cttacccaaa gtataatggc aggataacat ttatcaatct attgcttctt 42960
gaaaaacaga tatgatgtgc ttaattttca ttttacatct caaataccaa tgcctaagga 43020
attcacagtc attttacaaa tctttttgac aaatgccttc attaatcacc acctgtttac 43080
aagtgctaaa taacattttg gttacattct gtaacatttc ctgcacttaa tgtcatctct 43140
agaatactgg ctaatatgaa gcacctggac ttcaggaaca caaacctgaa actaacacac 43200
caaactaaac tgttatgtaa atgacagaaa tgacacattt tggtctgcaa catctctaga 43260
tggcttttgg accaattcaa cttttaccac taaaaatcgg tcacctgact atagtcattt 43320
tgagctcatg ataaatgaat tacagatgaa aaataaatag tttgatgaca atctttacaa 43380
aagtttatct tcaaagaata ccaccagtca caggtattct aggctcctat caacttattt 43440
ggtcagggca gacttcactt ttcatgataa ttatgttctg aaaattctac aaacttaatg 43500
attacaaaca aaagtcatag tttgctcata aatcaggcct aggtctggat tctagttctt 43560
ccatttttca tttgttcact gaggcaagtg acttaaaatt ccctagcctc agtttcctca 43620
catgtaaaat cagataatga ttcctattcc taagatggtt ttgaggcttc aacaagataa 43680
gatgggcctc actcaagcat gctcagtact ctgtctctct ctctccggtt atgcagaaat 43740
tctattagga ttctgcaaag taaaataaat atttcagtaa aaattatgcc ctttattaat 43800

gaatctagat tttcagattt tccttaaatt tacttagtaa cttaagggct caaatattat 43860
agagatttgt atctagtatt ttaaagaaat gaaaggtgtt aatcaaaatg ctgcacaaat 43920
aaatgctaca tttaacaaac agaatatcac aaccatacaa actaatcaga tataaagaag 43980
tcagcaacag aaatctgatg ttgcctttag atcacacaat taggcaaaca aaaatagagt 44040
tccatcctcc tttggtcaag gccatggttg aagactgaat accaaatagg gaaataggaa 44100
aagccaggaa atggcaaatt agcaaaaact ggactcctta atttttatat tcatttcat 44160
atctcacttc taaaacttta attaaattca aataaaaacc aaaatggaac tgagataaag 44220
ccaaaaggaa agttatgtag gtcaaatgag aacctatatt gtccttaggc tctttgttgc 44280
tttctgttta aggaaaaact gcccaagtgc cttgacacat taaagatcaa gcaggaggtt 44340
ctgccgagag tccccatctg gcagccaggt tttgtcaagc aaattttgag aattctctac 44400
cctcccactt tctatctaat tatagcactt tataaaaacc attctctctc tgtctctgtc 44460
tctctctctc tctctctctc acacacacac acacacacac acacacacac acacacaccc 44520
tttctctctc tctctctctg aaacttatct gtattataat aacacaacac taggtatgga 44580
ttaatctgac aattttcccc taaaacagaa taaattcaaa aaggaaaacc tttcctctgt 44640
acacatgcac tatattctga caataataat tcctaaatta agtataatac attttcccta 44700
caggagttta aagaagttac agtaaagaat ctcttgtata aatatatatg ccagaacttg 44760
acccaaataa gtgctgagag gtataaatct caaaacagtt tccggactct ttgtgaaatg 44820
tcttcagagt ctgcgatata ttttcttcaa ctaaattata caagtaagat attttgctgg 44880
gctgtgggaa tgccttacgg catgttactg tggagctcat ggtaaaatag aaagaatata 44940
aataattaaa ataaaattga caaatgataa atgatttaat aaattagaaa ttcaaatgcc 45000
gggcactttt ctagaacctg gacacaaagc atgaacctaa caataacccc gccttcatga 45060
aaaatatgga ctatttgaaa attatacctg caacactaaa taaatattct tcattcttcc 45120
agtatattga gatgtttact ttcaattaga caatttgctt tcctctctga acacatagtt 45180
atgtgatggc tctataaaag attttaaaat aactatagaa ggaactattg gtaaagactg 45240
tgggatacta aaaatggcta caaagaaagt tatgacaaaa cctctgagtt tgaatggaag 45300
tcctactaga ttagagtcta agcctgtgac attatgcttc tggttcttgt tcttaaatgc 45360
ttttctcatt aatagtatgt aacttacttc ctggaatgcc attcattaaa aaaatattta 45420
atatttgcta aatgtcaata tttatgccag cacttttaaa gtacagaaac atggagtttc 45480
tttacctcat gcaaatatgc tgtgagaaag acttaagagc ctattgccta ctttgtggta 45540
caacactgaa gactcaccat ccaaaacaaa cagacttagt aaattcttgt gatttgcagt 45600
agttctgttc tataaggtta ccacaaacac tgaaatcatc gctcctgggg gaatacaagg 45660
ttatgtttcc gtgagccctc ggtcacaaca tgttcattaa ctgatcaata cataaccttg 45720
ttctatgtgt gtttctgttt aaaaagagca cttcagtgct acatttggag tctgttttaa 45780
acagcaaaat cactaataaa aagcacaaaa atgtaaaagc atggcactac atacactgtg 45840
acaagaaggc ttgtttatag tatgacagct gagacaagaa ggtagagcct cgctttgatc 45900
aacctctgct gggaaatgag catcaggtga atcaattttt caccactctg aatgaccgta 45960
aaagtgctcc aagtactgac tttggggtta cacataaatt ttagtaagca tgtgaatctg 46020
ccaatatgaa atctacaaat aatgagtacc aaatgcatat gagtcaaata tttcagtgcg 46080
gtatctgact tgattgccac tgaaagacac agtttggaaa acccctaata aataccgttt 46140
agttactatg cagacaaaga gttctacact agagtgcttc aattaagatg tctgaggctt 46200
tcataaatgg atgtttttta aaatgttatt tcctacctga tatattctaa aggggatata 46260
acgaaatcca ttttcttctg caggatattc catgagtttc cgattgatgg cccaaaactg 46320
gtcaaatctg tctgtaatga 46340

<210> 67
<211> 773
<212> ADN
<213> Homo sapiens

<400> 67
actgagagac aggactagct ggatttccta ggctgactaa gaatccctaa gcctagctgg 60
gaaggtgacc acatccacct ttaaacacgg ggcttgcaac ttagctcaca cctgaccaag 120
gaaggtgacc acaccctcct ttaaacacag agcttgtaac tcagctcaca cccgaccaat 180
caggtagtaa agagagctca ctaaaatacc aattaggcta aaaacaggag gtaaagaaat 240
aatcaaatca tctatcgcct gagagcacag ggggagggac aatgatcggg atataaaccc 300
aggcatttga gccagatcag gtaaccctct ttgggtcccc tcacactgta tgggagctct 360
gttttcactc tattaaatct tgcaactgca cactcttctg gtccatgttt gttccggctc 420
aagctgagct tttgctcgcc gtccaccact gctgaatgcc gccattgcag acctgccctt 480
gacttccacc cctccggatc cggcagagtg tccgctgcac tcctgatcca gcgaggcacc 540
cattgccact cccgatcagg ctaaaggctt gccattgttc ctgcacagct aagtgcctgg 600
gttcatccta atcaggctga acactggtcg ctgggttcca cggttctctt ccatgactca 660
cagcttctaa tagagctata acactcacca catggcccaa ggttccattc gttggaatcc 720
atgaggccaa gaaccccagg tcagagaata aaaggcccgc cccatcttgg gag 773

<210> 68
<211> 10

<212> PRT
<213> Homo sapiens

<400> 68
Phe Leu Gly Glu Glu Cys Cys Tyr Tyr Val
1               5                   10

<210> 69
<211> 10
<212> PRT
<213> Homo sapiens

<400> 69
Leu Leu Phe Gly Pro Cys Ile Phe Asn Leu
1               5                   10

<210> 70
<211> 10
<212> PRT
<213> Homo sapiens

<400> 70
Cys Leu Pro Leu Asn Phe Arg Pro Tyr Val
1               5                   10

<210> 71
<211> 10
<212> PRT
<213> Homo sapiens

<400> 71
Gly Leu Leu Ser Gln Trp Met Pro Trp Ile
1               5                   10

<210> 72
<211> 9
<212> PRT
<213> Homo sapiens

<400> 72
Cys Leu Pro Ser Gly Ile Phe Phe Val
1               5

<210> 73
<211> 9
<212> PRT
<213> Homo sapiens

<400> 73
Trp Met Pro Trp Ile Leu Pro Phe Leu
1               5

<210> 74
<211> 10
<212> PRT
<213> Homo sapiens

<400> 74
Ile Arg Trp Val Thr Pro Pro Thr Gln Ile
1               5                   10

<210> 75
<211> 10
<212> PRT
<213> Homo sapiens

<400> 75
Leu Arg Asn Thr Gly Pro Trp Gly Leu Leu
1               5                   10

<210> 76
<211> 10
<212> PRT
<213> Homo sapiens

<400> 76
Leu Arg Thr His Thr Arg Leu Val Ser Leu
1               5                   10

<210> 77
<211> 10
<212> PRT
<213> Homo sapiens

<400> 77
Lys Arg Val Pro Ile Leu Pro Phe Val Ile
1               5                   10

<210> 78
<211> 10
<212> PRT
<213> Homo sapiens

<400> 78
Cys Arg Cys Met Thr Ser Ser Ser Pro Tyr
1               5                   10

<210> 79
<211> 10
<212> PRT
<213> Homo sapiens

<400> 79
Thr Arg Val His Gly Thr Ser Ser Pro Tyr
1               5                   10

<210> 80
<211> 10
<212> PRT
<213> Homo sapiens

<400> 80

Ala Arg Glu Lys His Val Lys Glu Val Ile
1               5                   10

<210> 81
<211> 10
<212> PRT
<213> Homo sapiens

<400> 81
Ser Arg Ile Glu Ala Val Lys Leu Gln Met
1               5                   10

<210> 82
<211> 10
<212> PRT
<213> Homo sapiens

<400> 82
Ser Gln Trp Met Pro Trp Ile Leu Pro Phe
1               5                   10

<210> 83
<211> 9
<212> PRT
<213> Homo sapiens

<400> 83
Cys Tyr Tyr Val Asn Gln Ser Gly Ile
1               5

<210> 84
<211> 9
<212> PRT
<213> Homo sapiens

<400> 84
Phe Tyr Tyr Lys Leu Ser Gln Glu Leu
1               5

<210> 85
<211> 9
<212> PRT
<213> Homo sapiens

<400> 85
Thr Tyr Thr Thr Asn Ser Gln Cys Ile
1               5

<210> 86
<211> 9
<212> PRT
<213> Homo sapiens

<400> 86
Ser Phe Leu Val Pro Pro Met Thr Ile
1               5

<210> 87
<211> 9
<212> PRT
<213> Homo sapiens

<400> 87
Tyr Tyr Val Asn Gln Ser Gly Ile Val
1               5

<210> 88
<211> 9
<212> PRT
<213> Homo sapiens

<400> 88
Leu Phe Asn Thr Thr Leu Thr Gly Leu
1               5

<210> 89
<211> 9
<212> PRT
<213> Homo sapiens

<400> 89
Leu Phe Gly Pro Cys Ile Phe Asn Leu
1               5

<210> 90
<211> 9
<212> PRT
<213> Homo sapiens

<400> 90
Arg Trp Val Thr Pro Pro Thr Gln Ile
1               5

<210> 91
<211> 10
<212> PRT
<213> Homo sapiens

<400> 91
Leu Pro Phe Leu Gly Pro Leu Ala Ala Ile
1               5                   10

<210> 92
<211> 10
<212> PRT
<213> Homo sapiens

<400> 92
Leu Pro Tyr His Ile Phe Leu Phe Thr Val
1               5                   10

<210> 93
<211> 10
<212> PRT
<213> Homo sapiens

<400> 93
Gly Ala Leu Gly Thr Gly Ile Gly Gly Ile
1               5                   10

<210> 94
<211> 10
<212> PRT
<213> Homo sapiens

<400> 94
Leu Pro Phe Val Ile Gly Ala Gly Val Leu
1               5                   10

<210> 95
<211> 9
<212> PRT
<213> Homo sapiens

<400> 95
Arg Arg Pro Leu Asp Arg Pro Ala Ser
1               5

<210> 96
<211> 9
<212> PRT
<213> Homo sapiens

<400> 96
Phe Arg Pro Tyr Val Ser Ile Pro Val
1               5

<210> 97
<211> 9
<212> PRT
<213> Homo sapiens

<400> 97
Arg Arg Ala Leu Asp Leu Leu Thr Ala
1               5

<210> 98
<211> 9
<212> PRT
<213> Homo sapiens

<400> 98
Trp Arg Met Gln Arg Pro Gly Asn Ile
1               5

<210> 99

<211> 10
<212> PRT
<213> Homo sapiens

<400> 99
Asp Arg Ile Gln Arg Arg Ala Glu Glu Leu
1               5                   10


<210> 100
<211> 10
<212> PRT
<213> Homo sapiens

<400> 100
Leu Arg Thr His Thr Arg Leu Val Ser Leu
1               5                   10


<210> 101
<211> 10
<212> PRT
<213> Homo sapiens

<400> 101
Glu Arg Val Ala Asp Ser Leu Val Thr Leu
1               5                   10


<210> 102
<211> 10
<212> PRT
<213> Homo sapiens

<400> 102
Leu Phe Gly Pro Cys Ile Phe Asn Leu Leu
1               5                   10


<210> 103
<211> 10
<212> PRT
<213> Homo sapiens

<400> 103
Gln Phe Tyr Tyr Lys Leu Ser Gln Glu Leu
1               5                   10


<210> 104
<211> 10
<212> PRT
<213> Homo sapiens

<400> 104
Gln Trp Met Pro Trp Ile Leu Pro Phe Leu
1               5                   10


<210> 105
<211> 10
<212> PRT

<213> Homo sapiens

<400> 105
Cys Tyr Tyr Val Asn Gln Ser Gly Ile Val
1               5                   10

<210> 106
<211> 10
<212> PRT
<213> Homo sapiens

<400> 106
Asn Phe Val Ser Ser Arg Ile Glu Ala Val
1               5                   10

<210> 107
<211> 9
<212> PRT
<213> Homo sapiens

<400> 107
Gly Pro Leu Val Ser Asn Leu Glu Ile
1               5

<210> 108
<211> 9
<212> PRT
<213> Homo sapiens

<400> 108
Leu Pro Leu Asn Phe Arg Pro Tyr Val
1               5

<210> 109
<211> 10
<212> PRT
<213> Homo sapiens

<400> 109
Leu Pro Phe Leu Gly Pro Leu Ala Ala Ile
1               5                   10

<210> 110
<211> 10
<212> PRT
<213> Homo sapiens

<400> 110
Glu Pro Lys Met Gln Ser Lys Thr Lys Ile
1               5                   10

<210> 111
<211> 10
<212> PRT
<213> Homo sapiens

<400> 111
Leu Pro Tyr His Ile Phe Leu Phe Thr Val
1               5                   10

<210> 112
<211> 9
<212> PRT
<213> Homo sapiens

<400> 112
Arg Glu Lys His Val Lys Glu Val Ile
1               5

<210> 113
<211> 10
<212> PRT
<213> Homo sapiens

<400> 113
Lys Pro Arg Asn Lys Arg Val Pro Ile Leu
1               5                   10

<210> 114
<211> 9
<212> PRT
<213> Homo sapiens

<400> 114
Val Val Leu Gln Asn Arg Arg Ala Leu
1               5

<210> 115
<211> 10
<212> PRT
<213> Homo sapiens

<400> 115
Ala Val Val Leu Gln Asn Arg Arg Ala Leu
1               5                   10

<210> 116
<211> 9
<212> PRT
<213> Homo sapiens

<400> 116
Leu Pro Phe Val Ile Gly Ala Gly Val
1               5

<210> 117
<211> 9
<212> PRT
<213> Homo sapiens

<400> 117
Asp Leu Tyr Ser Tyr Val Ile Ser Lys

1               5

<210> 118
<211> 10
<212> PRT
<213> Homo sapiens

<400> 118
Thr Glu Gln Asp Leu Tyr Ser Tyr Val Ile
1               5                   10

<210> 119
<211> 2615
<212> ADN
<213> Homo sapiens

<400> 119
gaattccggg aagccagacg gttaacacag acaaagtgct gccgtgacac tcggccctcc 60
agtgttgcgg agaggcaaga gcagcgaccg cgcacctgtc cgcccggagc tgggacgcgc 120
gcccgggcgg ccggacgaag cgaggaggga ccgccgaggc tgcccccaag tgtaactcca 180
gcactgtgag gtttcaggga ttggcagagg ggaccaaggg gacatgaaaa tggacatgga 240
ggatgcggat atgactctgt ggacagaggc tgagtttgaa gagaagtgta catacattgt 300
gaacgaccac ccctgggatt ctggtgctga tggcggtact tcggttcagg cggaggcatc 360
cttaccaagg aatctgcttt tcaagtatgc caccaacagt gaagaggtta ttggagtgat 420
gagtaaagaa tacataccaa agggcacacg ttttggaccc ctaataggtg aaatctacac 480
caatgacaca gttcctaaga acgccaacag gaaatatttt tggaggatct attccagagg 540
ggagcttcac cacttcattg acggctttaa tgaagagaaa agcaactgga tgcgctatgt 600
gaatccagca cactctcccc gggagcaaaa cctggctgcg tgtcagaacg ggatgaacat 660
ctacttctac accattaagc ccatccctgc caaccaggaa cttcttgtgt ggtattgtcg 720
ggactttgca gaaaggcttc actaccctta tcccggagag ctgacaatga tgaatctcac 780
acaaacacag agcagtctaa agcaaccgag cactgagaaa aatgaactct gcccaaagaa 840
tgtcccaaag agagagtaca gcgtgaaaga aatcctaaaa ttggactcca acccctccaa 900
aggaaaggac ctctaccgtt ctaacatttc acccctcaca tcagaaaagg acctcgatga 960
ctttagaaga cgtgggagcc ccgaaatgcc cttctaccct cgggtcgttt accccatccg 1020
ggcccctctg ccagaagact ttttgaaagc ttccctggcc tacgggatcg agagacccac 1080
gtacatcact cgctccccca ttccatcctc caccactcca agcccctctg caagaagcag 1140
ccccgaccaa agcctcaaga gctccagccc tcacagcagc cctgggaata cggtgtcccc 1200
tgtgggcccc ggctctcaag agcaccggga ctcctacgct tacttgaacg cgtcctacgg 1260
cacggaaggt ttgggctcct accctggcta cgcacccctg ccccacctcc cgccagcttt 1320
catcccctcg tacaacgctc actaccccaa gttcctcttg cccccctacg gcatgaattg 1380
taatggcctg agcgctgtga gcagcatgaa tggcatcaac aactttggcc tcttcccgag 1440
gctgtgccct gtctacagca atctcctcgg tgggggcagc ctgccccacc ccatgctcaa 1500
ccccacttct ctcccgagct cgctgccctc agatggagcc cggaggttgc tccagccgga 1560
gcatcccagg gaggtgcttg tcccggcgcc ccacagtgcc ttctccttta ccggggccgc 1620
cgccagcatg aaggacaagg cctgtagccc cacaagcggg tctcccacgg cgggaacagc 1680
cgccacggca gaacatgtgg tgcagcccaa agctacctca gcagcgatgg cagcccccag 1740
cagcgacgaa gccatgaatc tcattaaaaa caaaagaaac atgaccggct acaagaccct 1800
tccctacccg ctgaagaagc agaacggcaa gatcaagtac gaatgcaacg tttgcgccaa 1860
gactttcggc cagctctcca atctgaaggt ccacctgaga gtgcacagtg gagaacggcc 1920
tttcaaatgt cagacttgca acaagggctt tactcagctc gcccacctgc agaaacacta 1980
cctggtacac acgggagaaa agccacatga atgccaggtc tgccacaaga gatttagcag 2040
caccagcaat ctcaagaccc acctgcgact ccattctgga gagaaaccat accaatgcaa 2100
ggtgtgccct gccaagttca cccagtttgt gcacctgaaa ctgcacaagc gtctgcacac 2160
ccgggagcgg ccccacaagt gctcccagtg ccacaagaac tacatccatc tctgtagcct 2220
caaggttcac ctgaaaggga actgcgctgc ggccccggcg cctgggctgc ccttggaaga 2280
tctgacccga atcaatgaag aaatcgagaa gtttgacatc agtgacaatg ctgaccggct 2340
cgaggacgtg gaggatgaca tcagtgtgat ctctgtagtg gagaaggaaa ttctggccgt 2400
ggtcagaaaa gagaaagaag aaactggcct gaaagtgtct ttgcaaagaa acatggggaa 2460
tggactcctc tcctcagggt gcagccttta tgagtcatca gatctacccc tcatgaagtt 2520
gcctcccagc aacccactac ctctggtacc tgtaaaggtc aaacaagaaa cagttgaacc 2580
aatggatcct taagattttc agaaaacact tattt                            2615

<210> 120

<211> 29
<212> PRT
<213> Homo sapiens

<400> 120
Leu Gln Asn Arg Arg Ala Leu Asp Leu Leu Thr Ala Glu Arg Gly Gly
1               5                   10                  15

Thr Cys Leu Phe Leu Gly Glu Glu Cys Cys Tyr Tyr Val
            20                  25

<210> 121
<211> 21
<212> ADN
<213> Homo sapiens

<400> 121
cttcaaacaa caaccaggag g                                                21

<210> 122
<211> 20
<212> ADN
<213> Homo sapiens

<400> 122
ttggggaggt tggccgacga                                                  20

## Description

La présente invention est relative à une nouvelle famille de séquences nucléiques et de séquences protéiques déduites, qui présentent des motifs rétroviraux endogènes humains complets ou partiels, ainsi que des séquences flanquantes ou adjacentes desdites séquences, et contrôlées par ces dernières : modification de l'expression ou altération de la structure (polyadénylation, épissage alternatif...) desdites séquences flanquantes.

L'invention est également relative à la détection et/ou à l'utilisation desdites séquences nucléiques et desdites séquences protéiques correspondantes, dans le cadre d'applications diagnostiques, prophylactiques et thérapeutiques, en particulier pour des neuropathologies à composante autoimmune comme la sclérose en plaques.

L'invention concerne aussi l'obtention de sondes nucléiques double brins et simple brin anti-sens, de ribozymes, aptes à moduler la réplication virale (T.R. Cech, Science, 1987, 236, 1532-1539 ; R.H. Symons, Trends Biochem. Sci., 1989, 14, 445-450) des molécules recombinantes correspondantes, et des anticorps associés.

Les rétrovirus sont des virus qui se répliquent uniquement en utilisant la voie inverse du traitement classique de l'information génétique. Ce processus, nommé transcription inverse, est médié par une ADN polymérase ARN dépendante ou transcriptase reverse, codée par le gène *pol.* Les rétrovirus codent aussi au minimum pour deux gènes additionnels. Le gène *gag* code pour les protéines du squelette, matrice, nucléocapside et capside. Le gène *env* code pour les glycoprotéines d'enveloppe. La transcription rétrovirale est régulée par des régions promotrices ou *" enhancers* ", situées dans des régions hautement répétées ou LTR *(Long Terminal Repeat)* et qui sont présentes aux deux extrémités du génome rétroviral.

Lors de l'infection d'une cellule, la polymérase fait une copie ADN du génome ARN ; cette copie peut alors s'intégrer dans le génome humain. Les rétrovirus ne tuent pas les cellules qu'ils infectent, mais au contraire améliorent souvent leur rapidité de croissance. Les rétrovirus peuvent infecter des cellules germinales ou des embryons à un stade précoce ; ils peuvent dans ces conditions, intégrer la lignée germinale et être transmis par transmission mendélienne verticale, ce qui constitue la relation la plus étroite entre un hôte et son parasite. Ces virus endogènes peuvent dégénérer au cours des générations de l'organisme hôte et perdre leurs propriétés initiales. Cependant certains d'entre eux peuvent conserver tout ou partie de leurs propriétés ou des propriétés des motifs les composant, ou encore acquérir de nouvelles propriétés fonctionnelles présentant un avantage pour l'organisme hôte, ce qui expliquerait la préservation de leur séquence.

L'existence de motifs endogènes présentant de longs cadres de lecture ouverts et/ou soumis à une forte pression de sélection peut donc être indicatrice d'une fonction biologique préservée ou acquise, qui peut correspondre à un bénéfice pour l'organisme hôte. Ces séquences rétrovirales peuvent aussi subir, au cours des générations, des modifications discrètes qui vont être à même de réveiller certaines de leurs potentialités et engendrer ou favoriser des processus pathologiques. Il est apparu récemment nécessaire de faire le bilan et d'identifier ces séquences afin de pouvoir évaluer leur impact fonctionnel.

Les séquences rétrovirales endogènes humaines ou HERVs représentent une part importante du génome humain. Ces régions rétrovirales se présentent sous plusieurs formes :
- des structures rétrovirales endogènes complètes associant des motifs *gag, pol* et *env,* flanqués de séquences nucléiques répétées, qui montrent une analogie significative avec la structure LTR-*gag-pol-env*-LTR des rétrovirus infectieux,
- des séquences rétrovirales tronquées ; par exemple, les rétro-transposons sont privés de leur domaine *env* et les rétroposons ne possèdent pas les régions *env* et LTR.

Jusqu'à présent l'étude de ces régions du génome a été négligée chez l'Homme pour deux raisons essentielles :
- l'existence d'insertions/délétions qui peuvent décaler le cadre de lecture et de mutations qui modifient la séquence. Ces modifications entraînent des altérations de la structure et par conséquent de la fonction biologique de ces motifs.
- l'absence d'associations avérées avec des pathologies humaines.

La connaissance, récente de fragments significativement représentatifs du génome humain et une orientation des recherches vers une étude structure/fonction des motifs rétroviraux endogènes, ont permis de préciser l'intérêt de ces régions. L'implication de séquences endogènes tronquées ou complètes dans des pathologies chez l'animal est documentée ; par exemple leur association avec des processus tumoraux a été clairement mise en évidence (S.K. Chattopadhyay et coll., 1982, Nature, 295, 25-31). Une recherche visant à préciser l'association ou l'influence des HERVs dans des pathologies humaines se justifie donc aujourd'hui.

Une classification des éléments HERV a été proposée (Tönjes R.R. et al., AIDS & Hum. Retrovirol., 1996, 13, S261-S267; A.M. Krieg et al., FASEB J., 1992, 6, 2537-2544). Elle est basée sur une homologie de ces séquences avec des rétrovirus isolés chez les animaux, à l'aide de sondes rétrovirales hétérologues. En effet, en général, les HERVs présentent relativement peu d'homologie avec des rétrovirus infectieux humains connus.

Les familles de classe I présentent une homologie de séquence avec les rétrovirus de mammifères de type C ; on peut citer notamment la superfamille ERI, proche du virus MuLV *(murine leukemia virus)* et du virus BaEV (*baboon endogenous virus).*

Les familles de classe II présentent une homologie de séquence avec les rétrovirus de mammifères de type B tel que le MMTV *(mouse mammary tumour virus)* ou les rétrovirus de type D tel que le SRV *(squirrel monkey retrovirus).*

D'autres familles ont également été décrites ; parmi celles-ci, on peut citer des HERVs qui présentent, de manière exceptionnelle, une homologie partielle avec HTLV-1 (RTVL-H) ou des virus de primates ; HRES-1, par exemple, présente une homologie de séquence avec des HTLVs.

Les programmes de très grand séquençage du génome humain permettent aujourd'hui de disposer d'un nombre significatif de nouvelles séquences rétrovirales. L'usage de logiciels de traitement de données permet d'identifier et d'analyser ces gènes. Dans ce contexte une recherche systématique portant sur l'ensemble des informations disponibles à ce jour a été engagée afin d'identifier de nouvelles séquences rétrovirales endogènes humaines en fonction de certains critères d'analyse :
- présence de longs cadres de lecture ouverts conservés au cours de l'évolution de l'organisme hôte et pouvant laisser envisager une fonction biologique,
- analogie avec des séquences déjà caractérisées en dehors ou dans le domaine des rétrovirus,
- localisation dans des régions de susceptibilité pour certaines pathologies ou à proximité de gènes essentiels, par exemple dans les domaine du cancer, des régulation du système immunitaire ou dans certaines neuropathologies.

Les recherches effectuées par les Inventeurs, dans des bases de données de séquences leur ont permis d'identifier un ensemble de séquences ou de motifs rétroviraux endogènes dont l'expression normale ou pathologique peut favoriser ou perturber un effet protecteur vis-à-vis de processus pathologiques, ou intervenir dans le déclenchement ou l'aggravation de pathologies.

La présente invention a pour objet un fragment d'acide nucléique purifié, caractérisé en ce qu'il comprend tout ou partie d'une séquence codant pour une séquence rétrovirale endogène humaine, qui présente au moins des motifs rétroviraux de type *env,* répondant à la séquence SEQ ID NO:1 ou à une séquence présentant un niveau d'homologie avec ladite séquence SEQ ID NO:1 supérieur ou égal à 80% sur plus de 190 nucléotides ou supérieur ou égal à 70 % sur plus de 600 nucléotides pour les domaines de type *env.*

On entend par séquence homologue, aussi bien une séquence qui présente une identité complète ou partielle avec la séquence SEQ ID NO:1 précitée qu'une séquence qui présente une similarité partielle avec ladite séquence SEQ ID NO:1.

Selon un mode de réalisation avantageux dudit fragment, il présente à la fois des motifs rétroviraux correspondant à un domaine *env* et répondant à la séquence SEQ ID NO:1 et des motifs rétroviraux correspondant à un domaine *gag* et répondant à la séquence SEQ ID NO:2 ou à une séquence présentant un niveau d'homologie supérieur ou égal à 80 % sur plus de 190 nucléotides ou supérieur ou égal à 70 % sur plus de 600 nucléotides pour les domaines de type *env* et un niveau d'homologie supérieur ou égal à 90 % sur plus de 700 nucléotides ou supérieur ou égal à 70 % sur plus de 1200 nucléotides pour les domaines de type *gag,* lesquels motifs ne présentent aucune insertion ou délétion de plus de 200 nucléotides.

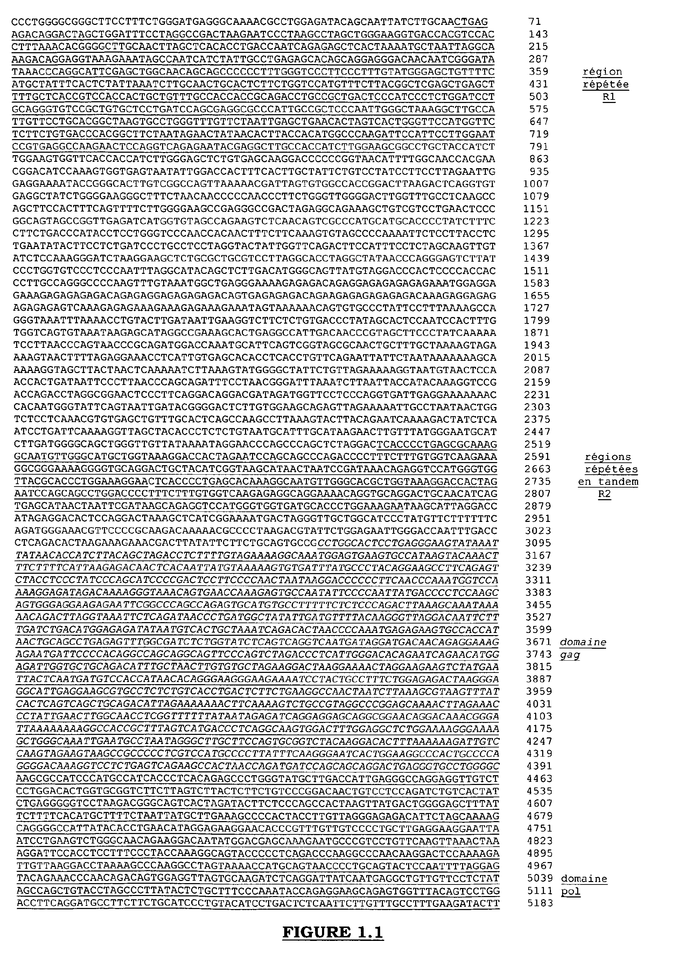

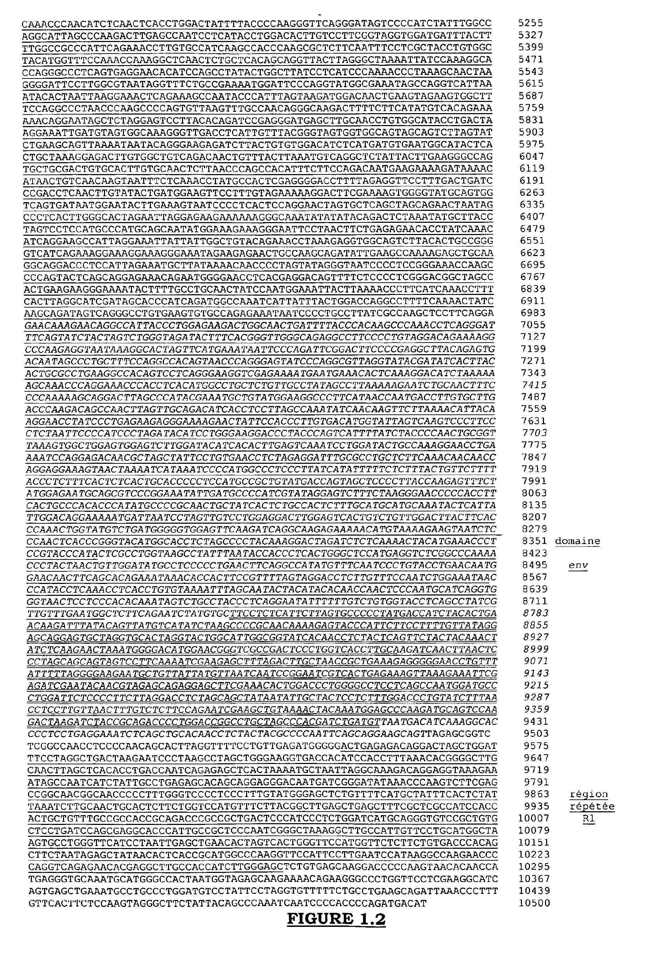

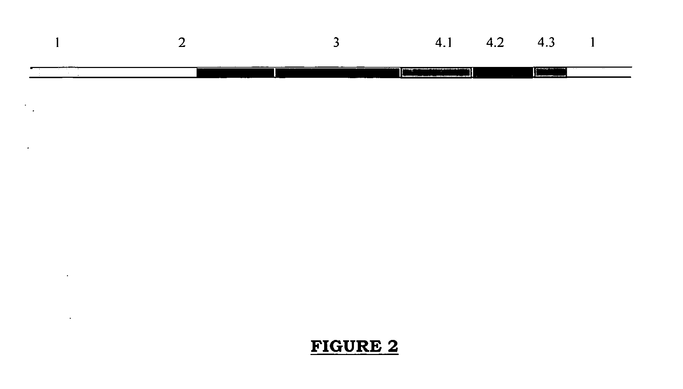

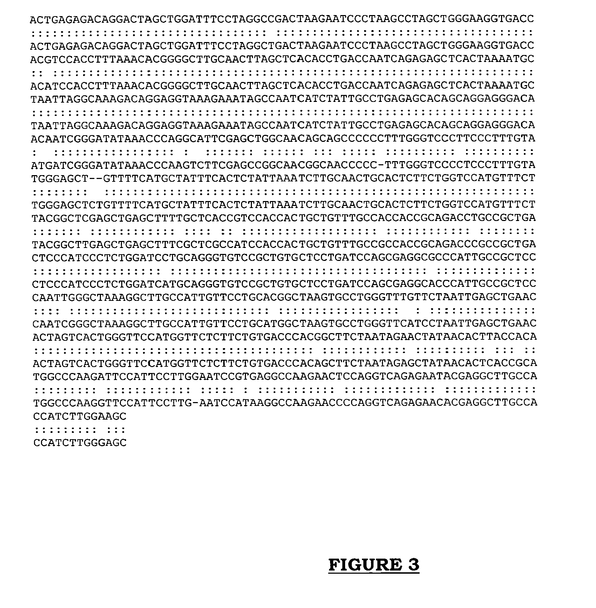

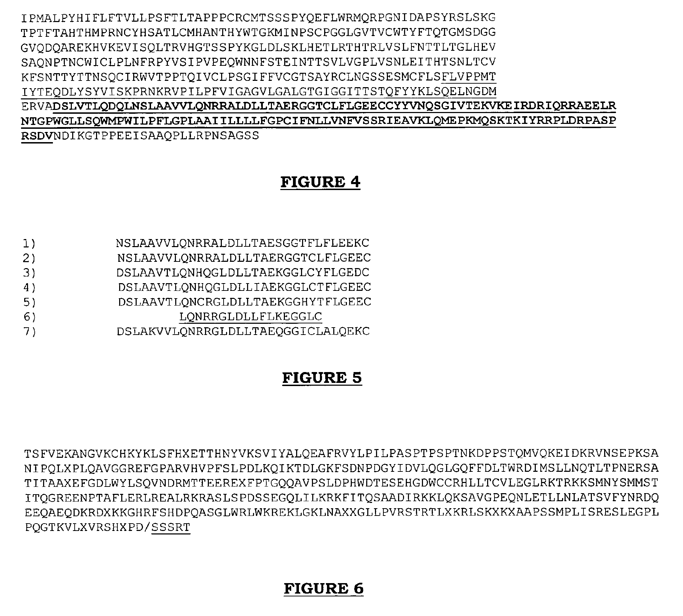

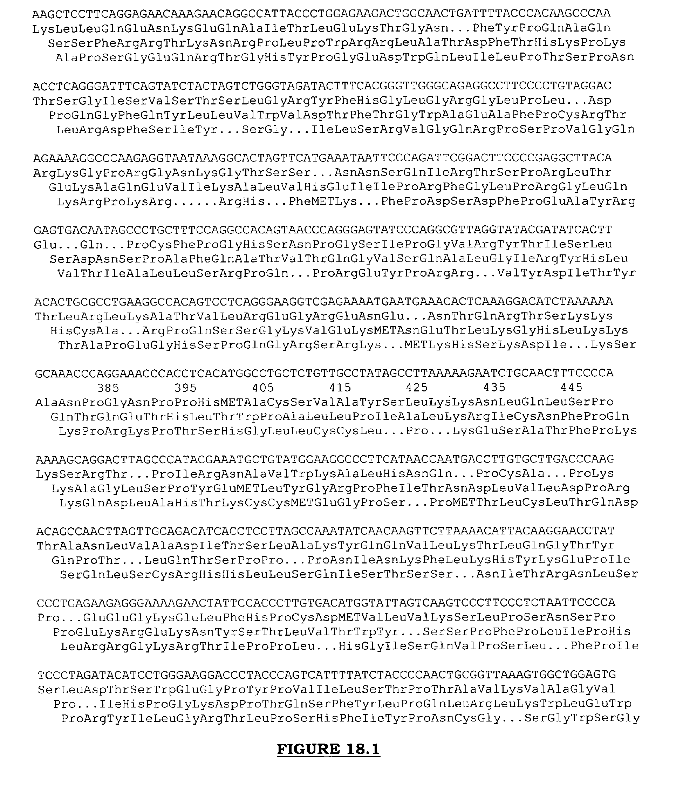

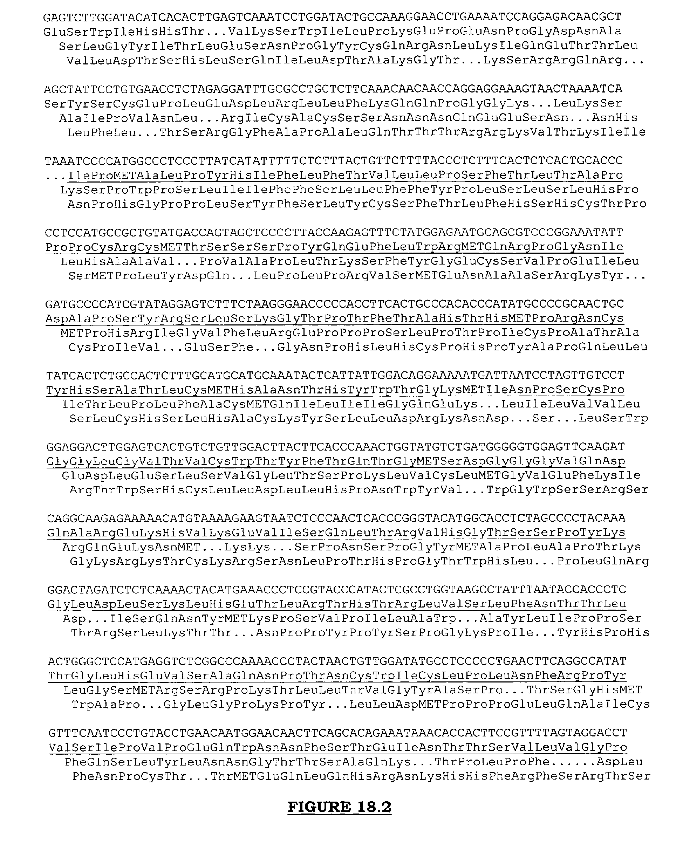

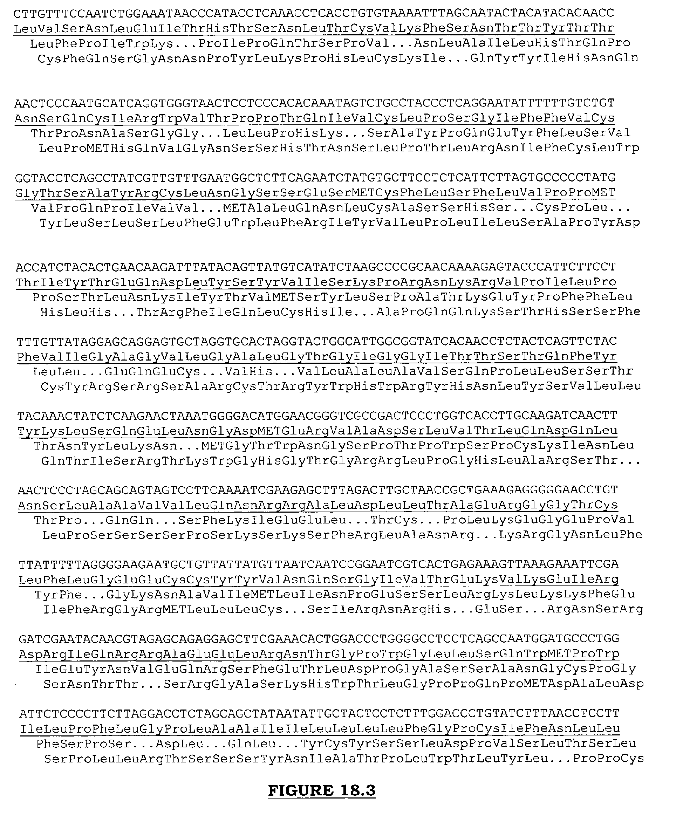

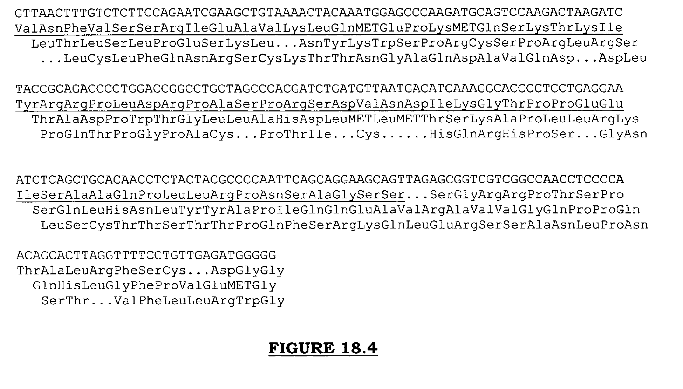

Lesdits fragments constituent une nouvelle famille de séquences rétrovirales endogènes humaines (famille HERV-7q) qui présente une homologie de séquence avec les rétrovirus MSRV, tels que décrits dans la Demande Internationale WO 97/06260 ; lesdits fragments selon la présente invention présentent :
- deux motifs nucléotidiques répétés de 711 pb (figure 3), présentant des signaux caractéristiques identifiés dans des LTRs *(Long Terminal Repeats) :* promoteurs de transcription de type boîtes TATAA ou CCAAT. Ces domaines répétés encadrent trois motifs déduits de type-gag, *pol* et *env* (figure 2).
- un motif de type *env* (positions 6965 nt - 9550 nt sur la séquence SEQ ID NO :3 ou sur la figure 1) qui contient un long cadre de lecture ouvert de 1620 nucléotides (positions 7874-9493 de la séquence ID NO:3 et figure 1), codant pour une protéine de séquence inédite de 540 acides aminés appelée envérine (figure 4 et SEQ ID NO:26) et fragment souligné de la figure 18. On retrouve à l'intérieur du domaine trans-membranaire de ce domaine *env,* un motif peptidique de type CKS-25/CKS-17 (figure 5), reconnu pour présenter des fonctions immunosuppressives sur les cellules lymphocytaires hôtes (M. Mitani et coll., 1987, Proc. Natl. Acad. Sci. USA, 84, 237-240). Un domaine de type doigt de zinc *(zinc-finger)* **HX₃₋₄HX₂₂₋₃₃CX₂C** (Kulkolski et coll., 1992, Mol. Cell. Biol., 12, 2331-2338), que l'on retrouve dans des domaines de type intégrase est identifié dans un autre cadre de lecture. Ce domaine *env* particulier signe la caractéristique de nouveaux motifs rétroviraux endogènes.
- le motif (positions 3065 nt - 4390 nt sur la séquence SEQ ID NO:3) de type-gag codant pour des motifs protéiques selon la figure 6 (SEQ ID NO:58) (positions 3118-4198 de la SEQ ID NO:3) a été identifié grâce à des analogies avec des domaines *gag* connus. On retrouve, par exemple, la région d'homologie majeure **QX₃EX₇R** (Benit et coll., 1997, J Virol., 71, 5652-5657). Le motif de fixation des acides nucléiques **CX₂CX₃₋₄HX₄C,** situé en position C-terminale, est identifié dans un autre cadre de lecture (Covey et coll., 1986, Nucleic Acids Res., 14, 623-633). En amont du domaine *gag* on détecte un motif de 182 nucléotides répété deux fois (figure 1).
- le domaine *pol* présente les consensus classiques d'une région *pol* de rétrovirus au niveau des domaines protéase, transcriptase reverse et RNAse H. On retrouve dans *pol* un motif proche du consensus **LLDTGA** (Weber et coll., 1988, Science, 243, 928-931). Les motifs **D** et **AF, LPQ** et **SP**, et **YVDD** (Xiong et Eickbush, 1990, EMBO J., 9, 3353-3362), sont respectivement retrouvés dans les 3°, 4° et 5° boîtes d'homologie. Les motifs **YTDGSS** et **TDS** sont présents dans la région de la RNAse H,
- les régions *gag* et *pol* pourraient être considérées comme jointives avec un passage de la région *gag* à la région *pol* par un décalage du cadre de lecture.

La présente invention englobe les séquences appartenant à la famille HERV-7q telle que définie ci-dessus (présence de la séquence SEQ ID NO:1 ou d'une séquence homologue ou présence à la fois des séquences SEQ ID NO:1 et SEQ ID NO:2) et notamment les séquences SEQ ID NO:3-22, 28 et 61 ; elle englobe également les séquences nucléiques complémentaires et les séquences inverses complémentaires des séquences précédentes ainsi que les fragments issus des régions codantes des séquences précédentes correspondant à un cadre glissant supérieur ou égal à 14 nucléotides ou leurs séquences complémentaires. (SEQ ID NO :37-57, 59-60 et 121-122).

Ces différents fragments peuvent avantageusement être utilisés comme amorces ou comme sondes (réactifs A) ; ils s'hybrident spécifiquement dans des conditions de forte stringence à une séquence de la famille HERV-7q.

Parmi ces fragments, on peut citer, de préférence les fragments suivants:
- un fragment de 182 nucléotides répété deux fois, situé en amont du domaine *gag* aux positions 2502-2611/2613-2865 de la SEQ ID NO:3;
   Amorces et sondes spécifiques de la région *gag*
- une amorce G1F, sens, localisée dans la région amont du domaine *gag de HERV-7q* : 5' GGACCATAGAGGACACTCCAGGACTA 3' (SEQ ID NO:37);
- une amorce G1R, anti-sens, localisée dans la région 3' terminale du domaine *gag* : 5' CCTCAGTCCTGCTGCTGGATCATCT 3' (SEQ ID NO:38)
- le fragment de 1505 nt amplifié par le couple G1F-G1R est utilisé afin de générer les sondes aptes à hybrider les différents produits d'amplification des PCR;
- une amorce G2F, sens nichée : (SEQ ID NO:39)
   5' CCTCCAAGCAGTGGGAGGAAGAGAATT 3'
- une amorce G2R, anti-sens nichée : (SEQ ID NO:40)
   5' CCTTCCCTGTGTTATTGTGGACATCATT 3'
- une amorce G4F, sens nichée : (SEQ ID NO:41)
   5' GGAAGAAGTCTATGAATTATTCAATGATGT 3'
- une amorce G3F, sens nichée: (SEQ ID NO:42)
   5'GGGACACAGAATCAGAACATGGAGATT3'
- une amorce G4R, anti-sens nichée : (SEQ ID NO:43)
   5' GCCTTCAGAAGAGTCAGGTGACAGAGA 3'
- une amorce G5R, anti-sens nichée : (SEQ ID NO:44)
   5'GAGCCTCCAAAGTCCACTTGCCTGA 3'
   Amorces et sondes spécifiques de la région env
- une amorce E1F, sens : (SEQ ID NO:45)
   5' GATTTCAGTATCTACTAGTCTGGGTAGAT 3'
- une amorce E1R, anti-sens : (SEQ ID NO:46)
   5' CTAGGAAATCCAGCTAGTCCTGTCTCA 3'
- le fragment de 2529 nt amplifié par le couple d'amorces E1F-E1R, est utilisé afin de générer les sondes aptes à hybrider les différents produits d'amplification des PCR.
- une amorce E2F, sens : (SEQ ID NO:47)
   5' CCAAGACAGCCAACTTAGTTGCAGACAT 3'
- une amorce E2R, antisens : (SEQ ID NO:48)
   5' GGACGCTGCATTCTCCATAGAAACTCTT 3'
- une amorce E3F, sens : (SEQ ID NO:49)
   5' GCAATACTACATACACAACCAACTCCCAA 3'
- une amorce E3R, anti-sens : (SEQ ID NO:50)
   5' GGGGGAGGCATATCCAACAGTTAGTA 3'
- une amorce E4F, sens : (SEQ ID NO:51)
   5'CCATCTACACTGAACAAGATTTATACACTT3'
- une amorce E4R, anti-sens : (SEQ ID NO:52)
   5'AATGCCAGTACCTAGTGCACCTAGCACT3'
- une amorce E5F, sens : (SEQ ID NO:53)
   5'CGAATACAACGTAGAGCAGAGGAGCTTCGAA3'
- une amorce E6F, sens : (SEQ ID NO:54)
   5'AGCCCAAGATGCAGTCCAAGACTAAGAT3'
- une amorce E5R : (SEQ ID NO:55)
   5'GCGTAGTAGAGGTTGTGCAGCTGAGAT 3'
- une amorce ExF : (SEQ ID NO:56)
   CCCTTACCAAGAGTTTCTATGGAGAAT
- une amorce ExR : (SEQ ID NO:57)
   ACCGCTCTAACTGCTTCCTGCTGAATT

Tous les oligonucléotides sont conçus pour pouvoir générer une amorce sens et une amorce anti-sens par un décalage de la séquence de l'amorce de référence de 1 à 7 nucléotides vers le côté 5' ou vers le côté 3': la modification de la séquence peut entraîner une modification de la taille de l'amorce de 1 à 7 nucléotides selon les cas. Les amorces choisies peuvent être optimisées selon les cas par un raccourcissement ou un allongement portant sur 1 à 9 nucléotides.

De manière préférée, l'hybridation, le clonage, le sous-clonage, l'obtention, la préparation et l'analyse des acides nucléiques, des peptides et des anticorps, le séquençage des acides nucléiques et des peptides, l'hybridation *in situ* et l'immunohistochimie sont réalisés dans les conditions décrites dans les ouvrages suivants :
- Current Protocols in Molecular Biology. Eds. F.M Ausubel, R. Brent & R.E Kingston et coll. Green Publishing associates and Wiley Interscience.
- Molecular Cloning: a laboratory manual. Eds. J. Sambrook, E.F. Fritsch & T. Maniatis. Cold Spring Harbor Laboratory Press. Cold Spring Harbor.
- The Practical Approach series. Eds. D. Rickwood & B.D. Ames. IRL Press and Oxford University Press. En particulier, antibodies I & II; DNA cloning I, II, III; Nucleic acid and protein sequence analysis; Nucleic acid hybridization; Nucleic acid sequencing ; Oligonucleotide synthesis; Protein purification applications; Protein purification methods; Protein sequencing; Transcription and translation; Gels electrophoresis of nucleic acids; Gels electrophoresis of proteins; Genome analysis; HPLC of macromolecules; Human genetic diseases; Microcomputing in biology; Molecular neurobiology; Mutagenicity testing; Essential molecular biology I & II.
- Proteome research: New frontiers in functional genomics. Eds M.R. Wilkins & coll.. Springer.

La séquence rétrovirale endogène humaine (SEQ ID NO:3), située sur le bras long du chromosome 7 correspond à la séquence HERV-7q ; elle présente 10,5 kb (fig. 1 et 2) et répond aux critères précédemment définis.

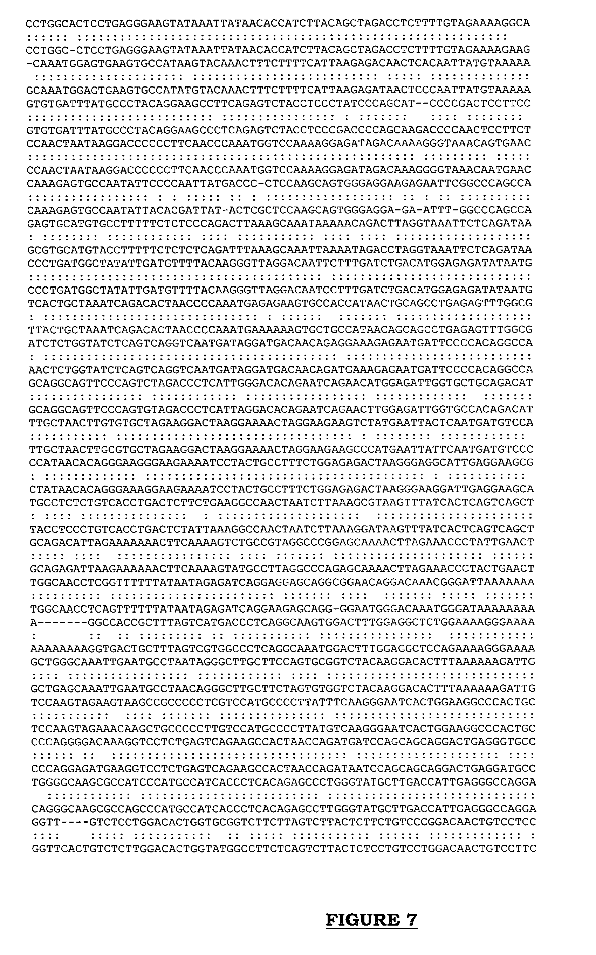

La recherche de domaines présentant des similitudes, tout ou partie, avec les régions *gag* et *env* de HERV-7q a abouti à l'identification de nouvelles séquences rétrovirales endogènes. Ces séquences peuvent présenter la structure d'un rétrovirus endogène complet comme la séquence rétrovirale endogène située à proximité du gène des sous-unités alpha et delta du récepteur des cellules-T, et dénommée en conséquence HERV-TcR ; à titre d'exemple la figure 7 montre la comparaison des alignements nucléiques des domaines *gag* respectifs de HERV-7q et HERV-TcR (séquence HG12, SEQ ID NO:19). On trouve aussi des structures rétrovirales partielles. Ces domaines rétroviraux similaires à HERV-7q sont identifiées dans des séquences nucléiques indépendantes comme le montre leur localisation chromosomique. Des motifs nucléiques (appelés ici, HEx ou HGx et respectivement analogues à des domaines de type *env* ou *gag*) ressemblant aux domaines *env* ou *gag* de HERV-7q ont été retrouvés, à l'aide des banques de données précitées :
- HE2 : chromosome 17 (SEQ ID NO:4),
- HE3 et HG3: chromosome 6 (SEQ ID NO:5 et 6),
- HE4 : chromosome X (SEQ ID NO:7),
- HE5 : chromosome X q22 (SEQ ID NO:8),
- HE6 et HG6 : chromosome 1 q23.3-q24.3 (SEQ ID NO:9 et 10),
- HE7 : chromosome 7 p15 (SEQ ID NO:11),
- HE8 et HG8 : chromosome 19 (SEQ ID NO:12 et 13),
- HE9 : chromosome X (SEQ ID NO:14),
- HE 10 : chromosome X q13.1-21.1 (SEQ ID NO:15),
- HE11 et HG11 : chromosome 7 q21-22 (SEQ ID NO:16 et 17),
- HE 12 et HG 12, dans HERV-TcR : chromosome 14 q11.2 (SEQ ID NO:18 et 19),
- HE13 (SEQ ID NO:61): chromosome 6 q24.1-24.3

La présente invention englobe également les fragments codants et non codants pour tout ou partie de l'envérine comprenant au moins 14 nucléotides et notamment les fragments codant pour la partie C-terminale de l'envérine, soit à partir de l'acide aminé 291, soit à partir de l'acide aminé 321, à compter de la première méthionine.

Ces fragments comprennent en particulier une zone critique où deux insertions de 12 nucléotides ont été caractérisées :
- une première insertion a été identifiée (séquence A), chez des individus de 2 groupes (malades et témoins). Cette insertion située entre les acides aminé 487 et 488, permet d'insérer le tétrapeptide VLQM. Une analyse comparative montre que cette insertion est identifiée dans une région homologue située dans la séquence HE13, appartenant à la famille HERV-7q. L'amplification de la séquence de type HE13, pourrait indiquer qu'il existe une altération de la séquence de l'envérine de HERV-7q, ce qui favoriserait l'amplification de la séquence contenue dans HE13. Cette observation permet aussi d'utiliser cette insertion comme élément spécifique d'amplification de séquences de type HE13.

Une deuxième insertion (séquence B) a été identifiée chez un patient présentant une SEP. L'insertion de 12 nucléotides est située au niveau de l'acide aminé 495 et code pour le tétrapeptide MQSM. Il est remarquable de constater que cette insertion est aussi identifiée dans une région homologue située dans HE 13.
Séquence A: TAAACTACAAATGGTTCTTCAAATGGAGCCCA (SEQ ID NO:59)
Séquence B: GATGCAGTCCAAGATGCAGTCCATGACTAAGA (SEQ ID NO:60).

Ces observations mettent en évidence des modifications de la séquence de l'envérine de type HERV-7q qui constituent la base d'une stratégie de détection par amplification spécifique d'allèles (AS-PCR), permettant de détecter ces différences dans une population et qui pourraient correspondre, soit à une mutation/délétion associée à une certaine susceptibilité, soit à un polymorphisme, soit à une mutation/délétion associée à une pathologie comme la sclérose en plaques.

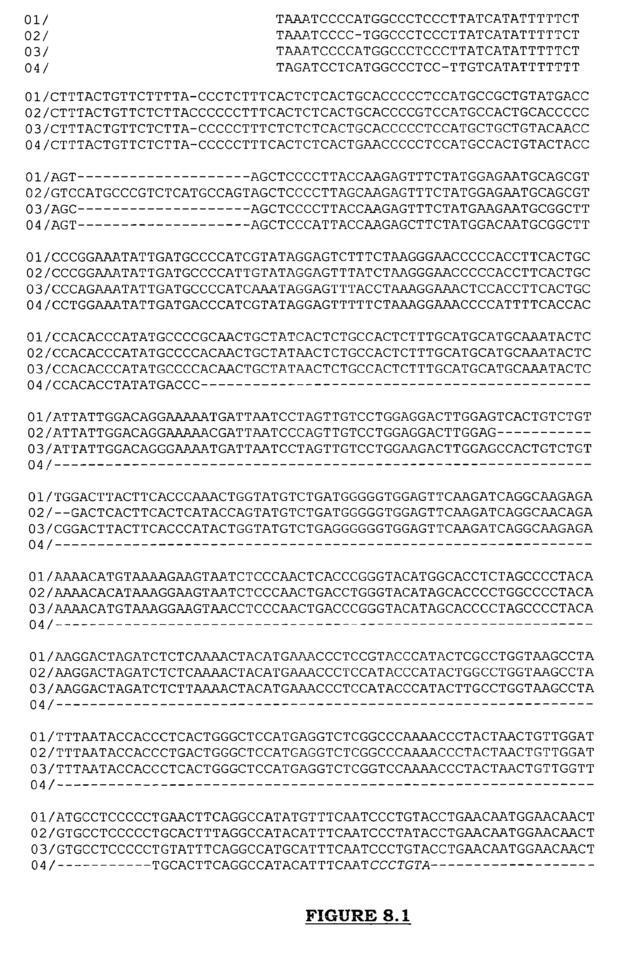

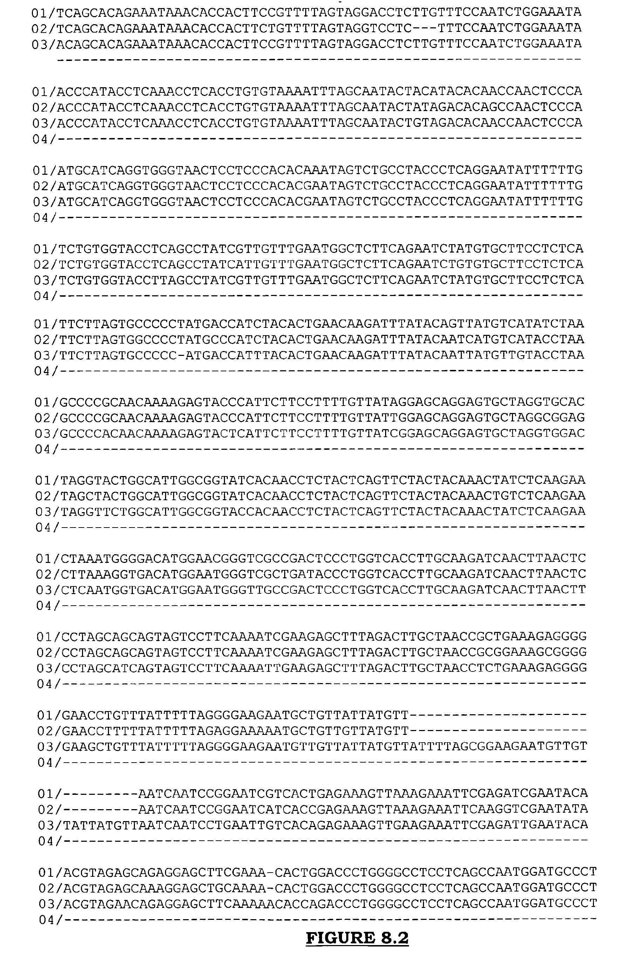

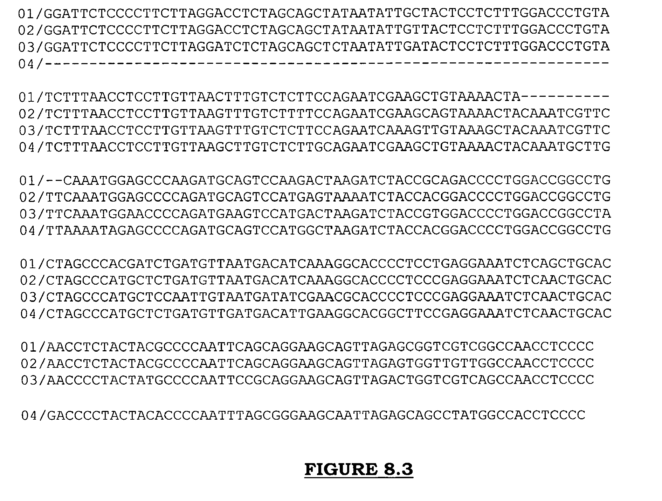

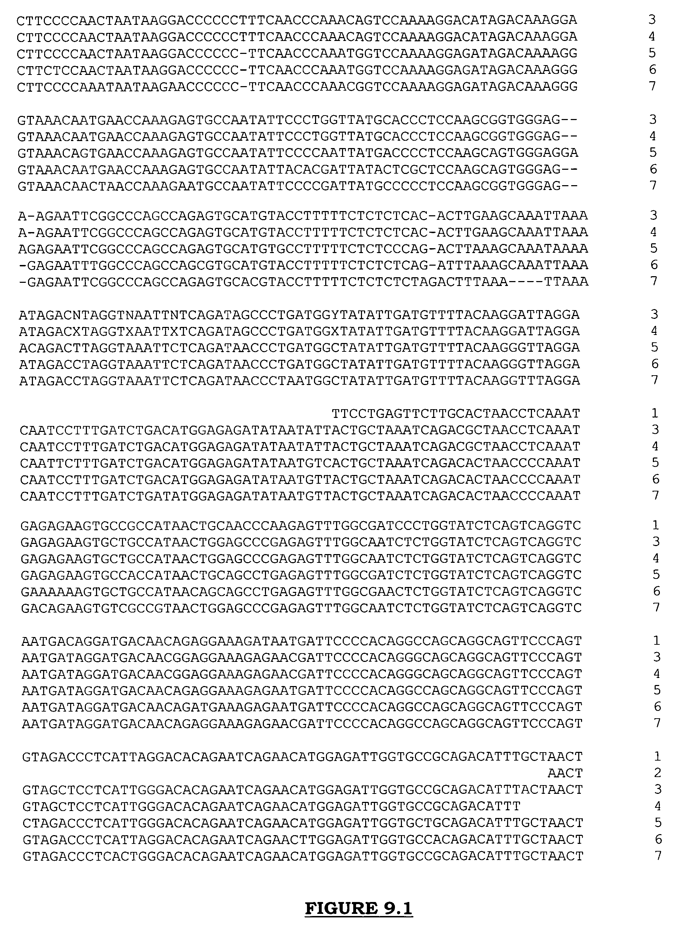

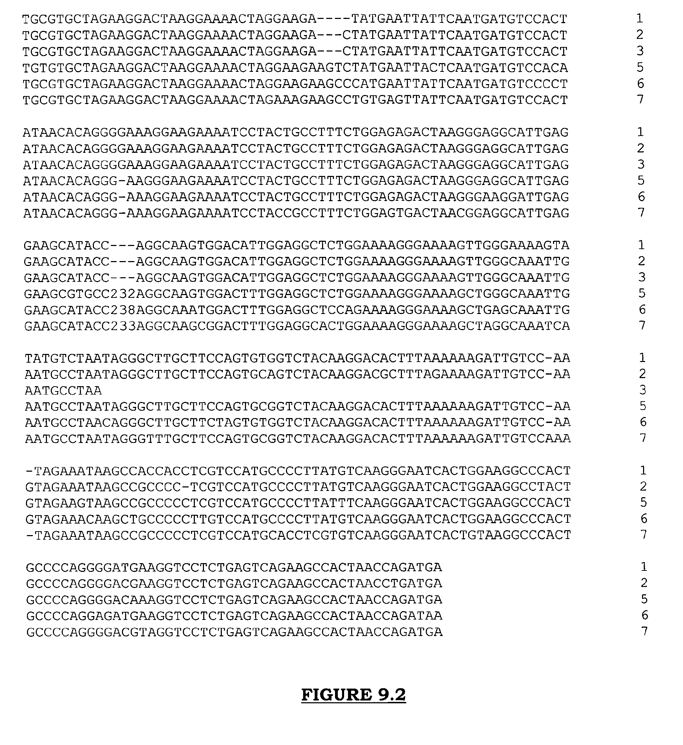

Les alignements des domaines *env* (fig. 8) et *gag* (fig. 9) explicitent les niveaux d'homologie observés entre les séquences décrites ci-dessus et les séquences homologues dans HERV-7q. Les analogies peuvent s'étendre aux motifs rétroviraux flanquants.

Une analyse des séquences étiquettes disponibles dans les banques de données montre que des transcrits appartenant à certains des membres de cette famille, en particulier HERV-7q, s'expriment essentiellement dans des tissus d'origine foetale ou placentaire.

Des séquences polypeptidiques générées par ces transcrits peuvent donc être potentiellement produites et des fonctions ou activités biologiques peuvent être envisagées, par analogie avec des polypeptides biologiquement actifs d'origine virale ou rétrovirale ; par exemple, les motifs peptidiques de type CKS-17 (Haraguchi et al., PNAS, 1995, 92, 5568-5571) (fig. 5) ou CKS-25 (Huang S.S et Huang J.S, J. Biol. Chem. 1998, 273, 4815-4818), qui présentent des fonctions immunomodulatrices sur les cellules lymphocytaires hôtes. Les différences de séquence observées et d'éventuelles modifications normales ou pathologiques, sont en particulier, à l'origine d'une modulation de la fonction.

HERV-7q représente le paradigme de la nouvelle famille de séquences rétrovirales endogènes humaines ou de motifs rétroviraux endogènes.

HERV-7q et certaines des séquences rétrovirales endogènes appartenant à sa famille, présentent un domaine de type *pol* analogue à des séquences rétrovirales de type *pol* comme par exemple la région *pol* identifiée dans le rétrovirus MSRV associé à la sclérose en plaques et décrit par H. Perron et al. (1997, Proc. Natl. Acad. Sci. USA, 94, 7583-7588 ; Demande Internationale PCT WO 97/06260).

Toutefois, les séquences selon la présente invention se distinguent des séquences rétrovirales exogènes infectieuses analogues à MSRV antérieurement décrites en ce que les séquences *gag* et *env,* selon l'invention sont significativement différentes selon les critères précédemment définis et en fonction de certaines caractéristiques spécifiques, par exemple le long cadre de lecture ouvert du domaine *env* de HERV-7q ; elles seraient à même de permettre de signer une pathologie lorsqu'elles présentent des insertions, des délétions, des décalages de cadre de lecture ou des mutations.

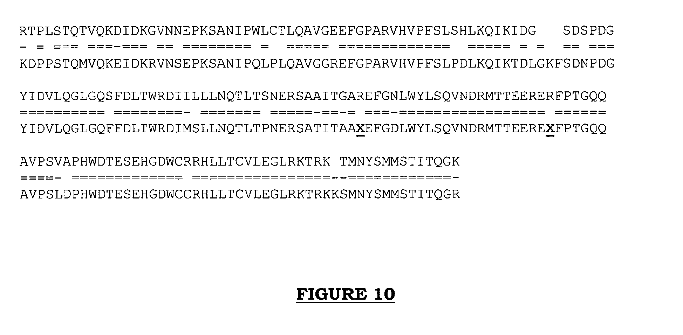

En effet, les différences observées entre les séquences humaines de type HERV-7q, qui sont isolées d'individus réputés normaux et les séquences issues de certains échantillons d'origine pathologique, ne sont pas distribuées au hasard. Des comparaisons menées entre la région *gag* provenant de particules rétrovirales infectieuses (N° d'accession EMBL: A60168, A60200, A60201, A60171...) et la séquence *gag* correspondante de HERV-7q (fig. 9), permettent d'observer que les mutations affectent préférentiellement des codons non-sens. Par exemple, deux codons non-sens dans HERV-7q sont remplacés par un codon arginine dans A60200, ce qui permet d'obtenir une séquence déduite de 109 acides aminés pour HERV-7q et de 166 acides aminés pour A60200. Les changements de base permettent en conséquence de prolonger le cadre de lecture et de coder potentiellement pour des structures polypeptidiques de plus grande taille (figure 10).

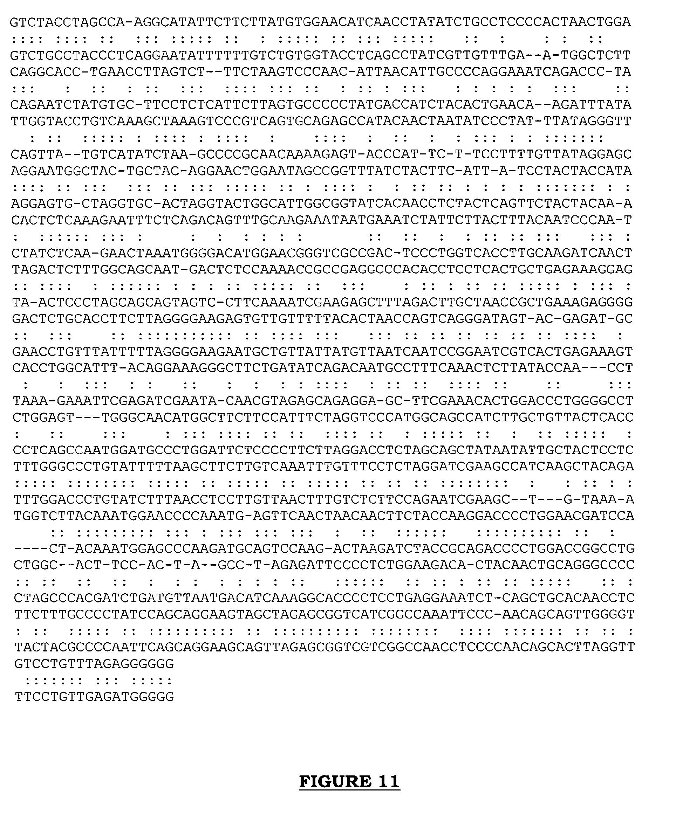

De même, une séquence de type *env* provenant de particules rétrovirales infectieuses, présente une analogie significative avec le domaine *env* de HERV-7q (figure 11). Ces analogies marquées entre séquences rétrovirales exogènes et endogènes pourraient être à l'origine du déclenchement ou de l'aggravation de certains processus pathologiques, en particulier de certaines maladies auto-immunes, comme la sclérose en plaques. A cet égard, on peut remarquer que certaines des séquences rétrovirales endogènes décrites dans l'invention se situent à proximité ou dans des régions réputées présenter une susceptibilité pour la sclérose en plaques : par exemple HERV-7q et la région 7q21-22 du chromosome 7, de même pour HE12 et HG12 dans HERV-TcR et la région du gène codant pour les chaînes alpha et delta du récepteur des cellules-T, HE2 et le chromosome 17, ou HE3, HE13 et HG3 et le chromosome 6, par exemple, les séquences HE11 et HG11, autour de la région 7q 21-22 ou encore HE4, HE5, HE6, HE9, HE10 ou HG10 sur le chromosome X. Ces séquences seraient donc à même de fournir des moyens de localisation ou d'identification des gènes de prédisposition.

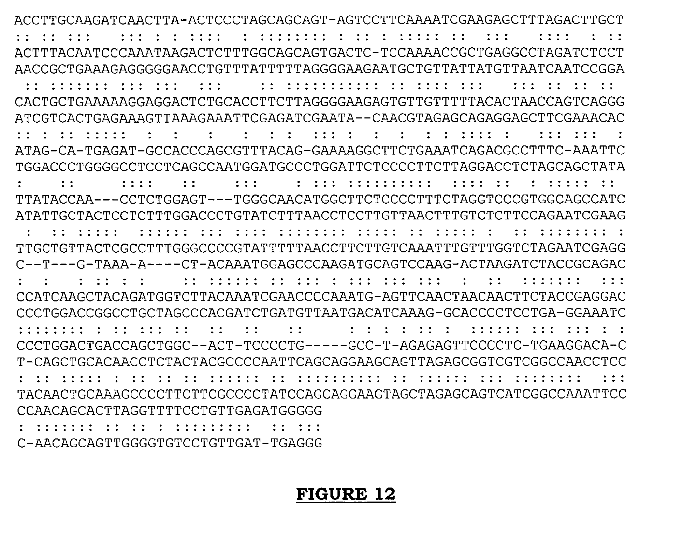

On n'observe aucune homologie significative avec des séquences rétrovirales endogènes déjà décrites, par contre, on peut relever une homologie limitée, permettant d'identifier une structure générale de domaine *env* ; toutefois, ladite homologie est inférieure aux critères définis selon l'invention entre les domaines *env* de la séquence HERV-7q (SEQ ID NO :1) et de la séquence HERV-9 (figure 12). La figure 11 montre des homologies étendues entre la séquence HERV-7q avec une séquence rétrovirale exogène (N° d'accession EMBL : A60170).

Les séquences rétrovirales endogènes humaines appartenant à la famille de HERV-7q, peuvent protéger contre des agressions liées à l'environnement ou constituer un bénéfice pour l'individu. Cet effet bénéfique pourrait être une des raisons possibles de la pression de sélection exercée sur certaines de ces séquences et du caractère potentiellement fonctionnel des structures protéiques déduites identifiées : par exemple le long cadre de lecture ouvert apte à coder pour une nouvelle protéine et correspondant au domaine *env* de HERV-7q.

Les séquences rétrovirales endogènes humaines appartenant à la famille de HERV-7q pourraient être associées par exemple, à des pathologies en relation avec les processus liés au cancer, aux neuropathologies à composante auto-immune ou à tout autre processus pathologique en association ou non avec des virus ou rétrovirus endogènes ou exogènes. Leur action pourrait porter sur la déclaration, l'aggravation, la modification du calendrier d'apparition ou encore la protection vis à vis de la maladie.

Dans le contexte d'application à des pathologies autoimmunes (comme par exemple le lupus, le syndrome de Sjögren, la polyarthrite rhumatoïde, la sclérose en plaques...), on peut relever des analogies significatives entre les motifs rétroviraux endogènes identifiés et des motifs retrouvés dans des structures rétrovirales caractérisées chez des patients présentant des pathologies autoimmunes comme la sclérose en plaques : par exemple des fragments de domaine *gag* (récemment disponibles dans les banques de données) provenant de particules rétrovirales infectieuses ou encore la séquence complète du domaine *pol* correspondant au virus MSRV associé à la sclérose en plaques. Ces motifs rétroviraux possèdent des analogies significatives avec les séquences endogènes homologues de type HERV-7q, ce qui permet d'envisager une association directe ou indirecte avec des processus pathologiques, dont la sclérose en plaques, en association ou non avec MSRV.

L'intérêt de ces séquences dépasse le cadre des maladies auto-immunes. En dehors de l'importance générale des motifs rétroviraux dans le déclenchement ou l'aggravation d'un processus tumoral, bien montré en particulier dans les modèles murins (H. Fan dans The retroviridiae, 1994, ed. J.A. Levy, Plenum, New York, p. 313-353), ces séquences pourraient se retrouver à proximité ou au sein de gènes importants et en altérer l'expression : par exemple HERV-TcR et les gènes des sous-unités alpha et delta du récepteur des cellules T impliquées dans des perturbations de la fonction immunitaire.

La présente invention englobe, en outre, l'utilisation de séquences associées aux séquences de la famille HERV-7q pour la détection et/ou le pronostic de différentes maladies auto-immunes (neuropathologies, en particulier) ; ces séquences codent pour tout ou partie d'un facteur, dont la fonction, la régulation/dérégulation ou l'altération (polyadénylation, épissage alternatif) est associée à l'expression normale ou pathologique ou à la régulation/dérégulation des motifs appartenant à la famille HERV-7q et correspondent à des transcrits ou des ADNc des séquences nucléotidiques codant pour des gènes situés dans des régions flanquantes ou encadrant des séquences rétrovirales de la famille HERV-7q.

On entend par région flanquante, toute région située à proximité (incluse dans ou incluant) une séquence rétrovirale endogène appartenant à la famille HERV-7q, telle que définie ci-dessus, jusque et y compris les gènes immédiatement contigus et/ou situés à une distance ne pouvant excéder 120 kb.

Les Inventeurs ont maintenant trouvé que la présence des séquences rétrovirales telles que définies ci-dessus, perturbent l'expression ou altèrent la structure des séquences flanquantes définies ci-après.

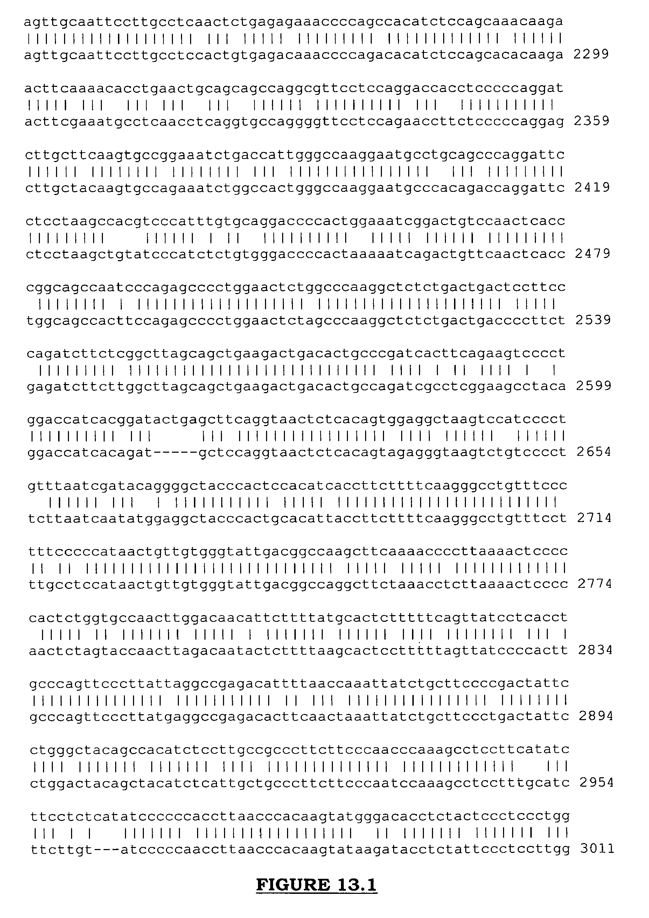

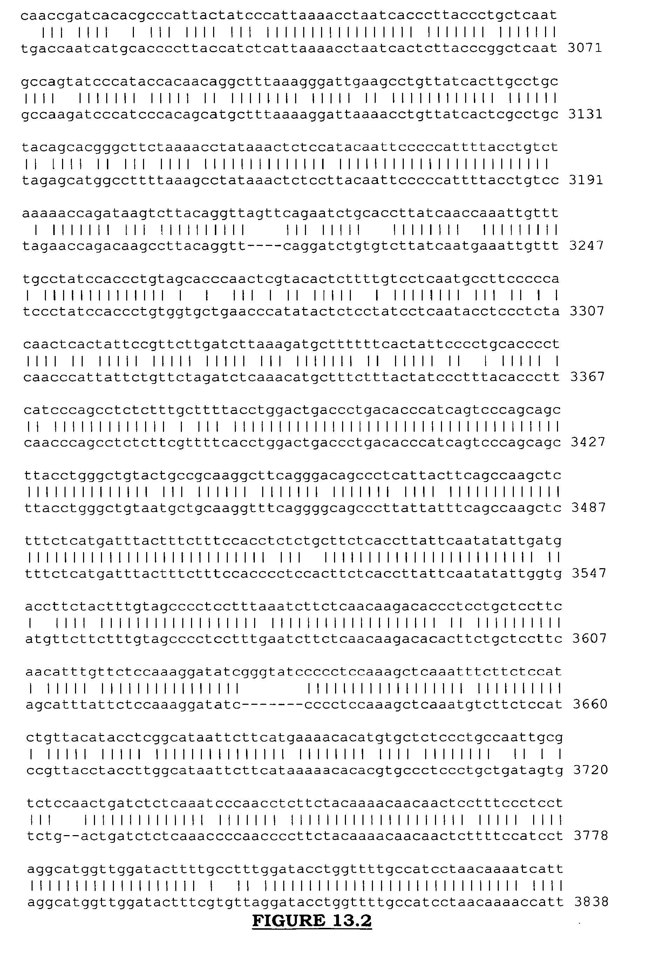

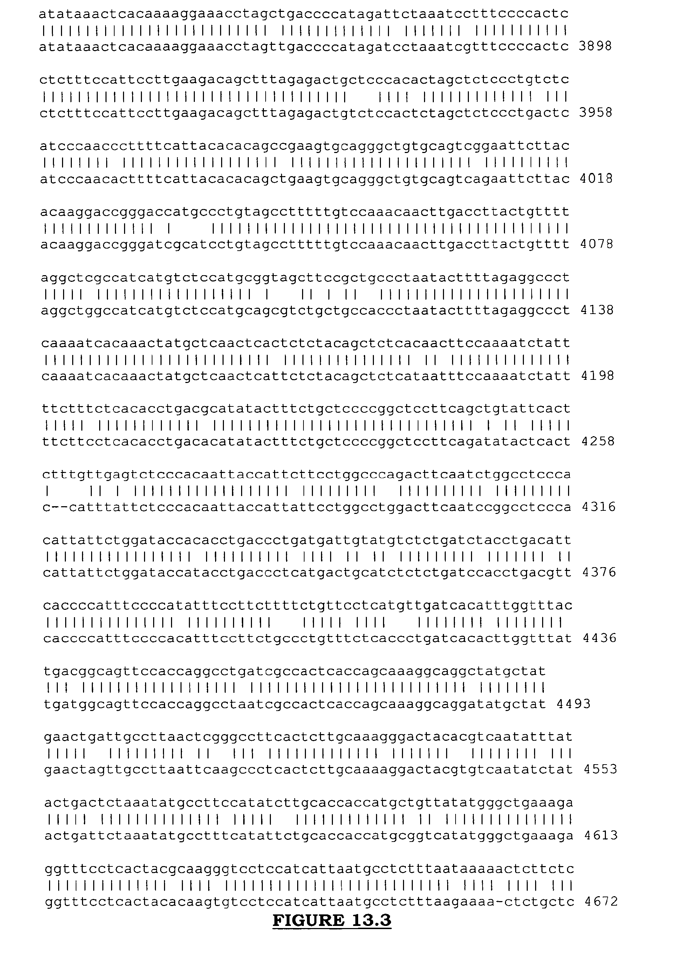

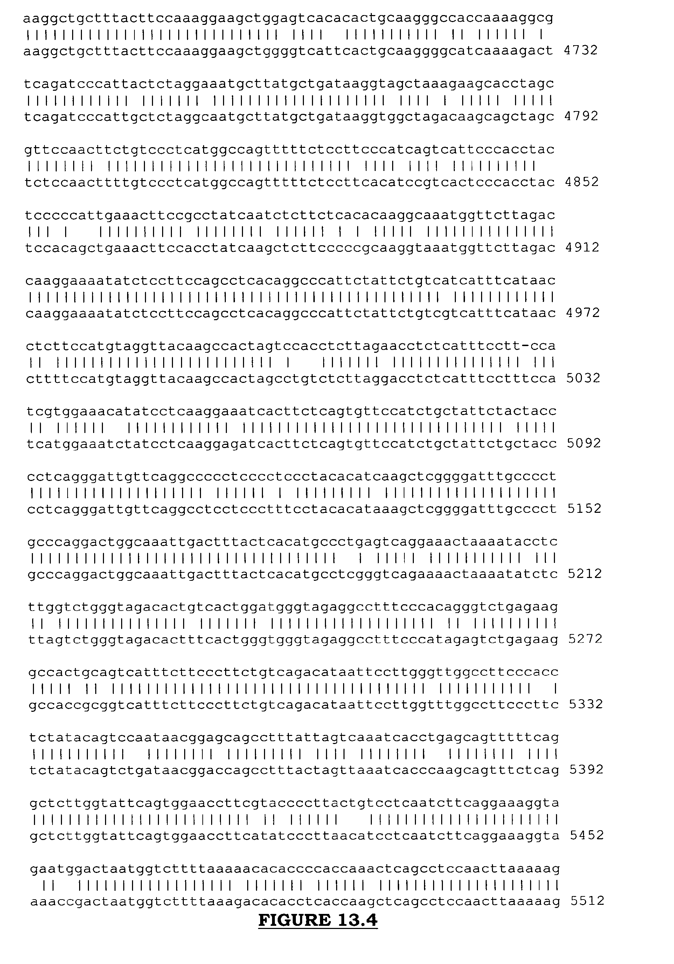

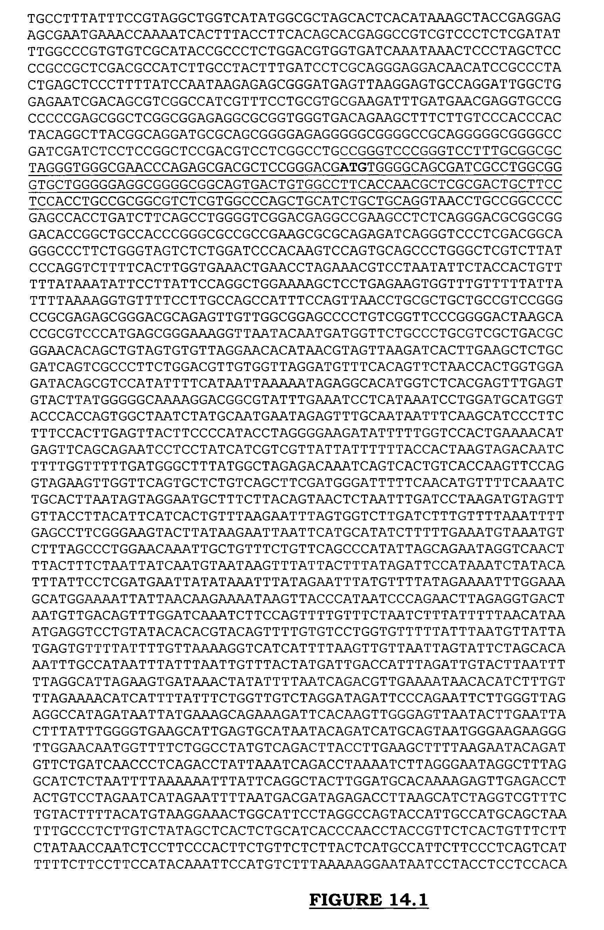

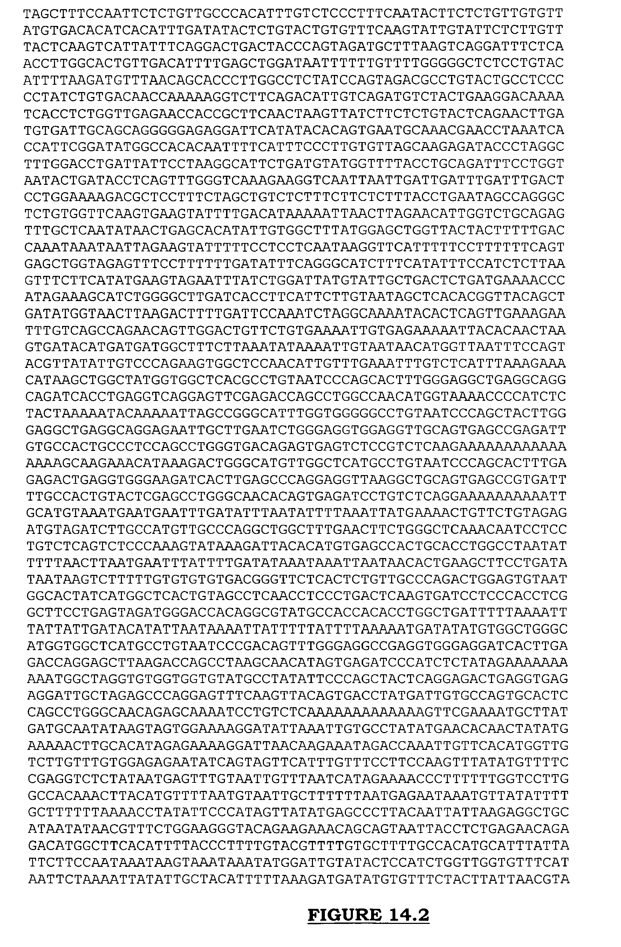

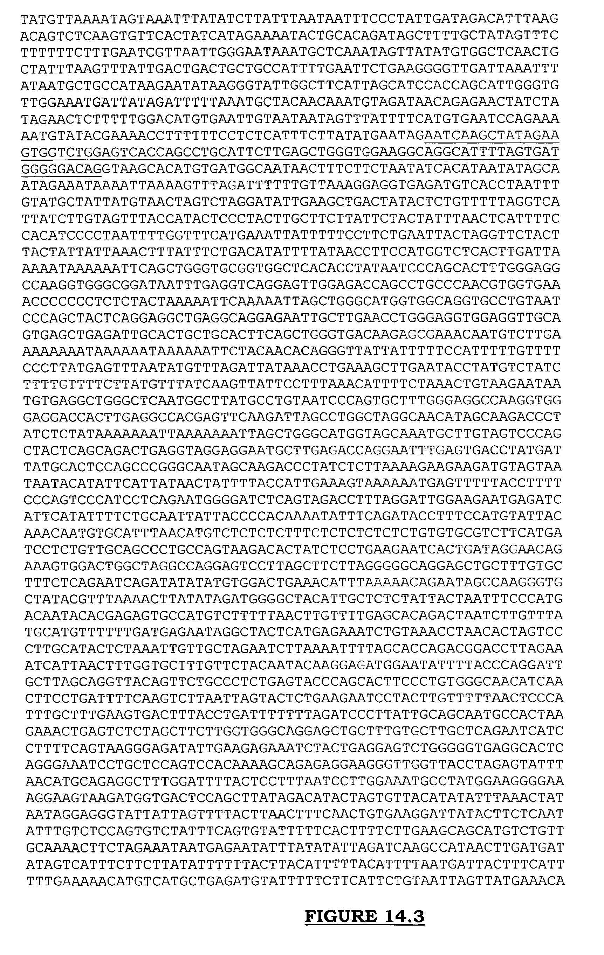

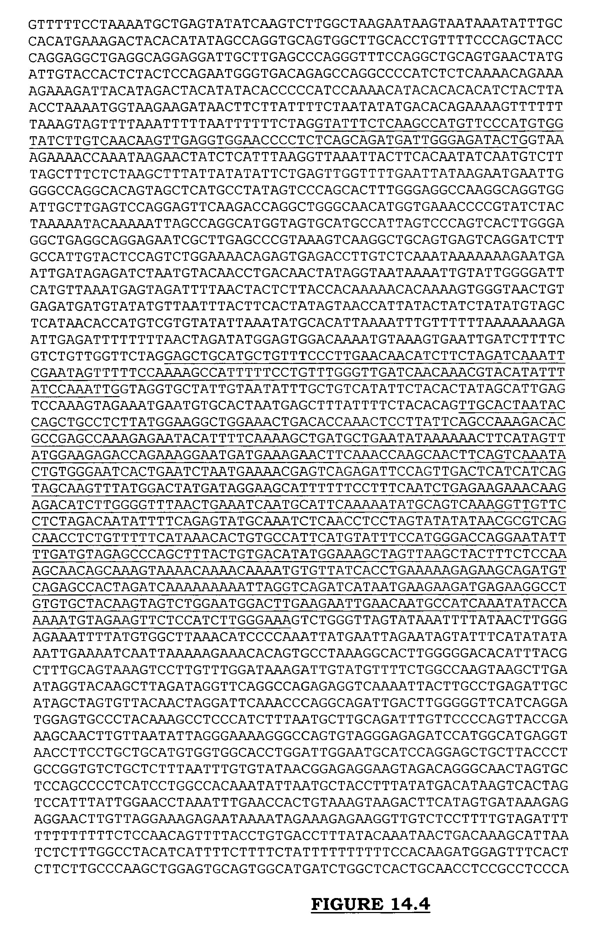

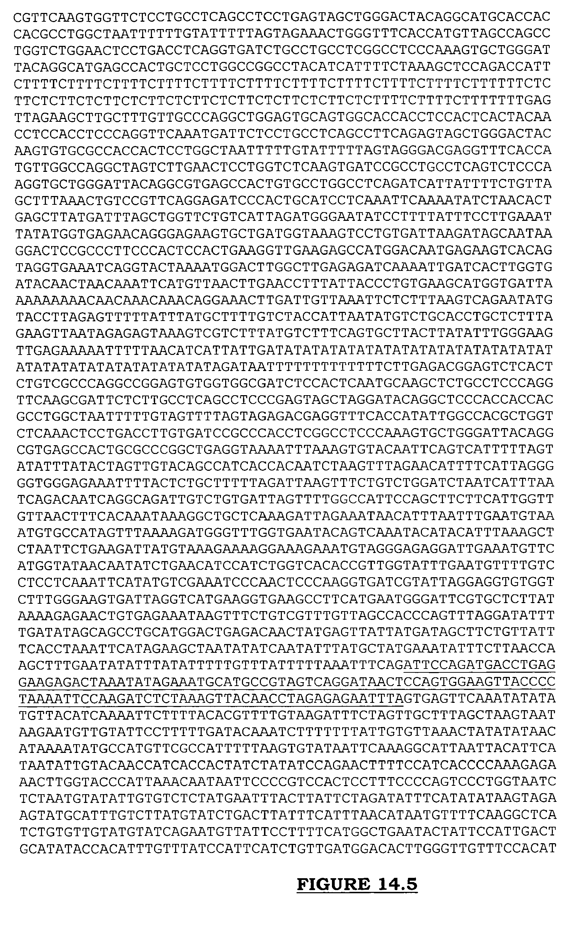

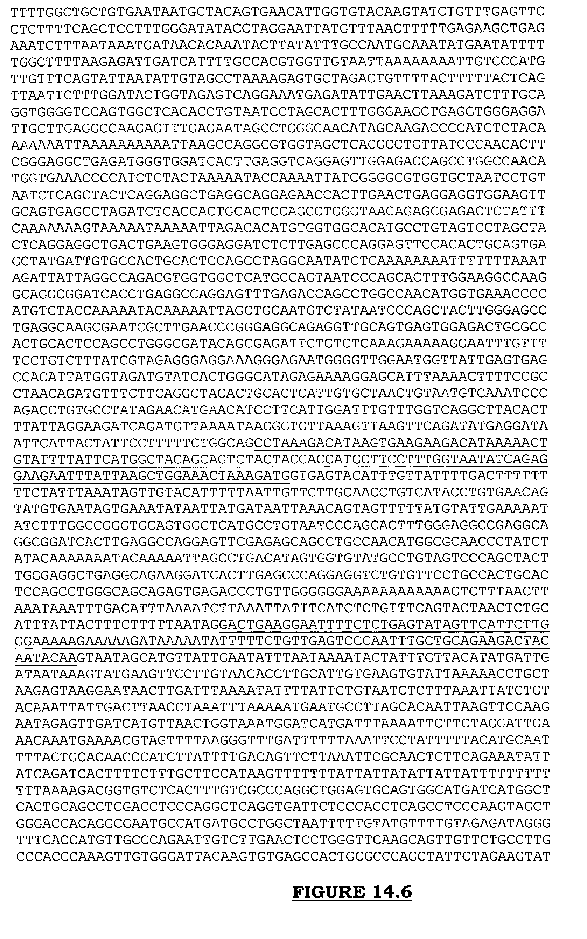

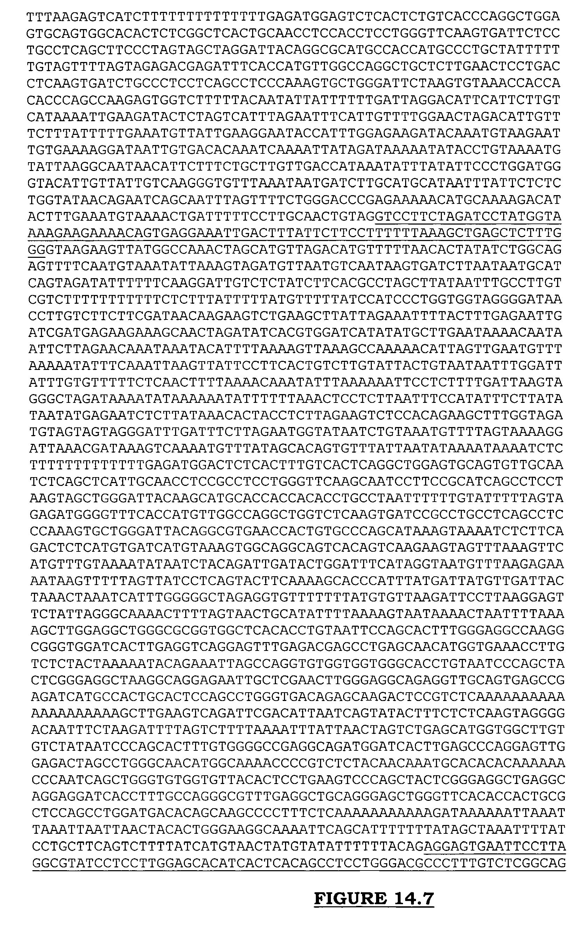

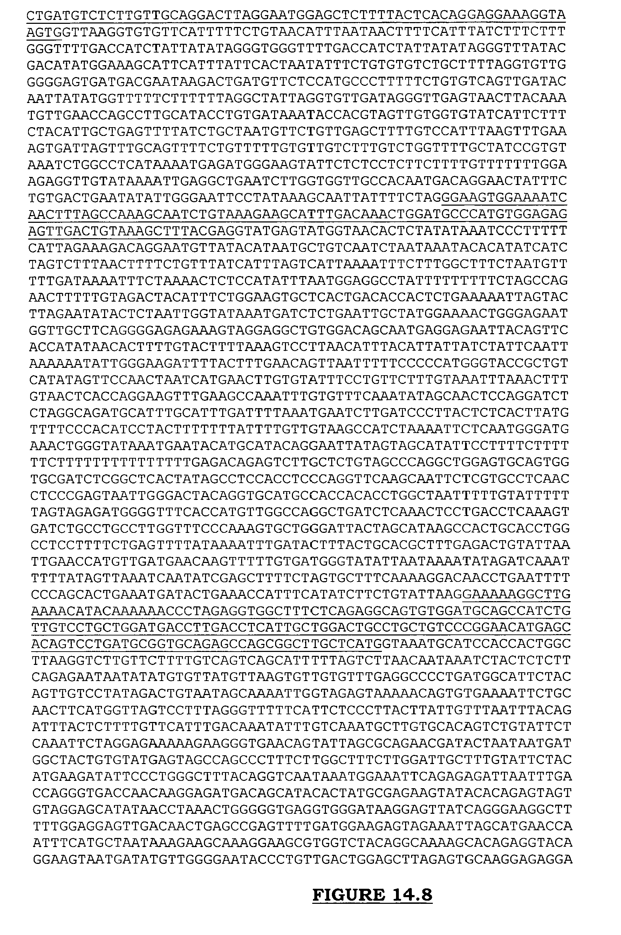

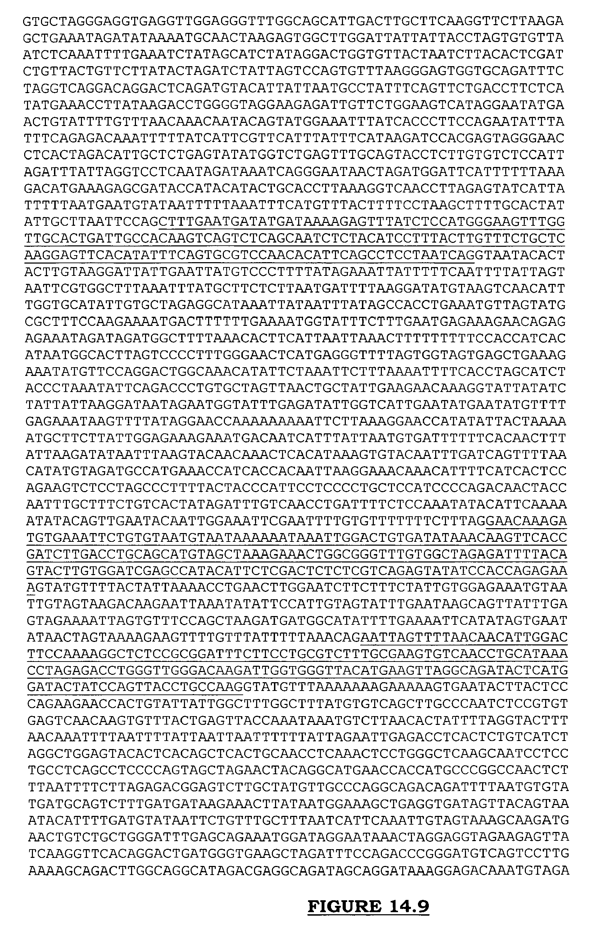

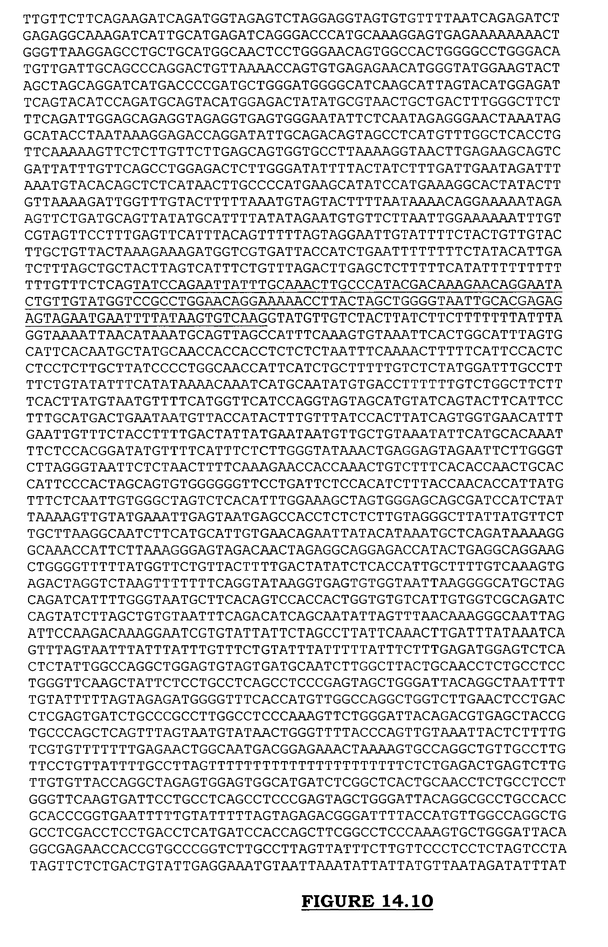

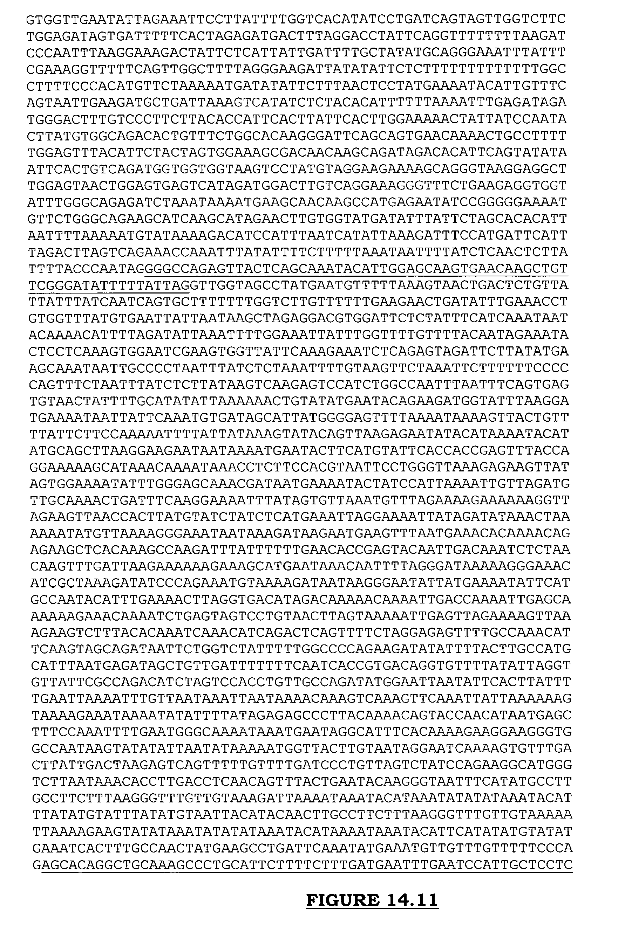

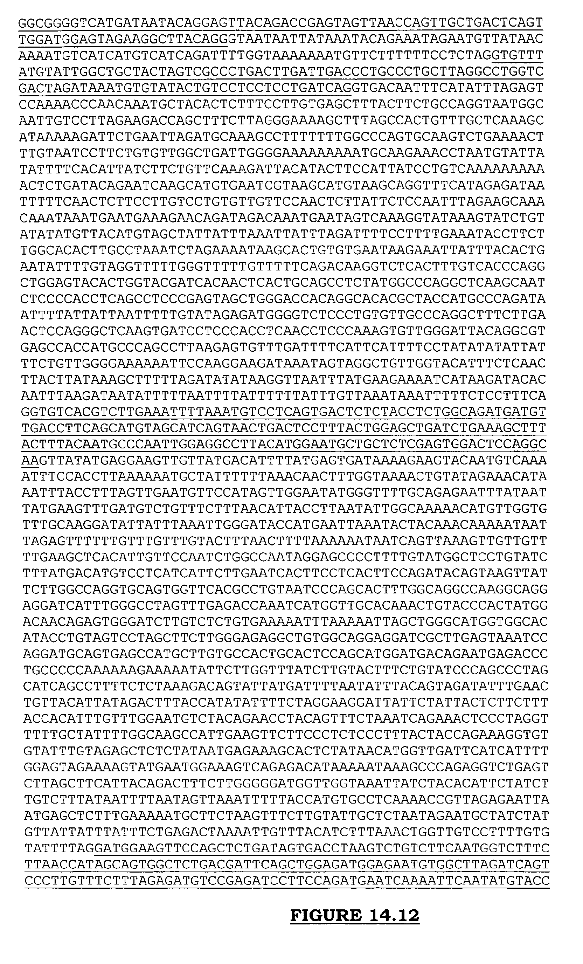

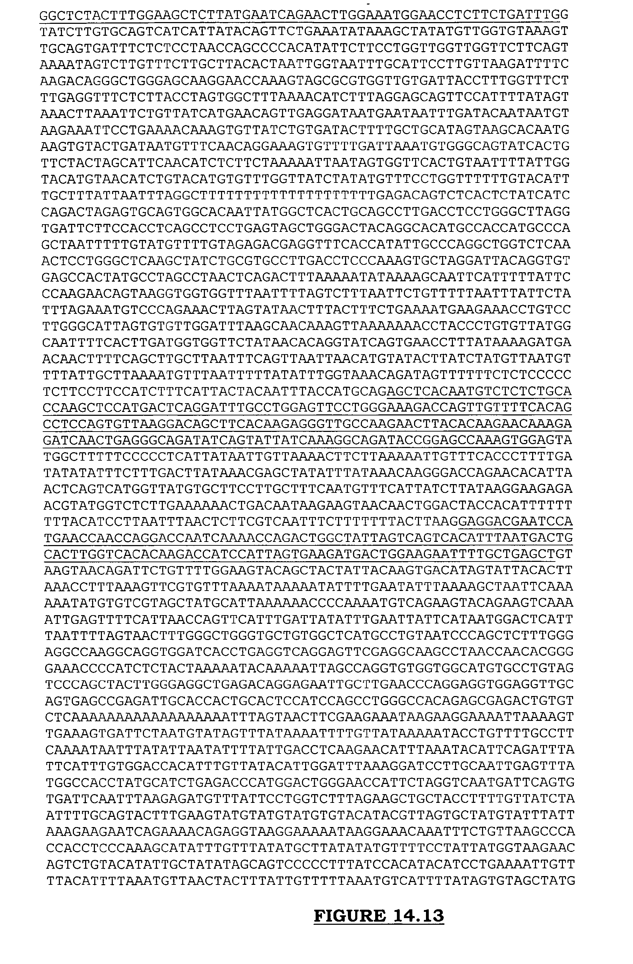

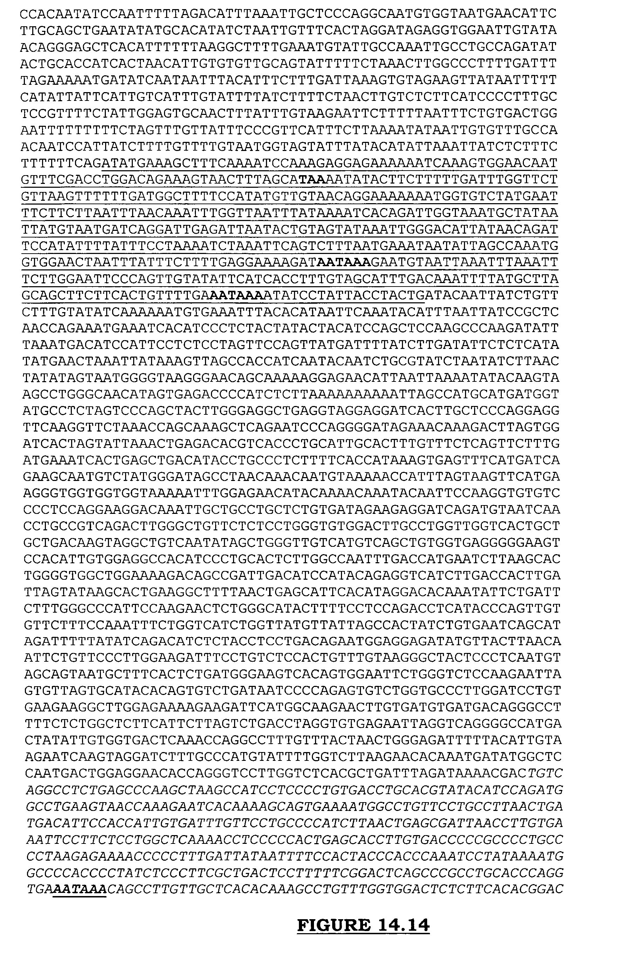

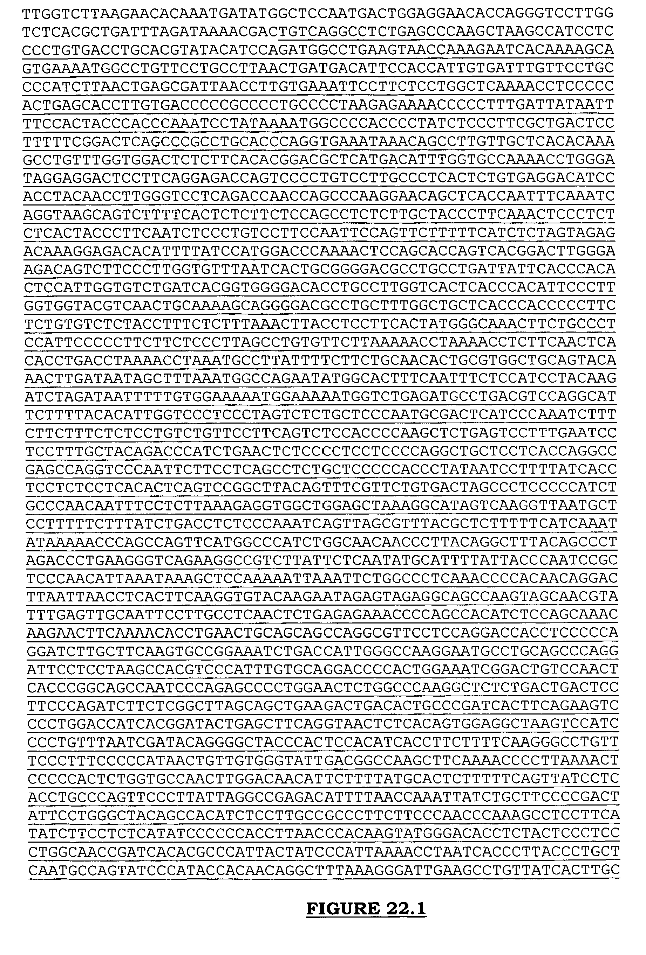

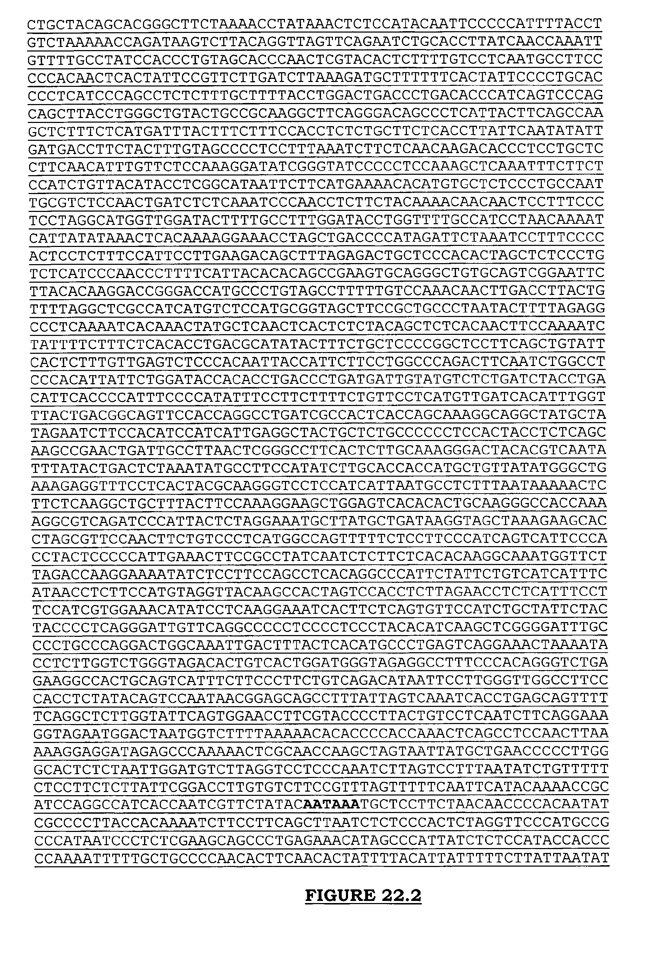

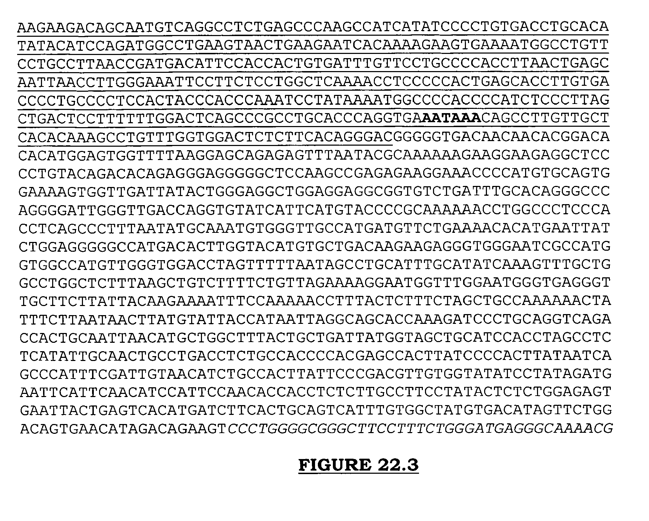

Les transcrits desdites séquences flanquantes (et leurs fragments, notamment ceux soulignés ou en italique dans les figures 14-16, 22-26, sont définies ci-après :
- à 1021 pb en amont de HERV-7q, on identifie une séquence rétrovirale endogène appelée RH7 (SEQ ID NO:62 et figure 22); cette séquence est située en 5' de la séquence HERV-7q ; dans la figure 22, la partie en italique correspond au début de la séquence HERV-7q ; la séquence RH7 est soulignée ; deux sites de polyadénylation putatifs sont en gras. Cette séquence SEQ ID NO:62 présente une homologie significative, sur plus de 6 kb, avec des séquences rétrovirales endogènes de type RGH (figure 13). Des séquences appartenant à cette famille s'expriment en particulier chez des patients présentant une arthrose rhumatoïde (Nakagawa et coll., (1997), Arthritis. Rheum., 40, 627- 638). La présente invention inclut également des fragments de la séquence SEQ ID NO:62, comprenant entre 14 et 50 nucléotides (utilisation comme amorces), de préférence entre 14 et 25 nucléotides ou au moins 25 nucléotides (utilisation comme sonde), lesquels fragments présentent les caractéristiques suivantes : les 4 nucléotides de l'extrémité 3' sont différents des motifs correspondant de la séquence RGH2 (séquence du bas dans la figure 13, n° accession à GenBank : D110 18).
- à moins de 9 kb en amont de HERV-7q, on identifie la séquence RAM75 (SEQ ID NO:63 et figure 14) contenant les 24 exons codants (qui couvrent près de 41 kb), du gène de l'ATPase péroxysomale PEX1. PEX1, en association avec PEX6 est responsable de l'importation des protéines péroxysomales et de la stabilisation du récepteur PEX5. Une perturbation/altération affectant PEX1 est responsable de diverses neuropathologies comme le syndrome de Zellweger, l'adrénoleucodystrophie néonatale et la forme infantile de la maladie de Refsum (Reuber et coll., (1997), Nature Genet., 17, 445- 448). On peut rappeler que la fonction principale des péroxy-somes est associée au métabolisme des acides gras, en particulier par des processus de β-oxydation. Une altération du gène identifié dans la séquence RAM75 ou de son expression, par modification de la fonction des régions 5' et 3' régulatrices ou encore par modification des épissages ou des processus de polyadénylation, en particulier sous l'influence de motifs rétroviraux voisins, seraient à même de perturber l'expression ou la structure de l'ATPase et par conséquent de perturber une des fonctions péroxysomales, en particulier le métabolisme des lipides, en particulier myéliniques, avec des conséquences pour certaines pathologies, dont des neuropathologies, comme la sclérose en plaques ; les parties soulignées (figure 14) correspondent aux 24 exons codants.

La présente invention inclut également les fragments de la séquence SEQ ID NO:63, inclus dans les 24 exons codants précités et comprenant au moins 14 nucléotides.

L'analyse du profil d'expression (transcrits et protéines) de la séquence RAM75 (SEQ ID NO:63) est un bon indicateur du diagnostic différentiel des neuropathologies à composante auto-immune.

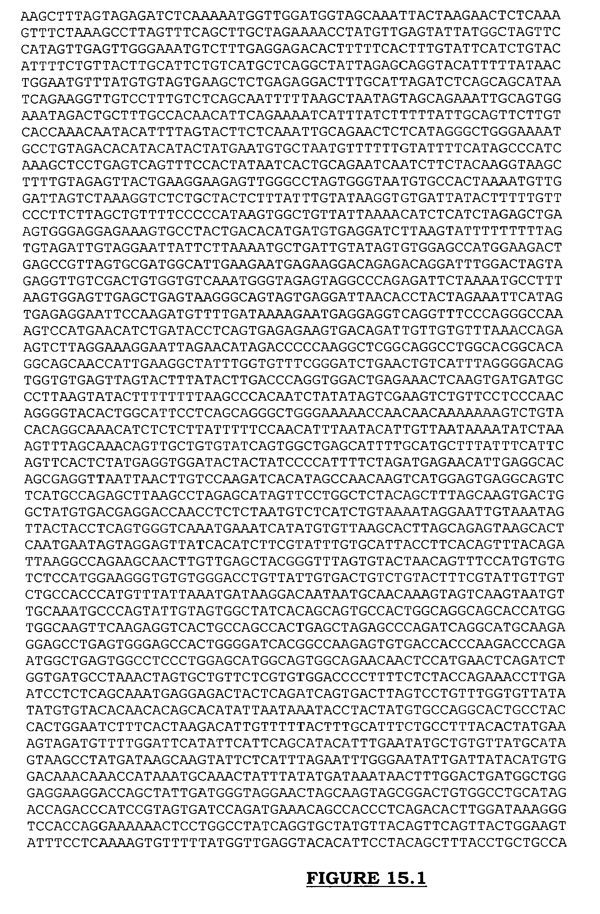

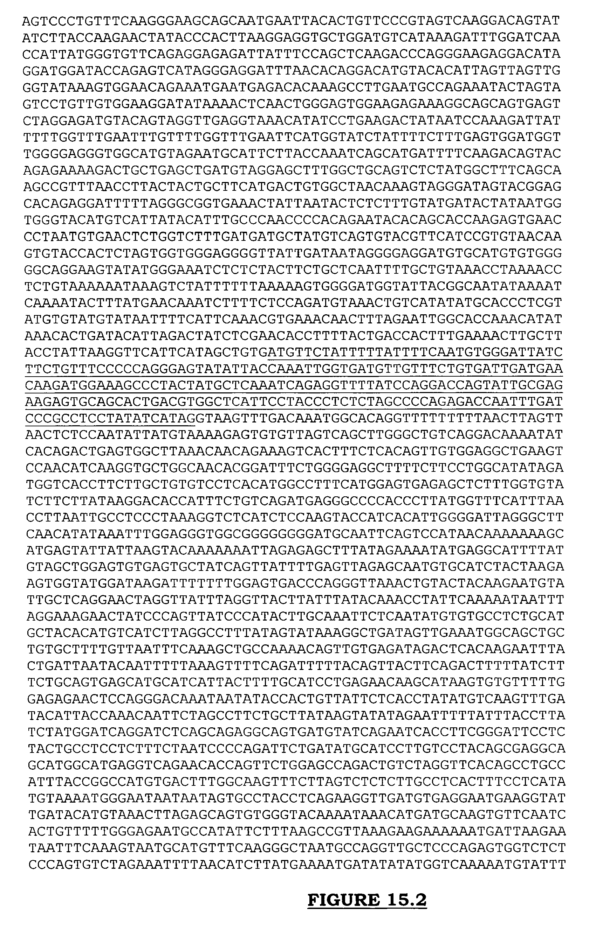

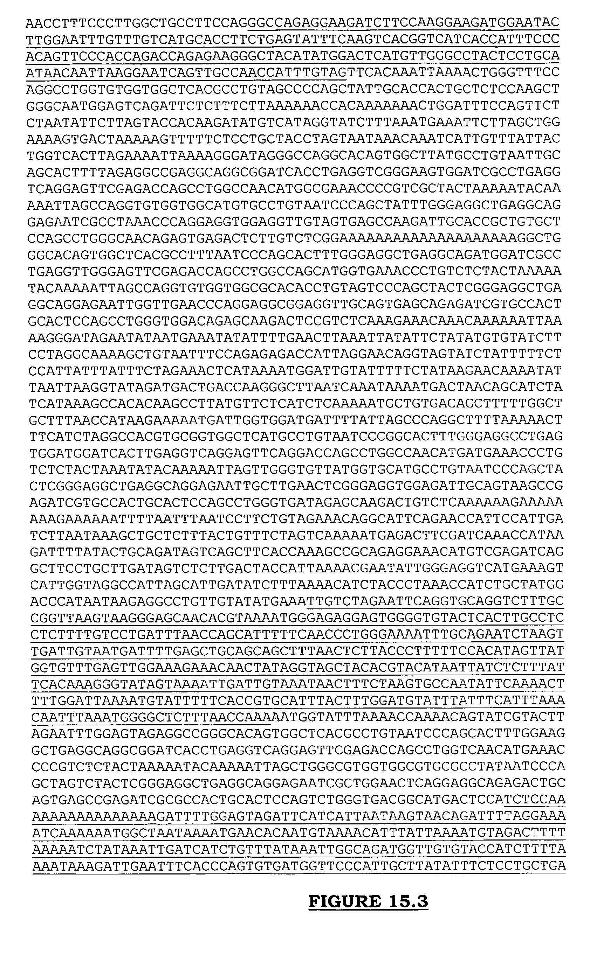

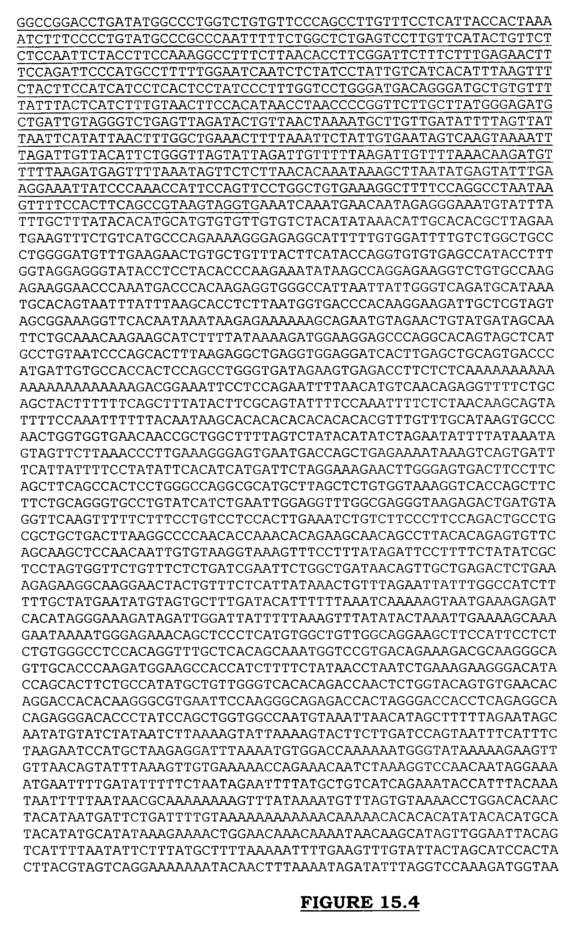

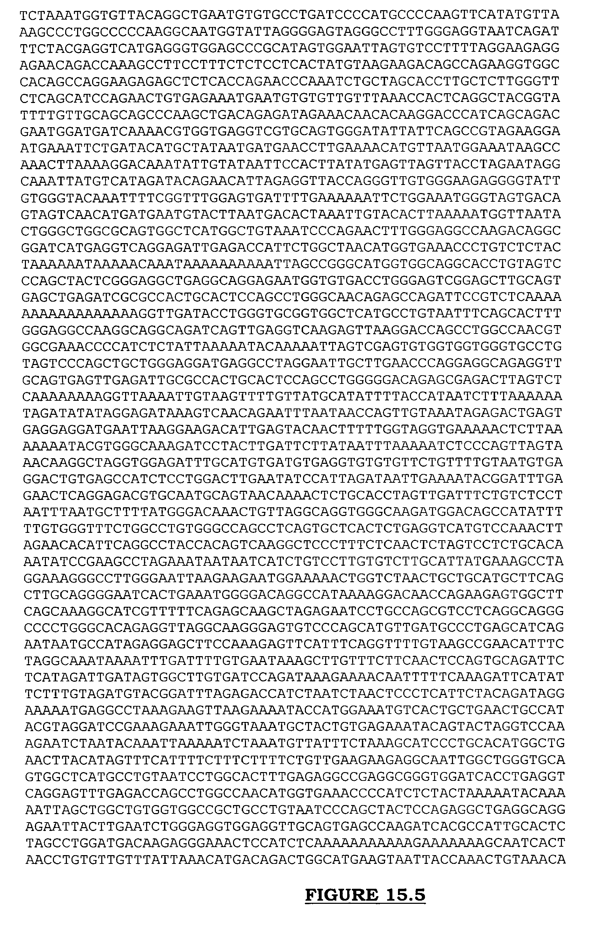

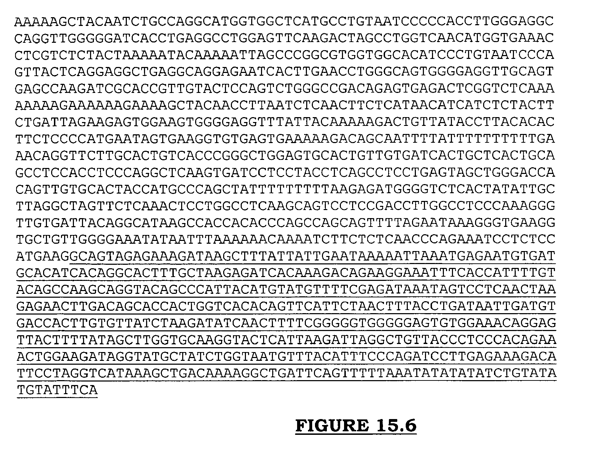

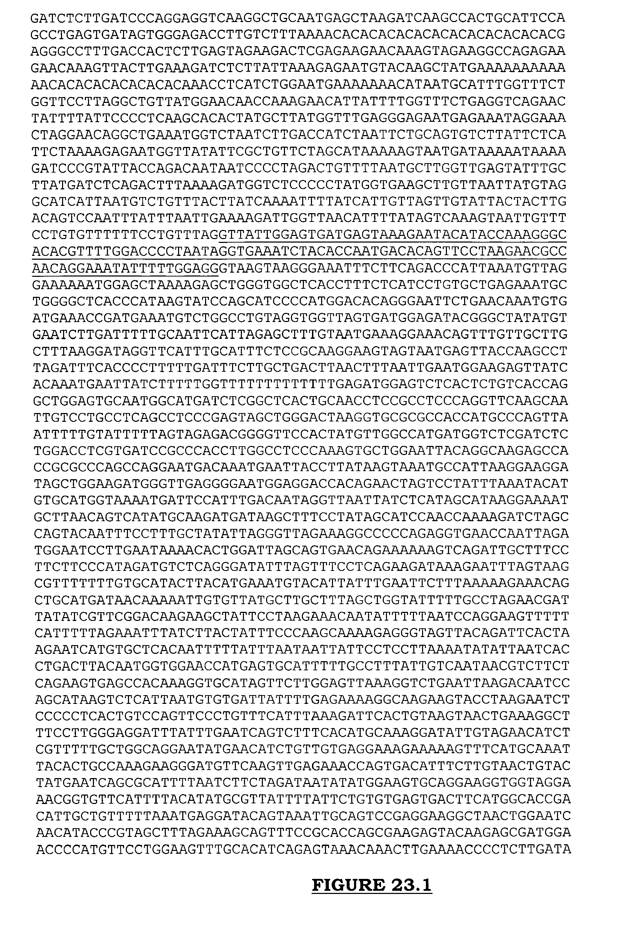

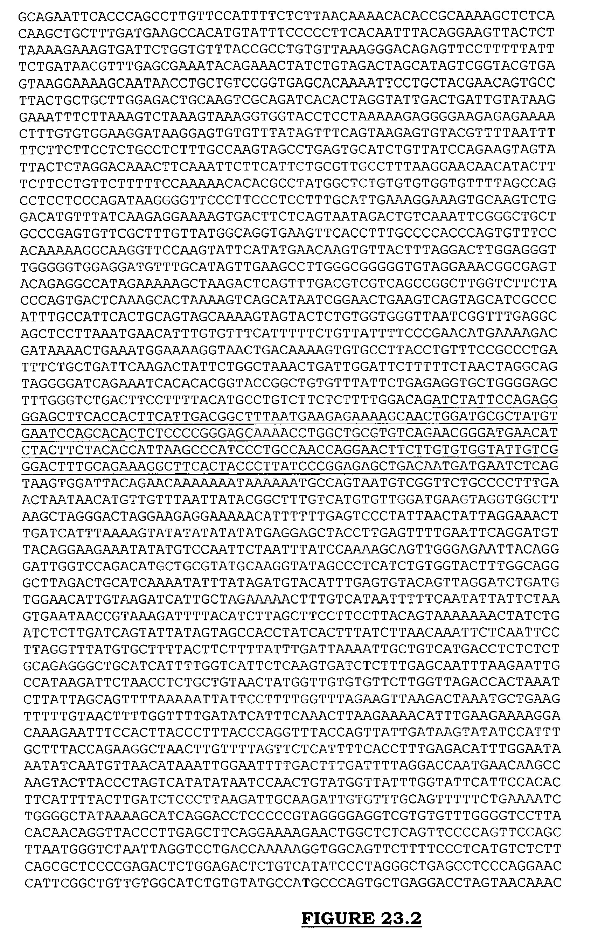

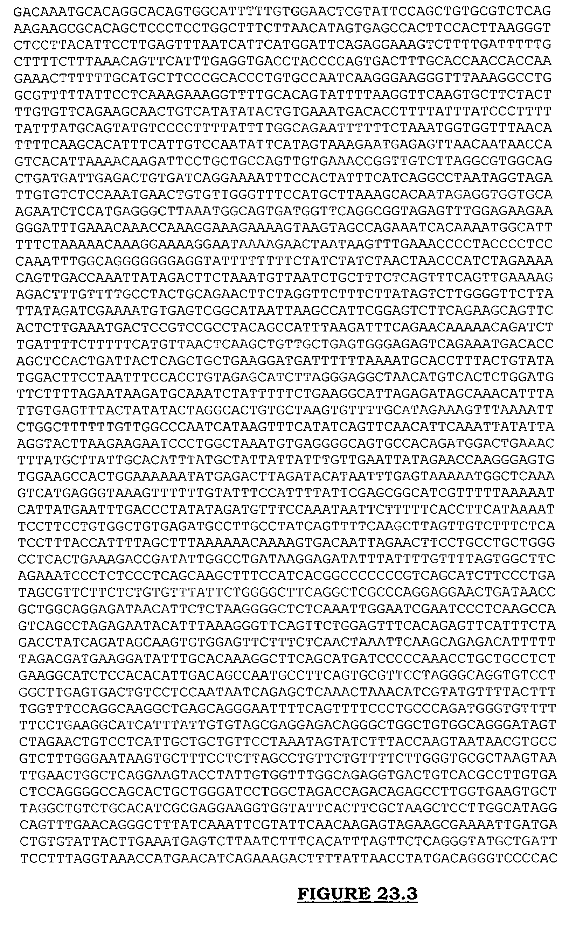

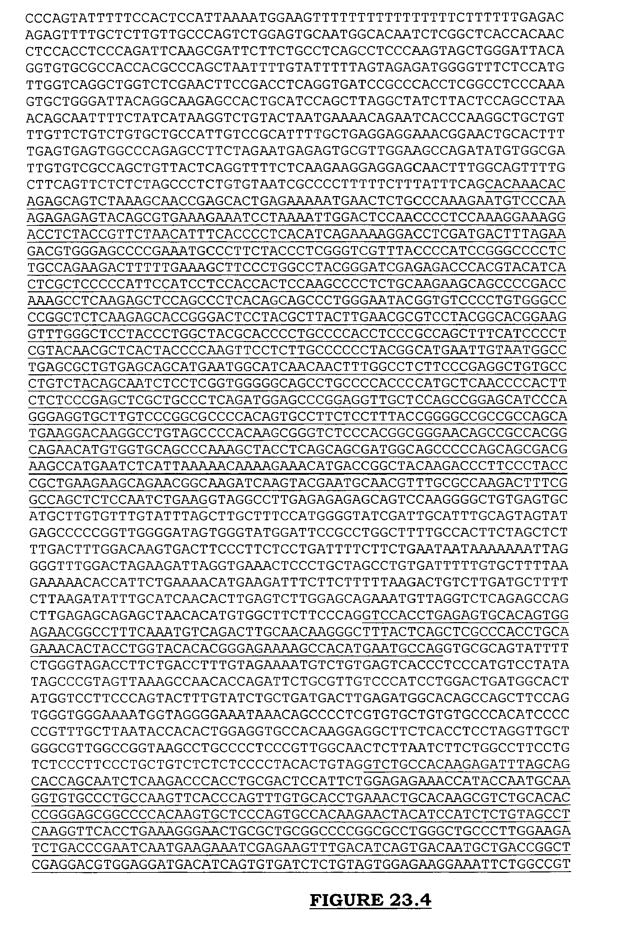

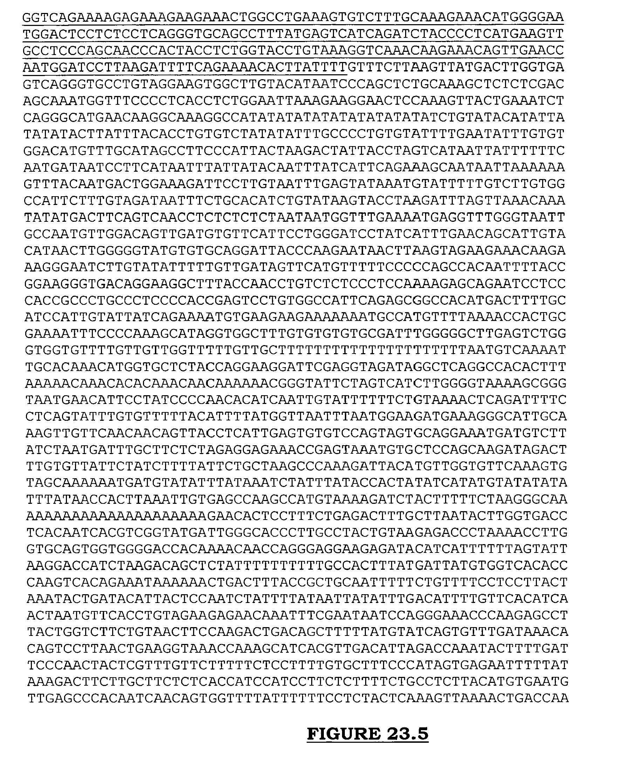

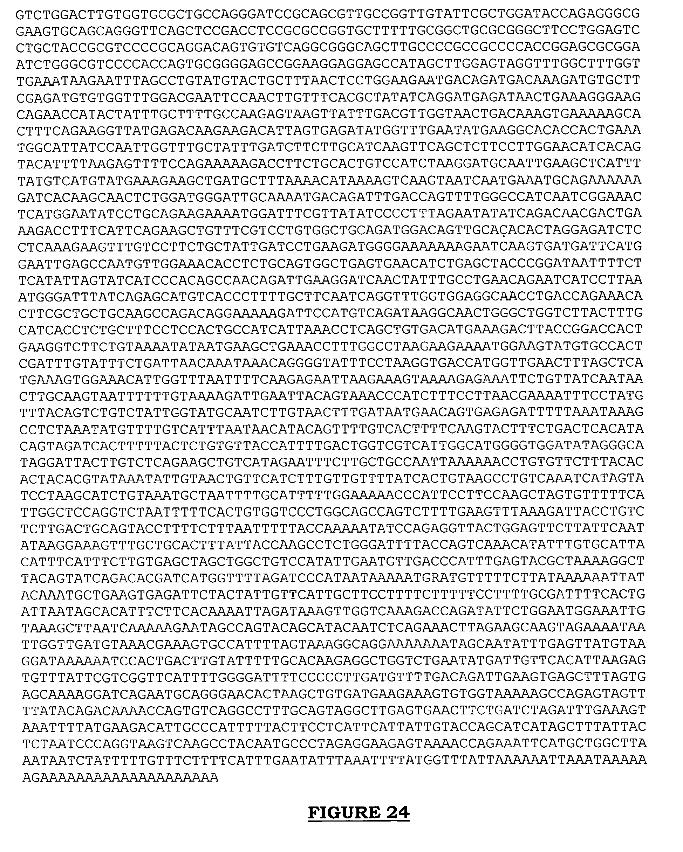

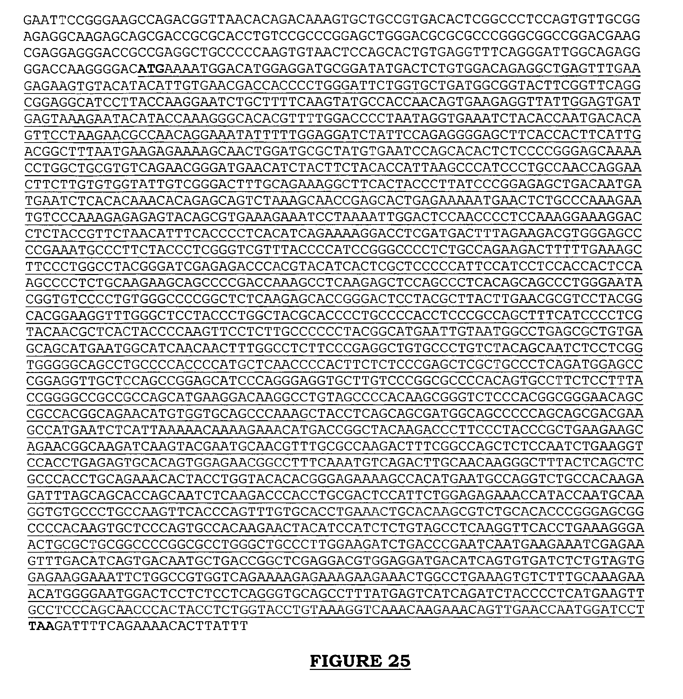

A la figure 14, les exons codants sont soulignés. Les codons d'initiation et non-sens ainsi que les sites putatifs de polyadénylation sont en gras et soulignés.
- à 0.7 kb en aval de la séquence HERV-7q et sur près de 17 kb (SEQ ID NO:64 et figure 15), on identifie la séquence nucléotidique RAV73, où l'on détecte des séquences étiquettes et des exons potentiels aptes à produire une ou plusieurs séquences polypeptidiques ; l'invention inclut également des fragments de cette séquence SEQ ID NO:64 compris dans les séquences étiquettes et les exons potentiels tels qu'ils apparaissent (parties soulignées) à la figure 15, lesquels fragments comportent au moins 14 nucléotides.
- à 120 kb en amont de la séquence HG3, et sur 15 kb, se trouve la séquence nucléotidique RBP3 (SEQ ID NO:65 et figure 23), qui couvre l'extrémité 3'du gène codant pour un facteur de transcription de la famille Blimp-1 (SEQ ID NO:119 et figure 25), une protéine de 789 acides aminés qui est un répresseur de l'expression du gène de l'interféron-béta (Keller et Maniatis, Genes Dev., (1991), 5, 868-879), qui est déjà associé à certaines pathologies malignes (Mock et coll., Genomics, (1996), 37, 24-28), et qui pourrait jouer un rôle dans la différenciation et la pathogenèse des cellules B. L'intérêt de l'association possible de la séquence rétrovirale endogène contenant les motifs HG3 et HE3 et de Blimp-1 est multiple, dans le cas de pathologies, et en particulier la sclérose en plaques. Blimp-1 agit en particulier sur les cellules B dont on connaît la contribution dans les processus inflammatoires associés à la sclérose en plaques. Blimp-1 est capable de bloquer l'induction virale du promoteur INFβ dont on connaît l'aptitude à réduire la fréquence des poussées et la progression lésionnelle chez des patients atteints de SEP. Une perturbation de l'expression ou de la structure de Blimp-1, en relation avec un élément rétroviral de type HERV-7q, est associée en conséquence à des neuropathologies ou à des maladies à caractère auto-immun, comme la sclérose en plaques ; cette séquence nucléotidique RBP3 (SEQ ID NO:65) contient des motifs nucléotidiques identifiés dans la séquence nucléique codant pour le gène Blimp-1; l'invention inclut aussi la détection des séquences ARNm de la protéine Blimp-1 (SEQ ID NO:119).
- la séquence rétrovirale endogène de type HERV-7q, contenant HE3 et HG3, se trouve située dans la région HI3 correspondant à un intron s'étendant sur plus de 46 kb (SEQ ID NO:66), d'un gène qui pourrait coder pour l'analogue d'APS (figure 24), une protéine de 275 acides aminés spécifique d'apoptose, surexprimée dans différents cellules en culture après déclenchement d'un processus apoptotique (Hammond et coll., FEBS Lett., (1998), 425, 391- 395). L'intron se situe au niveau de l'acide aminé 231 d'APS. L'extrémité de HE3 est à plus de 12 kb de l'extrémité 5' de l'intron, alors que HG3 est situé à plus de 28 kb de l'extrémité 3' de l'intron. Des processus apoptotiques sont associés à la sclérose en plaques. En particulier, il a été décrit un processus apoptotique affectant des astrocytes et des oligodendrocytes en présence d'une fraction purifiée de liquide céphalo-rachidien de patients atteints de sclérose en plaques (Ménard et coll., J. Neurol. Sci., (1998), 154, 209- 221).

Enfin, il faut souligner que la région nucléique contenant HE3, HG3, HI3 et RBP3 est localisée au niveau du bras court du chromosome 6, en 6p21, qui est une région proposée de susceptibilité à la sclérose en plaques (The Multiple Sclerosis Genetic Group, Nature Genet., (1996), 13, 469- 472).

L'interaction entre les séquences de type HERV-7q et les séquences flanquantes et l'importance de l'établissement d'un profil d'expression incluant une ou plusieurs des séquences précitées pour établir un diagnostic différentiel d'une neuropathologie apparaît encore plus du fait que l'on observe que les séquences HG12 et HE12 sont situées dans une région intronique du gène codant pour les sous-unités alpha et delta des récepteurs des cellules T. Les récepteurs des cellules T sont impliqués dans les processus de régulation immunitaire et leur influence a été proposée dans le cas de maladies auto-immunes, dont la sclérose en plaques.

L'invention a également pour objet les transcrits générés à partir des séquences précitées ainsi que celles présentant éventuellement des modifications avec les séquences de référence décrites dans l'invention lorsqu'ils sont exprimés chez certains patients.

En effet, les systèmes de régulation de l'expression des protéines rétrovirales de HERV-7q, qui sont présents dans les motifs de type LTR, pourraient influencer l'expression de gènes situés dans le voisinage chromosomique proche ou éloigné et induire des perturbations à caractère immunologique et/ou neurologique. Par exemple la séquence rétrovirale endogène HERV-TcR, se trouve à proximité immédiate des gènes des sous-unités alpha et delta du récepteur des cellules-T précédemment décrit. Les motifs de type LTR pourraient aussi coder pour des superanti-gènes (Acha-Orbea et Palmer, 1991, Immunol. Today, 12, 356-361). D'une manière générale des protéines rétrovirales de type HERV-7q ou apparenté, ou leurs formes tronquées ou partielles pourraient être impliquées dans des phénomènes de cytotoxi-cité ou de superantéginicité, comme par exemple celles issues du long cadre de lecture ouvert identifié dans le domaine *env* (figure 4).

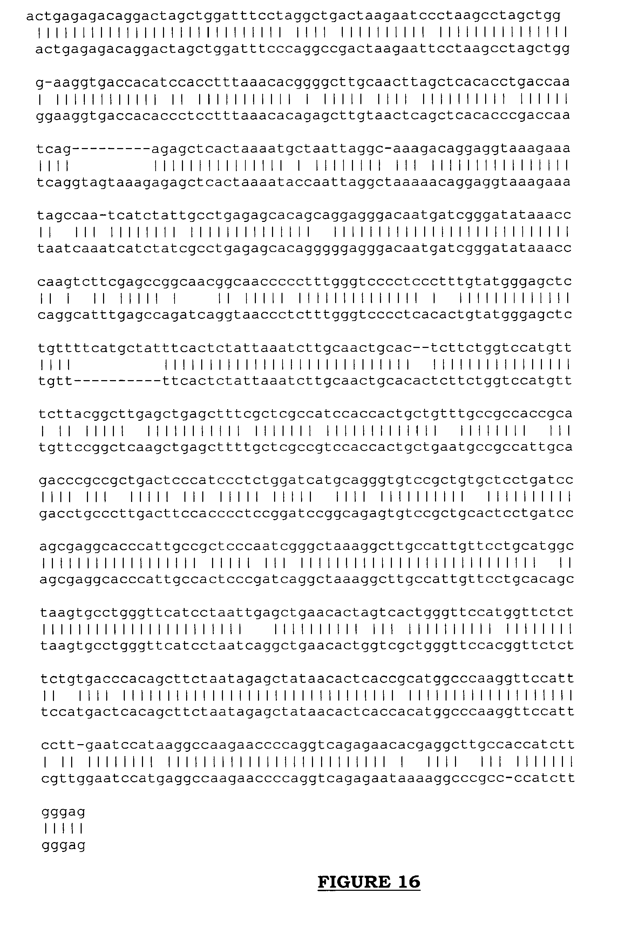

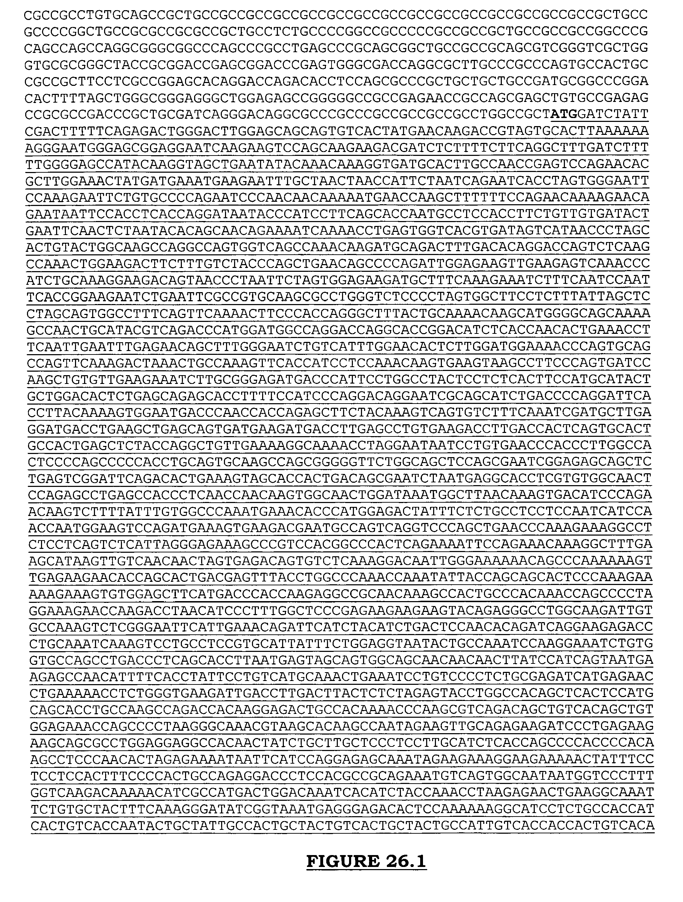

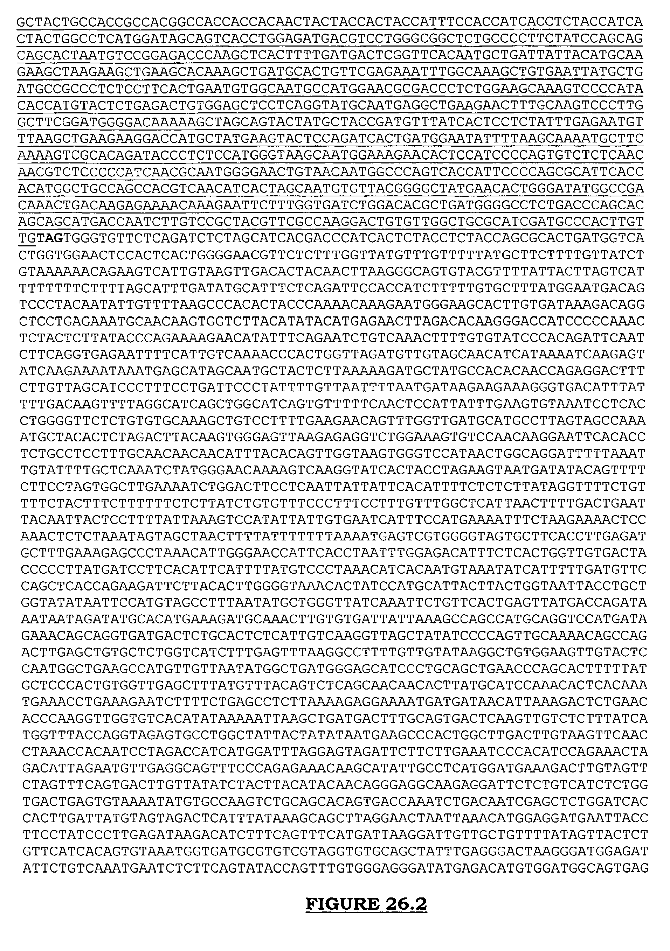

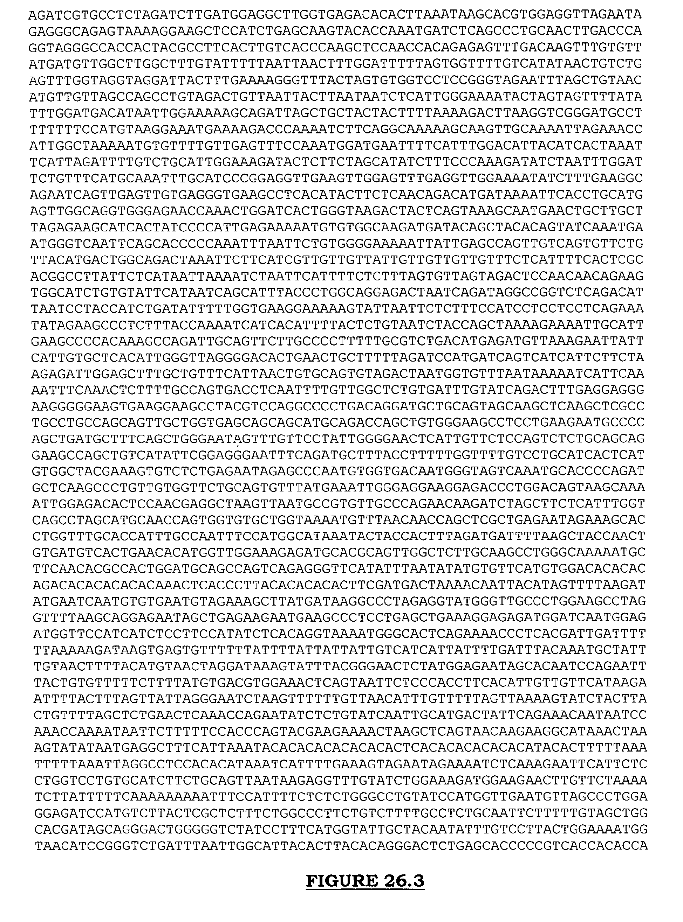

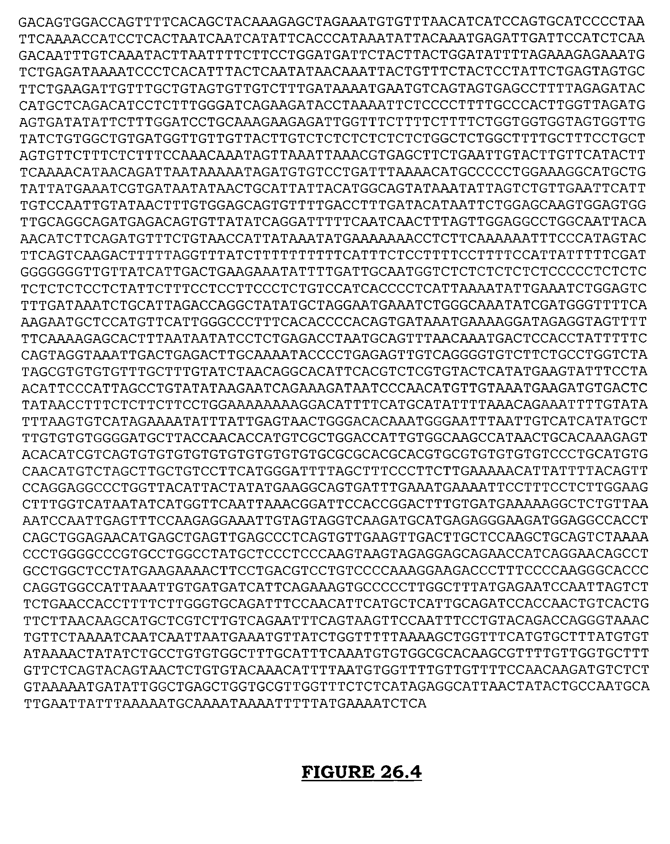

Des séquences du type des LTR 5' et 3' de HERV-7q, fortement conservées sont concernées par de tels effets régulateurs. A titre d'exemple on décrit LTX, une séquence comparable à celle d'une LTR de HERV-7q (SEQ ID NO:67 et figure 16), et qui se trouve au coeur d'un intron de plus de 49 kb, mais à 2 kb du site 5' donneur, du gène de FMR2 associé au X-fragile et codant pour une protéine de 1311 acides aminés (figure 26). Les LTR modulent l'épissage alternatif (Kapitonov et Jurka, (1999), J. Mol. Evol., 48, 248- 251), l'expression du gène, la fixation sur des protéines nucléaires (Akopov et coll., (1998), FEBS Lett., 421, 229- 233), ou permettent l'obtention d'un signal de polyadénylation alternatif (Goodchild et coll., (1992), Gene, 121, 287- 294).

D'une manière générale, on peut remarquer l'existence de plusieurs séquences rétrovirales endogènes de type HERV-7q (HE4, HE5, HE9, HE10), situées au niveau du chromosome X qui représente le chromosome associé au plus grand nombre de pathologies.

A cet égard, on peut relever que des motifs rétroviraux issus de régions défectives sont aptes à présenter des fonctions biologiques: par exemple, la protéine d'enveloppe p15E issue de motifs rétroviraux défectifs, possède une activité anti-inflammatoire et immunosuppressive (Snyderman et Ciancolo, 1984, Immunol. Today, 5, 240-244).

Ces structures sont vraisemblablement à même de provoquer des brèches ou d'amplifier des dérégulations dans les processus de défense immunitaire. Certains des motifs des domaines *gag, env* et de type LTR peuvent être associés à une fonction particulière ou peuvent contribuer à la fonction normale ou pathologique des domaines flanquants tels que définis ci-dessus (SEQ ID NO:62-67). Des recombinaisons avec un élément d'origine exogène, rétroviral ou non, peut donner lieu à la production de motifs nucléiques ou protéiques qui pourraient soit protéger, soit déclencher, ou favoriser ou aggraver une pathologie. De même, une structure rétrovirale contenant des éléments rétroviraux endogènes selon l'invention seraient à même de provoquer un processus pathologique après passage par un cycle transitoire exogène puis réintégration dans une région sensible ou critique du génome humain.

On peut ainsi obtenir des profils d'expression (transcrits et éventuellement protéines) qui correspondent aux neuropathologies précitées.

De même, la combinaison de motifs appartenant à la famille de HERV-7q, ou d'éléments induits par des motifs appartenant à la famille de HERV-7q, avec des motifs d'origine ou induits de manière exogène seraient à même de pouvoir déclencher, ou aggraver un processus pathologique ou au contraire de favoriser une protection ou une rémission partielle ou une guérison totale et définitive.

La détection rendue possible des domaines de type HERV-7q, suggère des applications possibles à la fois au niveau prophylactique, du pronostic et du diagnostic : par exemple des approches immunologiques ou d'amplification génique permettant de comparer des individus normaux servant de référence avec des patients, seraient à même de favoriser le dépistage, d'améliorer la détection précoce de la déclaration de la maladie et/ou de suivre l'évolution d'une pathologie chez des patients pouvant présenter une susceptibilité ou ayant déclaré la maladie ou encore chez des individus considérés comme normaux, selon les critères cliniques actuels.

Les sondes nucléiques et immunologiques spécifiques, telles que définies, dans la présente invention sont à même de favoriser l'identification et la détection de motifs anormalement exprimés dans le cadre de pathologies associées au cancer, ou de neuropathologies en particulier auto-immunes, au premier rang desquelles la sclérose en plaques.

La présente invention a également pour objet des séquences nucléiques hybrides, caractérisées en ce qu'elles comprennent des séquences ou motifs appartenant à la famille de HERV-7q, ou d'éléments induits par des motifs appartenant à la famille de HERV-7q, avec des motifs d'origine ou induits de manière exogène (séquences rétrovirales exogènes) ; de telles séquences hybrides sont vraisemblablement à même de pouvoir déclencher, ou aggraver un processus pathologique ou au contraire de favoriser une protection ou une rémission partielle ou une guérison totale et définitive.

La présente invention a également pour objet un réactif de diagnostic pour la détection différentielle de séquences nucléiques endogènes humaines complètes ou partielles, présentant des motifs rétroviraux, sélectionnés parmi les séquences SEQ ID NO :1 et/ou SEQ ID NO :2, caractérisé en ce qu'il est sélectionné dans le groupe constitué par les séquences SEQ ID NO:1-22, 28, 37-57, 59-61 et 121-122, les séquences nucléiques complémentaires et les séquences inverses complémentaires des séquences précédentes, par les fragments nucléotidiques capables de définir ou d'identifier les séquences SEQ ID NO:1 et/ou SEQ ID NO:2 et toute séquence flanquante ou les chevauchant ainsi que par les fragments issus des régions codantes des séquences SEQ ID NO:1-22 et 61, correspondant à un cadre glissant supérieur ou égal à 14 nucléotides ou leurs séquences complémentaires, éventuellement marquées avec un marqueur approprié ainsi que par les séquences telles que définies aux figures 18-21.

Les séquences des sondes nucléiques, ribonucléiques et oligonucléotidiques utilisées seront choisies dans les régions *env* et *gag* ou leur régions flanquantes : par exemple les oligonucléotides amorces pour HERV-7q, seront choisis dans les régions situées entre les nucléotides 3065 et 4390, les nucléotides 6965 et 9550 ou les nucléotides 2502-2865 de la SEQ ID NO:3, ainsi que dans toute séquence adjacente (amont ou aval) capable de permettre une amplification spécifique (figure 1).

Parmi les marqueurs appropriés, on peut citer, les isotopes radioactifs, les enzymes, les fluorochromes, des marqueurs chimiques (biotine), les haptènes (digoxygénine) et les anticorps ou analogues de bases appropriées.

De manière préférée :
- ledit réactif est sélectionné parmi les séquences SEQ ID NO:37-57 et est apte à être utilisé comme amorce.
- ledit réactif est sélectionné parmi les séquences suivantes :
   un fragment de 1505 nt amplifié par le couple d'amorces SEQ ID NO:37 et SEQ ID NO:38 (amorces G1F et G1R),
   un fragment de 2529 nt amplifié par le couple d'amorces SEQ ID NO:45 et SEQ ID NO:46 (amorces E1F et E1R),
   un fragment de 182 nucléotides répété deux fois, situé en amont du domaine *gag* aux positions 2502-2611/2613-2865,
   des fragments codants ou non-codants pour tout ou partie de l'envérine, comprenant au moins 14 nucléotides et notamment les fragments codant pour la partie C-terminale de l'envérine, soit à partir de l'acide aminé 291, soit à partir de l'acide aminé 321, à compter de la première méthionine,
et est apte à être utilisé comme sonde.

La présente invention a également pour objet un procédé de détection rapide et différentiel des séquences nucléiques rétrovirales endogènes de type *env* ou *env* et *gag,* de leurs variants normaux ou pathologiques, par hybridation et/ou amplification génique, réalisé à partir d'un échantillon biologique, lequel procédé est caractérisé en ce qu'il comprend :
(a) une étape dans laquelle l'on met en contact un échantillon biologique à analyser avec au moins une sonde telle que définie ci-dessus et
(b) une étape dans laquelle on détecte par tout moyen approprié, le ou les produits résultant de l'interaction séquence nucléotidique-sonde.

Conformément audit procédé, il peut comprendre :
* préalablement à l'étape (a) :
   - une étape de préparation du tissu ou du liquide biologique concerné,
   - une étape d'extraction de l'acide nucléique à détecter, et
   - au moins un cycle d'amplification génique et
* postérieurement à l'étape (b) :
   - une étape de comparaison des séquences nucléiques obtenues dans ledit échantillon biologique avec les séquences rétrovirales endogènes humaines selon l'invention par tout moyen approprié et notamment par séquençage, Southern-blot, coupure de restriction, SSCP ou toute autre méthode permettant d'identifier une insertion ou une délétion ou encore une simple mutation entre les différentes séquences comparées.

Conformément à l'invention, les séquences rétrovirales endogènes humaines selon l'invention sont ainsi comparées aux séquences nucléiques présentes dans l'échantillon biologique à analyser et permettent la détection de séquences homologues de patients atteints de pathologies, susceptibles de mettre en jeu une modification de leur génome.

De manière avantageuse, lesdites comparaisons géniques sont menées à partir d'ADN génomique provenant d'individus témoins et de patients.

Une amplification génique classique par PCR sera menée à l'aide d'amorces 5' -sens et 3' -antisens encadrant ou comprenant la zone à étudier (zone *env* ou zone *gag*).

Également de manière avantageuse, les séquences des sondes nucléiques, ribonucléiques et oligonucléotidiques utilisées sont choisies dans les régions *env* et *gag* ou leurs régions flanquantes : par exemple les oligonucléotides amorces pour HERV-7q, seront choisis dans les régions situées entre les nucléotides 3065 et 4390 et les nucléotides 6965 et 9550, ainsi que dans toute séquence adjacente (amont ou aval) capable de permettre une amplification spécifique (figure 1), comme précisé ci-dessus. Elles sont de préférence sélectionnées dans le groupe constitué par
un fragment de 1505 nt amplifié par le couple d'amorces SEQ ID NO:37 et SEQ ID NO:38 (amorces G1F et G1R),
un fragment de 2529 nt amplifié par le couple d'amorces SEQ ID NO:45 et SEQ ID NO:46 (amorces E1F et E1R).

L'étape d'amplification génique est notamment réalisée à l'aide d'une des techniques d'amplification génique suivante : amplification par la Qβ-réplicase, PCR, LCR, ERA, CPR ou SDA.

La présente invention a également pour objet des séquences chimères, caractérisées en ce qu'elles sont constituées par un fragment de 17 à 40 nucléotides d'une séquence flanquante telle que définie ci-dessus associée à un motif rétroviral endogène de type HERV-7q comprenant entre 17 et 40 nucléotides, tel que défini ci-dessus.

La présente invention a également pour objet un procédé de détection des transcrits, tels que définis ci-dessus, caractérisé en ce qu'il comprend :
- le prélèvement des ARN messagers provenant d'échantillons biologiques (tissus, cellules, fluides biologiques) témoins et d'un échantillon analogue prélevé chez des patients et
- l'analyse qualitative et/ou quantitative desdits ARNm, par hybridation *in situ,* par dot-blot, Northern-blot, RNAse mapping ou RT-PCR, à l'aide d'un réactif de diagnostic tel que défini ci-dessus.

La présente invention a également pour objet une méthode de détection et/ou d'évaluation d'une sur-expression/sous-expression ou d'une modification d'au moins l'une des séquences ou fragments de séquences rétrovirales endogènes de type HERV-7q et/ou de leurs séquences flanquantes associées, caractérisée en ce qu'elle comprend :
- le dépôt sur un support approprié comme par exemple un filtre de nylon, une lame de verre ou leur équivalent, de l'ADNc ou son équivalent provenant de clones, de produits de PCR obtenus à partir d'ADN génomique, de produits de RT-PCR provenant de transcrits ou encore de séquences oligonucléotidiques spécifiques, lesdites séquences d'ADN étant des séquences ou des fragments de séquences rétrovirales endogènes de type HERV-7q et/ou leurs séquences flanquantes, telles que définies ci-dessus, constituées par les transcrits et ADNc des séquences génomiques, qui codent pour tout ou partie d'un facteur, dont la fonction, la régulation/dérégulation ou l'altération est associée à l'expression normale ou pathologique ou à la régulation/dérégulation de motifs appartenant à ladite famille HERV-7q, ces séquences correspondant à des séquences nucléotidiques codant pour des gènes situés dans des régions flanquantes situées en amont et/ou en aval d'une séquence rétrovirale de ladite famille HERV-7q et dont l'une des extrémités ne peut se trouver à une distance excédant 120 kb, et/ou une séquence chimère telle que définie ci-dessus,
- l'hybridation dudit support avec au moins une sonde marquée de manière adéquate obtenue, par exemple, par rétrotransposition d'un mélange d'ARN provenant de cellules, de tissus ou de liquides biologiques provenant de témoins réputés normaux, de membres de populations ethniques différentes, de patients atteints de pathologies souvent associées à une expression de rétrovirus, comme les processus tumoraux, ou comme les maladies auto-immunes, et
- la détection des hybrides formés.

Selon un mode de mise en oeuvre avantageux de ladite méthode, ledit transcrit ou ADNc est sélectionné dans le groupe constitué par les séquences SEQ ID NO:62-67 et 119 et leurs fragments correspondant à un cadre glissant supérieur ou égal à 14 nucléotides ou leurs séquences complémentaires.

Selon un autre mode de mise en oeuvre avantageux de ladite méthode, ledit support comprend en outre toute séquence rétrovirale endogène ou exogène.

La méthode des puces à ADN (Bowtell, (1999), Nature Genet., 21, 25- 32), est utilisée pour évaluer la modification de l'expression de tout ou partie de certaines des séquences d'origine rétrovirale de type HERV-7q et des séquences flanquantes. Brièvement de l'ADN provenant de clones, de produits de PCR obtenus à partir d'ADN génomique, de produits de RT-PCR provenant de transcrits ou encore de séquences oligonucléotidiques spécifiques, sont déposées sur un support, comme par exemple un filtre de nylon, une lame de verre ou leur équivalent. Les séquences nucléiques déposées couvrent les différents domaines rétroviraux décrits ci-dessus, ainsi que les séquences contiguës et les gènes flanquants. Afin de détecter d'éventuels processus d'épissage alternatifs, des ADN spécifiques sont synthétisés par pas de 500-600 nucléotides avec un chevauchement de 250- 300 nucléotides de part et d'autre. Les épissages alternatifs déjà identifiés feront l'objet d'une synthèse spécifique. L'hybridation s'effectue à l'aide d'une sonde obtenue, par exemple, par rétrotransposition d'un mélange d'ARN provenant de cellules, de tissus ou de liquides biologiques provenant de témoins réputés normaux, de membres de populations ethniques différentes, de patients atteints de pathologies souvent associées à une expression de rétrovirus, comme les processus tumoraux, ou comme les maladies auto-immunes, dont la sclérose en plaques. Dans ce cas une fraction de µg et jusqu'à quelques µg d'ARNm ou jusqu'à quelques µg ou dizaines de µg d'ARN, selon la méthode utilisée et la taille de la puce d'ADN concernée, sont suffisants pour la synthèse de la sonde nucléique. La sonde nucléique est marquée de manière adéquate, afin d'autoriser une détection ultérieure, comme par exemple par fluorescence ou par une méthode équivalente.

L'usage de sondes bi-, voire multicolores permet de préciser l'expression concertée de plusieurs gènes en parallèle, en bénéficiant de plus d'une normalisation précise. L'acquisition des résultats est effectuée automatiquement, comme par exemple par un système de balayage laser ou son équivalent.

Deux types de puces à ADN sont conçues, d'une part des puces présentant un ensemble exhaustif de séquences, et d'autre part des puces à ADN spécifiques permettant un ciblage sur une application plus spécifique.

Par exemple, une séquence critique en ce qu'elle contiendrait une différence portant sur une délétion, voire une mutation, est détectée à l'aide d'oligonucléotides spécifiques (Wang et coll., (1998), Science, 280, 1077- 1082). Le polymorphisme associé à une base ou à une mutation est détecté à l'aide de quatre oligonucléotides possédant une des quatre possibilités de séquence au niveau d'une base (A, C G ou T): pour chaque différence ponctuelle les 4 oligonucléotides sont déposés et les intensités d'hybridation sont comparées. De plus, un épissage alternatif est détecté en utilisant des ADN correspondant à un seul exon effectif ou putatif : le gène est donc analysé exon par exon. Les puces à ADN concernent aussi, par extension, toute séquence rétrovirale endogène ou exogène, comme par exemple ERV-9, ERV-K, ERV-L, ERV-H, ERV-4, ERV-6, ERV-8, ERV-10, ERV-15, ERV-16, ERV-17, ERV-18, ERV-21, ERV-24, ERV-33, ERV-34, ERV-36, ERV-40, ERV-42, ERV-MLN, ERV-FRD, ERV-FTD...), ainsi que toutes les séquences exoniques putatives (identifiées par l'existence de séquences étiquettes et des transcrits correspondants) ou effectives, et qui sont situées de part et d'autre jusqu'à une distance de 120 kb des séquences rétrovirales endogènes de type HERV-7q.

L'étude comparative est menée entre un échantillon témoin et l'échantillon à tester, dans une perspective prophylactique, diagnostique ou thérapeutique, comme par exemple: la détection précoce d'une modification de l'expression d'une des séquences, dans une cellule, un tissu ou un organisme, l'identification d'une séquence associée à une susceptibilité ou à une pathologie quelconque, le suivi de l'évolution de la pathologie, ou encore le suivi d'un traitement et l'évaluation de son efficacité.

En dehors des applications déjà énoncées, l'intérêt de la méthode permet, d'une manière plus générale, de faire un bilan des variations constatées chez un individu, ce qui constitue en quelque sorte une carte d'identité, ce qui facilite une approche épidémiologique permettant d'établir de nouvelles corrélations entre un profil particulier observé et une pathologie, en dehors de tout *a priori* concernant cette pathologie.

La présente invention a également pour objet un kit de détection et/ou d'évaluation d'une maladie auto-immune et notamment des neuropathologies à étiologie auto-immune, caractérisé en ce qu'il comprend outre les tampons nécessaires à la mise en oeuvre des procédés tels que définis ci-dessus :
- des réactifs A de diagnostic tels que définis ci-dessus, et
- des réactifs B constitués par les transcrits et ADNc des séquences génomiques, qui codent pour tout ou partie d'un facteur, dont la fonction, la régulation/dérégulation ou l'altération est associée à l'expression normale ou pathologique ou à la régulation/dérégulation de motifs appartenant à ladite famille HERV-7q, ces séquences correspondant à des séquences nucléotidiques codant pour des gènes situés dans des régions flanquantes situées en amont et/ou en aval d'une séquence rétrovirale de ladite famille HERV-7q dont l'une des extrémités ne peut se trouver à une distance excédant 120 kb,
lesquels réactifs sont de préférence fixés sur un support approprié.

Selon un mode de réalisation avantageux dudit kit, lesdits réactifs B sont sélectionnés dans le groupe constitué par les séquences SEQ ID NO:62-67 et 119 et leurs fragments correspondant à un cadre glissant supérieur ou égal à 14 nucléotides ou leurs séquences complémentaires, ainsi que les séquences représentées aux figures 13-17, 22-26.

La présente invention a également pour objet des produits de traduction, caractérisés en ce qu'ils sont codés par une séquence nucléotidique telle que définie ci-dessus.

La présente invention a également pour objet un peptide, caractérisé en ce qu'il est susceptible d'être exprimé à l'aide d'une séquence nucléotidique sélectionnée dans le groupe constitué par les séquences SEQ ID NO:1-22, 28 et 61, telles que définies ci-dessus, selon les combinaisons offertes par l'usage des différents cadres de lecture possibles (voir également figures 18-21).

Ledit peptide englobe également les peptides ou polypeptides dérivés comprenant entre 5 et 540 aminoacides (SEQ ID NO:23-36 et SEQ ID NO:58 et leurs fragments d'au moins 5 aminoacides) et notamment un fragment de 538 aminoacides, commençant à la première méthionine de la séquence SEQ ID NO:26 (envérine).

Selon un mode de réalisation avantageux desdits peptides, ils sont notamment sélectionnés parmi les séquences SEQ ID NO:23-36, 58, notamment la séquence SEQ ID NO:26 et ses fragments C-terminaux, soit à partir de l'acide aminé 291, soit à partir de l'acide aminé 321, à compter de la première méthionine.

Selon un autre mode de réalisation avantageux desdits peptides, ils sont obtenus à partir des séquences nucléiques telles que définies ci-dessus, dans lesquelles au moins un codon non-sens peut être remplacé par un codon codant pour l'un des aminoacides suivants : Phe (F), Leu (L), Ser (S), Tyr (Y), Cys (C), Trp (W), Gln (Q), Arg (R), Lys (K), Glu (E) ou Gly (G).

L'invention englobe ainsi les peptides déduits ou les protéines déduites correspondant à tout ou partie des séquences nucléiques décrites dans l'invention, et présentant éventuellement des modifications avec les séquences de références décrites dans l'invention, lorsqu'ils sont exprimés chez certains patients. En particulier, l'invention englobe les séquences complètes ou partielles obtenues selon les 3 cadres de lecture sens et les 3 cadres de lecture inverses et complémentaires. (voir figures 18-21)

De manière avantageuse, l'analyse de la structure du domaine env de HERV-7q, appelé envérine, a permis de mettre successivement en évidence:
- un peptide signal N-terminal (région 1- 21) et deux domaines transmembranaires (région 320-340; 455-477), responsables d'interactions avec des motifs protéiques ou lipidiques membranaires,
- un motif immunomodulateur de type CKS-17(Haraguchi et coll., (1995), 92, 5568- 5571)/ CKS-25. On peut noter à cet égard, la présence d'un motif **RalD** à l'intérieur du peptide de type CKS-17/CKS-25 de HERV-7q et un motif **RvaD** en position 363 qui correspondent au consensus W/RxxD, proposé pour le site actif des TGF-β (Huang et al., J. Biol. Chem., 1997, 272, 27155- 27159), de puissants facteurs associés à la croissance, à la différenciation et à la morphogenèse et qui sont associés à de nombreuses pathologies humaines, comme les processus tumoraux (Tang et coll., (1998), Nat. Med., 4, 802- 807) ou les maladies neurodégénératives (Flanders et coll., (1998), Prog. Neurobiol., 54, 71- 85). Les peptides selon l'invention contenant ces motifs peuvent avantageusement servir d'antagonistes en inhibant la fixation des TGF- β sur leurs récepteurs naturels.
- des motifs de N-glycosylation. La glycosylation des protéines d'enveloppe des rétrovirus semble être directement associée à leurs propriétés fonctionnelles, par exemple en influençant le nombre des déterminants disponibles dans les cellules-T ou en favorisant la reconnaissance des antigènes par les cellules-T. La glycosylation pourrait jouer un rôle dans la déclaration ou l'extension d'une pathologie à incidence autoimmune. Les glycosylations sont nécessaires au maintien de la conformation de certains épitopes, en particulier lors de la réalisation d'une protéine d'enveloppe recombinante à fin de mise au point d'un réactif de diagnostic et pour favoriser l'efficacité d'un éventuel vaccin. Positions 171, 210, 216, 236, 244, 283 et 411. Nombre prévu au hasard : 3.2
- des sites de prénylation. La prénylation est un mécanisme essentiel de la fixation à la membrane cellulaire et pour le ciblage de certaines protéines. Ce processus de ciblage pourrait être essentiel pour l'élaboration d'agents thérapeutiques spécifiques aptes à interférer dans la réalisation et la régulation du trafic de complexes cellulaires mettant en jeu des protéines impliquées dans les interactions, la croissance et les mouvements cellulaires. Positions 188 et 290. Nombre prévu au hasard : 1.8
- des sites de ciblage dans le réticulum endoplasmique. Ces sites permettraient d'assurer le ciblage vers le réticulum endoplasmique afin d'effectuer les modifications nécessaires pour favoriser le franchissement membranaire. Positions 353 et 431. Nombre prévu au hasard : 0.2.

Par ailleurs, les Inventeurs ont montré qu'un certain nombre de peptides issus de la protéine env de HERV-7q (envérine) présentent une affinité/demi-vie élevées pour des allèles HLA de classe I. Une analyse par CADD a permis de sélectionner des peptides candidats, dont les meilleurs scores sont indiqués dans le Tableau I:

**TABLEAU I**

| localisation | séquence | molécule HLA | score | Séquence n° |
|---|---|---|---|---|
| 399 | FLGEECCYYV | A-0201 | 7214 | SEQ ID NO:68 |
| 462 | LLFGPCIFNL | A-0201 | 1792 | SEQ ID NO:69 |
| 189 | CLPLNFRPYV | A-0201 | 1453 | SEQ ID NO:70 |
| 439 | GLLSQWMPWI | A-0201 | 488 | SEQ ID NO:71 |
| 263 | CLPSGIFFV | A-0201 | 5103 | SEQ ID NO:72 |
| 444 | WMPWILPFL | A-0201 | 897 | SEQ ID NO:73 |
| 252 | IRWVTPPTQI | B-2705 | 3000 | SEQ ID NO:74 |
| 432 | LRNTGPWGLL | B-2705 | 2000 | SEQ ID NO:75 |
| 158 | LRTHTRLVSL | B-2705 | 2000 | SEQ ID NO:76 |
| 316 | KRVPILPFVI | B-2705 | 1800 | SEQ ID NO:77 |
| 25 | CRCMTSSSPY | B-2705 | 1000 | SEQ ID NO:78 |
| 137 | TRVHGTSSPY | B-2705 | 1000 | SEQ ID NO:79 |
| 124 | AREKHVKEVI | B-2705 | 600 | SEQ ID NO:80 |
| 478 | SRIEAVKLQM | B-2705 | 600 | SEQ ID NO:81 |
| 442 | SQWMPWILPF | B-2705 | 500 | SEQ ID NO:82 |
| 405 | CYYVNQSGI | Kd | 2400 | SEQ ID NO:83 |
| 346 | FYYKLSQEL | Kd | 2400 | SEQ ID NO:84 |
| 244 | TYTTNSQCI | Kd | 2400 | SEQ ID NO:85 |
| 291 | SFLVPPMTI | Kd | 1600 | SEQ ID NO:86 |
| 406 | YYVNQSGIV | Kd | 1200 | SEQ ID NO:87 |
| 167 | LFNTTLTGL | Kd | 1152 | SEQ ID NO:88 |
| 463 | LFGPCIFNL | Kd | 960 | SEQ ID NO:89 |
| 253 | RWVTPPTQI | Kd | 480 | SEQ ID NO:90 |
| 449 | LPFLGPLAAI | B-5102 | 2200 | SEQ ID NO:91 |
| 3 | LPYHIFLFTV | B-5102 | 1210 | SEQ ID NO:92 |
| 331 | GALGTGIGGI | B-5102 | 798 | SEQ ID NO:93 |
| 321 | LPFVIGAGVL | B-5102 | 550 | SEQ ID NO:94 |
| 499 | RRPLDRPAS | B-2705 | 600 | SEQ ID NO:95 |
| 194 | FRPYVSIPV | B-2705 | 600 | SEQ ID NO:96 |
| 383 | RRALDLLTA | B-2705 | 600 | SEQ ID NO:97 |
| 39 | WRMQRPGNI | B-2705 | 600 | SEQ ID NO:98 |
| 423 | DRIQRRAEEL | B14 | 1800 | SEQ ID NO:99 |
| 158 | LRTHTRLVSL | B14 | 600 | SEQ ID NO:100 |
| 359 | ERVADSLVTL | B14 | 540 | SEQ ID NO:101 |
| 463 | LFGPCIFNLL | Kd | 1658 | SEQ ID NO:102 |
| 345 | QFYYKLSQEL | Kd | 1152 | SEQ ID NO:103 |
| 443 | QWMPWILPFL | Kd | 691 | SEQ ID NO:104 |
| 405 | CYYVNQSGIV | Kd | 500 | SEQ ID NO:105 |
| 474 | NFVSSRIEAV | Kd | 480 | SEQ ID NO:106 |
| 221 | GPLVSNLEI | B-5102 | 1320 | SEQ ID NO:107 |
| 190 | LPLNFRPYV | B-5102 | 726 | SEQ ID NO:108 |
| 449 | LPFLGPLAAI | B-5101 | 1144 | SEQ ID NO:109 |
| 488 | EPKMQSKTKI | B-5101 | 968 | SEQ ID NO:110 |
| 3 | LPYHIFLFTV | B-5101 | 629 | SEQ ID NO:111 |
| 125 | REKHVKEVI | Kk | 1000 | SEQ ID NO:112 |
| 312 | KPRNKRVPIL | B7 | 800 | SEQ ID NO:113 |
| 378 | VVLQNRRAL | Db | 792 | SEQ ID NO:114 |
| 377 | AVVLQNRRAL | Db | 660 | SEQ ID NO:115 |
| 321 | LPFVIGAGV | B-5101 | 629 | SEQ ID NO:116 |
| 304 | DLYSYVISK | A3 | 540 | SEQ ID NO:117 |
| 301 | TEQDLYSYVI | Kk | 500 | SEQ ID NO:118 |

Ce Tableau I indique une estimation de la demi-vie de dissociation d'un peptide de l'envérine avec un allèle du système HLA de classe I (les tables de coefficients de Parker: J. Immunol, (1994), 152, 163- 175). La localisation indique la position du premier acide aminé des peptides testés dans la séquence de l'envérine. Le code à une lettre est utilisé pour la séquence des acides aminés. Les scores autour de 500 ou supérieurs à 500 ont été retenus. A titre de comparaison, une analyse a été effectuée sur une concaténation de peptides (polypeptide de 4968 acides aminés) réputés pour fixer les molécules du complexe majeur d'histocompatibilité de classe 1 (Rammensee, Immunogenetics, (1995), 41, 178- 228): les dix meilleurs scores enregistrés pour des nonapeptides et le type HLA, A_0201 sont respectivement de 4984, 4047, 2406, 1267, 800, 705, 607, 591, 591 et 577.

Il ressort de ce Tableau I que certaines molécules du complexe majeur d'histocompatibilitè de type I sont aptes à fixer des peptides issus de l'envérine, ainsi assimilés à des peptides d'origine virale ou tumorale, au niveau du réticulum endoplasmique. Les complexes formés au niveau du réticulum endoplasmique sont alors transportés à la surface cellulaire, ce qui entraîne la destruction de la cellule cible par les lymphocytes-T cytotoxiques. Les peptides identifiés comportent généralement 8 à 10 acides aminés. Des études ont montré que certains allèles du système HLA de classe I sont ainsi associées à certaines pathologies, en particulier à caractère auto-immun, comme HLA-B27 avec la spondylarthrite ankylo-sante ou HLA-B51 avec la maladie de Behçet.

Un peptide apte à fixer une molécule particulière de classe I est par conséquent apte à fonctionner comme un épitope de cellule-T.

En conséquence, la présente invention inclut également les fragments 399-471 et 244-271 de l'envérine qui regroupent avantageusement plusieurs épitopes de forte affinité pour différents haplotypes du système HLA de classe I. L'usage de tout ou partie de ces polypeptides est en conséquence apte à favoriser une augmentation du répertoire des cellules-T, en permettant une meilleure efficacité de la réponse immunitaire dans le cadre des différentes stratégies immunothérapeutiques, prophylactique ou vaccinales). Ces peptides pourront être avantageusement délivrés par exemple par l'usage, de vecteurs viraux, de particules virales ou synthétiques, de lipopeptides, d'adjuvants classiques, d'acides nucléiques nus ou adsorbés sur des particules, ou de liposomes.

Au sens de la présente invention, les peptides peuvent être chimiquement ou biochimiquement modifiés; certaines des acides aminés peuvent être remplacés par un acide aminé analogue, selon les critères classiques d'homologies (A ou G ; S ou T ; I , L ou V;F,Y ou W ; N ou Q ; D ou E).

La présente invention a également pour objet des compositions immunogènes ou vaccinales, pour la protection contre les maladies auto-immunes, notamment chez les sujets à risque, caractérisée en ce qu'elle comprend au moins un peptide comprenant au moins un motif de type CKS et/ou au moins un peptide constitué par un motif présentant une affinité avec l'un des haplotypes du système HLA de classe I ou de classe II et un véhicule pharmaceutiquement acceptable.

Selon un mode de réalisation avantageux de ladite composition, ledit motif est sélectionné dans le groupe constitué par les peptides tels que définis dans le Tableau I ci-dessus.

Selon un autre mode de réalisation avantageux de ladite composition, ledit peptide présente la séquence suivante :
séquence CKH: LQNRRALDLLTAERGGTcl**FLGEECCYYV** (SEQ ID NO:120).

Il est remarquable de constater au niveau de la position 380 de la protéine envérine, la contiguïté des motifs de type CKS-17 (souligné) et du peptide présentant le score le plus élevé (en gras ; voir peptide en position 399 dans le Tableau I, SEQ ID NO:68) dans la séquence CKH.

L'activation clonale des sous-groupes de lymphocytes, par exemple de lymphocytes cytotoxiques, par les peptides du Tableau I et par extension leurs homologues, est bloquée par des manoeuvres usuelles d'immunothérapie comme par exemple la sérothérapie et la vaccination.

L'association de deux séquences ou des séquences analogues au peptide CKH (SEQ ID NO:120), est à même d'entraîner un processus synergique dans la réponse immunitaire, qui pourrait mettre en jeu des voies de signalisation et d'activation complémentaires, aptes à moduler l'activation lymphocytaire.

La vaccination concerne la production d'anticorps dirigés contre les peptides du tableau I, selon les règles de l'art et selon les méthodes de libération contrôlées par implants artificiels ou cellulaires mettant en oeuvre une composition telle que définie ci-dessus et par usage des manoeuvres de thérapie génique, comme par exemple par expression des séquences nucléiques codant pour les peptides du Tableau I. En conséquence l'invention a également pour objet des compositions immunogènes ou vaccinale caractérisée en ce qu'elles comprennent un vecteur incluant au moins une séquence nucléique codant un peptide tel que défini dans le Tableau I, éventuellement associée à une séquence codant un motif de type CKS-17.

La sérothérapie concerne l'utilisation d'anticorps neutralisants produits à partir des peptides du Tableau I et leurs homologues.

Les produits protéiques générés par les séquences rétrovirales endogènes ou produits parallèlement peuvent avantageusement être caractérisés par des micro-méthodes d'analyse et de quantification des peptides et des protéines: HPLC/FPLC ou équivalent, électrophorèse capillaire ou équivalent, techniques de microséquençages (méthode d'Edman ou équivalent, spectrométrie de masse...).

L'invention a également pour objet des anticorps dirigés contre l'un ou plusieurs des peptides décrits ci-dessus et leur utilisation soit pour la mise en oeuvre d'une méthode de détection *in vitro,* notamment différentielle de la présence d'une telle séquence chez un individu, soit pour la préparation d'une composition apte à être utilisée en sérothérapie dans les neuropathologies à composante auto-immune.

Lesdits anticorps sont avantageusement des anticorps polyclonaux ou monoclonaux obtenus par une réaction immunologique d'un organisme humain, mammifères, oiseaux ou autres espèces vis-à-vis des protéines, telles que définies ci-dessus.

La présente invention a pour objet un procédé de dépistage immunologique différentiel de séquences rétrovirales endogènes humaines de la famille HERV-7q normales ou pathologiques, caractérisé en ce qu'il comprend la mise en contact d'un échantillon biologique avec un anticorps selon l'invention, la lecture du résultat étant révélée par un moyen approprié, notamment EIA, ELISA, RIA, fluorescence.

A titre d'illustration, une telle méthode de diagnostic *in vitro* selon l'invention comprend la mise en contact d'un échantillon biologique prélevé chez un patient, avec des anticorps selon l'invention et la détection à l'aide de tout procédé approprié, notamment à l'aide d'anti-immunoglobulines marquée, des complexes immunologiques formés entre les protéines produites normalement ou pathologiquement et les anticorps.

Des anticorps monoclonaux ou polyclonaux, produits à partir d'antigènes correspondants à des peptides de synthèse, de polypeptide ou protéines recombinants, permettent de suivre l'expression des peptides ou protéines produits normalement ou pathologiquement. L'analyse est de préférence effectuée par ELISA, ou équivalent, Western-blot ou équivalent, ou par immunohistochimie.

Les peptides ou protéines, issus des séquences rétrovirales endogènes ou dont l'expression est associée à l'expression de ces séquences rétrovirales endogènes, sont recherchés et identifiés.

La présente invention a également pour objet un procédé d'identification et de détection de motifs rétroviraux endogènes, anormalement exprimés dans le cadre de pathologies associées au cancer, ou de neuropathologies en particulier auto-immunes, au premier rang desquelles la sclérose en plaques, caractérisé en ce qu'il comprend l'analyse comparée des séquences extraites d'un échantillon biologique avec les séquences selon l'invention.

La présente invention a également pour objet l'application des séquences nucléiques ou des séquences protéiques selon l'invention au diagnostic, au pronostic, à l'évaluation de la susceptibilité génétique, à toutes maladies humaines induites, innées ou acquises en particulier celles à composantes cancéreuses, auto-immunes et/ou à incidence neurologique, comme la sclérose en plaques, les syndromes associés et les maladies neurodégénératives où intervient tout ou partie des séquences nucléiques selon l'invention et des formes endogènes ou exogènes apparentées.

La présente invention a également pour objet des séquences nucléiques hybrides, caractérisées en ce qu'elles comprennent des séquences ou motifs nucléiques selon l'invention, combinés avec des séquences ou motifs d'origine endogène ou d'origine ou induits de manière exogène.

La présente invention a, en outre, pour objet un vecteur recombinant de clonage ou d'expression, caractérisé en ce qu'il comprend une séquence nucléique conforme à l'invention.

Des manoeuvres thérapeutiques peuvent être envisagées par usage de certaines des séquences nucléiques contenues dans HERV-7q et les séquences de la même famille ou des structures polypeptidiques déduites ou par utilisation de peptides ou protéines, ou d'anticorps spécifiques.

Conformément à l'invention, tout ou partie des séquences nucléiques rétrovirales endogènes de type HERV-7q, peut être utilisée à usage de vecteur ou d'éléments de vecteurs à vocation thérapeutique, en particulier les séquences LTR et la région *gag* (SEQ ID NO :2, 21 et 22)..

L'intérêt de telles séquences réside, dans l'innocuité du vecteur ainsi formé, dans la possibilité d'une insertion spécifique ciblée dans une région bien définie par une stratégie analogue à la recombinaison homologue, dans le ciblage cellulaire, éventuellement transitoire dans le cas d'une expression placentaire chez la femme. Un autre aspect concerne la possibilité d'associer aux gènes d'intérêts les motifs rétroviraux biologiquement actifs (peptides immunomodulateurs, tels que représentés aux séquences SEQ ID NO 68-118, ci-après, peptide fusogène...).

La présente invention a également pour objet des animaux transgéniques, caractérisés en ce qu'ils comprennent tout ou partie d'une séquence de type HERV-7q (SEQ ID NO:1-22 et 61).

Le Tableau II ci-après établit les correspondances entre les numéros des séquences telles qu'elles apparaissent dans la liste de séquences et le nom des différentes séquences.

**TABLEAU II**

| **SEQ ID NO:** | **DÉSIGNATION** |
|---|---|
| 1 | Acide nucléique: 7 env |
| 2 | Acide nucléique: gag |
| 3 | Acide nucléique: HERV-7q |
| 4 | Acide nucléique: HE2 |
| 5 | Acide nucléique: HE3 |
| 6 | Acide nucléique: HG3 |
| 7 | Acide nucléique: HE4 |
| 8 | Acide nucléique: HE5 |
| 9 | Acide nucléique: HE6 |
| 10 | Acide nucléique: HG6 |
| 11 | Acide nucléique: HE7 |
| 12 | Acide nucléique: HE8 |
| 13 | Acide nucléique: HG8 |
| 14 | Acide nucléique: HE9 |
| 15 | Acide nucléique: HE 10 |
| 16 | Acide nucléique: HE11 |
| 17 | Acide nucléique: HG11 |
| 18 | Acide nucléique: HE 12 |
| 19 | Acide nucléique: HG12 |
| 20 | Acide nucléique : R1 |
| 21 | Acide nucléique : R1F |
| 22 | Acide nucléique + protéine env déduite : HERV-7q |
| 23 | Fragment de protéine env déduite selon SEQ ID NO :22 |
| 24 | Fragment de protéine env déduite selon SEQ ID NO :22 |
| 25 | Fragment de protéine env déduite selon SEQ ID NO :22 |
| 26 | Protéine : envérine |
| 27 | Fragment de protéine env déduite selon SEQ ID NO :22 |
| 28 | Acide nucléique + protéine déduite de gag : HERV-7q |
| 29 | Fragment de protéine gag déduite selon SEQ ID NO :28 |
| 30 | Fragment de protéine gag déduite selon SEQ ID NO :28 |
| 31 | Fragment de protéine gag déduite selon SEQ ID NO :28 |
| 32 | Fragment de protéine gag déduite selon SEQ ID NO :28 |
| 33 | Fragment de protéine gag déduite selon SEQ ID NO :28 |
| 34 | Fragment de protéine gag déduite selon SEQ ID NO :28 |
| 35 | Protéine env : cadre de lecture 1 |
| 36 | Protéine gag |
| 37 | Acide nucléique : G1F (amorce) |
| 38 | Acide nucléique : G1R (amorce) |
| 39 | Acide nucléique: G2F (amorce) |
| 40 | Acide nucléique : G2R (amorce) |
| 41 | Acide nucléique: G4F (amorce) |
| 42 | Acide nucléique: G3F (amorce) |
| 43 | Acide nucléique: G4R (amorce) |
| 44 | Acide nucléique: G5R (amorce) |
| 45 | Acide nucléique: E1F (amorce) |
| 46 | Acide nucléique: E1R (amorce) |
| 47 | Acide nucléique : E2F (amorce) |
| 48 | Acide nucléique : E2R (amorce) |
| 49 | Acide nucléique : E3F (amorce) |
| 50 | Acide nucléique: E3R (amorce) |
| 51 | Acide nucléique : E4F (amorce) |
| 52 | Acide nucléique : E4R (amorce) |
| 53 | Acide nucléique: E5F (amorce) |
| 54 | Acide nucléique: E6F (amorce) |
| 55 | Acide nucléique : E5R (amorce) |
| 56 | Acide nucléique : ExF (amorce) |
| 57 | Acide nucléique : ExR (amorce) |
| 58 | Protéine gag |
| 59 | Acide nucléique: Séquence A (séquence d'insertion) |
| 60 | Acide nucléique : Séquence B (séquence d'insertion) |
| 61 | Acide nucléique : HE13 |
| 62 | Acide nucléique : RH7 |
| 63 | Acide nucléique : RAM75 |
| 64 | Acide nucléique : RAV73 |
| 65 | Acide nucléique : RBP3 |
| 66 | Acide nucléique : HI3 |
| 67 | Acide nucléique : LTX |
| 68 | Peptide Tableau I |
| 69 | Peptide Tableau I |
| 70 | Peptide Tableau I |
| 71 | Peptide Tableau I |
| 72 | Peptide Tableau I |
| 73 | Peptide Tableau I |
| 74 | Peptide Tableau I |
| 75 | Peptide Tableau I |
| 76 | Peptide Tableau I |
| 77 | Peptide Tableau I |
| 78 | Peptide Tableau I |
| 79 | Peptide Tableau I |
| 80 | Peptide Tableau I |
| 81 | Peptide Tableau I |
| 82 | Peptide Tableau I |
| 83 | Peptide Tableau I |
| 84 | Peptide Tableau I |
| 85 | Peptide Tableau I |
| 86 | Peptide Tableau I |
| 87 | Peptide Tableau I |
| 88 | Peptide Tableau I |
| 89 | Peptide Tableau I |
| 90 | Peptide Tableau I |
| 91 | Peptide Tableau I |
| 92 | Peptide Tableau I |
| 93 | Peptide Tableau I |
| 94 | Peptide Tableau I |
| 95 | Peptide Tableau I |
| 96 | Peptide Tableau I |
| 97 | Peptide Tableau I |
| 98 | Peptide Tableau I |
| 99 | Peptide Tableau I |
| 100 | Peptide Tableau I |
| 101 | Peptide Tableau I |
| 102 | Peptide Tableau I |
| 103 | Peptide Tableau I |
| 104 | Peptide Tableau I |
| 105 | Peptide Tableau I |
| 106 | Peptide Tableau I |
| 107 | Peptide Tableau I |
| 108 | Peptide Tableau I |
| 109 | Peptide Tableau I |
| 110 | Peptide Tableau I |
| 111 | Peptide Tableau I |
| 112 | Peptide Tableau I |
| 113 | Peptide Tableau I |
| 114 | Peptide Tableau I |
| 115 | Peptide Tableau I |
| 116 | Peptide Tableau I |
| 117 | Peptide Tableau I |
| 118 | Peptide Tableau I |
| 119 | Acide nucléique : BLIMP-1 |
| 120 | Peptide : CKH |
| 121 | Acide nucléique : F645 (amorce) |
| 122 | Acide nucléique : PS5D (amorce) |

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :
- Figure 1. Séquence nucléique humaine HERV-7q, dont l'analyse et le traitement permettent de caractériser une nouvelle structure rétrovirale endogène. Les régions nucléiques répétées de type R1 et R2 et les domaines *gag, pol* et *env* sont soulignés. Les domaine de type *gag* et *env* sont en italiques. La région homologue à une partie 3' non-codante de Rab7 est doublement soulignée.
- Figure 2. Cartographie de la région rétrovirale endogène humaine HERV-7q. La partie haute de la figure correspond à une région anonyme du génome humain située sur le bras long du chromosome 7. On peut identifier les domaines répétés (1), *gag* (2), *pol* (3) et *env* (4) de HERV-7q. La région *env* C-terminale (4.3) se prolonge en amont en un long cadre de lecture ouvert (4.2). Le domaine 4.1, correspond à la région N-terminale du domaine *env.*
- Figure 3. Comparaison des séquences nucléiques répétées situées aux bornes de HERV-7q. Les régions nucléiques répétées 5'(haut) et 3'(bas), sont comparées et les bases identiques sont indiquées par deux points.
- Figure 4. Séquence déduite présentant un cadre de lecture ouvert, dans le domaine de type-env de HERV-7q selon la règle du plus long cadre de lecture ouvert.
- Figure 5. Séquences autour du domaine CKS-17 identifiées dans différents domaines *env* déduits de la famille de HERV-7q et comparaison avec des motifs CKS-17 de référence.
   1) HE2 - 2) HERV-7q - 3) N° d'accès à GenBank: M85205 - 4) HE7 - 5) HE9 - 6) CKS-17: le motif peptidique doué de propriétés immunomodulatrices est souligné - 7) gp20 de rétrovirus de type-D (SRV-Pc).
- Figure 6. Séquence déduite possible du domaine de type-gag identifié dans HERV-7q établie selon la règle du plus long cadre de lecture ouvert. X et / correspondent respectivement à un codon non-sens et à un décalage de cadre de lecture. La séquence soulignée correspond au début du domaine *pol.*
- Figure 7. Comparaison des régions nucléiques couvrant la région *gag* de HERV-7q (haut) et HERV-TcR (bas) et leurs régions flanquantes. Les bases identiques sont spécifiées par deux points.
- Figure 8. Exemple d'alignements nucléiques du domaine de type *env* de HERV-7q avec des domaines de type *env* similaires présents dans des séquences rétrovirales endogènes humaines de la même famille. Les codons non sens sont soulignés : 1) HERV-7q - 2) HE2 - 03) HE3 - 04) HE4.
- Figure 9. Alignements nucléiques entre le domaine *gag* de HERV-7q et les domaines correspondants appartenant à la même famille. Comparaison avec des fragments de domaines *gag* isolés d'agents rétroviraux infectieux. Séquences d'origine rétrovirale infectieuse: N° d'accession dans la banque de données EMBL : 1) A60168 - 2) A60201 - 3) A60200 - 4) A60171. Séquences rétrovirales endogènes humaines: 5) HERV-7q - 6) HG11 - 7) HG3. Les chiffres indiqués dans les séquences endogènes, correspondent au nombre de nucléotides insérés afin d'optimiser l'alignement avec les séquences de type *gag* identifiées dans des rétrovirus d'origine infectieuse.
- Figure 10. Alignement d'un motif *gag* protéique déduit (haut) appartenant à un rétrovirus infectieux (N° d'accession EMBL : A60200) avec le motif *gag* protéique déduit (bas) identifié dans HERV-7q. Les codons non-sens sont en gras et soulignés. Les acides aminés identiques sont spécifiés par 2 tirets. Un tiret indique une délétion ou un acide aminé homologue.
- Figure 11. Alignement d'un motif *env* (haut) appartenant à un rétrovirus infectieux (N° d'accession EMBL : A60170) avec le motif *env* (bas) identifié dans HERV-7q. Les nucléotides homologues sont spécifiés par deux points et les délétions par un tiret.
- Figure 12. Comparaison entre le domaine *env* de HERV-7q (haut) et le domaine *env* de HERV-9 (bas). L'homologie de 66 % se limite à la région 3' du domaine *env* de HERV-7q et HERV-9, respectivement entre les nucléotides 8976 nt et 9500 nt de HERV-7q et les nucléotides 2898 nt et 3465 nt de HERV-9 (N° d'accession à GenBank : X57147). De nombreuses insertions/délétions sont aussi observées.
- Figure 13. Homologie entre une partie de la séquence du transcrit codant pour RH7 (haut, SEQ ID NO:62) et un motif de RGH2 (bas - N° d'accession à GenBank: D11018).
- Figure 14. Identification de la séquence du transcrit codant pour RAM75 (SEQ ID NO:63), correspondant au gène d'une ATPase de type PEX1. Les exons codants sont soulignés. Les codons d'initiation et non-sens ainsi que les sites putatifs de polyadénylation sont en gras et soulignés. La région en italique correspond au début de la séquence rétrovirale endogène RH7.
- Figure 15. Séquence du transcrit codant pour RAV73 (SEQ ID NO:64), située à 0.7 kb en aval de HERV-7q ; les séquences nucléiques aptes à coder pour un ou plusieurs polypeptides sont soulignées.
- Figure 16. Comparaison entre la séquence LTR 3' (haut) de HERV-7q et la séquence intronique LTX (SEQ ID NO:67), située dans le gène FMR2, associé au X-fragile (bas).
- Figure 17. Mise en évidence de modifications sur la séquence nucléotidique (ID NO:3), chez des patients atteints de SEP. Les bases modifiées, chez au moins un patient, sont soulignées. Les amorces utilisées sont en italiques (séquences SEQ ID NO:121 et 122). L'ATG d'initiation et le codon non-sens sont en gras.
- Figure 18. Partie codante *env* de la séquence HERV-7q (séquence ID NO:3), avec 3 cadres de lecture.
- Figures 19, 20, 21. Présentation séparée de la protéine *env* selon les 3 cadres de lecture.
- Figure 22. Séquence nucléique contenant la séquence rétrovirale RH7 située en 5' de la séquence HERV-7q. La séquence en italique correspond au début de la séquence HERV-7q. La séquence RH7 est soulignée. Deux sites de polyadénylation putatifs sont gras.
- Figure 23. Séquence du transcrit codant pour RBP3 contenant des motifs nucléotidiques identifiés dans la séquence nucléique codant pour le gène Blimp-1.
- Figure 24. Séquence du transcrit codant pour APS.
- Figure 25. Séquence du transcrit codant pour Blimp-1 ; la partie codante est soulignée ; les codons d'initiation et de terminaison sont en gras.
- Figure 26. Séquence du transcrit codant pour FMR2. La partie codante est soulignée. Les codons d'initiation et non-sens sont en gras.

Il doit être bien entendu, toutefois, que ces exemples sont donnés uniquement à titre d'illustration de l'objet de l'invention, dont ils ne constituent en aucune manière une limitation.

### EXEMPLE 1 : Détection, par amplification génique, d'une séquence nucléique appartenant à un domaine de type gag ou env selon l'invention, dans un échantillon d'ADN génomique d'origine humaine ou de mammifères.

L'amplification génique s'effectue à partir d'ADN génomique isolé à partir du sang. Un traitement anticoagulant est effectué avec 1 ml d'une solution de citrate (pour un litre : 4,8 g de d'acide citrique, 13,2 g de citrate de sodium, 14,7 g de glucose) pour 6 ml de sang frais. Après centrifugation de 20 ml de sang pendant 15 mn à 13.0000 g, le surnageant est éliminé et la fraction enrichie en globules blancs est transférée dans un nouveau tube, puis recentrifugée dans les mêmes conditions que précédemment. La fraction enrichie en globules blancs est resuspendue dans un tampon d'extraction (10 nm Tris-HCl, 0,1 M EDTA, 20 µg/ml de RNAse pancréatique traitée afin d'éliminer les DNAses, 0,5 % SDS, pH 8,0), puis incubée pendant 1 heure à 37°C. La protéinase K est ajoutée à une concentration finale de 100 µg/ml. La suspension des cellules lysées est incubée à 50°C durant 3 heures sous agitation périodique, puis traitée par un volume égal de phénol équilibré par du Tris-HCl 0,5 M, pH 8,0. L'émulsion formée est placée sur une roue pendant une heure, puis centrifugée à 5000 g pendant 15 mn à température ambiante. La solution aqueuse est traitée déprotéinisé par une triple extraction phénolique afin d'obtenir un niveau de purification correspondant à un rapport final d'absorbance A260/A280 supérieur à 1,75. La fraction aqueuse est précipitée par 0,2 vol. d'acétate de sodium 10 M et 2 vol. d'éthanol. L'ADN est alors soit prélevé avec l'extrémité d'une pipette pasteur recourbée, soit centrifugé à 5000 g pendant 5 mn à température ambiante. L'ADN ou le culot d'ADN est lavé deux fois par de l'éthanol à 70 %, puis repris dans 1 ml de TE pH 8,0 afin d'être élué sous agitation douce pendant 12 à 24 heures.

Des oligonucléotides spécifiques des séquences endogènes décrites selon l'invention sont choisis pour amplifier la région *gag* ou *env* des régions rétrovirales endogènes décrites selon l'invention. L'ADN génomique étudié provient de patients présentant des pathologies comme la sclérose en plaques et d'individus réputés sains.

Les ADN polymérases thermostables utilisées ont été choisies pour leur grande fidélité lors du processus d'amplification, comme la Ventᵣ ADN polymérase (Biolabs) ou équivalent, et sont utilisées selon les conditions préconisées par le fournisseur.

La stratégie d'amplification utilise selon les cas une simple PCR, ou une PCR nichée ou semi-nichée.

Oligonucléotides utilisés pour amplifier la région *gag* :
- amorce G1F, sens, localisée dans la région amont du domaine *gag de HERV-7*q (SEQ ID NO:37),
- amorce G1R, anti-sens, localisée dans la région 3' terminale du domaine *gag* (SEQ ID NO:38),

Le fragment de 1505 nt amplifié par le couple G 1 F-G 1 R : 1505 nt est utilisé afin de générer les sondes aptes à hybrider les différents produits d'amplification des PCR.
- amorce G2F, sens nichée (SEQ ID NO:39),
- amorce G2R, anti-sens nichée (SEQ ID NO:40),
- amorce G4F, sens nichée (SEQ ID NO:41),
- amorce G3F, sens nichée (SEQ ID NO:42),
- amorce G4R, anti-sens nichée (SEQ ID NO:43),
- amorce G5R, anti-sens nichée (SEQ ID NO:44),
Oligonucléotides utilisés pour amplifier la région *env* de HERV-7q :
- amorce E1F, sens (SEQ ID NO:45),
- amorce E1R, anti-sens (SEQ ID NO:46),

Le fragment de 2529 nt amplifié par le couple d'amorces E1F-E1R, est utilisé afin de générer les sondes aptes à hybrider les différents produits d'amplification des PCR.
- amorce E2F, sens (SEQ ID NO:47),
- amorce E2R, antisens (SEQ ID NO:48),
- amorce E3F, sens (SEQ ID NO:49),
- amorce E3R, anti-sens (SEQ ID NO:50),
- amorce E4F, sens (SEQ ID NO:51),
- amorce E4R, anti-sens (SEQ ID NO:52),
- amorce E5F, sens (SEQ ID NO:53),
- amorce E6F, sens(SEQ ID NO:54)
- amorce E5R(SEQ ID NO:55).
- amorce ExF (SEQ ID NO:56)
- amorce ExR (SEQ ID NO:57)

La PCR est réalisée à partir de 50 à 200 ng d'ADN génomique. Les conditions de PCR sont celles préconisées par le fournisseur. Les conditions cycliques d'amplification sont réalisées dans 50 µl: une dénaturation de 94°C pendant 1 min., une hybridation de 70°C pendant 1 min., et une élongation à 72 °C pendant 1 à 2 min., selon les fragments amplifiés. Après 35 cycles, une réaction terminale est menée à 72°C pendant 10 min. Le séquençage automatique des échantillons amplifiés est réalisé à l'aide d'un séquenceur Applied Biosystems de type ABI 377 ou autre modèle comparable, selon les protocoles fournis par le constructeur.

Dans le cas d'une PCR nichée ou semi-nichée, les mêmes conditions expérimentales sont utilisées, à la seule différence que l'échantillon d'ADN génomique est remplacé par 5 à 10 µl du produit d'amplification issu de la première PCR.

Deux amplifications indépendantes sont réalisées à partir du même échantillon. Une réaction de contrôle est réalisée en remplaçant l'échantillon d'ADN par de l'eau afin de détecter d'éventuels contaminants.

### EXEMPLE 2 : Détection par amplification génique d'une séquence nucléique selon l'invention dans un échantillon biologique d'ADN génomique prélevé chez des patients présentant une pathologie candidate déclarée ou la suspicion de cette pathologie.

Le protocole d'amplification est le même que dans l'exemple 1, mis à part l'origine de l'échantillon qui provient de patients présentant une pathologie candidate. Un échantillon d'ADN génomique réputé normal est systématiquement intégré dans l'ensemble des échantillons pathologiques amplifiés puis analysés.

Les produits de PCR sont séparés sur un gel d'agarose à 1,5 %, puis transférés en présence de soude 0,4 N sur une membrane de nylon chargé. Une hybridation est réalisée avec une sonde spécifique correspondant aux fragments de PCR amplifiés soit par les couples G1F-G1R soit par le couple E1F-E1R. La sonde est marquée par incorporation de dUTP-digoxygénine selon le protocole du fournisseur (Boehringer Mannheim). L'hybridation est effectuée dans un tampon d'hybridation (5XSSC, 50 % formamide, 0,1 % lauroyl-sarcosine, 0,02 % SDS, 2 % de réactif de blocage Boehringer) pendant une nuit à 42°C. Le Southern est lavé 2 fois 5 min. à température ambiante dans une solution de 2XSSC, 0,1% SDS. Puis un lavage à haute stringence est effectué à deux reprises pendant 15 min. à 55°C dans une solution 0,1XSSC, 0,1 % SDS. L'hybridation est révélée selon le protocole du fournisseur (Boehringer Mannheim), en présence d'un substrat chimioluminescent de la phosphatase alcaline, de type CSPD ou CDP-STAR. Le filtre est révélé après une exposition de 15min. à 60 min.

Une analyse par SSCP (" *single strand conformation polymor-phism* ") permet de détecter des modifications discrètes de la séquence des fragments amplifiés par PCR. La PCR est menée en présence de dCTP marqués au P³². L'échantillon a analyser est dénaturé à 95°C pendant 10 min., en présence de tampon de charge, puis immédiatement chargé sur un gel de polyacrylamide à 10%, contenant 7.5% de glycérol. La migration s'effectue à 4°C à 8-10 W. Le gel est séché puis auto-radiographié.

Les fragments de PCR susceptibles de présenter une altération de leur séquence nucléotidique sont séquencés selon l'exemple 1.

Une hybridation à l'aide d'un oligonucléotide spécifique (17 mers à 20 mers) correspondant à la région nucléotidique modifiée permet d'identifier les échantillons présentant une modification identique (méthode ASO). Brièvement le southern est hybridé avec un oligonucléotide marqué distalement soit au P³², soit en présence de digoxygénine (selon le protocole de Boehringer Mannheim) puis lavé dans des conditions stringentes à 65%C dans une solution 6XSSC, 0.05% pyrophosphate de sodium.

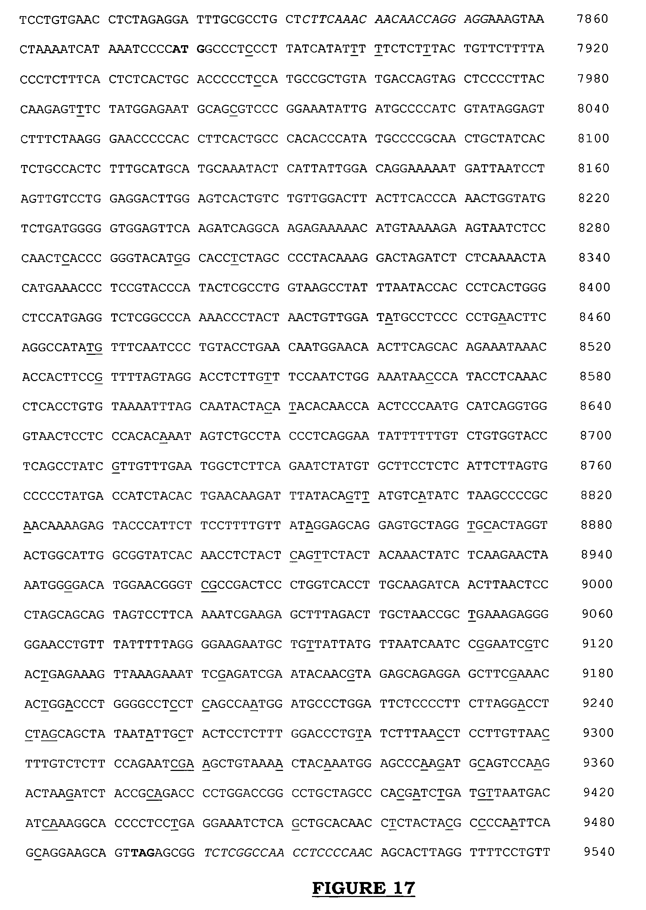

Par exemple, un séquençage nucléotidique automatique a été réalisé sur six fragments de PCR, provenant de 5 patients atteints de SEP et un témoin réputé normal, et qui ont été amplifiés à partir des amorces F645: CTTCAAACAACAACCAGGAGG (SEQ ID NO:121) (située à 26 nucléotides en amont de la méthionine d'initiation de l'envérine) et PS5D: TTGGGGAGGTTGGCCGACGA (SEQ ID NO:122) (située à 6 nucléotides en aval du codon non-sens de l'envérine. Des modifications de la séquence de l'envérine ont été observés sur l'ADN de certains des patients (figure 17).

### EXEMPLE 3 : Détection d'une protéine selon l'invention dans un échantillon biologique.

### - Préparation d'une fraction protéique purifiée de liquide céphalo-rachidien de patients atteints de SEP

Après un traitement à 56°C pendant 30 min, et élimination des immunoglobulines sur une colonne de protéine G HiTrap (Pharmacia), l'équivalent de 10 ml de LCR est déposé sur une colonne de DEAE Sepharose CL-6B (Pharmacia). L'élution est réalisée en Tris-HCl 20 mM pH 8,8, et un gradient de 0 à 0,4 M de NaCl, puis la fraction est dialysée 2 fois contre du tampon phosphate-NaCl (PBS). Après concentration sur Ultrafree-MC (Millipore), la fraction est déposée sur une colonne de Superose 12 (FPLC Pharmacia) et éluée en présence de PBS. Après séparation par électrophorèse en gel de polyacrylamide-SDS, et électo-transfert sur une membrane d'Immobilon-P (Millipore), les bandes protéiques sont soumises à une hydrolyse trypsique ménagée.

### - Analyse de la fraction protéique par spectrométrie de masse

Les peptides digérés en présence de trypsine, sont analysés par la méthode de MALDI-TOF, qui permet l'analyse de peptides présents en mélange. (COTTRELL J.S., Pept. Res., 1997, 7, 115-124). Les peptides caractérisés en fonction de leur masse sont comparés aux protéines et aux protéines associées selon l'invention.

### EXEMPLE 4 : Détection d'anticorps spécifiques anti-domaine env de HERV-7q.

L'identification d'un long cadre de lecture ouvert au sein de la séquence *env* de HERV-7q, a permis de déterminer une séquence protéique déduite SEQ ID NO:22 et 35 et figures 18-20 d'une région dudit gène.

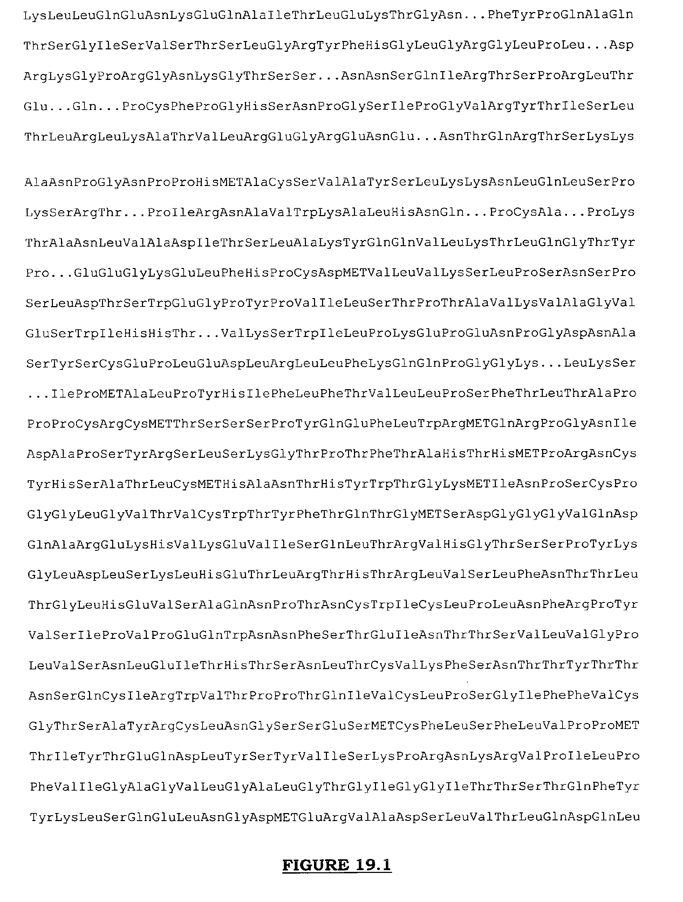

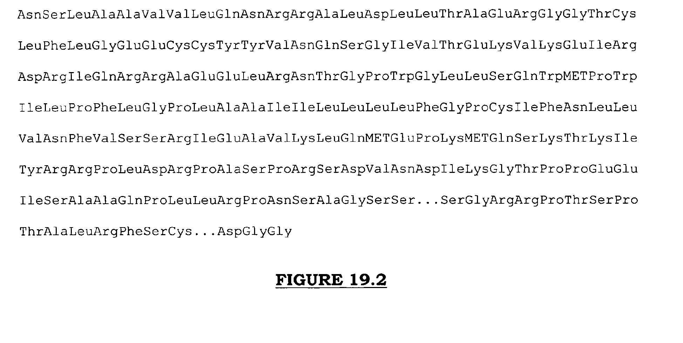

Les séquences de protéines déduites des séquences ID NO:22, 35 et des figures 18-20 sont positionnées comme suit par rapport à la figure 1 ou à la séquence ID NO:3 :
SEQ ID NO:22 (cadre de lecture 1) et figure 19 : début de la séquence codante : position 7874, fin de la séquence codante 1^{er} codon non-sens (position 9493)
SEQ ID NO:35: début de la séquence codante : position 7874, fin de la séquence codante 1^{er} codon non-sens (position 9493) (cadre de lecture 1)
Figure 19 : début de la séquence codante : position 6970, fin de la séquence codante 1^{er} codon non-sens (position 9493) (cadre de lecture 1)

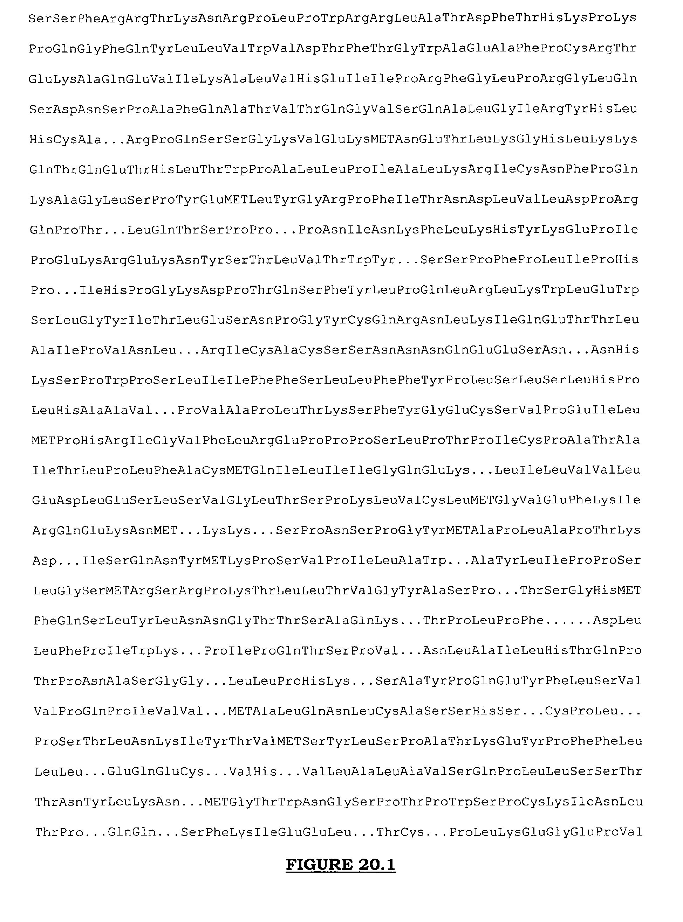

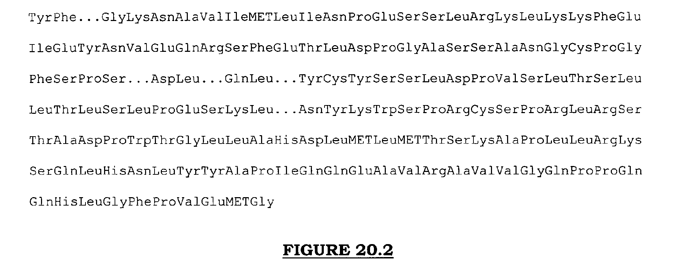

Figure 20 : début de la séquence codante : position 6971, la fin du cadre de lecture est décalée selon le cas de 1, 2 ou 3 codons

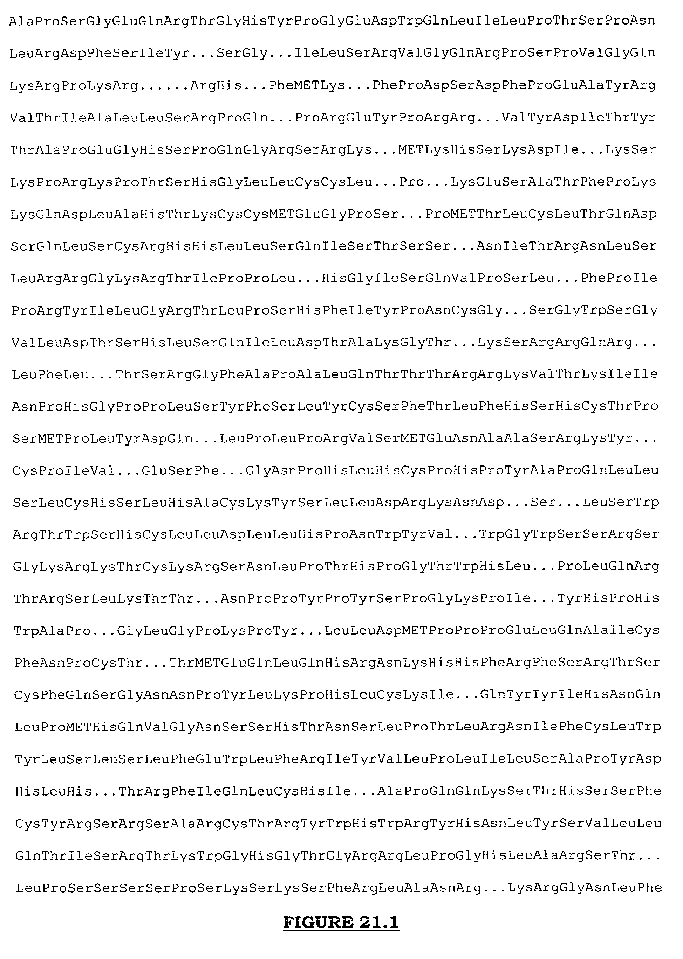

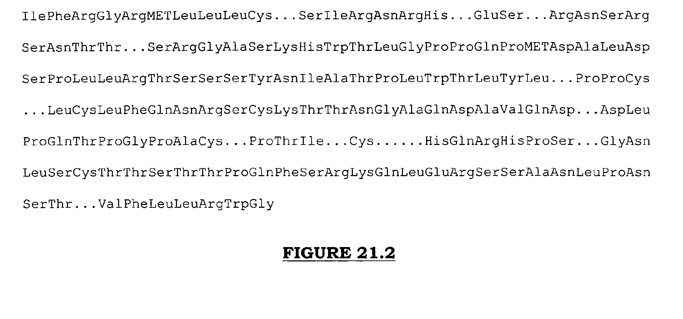

Figure 21 : début de la séquence codante : position 6972, la fin du cadre de lecture est décalée selon le cas de 1, 2 ou 3 codons

Différents peptides correspondant à tout ou partie des SEQ ID NO:22 (voir SEQ ID NO:23-27 et 35) ont été synthétisés par génie génétique afin de tester leur spécificité antigénique vis-à-vis de séra ou de tissus de patients atteints de SEP, par exemple. Brièvement, tout ou partie de la région env de HERV-7q est sous clonée dans les vecteurs pQE30, 31 et 32. Les vecteurs pQE30, 31 et 32 contiennent en 5' du multi-site de clonage les séquences consensuelles pour la transcription (le promoteur fort du bactériophage T5, 2 opérateurs de l'opéron lactose), la traduction (un site d'accrochage ribosomal synthétique). De même, pQE30, 31 et 32 possèdent en 3', le terminateur de transcription du phage 1 ainsi qu'un codon "Stop" pour la traduction. L'expression de la protéine s'effectue après transformation dans *E. coli* M15. Le plasmide pQE30, 31 et 32 possèdent en amont du site de polyclonage la séquence codante pour une suite de 6 histidines présentant une affinité pour les ions nickel. Cet enchaînement permet la purification de la protéine chimérique exprimée, par adsorption sur une résine constituée d'un ligand chélatant, l'acide nitrilotriacétique (NTA), chargé de 4 ions nickel (résine NI-NTA, Qiagen).

La transformation s'effectue par électroporation ou traitement au chlorure de calcium. Par exemple, une colonie d'*E. coli* M15 est incubée dans 100 ml de milieu LB contenant 250 µg de kanamycine, sous agitation à 37°C jusqu'à l'obtention d'une DO⁶⁰⁰ de 0,5. Après une centrifugation de 5 minutes à 2000g à 4°C, le culot bactérien est repris dans 30 ml de solution TFB1 (100 mM de chlorure de rubinium, 50 mM de chlorure de manganèse, 30 mM d'acétate de potassium, 10 mM CaCl2, 15% glycérol, pH 5.8), à 4°C pendant 90 minutes. Après une centrifugation de 5 minutes à 2000g à 4°C, le culot bactérien est repris dans 4 ml de solution TFB2 (10 mM de chlorure de rubidium, 10 mM de MOPS, 75 mM CaCl2, 15% de glycérol , pH 8). Les cellules peuvent être gardées à -70°C par aliquot de 500 ml. 20 µl de la ligation et 125 µl de cellules compétentes sont mélangés et placés dans la glace 20 minutes. Après un choc thermique de 42°C pendant 90 secondes, les cellules sont agitées 90 minutes à 37°C dans 500 ml de milieu Psi-broth (milieu LB complémenté par 4 mM de MgSO₄, 10mM de chlorure de potassium). Les cellules transformées sont étalées sur des boîtes LB-agar complémentées par 25 µg/ml de kanamycine, et 100µg/ml d'ampicilline, et les boîtes sont incubées une nuit à 37°C.

Les clones potentiellement recombinants sont repiqués de manière ordonnée sur un filtre de nylon déposé sur une boîte LB-agar complémentée par 25 µg/ml de kanamycine et 100 µg/ml d'ampicilline. Après une nuit à 37°C, les clones recombinants sont repérés par hybridation de l'ADN plasmidique avec la sonde nucléotidique amplifiée par PCR avec le couple d'amorces selon SEQ ID NO:45 et SEQ ID NO:46.

Une colonie indépendante, contenant l'insert, est inoculée à 20 ml de milieu LB complémentée par 25 µg/ml de kanamycine et 100 µg/ml d'ampicilline. Après une nuit à 37°C sous agitation, 500 ml de même milieu sont incubés au 1/50° par cette préculture jusqu'à l'obtention d'une D0⁶⁰⁰ de 0,8, puis 1 à 2 mM final d'IPTG est ajouté. Après 5 heures, les cellules sont centrifugées 20 minutes à 4000 g.

Une partie du culot cellulaire est repris dans 5 ml de tampon de sonication (50 mM de phosphate de sodium pH 7,8, 300 mM NaCl) puis placé dans la glace. Après une rapide sonication, les cellules sont centrifugées 20 minutes à 10000 g. Une partie du culot cellulaire est repris dans 10 ml d'une solution 30 mM Tris/HCl-20% sucrose pH8. Les cellules sont incubées 5 à 10 minutes sous agitation, après adjonction de 1 mM EDTA. Après une centrifugation de 10 minutes à 8000 g à 4°C, le culot est repris dans 10 ml de 5 mM de MgSO4 glacé. Après 10 minutes dans la glace sous agitation, les cellules sont centrifugées 10 minutes à 8000 g à 4°C.

Le culot est repris par 5 ml/g dans du tampon A (6 M GuHCl (chlorhydrate de guanidine), 0,1 M phosphate de sodium, 0,01 M Tris/HCl, pH 8), 1 heure à température ambiante. Le lysat est centrifugé 15 minutes à 10000 g à 4°C, et le surnageant est complémenté par 8 ml de résine Ni-NTA, prééquilibrée dans du tampon A. Après 45 minutes à température ambiante, la résine est coulée dans une colonne, lavée par 10 fois le volume de la colonne par du tampon A puis par 5 fois le volume da la colonne par du tampon B (8 M urée, 0,1 M phosphate de sodium, 0,01 M Tris/HCl, pH 8). La colonne est lavé par du tampon C (8 M urée, 0,1 M phosphate de sodium, 0,01 M Tris/HCl, pH 6,3) jusqu'à ce que l'A280 soit inférieur à 0,01. La protéine recombinante est éluée par 10 à 20 ml de tampon D (8 M urée, 0,1 M phosphate de sodium, 0,01 M Tris/HCl, pH 5,9) puis par 10 à 20 ml de tampon E (8 M urée, 0,1 M phosphate de sodium, 0,01 M Tris/HCl, pH 4,5), puis par 20 ml de tampon F (6 M HCl, 0,2 M acide acétique). Après une analyse en SDS-PAGE, la ou les fractions purifiées contenant la protéine chimérique ont permis l'obtention d'anticorps chez le lapin. Les anticorps obtenus sont testés par Western-blot après révélation par un anticorps secondaire couplé à la phosphatase alcaline.

Des anticorps sont obtenus de la même manière, à partir de peptides synthétisés chimiquement selon la technique de Merrifield (G. Barany and B. Merrifield, 1980, dans The peptides, 2, 1-284, E. Gross et J. Meienhofer, Academic Press, New York).

Les anticorps spécifiques obtenus sont utilisés à fin de détection de l'expression sérique ou tissulaire de tout ou partie des séquences rétrovirales endogènes selon l'invention, dans les cas normaux et pathologiques.

Les protéines d'origine sérique ou tissulaire, sont séparées sur gel d'acrylamide-SDS puis transférées sur un filtre de nitrocellulose à l'aide d'un appareil Novablot 2117-2250 (LKB). Le transfert est effectué sur une feuille de Hybond C-extra (Amersham) en utilisant un tampon CAPS 100 mM pH 11, méthanol, eau (V/V/V: 1/1/8) contenant 1 mM de CaCl₂. Après un transfert de 1 heure à 0,8 mA/cm², la feuille est saturée une heure à température ambiante dans du PBS-0,5 % gélatine. La feuille est mise en présence de l'anticorps spécifique à la concentration de 1/1000 dans du PBS-0,25 % gélatine. Au bout de 2 heures, le filtre est lavé 3 fois 15 minutes dans du PBS-0,1 % de Tween-20, puis le filtre est incubé 30 minutes en présence d'un anticorps secondaire couplé à la phosphatase alcaline (Promega), dilué au 1/7500 dans du PBS-0,25% gélatine. Après trois lavages dans du PBS-0,1 % de Tween-20, le filtre est équilibré dans un tampon (100 mM de Tris-HCl pH 9,5, 100 mM de NaCl, 5 mM de MgCl₂). La révélation est effectuée en présence de 45 µl de NBT à 75 mg/ml et 35 µl de BCIP à 50 mg/ml, pour 10 ml de tampon de phosphatase alcaline.

Les protéines chimériques obtenues par génie génétique, sont utilisées aussi à fin de tests d'activité biologique, comme par exemple pour le test d'activité biologique du peptide de type CKS-17 identifié dans le domaine *env* de HERV-7q (figure 5).

### EXEMPLE 5 : Obtention de sondes ribonucléiques codant pour les séquences env de HERV-7q.

Les fragments de PCR obtenus sont sous clonés dans le plasmide PGEM 4Z (Promega) qui possède de par et d'autre de son site de polyclonage, les séquences promotrices pour les ARN polymérase SP6 et T7.

La méthode de compétence utilisée est l'électroporation. Le plasmide et le fragment de PCR sont hybridés dans un rapport de 50 ng de vecteur (coupé à Sma I) pour 100 ng de fragment de PCR (rendu à bout franc par traitement par le fragment de Klenow de l'ADN polymérase). L'incubation a lieu une nuit à 22°C, dans le tampon de ligation (66 mM Tris-HCl pH 7,5, 5 mM MgCl2, 1 mM dithioerythritol, 1 mM ATP) en présence de1u. de T4 ADN ligase puis est arrêtée par dénaturation 10 minutes à 65°C. Parallèlement, la souche d*'E*. *Coli* JM 105 est ensemencée une nuit à 37°C dans du milieu LB. Cette préculture est diluée au 1/500 et placée à 37°C jusqu'à une DO⁶⁰⁰ égale à 1. Pour la suite du mode opératoire les cellules seront toujours conservées au froid. Après une centrifugation de 5 minutes à 3500 g à 4°C, le culot cellulaire est resuspendu dans 1/4 vol. d'eau glacée ultra-pure. Cette étape est répétée 5 à 6 fois. Puis le culot est resuspendu dans 1/4000 vol. d'eau; 10 % de glycérol stérile sont ajoutés permettant la conservation des cellules électrocompétentes, par aliquots de 10 µl à 20°C. A 50 µl de cellules électrocompétentes est ajouté 1 µl de la ligation ; le tout est soumis à une décharge électrique de 12,5 kV/cm, appliquée pendant 5,8 ms. Les cellules sont rapidement remises en suspension dans le milieu SOC, incubées 1 heure à 37°C, puis étalées, en présence de 2% X-Gal dans du diméthylformamide, et 10 mM d'IPTG, sur une boîte de gélose LB-agar supplémentée en ampicilline (100 µg/ml). Après une nuit à 37°C, les clones blancs potentiellement recombinants, sont repiqués de manière ordonnée sur une boîte LB/ampicilline et parallèlement sur un filtre de nylon déposé sur une boîte LB/ampicilline. Ces deux boîtes sont incubées une nuit à 37°C. Les clones recombinants sont alors repérés par hybridation avec une sonde nucléique amplifiée par PCR avec le couple d'amorces selon SEQ ID NO:45 et SEQ ID NO:46 et marquée à la digoxygénine.

Les clones recombinants sont cultivés dans 50 ml de milieu LB/ampicilline (100 µg/ml) en agitation pendant une nuit à 37°C. Après une centrifugation à 3500 g pendant 15 minutes à 4°C, le culot bactérien est repris dans 4ml de tampon P1 (50 mM Tris-HCl, 10mM EDTA, 400 µg/ml RNase A, pH 8) et 4ml de tampon P2 (200 mM NaOH, 1% SDS). Le mélange est incubé à température ambiante pendant 5 minutes. Après adjonction de 4ml de tampon P3 (2,55 M d'acétate de potassium, pH 4,8) le mélange est centrifugé à 12000 g pendant 30 minutes à 4°C. Le surnageant est appliqué sur une colonne Qiagen-type 100, prééquilibrée avec 2 ml de tampon QBT (750 mM NaCl, 50 mM MOPS, 15% éthanol, pH 7), la colonne est lavée avec 2 fois 4ml de tampon QC (1M NaCl, 50 mM MOPS, 15 % éthanol, pH 7) et l'ADN est élué avec 2ml de tampon QF (1,2 M NaCl, 50mM MOPS, 15 % éthanol, pH 8). L'ADN est précipité avec 0,8 vol. d'isopropanol, et centrifugé à 12000 g à 4°C pendant 30 minutes. Le culot est lavé avec de l'éthanol à 70 % glacé, puis l'ADN plasmidique est repris par 2 fois 150 µl de tampon TE.

Les sondes ribonucléiques sont utilisées comme sondes spécifiques, en particulier pour la détection des transcrits exprimés par les séquences rétrovirales endogènes selon l'invention.

### EXEMPLE 6 : Construction d'une souris transgénique contenant tout ou partie du gène de l'envérine.

Une souris transgénique contenant tout ou partie de la séquence HERV-7q (SEQ ID NO:3) est construite afin d'identifier les séquences responsables de la spécificité tissulaire, et pour évaluer le rôle de tout ou partie des motifs rétroviraux endogènes de type HERV-7q, en particulier tout ou partie des motifs peptidiques de l'envérine. La technique de micro-injection utilisée se réfère à la technique classique (Hogan et coll., (1994), Manipulating the mouse embryo, Cold Spring Harbor, Cold Spring Harbor Laboratory Press) ou à ses équivalents. Des formes identiques à la molécule humaine normale de motifs de type HERV-7q, dont l'envérine, ou des formes mutées, délétées, présentant des insertions ou tronquées sont testées afin de déterminer les motifs critiques tant sur le plan normal que pathologique, et plus particulièrement au cours du développement foetal et lors des processus tumoraux.

### Bibliographie :

- Benit L. et al., 1997. Cloning of a new murine endogenous retrovirus MuERV-L, with strong similarity of the human HERV-L element and with a gag coding sequence closely related to the Fv1 restriction gene. J. Virol. 71, 5652-5657.
- Coffin J.M. 1985. Endogenous retrovirus. In: " RNA tumor viruses " (Weiss R.A., Varmus H.E., Teich N.M., and Coffin J.M. eds), Cold Spring Harbor Laboratory Press, Cold Spring Harbor, New York.
- Conrad B., Weissmahr R.N., Boni J., Arcari R., Schupbach J., and Mach B. 1997. A human endogenous retroviral superantigen as candidate autoimmunogene in type 1 diabetes. Cell 90, 303-313.
- Covey S.N. 1986. Amino acid sequence homology in gag region of reverse transcribing elements and the coat protein gene of cauliflower mosaic virus. Nucleic Acids Res. 14, 623-633.
- Hertig C., Coupar B.E., Gould A.R., and Boyle D.B. 1997. Field and vaccine strains of fowlpox virus carry integrated sequences from the avian retrovirus, reticuloendotheliosis virus. Virology 235, 367-376.
- Hohenadl C., Leib-Mösch C., Hehlemann R., and Erfle Y. 1996. Biological significance of human endogenous retroviral sequences. J. Acqui. Imm. Def. Synd. Hum. Retrovir. 13, S268-S273.
- Kulkoski J.K., Jones S., Katz R.A., Mack J.P.G., and Skalka A.M. 1992. Residues critical for retroviral integrative recombination in a region that is highly conserved among retroviral/retrotransposon integrases and bacterial insertion sequence transposases. Mol. Cell. Biol. 12, 2331-2338.
- La Mantia G. et al, N.A.R., 1991, 19, 7, 1513-1520
- Patience C., Wilkinson D.A., and Weiss R.A. 1997. Our retroviral heritage. Trends Genet. 13, 116-120.
- Pearson W.R. 1994. Using the FASTA program to search protein and DNA sequence databases. Methods Mol. Biol. 24, 307-331.
- Perron H., Garson J.A., Bedin F., Beseme F., Paranhos-Baccala G., Komurian-Pradel F., Mallet F., Tuke P.W., Voisset C., Blond J.L., Lalande B., Seigneurin J.M., Mandrand B. and the Collaborative Research Group on Multiple Sclerosis. 1997. Molecular identification of a novel retrovirus repeatedly isolated from patients with multiple sclerosis. Proc. Natl. Acad. Sci. USA 94, 7583-7588.
- Tönjes R.R. et al., J. AIDS and Hum. Retrovirol, 1996, 13, S261-S267
- Vitelli R., Chiarillo M., Lattero D., Bruni C.B., and Bucci C. 1996. Molecular cloning and expression analysis of the human Rab7 GTP-ase complementary deoxyribonucleic acid. Biochem. Biophys. Res. Commun. 229, 887-890.
- Weber L.T., Miller M., Jaskolski M., Leis J., Skalka M., and Wlodawer A. 1989. Molecular modeling of the HIV-1 protease and its substrate binding site. Science 243, 928-931.
- Wilkinson D., Mager D.L., and Leong J.A.C. 1994. Endogenous human retroviruses. In: "The Retroviridae" (Levy, J.A. ed), Plenum Press New York, , Vol. 3, 465-535.
- Xiong Y., and Eickbush, T. 1990. Origin and evolution of retroelements based upon their reverse transcriptase sequences. EMBO J. 9, 3353-3362.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

## Revendications

1. Polynucléotide isolé de séquence SEQ ID NO:2, correspondant au gène *gag* d'un rétrovirus endogène humain dénommé HERV-7q.

2. Polyribonucléotide susceptible d'être obtenu par transcription de la séquence telle que définie à la revendication 1.

3. Sonde ou amorce pour la détection de séquences rétrovirales endogènes humaines de la famille HERV-7q, **caractérisée en ce qu'**elle est spécifique du gène *gag* et qu'elle est sélectionnée dans le groupe constitué par:
a) la sonde de 1505 nucléotides située des positions 2875 à 4380 de la séquence SEQ ID NO: 3, susceptible d'être amplifiée par le couple d'amorces SEQ ID NO: 37 et SEQ ID NO: 38, et
b) les sondes ou amorces présentant les séquences SEQ ID NO: 37 à 44.

4. Protéine isolée, **caractérisée en ce qu'**elle est codée par le polynucléotide selon la revendication 1.

5. Peptide, **caractérisé en ce qu'**il est sélectionné dans le groupe constitué par les séquences SEQ ID NO: 29 à 34, 36 et 58.

6. Vecteur d'expression comprenant l'une des séquences définies aux revendications 1 et 3(a).

7. Vecteur d'expression, **caractérisé en ce qu'**il code pour au moins un peptide selon la revendication 5.

8. Animal transgénique non-humain, **caractérisé en ce qu'**il comprend un polynucléotide selon la revendication 1.

9. Anticorps, **caractérisé en ce qu'**il est dirigé spécifiquement contre un peptide selon la revendication 5.

10. Polynucléotide de séquence sélectionnée dans le groupe constitué par les séquences telles que définies à l'une quelconque des revendications 1 à 3 et les séquences complémentaires des séquences précédentes, pour utilisation comme réactif de diagnostic.

11. Anticorps selon la revendication 9 pour utilisation comme réactif de diagnostic.

12. Utilisation d'au moins un anticorps selon la revendication 9 pour la préparation d'un réactif de diagnostic d'une neuropathologie humaine à composante autoimmune.

13. Utilisation selon la revendication 12, **caractérisée en ce que** ladite pathologie est la sclérose en plaques.

14. Procédé de diagnostic d'une neuropathologie humaine à composante autoimmune, à partir d'un échantillon biologique, lequel procédé est **caractérisé en ce qu'**il comprend l'analyse de l'expression du transcrit tel que défini à la revendication 2 ou de la protéine selon la revendication 4.

15. Procédé selon la revendication 14, **caractérisé en ce que** ladite analyse est une analyse comparative entre un échantillon provenant d'un patient et un échantillon d'un individu normal servant de référence.

16. Procédé selon la revendication 14 ou la revendication 15, **caractérisé en ce qu'**il comprend :
(a) une étape dans laquelle l'on met en contact ledit échantillon biologique contenant des séquences nucléiques et/ou protéiques à analyser, avec au moins un réactif de diagnostic tel que défini à la revendication 10 ou 11, et
(b) une étape dans laquelle on détecte par tout moyen approprié les produits résultants de l'interaction entre lesdites séquences nucléiques ou protéiques et ledit réactif de diagnostic.

17. Procédé selon la revendication 16, **caractérisé en ce que** l'étape b) de détection comprend l'hybridation avec une sonde selon la revendication 3.

18. Procédé selon la revendication 16 ou 17, **caractérisé en ce que** le réactif de l'étape a) est un réactif nucléotidique tel que défini à la revendication 10, préalablement déposé sur un support approprié.

19. Procédé selon la revendication 16 ou 17, **caractérisé en ce qu'**il comprend :
* préalablement à l'étape (a) :
une étape de préparation du tissu ou du liquide biologique concerné, et
. une étape d'extraction de l'acide nucléique,
* préalablement à l'étape b) :
l'amplification dudit acide nucléique, à l'aide d'au moins une amorce selon la revendication 3 et
* postérieurement à l'étape (b):
. une étape de comparaison des séquences nucléiques amplifiées avec la séquence SEQ ID NO: 1.

20. Procédé selon la revendication 16, **caractérisé en ce que** l'étape b) comprend la mise en contact dudit échantillon biologique avec un anticorps selon la revendication 9, et la détection des complexes immunologiques formés, par tout moyen approprié.

21. Kit de diagnostic d'une neuropathologie humaine à composante autoimmune, **caractérisé en ce qu'**il comprend un réactif de diagnostic tel que défini à la revendication 10 ou 11.
